(19) Europäisches Patentamt / European Patent Office / Office européen des brevets

(11) **EP 3 852 524 B1**

(12) **EUROPEAN PATENT SPECIFICATION**

(45) Date of publication and mention
of the grant of the patent:
**28.06.2023 Bulletin 2023/26**

(21) Application number: **19782837.9**

(22) Date of filing: **20.09.2019**

(51) International Patent Classification (IPC):
**A01N 1/02** $^{(2006.01)}$    **A61K 35/17** $^{(2015.01)}$
**C12N 5/0783** $^{(2010.01)}$

(52) Cooperative Patent Classification (CPC):
**A01N 1/0284; A61K 35/17; C12N 5/0636;**
C12N 2501/2302; C12N 2501/2315;
C12N 2501/2321

(86) International application number:
**PCT/US2019/052108**

(87) International publication number:
**WO 2020/061429 (26.03.2020 Gazette 2020/13)**

(54) **EXPANSION OF TILS FROM CRYOPRESERVED TUMOR SAMPLES**

EXPANSION VON TILS AUS KRYOKONSERVIERTEN TUMORPROBEN

EXPANSION DE TIL À PARTIR D'ÉCHANTILLONS DE TUMEUR CRYOCONSERVÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.09.2018 US 201862733937 P
29.07.2019 US 201962879881 P**

(43) Date of publication of application:
**28.07.2021 Bulletin 2021/30**

(73) Proprietor: **Iovance Biotherapeutics, Inc.
San Carlos, CA 94070 (US)**

(72) Inventors:
• **VEERAPATHRAN, Anand
Tampa, FL 33647 (US)**
• **ONIMUS, Kenneth
Wesley Chapel, FL 33545 (US)**

(74) Representative: **Secerna LLP
The Old Fire Station
18 Clifford Street
York YO1 9RD (GB)**

(56) References cited:
**WO-A1-2018/081473**

• **CARINA G. J. M. HILDERS ET AL: "Isolation and
characterization of tumor-infiltrating
lymphocytes from cervical carcinoma",
INTERNATIONAL JOURNAL OF CANCER, vol. 57,
no. 6, 15 June 1994 (1994-06-15), pages 805-813,
XP055144013, ISSN: 0020-7136, DOI:
10.1002/ijc.2910570608**
• **NICOLETTE G. ALKEMA ET AL: "Biobanking of
patient and patient-derived xenograft ovarian
tumour tissue: efficient preservation with low and
high fetal calf serum based methods",
SCIENTIFIC REPORTS, vol. 5, no. 1, 6 October
2015 (2015-10-06) , XP055591885, DOI:
10.1038/srep14495 & Mona Sheikhi ET AL:
"Clinical grade vitrification of human ovarian
tissue: an ultrastructural analysis of follicles and
stroma in vitrified tissue", Human Reproduction,
vol. 26, no. 3, 8 January 2011 (2011-01-08), pages
594-603, XP055656954, GB ISSN: 0268-1161, DOI:
10.1093/humrep/deq357**

- **Seth Wardell ET AL: "A Cryopreserved Tumor Infiltrating Lymphocyte (TIL) product for LN-144, Generated with an Abbreviated Method Suitable for High Throughput Commercial Manufacturing Exhibits Favorable Quality Attributes for Adoptive Cell Transfer", , 8 November 2017 (2017-11-08), XP055656206, Retrieved from the Internet: URL:http://www.iovance.com/wp-content/uplo ads/2017/11/SITC2017_Seth_poster_FINAL_SW D E_PRINT_7Nov2017.pdf [retrieved on 2020-01-09]**
- **ARIAN SADEGHI ET AL: "Rapid expansion of T cells: Effects of culture and cryopreservation and importance of short-term cell recovery", ACTA ONCOLOGICA., vol. 52, no. 5, 1 June 2013 (2013-06-01), pages 978-986, XP055656852, GB ISSN: 0284-186X, DOI: 10.3109/0284186X.2012.737020**

Remarks:

The complete document including Reference Table(s) and the Sequence Listing(s) can be downloaded from the EPO website

**Description**

FIELD OF THE INVENTION

The invention described herein relates generally to the expansion of lymphocytes and more particularly, but not exclusively, to expansion of lymphocytes from cryopreserved tumor tissue.

BACKGROUND OF THE INVENTION

Treatment of refractory cancers using adoptive transfer of tumor infiltrating lymphocytes (TILs) represents a potentially powerful approach to treat patients with poor prognoses. Gattinoni, et al., Nat. Rev. Immunol. 2006, 6, 383-393. Successful immunotherapy requires a large number of TILs; therefore, a robust and reliable process is needed for manufacturing and commercialization. This scaling for commercialization has been a profound challenge because of the legion technical, logistical, and regulatory issues with cell expansion. IL-2-based TIL expansion followed by a "rapid expansion process" (REP) has become a preferred method for TIL expansion because of its efficiency. Dudley, et al., Science 2002, 298, 850-54; Dudley, et al., J. Clin. Oncol. 2005, 23, 2346-57; Dudley, et al., J. Clin. Oncol. 2008, 26, 5233-39; Riddell, et al., Science 1992, 257, 238-41; Dudley, et al., J. Immunother. 2003, 26, 332-42. A REP can result in a 1,000-fold expansion of TILs over a 14-day period, although it requires a large excess (*e.g.*, 200-fold) of irradiated allogeneic peripheral blood mononuclear cells (PBMCs, also known as mononuclear cells (MNCs)), often from multiple donors, as feeder cells, as well as anti-CD3 antibody (OKT3) and high doses of IL-2. Dudley, et al., J. Immunother. 2003, 26, 332-42. TILs that have undergone a REP procedure have produced successful adoptive cell therapy following host immunosuppression in some melanoma patients.

Current TIL manufacturing processes are limited by duration, cost, sterility concerns, and other factors described herein such that the potential to commercialize such processes is severely limited. Among the many limitations of the current processes is the dependence on fresh tumor tissue to initiate manufacturing, which often necessitates expensive and costly local manufacturing facilities because of the difficulty in shipping fresh tumor longer distances. There is an urgent need to provide TIL manufacturing processes and therapies that are not dependent on fresh tumor tissue and that are appropriate for commercial scale manufacturing and regulatory approval for use in geographically widely dispersed human patients. Examples of methods for manufacturing TILs are described in WO 2018/081473 A1 and Hilders et al. International Journal of Cancer, 1994, vol.57, no. 6, pp 805-813. Both describe methods of cryopreserving tumor tissue for the manufacture of TILs wherein the method comprises first fragmenting tumor tissue and then freezing the fragments by cryopreservation.

SUMMARY OF THE INVENTION

The invention is set out in the appended set of claims.

In a first aspect, the invention provides a method of cryopreserving tumor tissue for the manufacture of tumor infiltrating lymphocytes (TILs), the method comprising:

(i) fragmenting the tumor tissue;

(ii) incubating the fragments in a cryopreservation medium for 30 minutes to 80 minutes at 2°C to 8°C; and,

(iii) freezing the fragments wherein the freezing is flash freezing using the vapor phase of liquid nitrogen.

In some embodiments, the tumor tissue is:

(i) fragmented into approximately spherical fragments having a diameter of 1.5 mm to 6 mm, optionally wherein the approximately spherical fragments have a diameter of 6mm or 3mm;

(ii) fragmented into generally rectangular fragments having a shortest edge length of at least 1.5 mm and a longest edge length of 6 mm;

(iii) fragmented into generally cubical fragments having edge lengths of between 1.5 mm and 6 mm, optionally wherein the cubical fragments have edge lengths of 6 mm or 3mm;

(iv) from a dissected tumor, optionally wherein the dissected tumor is less than eight hours old; or

(v) is washed in a physiologically buffered isotonic saline solution prior to incubation, optionally wherein the washing comprises three serial washes of at least three minutes each, with the physiologically buffered isotonic saline solution replaced after each serial wash.

In some embodiments, the cryopreservation medium comprises:

(i) 2% v/v to 15% v/v dimethyl sulfoxide (DMSO), optionally wherein the cryopreservation medium comprises 10% v/v DMSO; or

(ii) at least one antimicrobial agent, optionally wherein the at least one antimicrobial agent is gentamicin at a concentration of at least 50 $\mu$g/mL.

In some embodiments, the freezing takes place at a temperature in the range from of - 125°C to -196°C, optionally wherein the freezing takes place at a temperature of -140°C to - 175°C, further optionally wherein the freezing takes place at -145°C.

In some embodiments, the method further comprises the step of (iv) storing the frozen fragments below at least -130°C, optionally wherein the frozen fragments are stored in the vapor phase of liquid nitrogen or are stored submerged in liquid nitrogen.

In some embodiments, the tumor fragments are incubated in the cryopreservation medium for 30 minutes to 70 minutes and optionally for 30 minutes to 60 minutes.

In another aspect, the invention provides a cryopreserved tumor fragment for the manufacture of tumor infiltrating lymphocytes (TILs), prepared by a process comprising the steps of:

(i) fragmenting a dissected tumor or tumor biopsy sample;

(ii) incubating the fragments in a cryopreservation medium; and,

(iii) freezing the fragments wherein the freezing is flash freezing using the vapor phase of liquid nitrogen,

wherein step (ii) further comprises incubating the fragments for 30 minutes to 60 minutes at 2°C to 8°C in a cryopreservation medium comprising 10% v/v DMSO.

In another aspect, the invention provides a method for preparing tumor infiltrating lymphocytes (TILs) for adoptive T-cell therapy, the method comprising:

(a) storing the tumor tissue in a frozen state, the method of storing the tumor tissue comprising:

(i) trimming the tumor tissue to remove excess non-tumor tissue;

(ii) placing the tumor tissue in a closable vessel containing a storage medium;

(iii) incubating the vessel, which contains the tumor tissue and the storage medium, at a temperature of 2 to 8°C for a period of 30 minutes to 60 minutes;

(iv) freezing the vessel, wherein the freezing is flash freezing using the vapor phase of liquid nitrogen; and

(v) storing the vessel at or below -130°C;

(b) thawing the vessel;

(c) treating the tumor tissue in a gas permeable container with a first cell culture medium comprising interleukin 2 (IL-2) and optionally OKT-3 to provide TILs;

(d) expanding the TILs in a gas permeable container using a second cell culture medium comprising cell culture medium, irradiated feeder cells, OKT-3, and IL-2, to provide an expanded number of TILs.

In some embodiments, the tumor tissue in step (i) is:

(a) trimmed to between 1.5 mm and 6 mm in diameter, optionally wherein the tumor tissue in step (i) is trimmed to 6 mm by 6 mm by 6 mm; and/or

(b) first washed in Hank's Balanced Salt Solution (HBSS) before performing step (i), optionally wherein the washing comprises three serial washes of at least three minutes each, with the HBSS replaced after each serial wash.

In some embodiments, the storage medium further comprises:

(i) an antibacterial agent and an antifungal agent, optionally wherein the storage medium comprises gentamicin at a concentration of at least 50 μg/mL; and/or

(ii) 2 % v/v to 12 % v/v dimethyl sulfoxide (DMSO), optionally wherein the storage medium comprises 10% DMSO.

In some embodiments, step (b) comprises immersing the vessel in water bath at a temperature of 37°C for 5 minutes.

In some embodiments, the tumor tissue is selected from the group consisting of melanoma tumor tissue, head and neck tumor tissue, breast tumor tissue, renal tumor tissue, pancreatic tumor tissue, glioblastoma tumor tissue, lung tumor tissue, colorectal tumor tissue, sarcoma tumor tissue, triple negative breast tumor tissue, cervical tumor tissue, ovarian tumor tissue, or HPV-positive tumor tissue.

In some embodiments, the method further comprising a split between step (c) and step (d), optionally wherein the split occurs 16 days after step (c) is initiated.

In some embodiments, the first culture step is 11 days, or wherein steps (c) through (d) are performed over a period of 22 days, or over a period of less than 20 days.

In some embodiments:

(a) step (iv) comprises freezing the vessel at -125 °C to -150 °C;

(b) step (v) comprises storing the vessel below at least -130 °C; or

(c) step (v) comprises storing the vessel submerged in liquid nitrogen.

In another aspect, the invention provides a therapeutic population of TILs for use in a method for treating a subject with cancer, the method comprising providing expanded tumor infiltrating lymphocytes (TILs) for administration, produced by a method comprising:

(a) storing the tumor tissue in a frozen state, the method of storing the tumor tissue comprising:

(i) trimming the tumor tissue to remove excess non-tumor tissue;

(ii) placing the tumor tissue in a closable vessel containing a storage medium;

(iii) incubating the vessel, which contains the tumor tissue and the storage medium, at 2 - 8°C for 30 minutes;

(iv) flash freezing the vessel using the vapor phase of liquid nitrogen; and

(v) storing the vessel at the vapor phase of liquid nitrogen temperature,

(b) thawing the tumor tissue;

(c) adding the tumor fragments into a closed system;

(d) performing a first expansion by culturing the first population of TILs in a cell culture medium comprising IL-2 to produce a second population of TILs, wherein the first expansion is performed in a closed container providing a first gas-permeable surface area, wherein the first expansion is performed for 3-14 days to obtain the second population of TILs, wherein the second population of TILs is at least 50-fold greater in number than the first population of TILs, and wherein the transition from step (c) to step (d) occurs without opening the system;

(e) performing a second expansion by supplementing the cell culture medium of the second population of TILs with additional IL-2, OKT-3, and antigen presenting cells (APCs), to produce a third population of TILs, wherein the second expansion is performed for 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (d) to step (e) occurs without opening the system;

(f) harvesting the therapeutic population of TILs obtained from step (e), wherein the transition from step (e) to step (f) occurs without opening the system;

(g) transferring the harvested TIL population from step (f) to an infusion bag, wherein the transfer from step (f) to (g) occurs without opening the system;

(h) cryopreserving the infusion bag comprising the harvested TIL population from step (g) using a cryopreservation process; and

(i) providing a therapeutically effective dosage of the third population of TILs for administration from the infusion bag in step (h) to the subject.

In some embodiments, the method for treating the subject with cancer further comprises the steps of (j) concomitantly providing a therapeutically effective dosage of aldesleukin or a biosimilar thereof for administration to the subject; and (k) concomitantly providing a therapeutically effective dosage of a PD-1/PD-L1 inhibitor for administration to the subject, optionally wherein the PD-1/PD-L1 inhibitor is selected from the group consisting of pembrolizumab, nivolumab, avelumab, durvalumab, atezolizumab, and biosimilars thereof.

In some embodiments, the cancer is selected from the group consisting of melanoma, cervical cancer, head and neck squamous cell cancer, non-small cell lung cancer, bladder cancer, ovarian cancer, pancreatic cancer, and sarcoma.

In another aspect, the invention provides a method for expanding tumor infiltrating lymphocytes (TILs) from frozen tumor tissue comprising:

(a) providing tumor tissue obtained from a patient;

(b) incubating the tumor tissue for at least 30 minutes in a storage medium at a temperature in the range of 2°C to 8°C;

(c) flash freezing the tumor tissue;

(d) storing the tumor tissue in a frozen state;

(e) thawing the tumor tissue;

(f) treating the tumor tissue in a gas permeable container with a first cell culture medium and interleukin 2 (IL-2) to provide TILs;

(g) removing at least a plurality of the TILs; and

(h) expanding the TILs with a second cell culture medium comprising cell culture media, irradiated feeder cells, OKT-3 antibody, and IL-2 in a gas permeable container to provide an expanded number of TILs.

In some embodiments, the tumour tissue in step (b) is incubated for 30 minutes and/or the first cell culture medium in step (f) comprises OKT-3 antibody.

In another aspect, the invention provides a method for expanding TILs from frozen tumor tissue comprising:

(a) providing tumor tissue obtained from a patient;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range of 2°C to 8°C for 20 minutes to 70 minutes;

(e) flash freezing the vessel;

(f) storing the vessel such that the vessel remains frozen;

(g) thawing the vessel;

(h) treating the tumor tissue in a gas permeable container with a first cell culture medium and interleukin 2 (IL-2) to provide TILs;

(i) removing at least a plurality of the TILs; and

(j) expanding the TILs with a second cell culture medium comprising cell culture media, irradiated feeder cells, OKT-3, and IL-2 in a gas permeable container to provide an expanded number of TILs.

In another aspect, the invention provides a therapeutic population of TILs for use in a method of treating cancer for a human subject in need thereof, the method comprising providing expanded tumor infiltrating lymphocytes (TILs) for administration produced by a method comprising:

(a) providing tumor tissue obtained from the subject;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range of 2°C to 8°C for 20 minutes to 70 minutes;

(e) flash freezing the vessel using the vapor phase of liquid nitrogen;

(f) storing the vessel at the vapor phase of liquid nitrogen temperature;

(g) thawing the tumor tissue;

(h) adding the tumor tissue into a closed system;

(i) performing a first expansion by culturing a first population of TILs from the tumor tissue in a cell culture medium comprising IL-2 to produce a second population of TILs, wherein the first expansion is performed in a closed container providing a first gas-permeable surface area, wherein the first expansion is performed for 3-14 days to obtain the second population of TILs, wherein the second population of TILs is at least 50-fold greater in number than the first population of TILs, and wherein the transition from step (h) to step (i) occurs without opening the system;

(j) performing a second expansion by supplementing the cell culture medium of the second population of TILs with additional IL-2, OKT-3, and antigen presenting cells (APCs), to produce a third population of TILs, wherein the second expansion is performed for 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (i) to step (j) occurs without opening the system;

(k) harvesting the therapeutic population of TILs obtained from step (j), wherein the transition from step (j) to step (k) occurs without opening the system;

(l) transferring the therapeutic population of TILs harvested in step (k) to an infusion bag, wherein the transfer from step (k) to (1) occurs without opening the system;

(m) cryopreserving the infusion bag comprising the therapeutic population of TILs from step (1) using a cryopreservation process; and

(n) providing a therapeutically effective dosage of the therapeutic population of TILs for administration from the infusion bag in step (m) to the subject.

In some embodiments, the TILs in step (f):

(i) are cultured over a period of from 3 to 5 days, and wherein the TILs in step (h) are cultured over a period of from 11 to 13 days;

(ii) are cultured over a period of 3 days, and wherein the TILs in step (h) are cultured over a period of 13 days; or

(iii) are cultured over a period of 5 days, and wherein the TILs in step (h) are cultured over a period of 11 days.

In some embodiments, the method further comprises (i) harvesting the TILs, optionally wherein the harvested TILs represent at least 10000-fold increase in number.

BRIEF DESCRIPTION OF THE DRAWINGS

The foregoing summary, as well as the following detailed description of the invention, will be better understood when read in conjunction with the appended drawings.

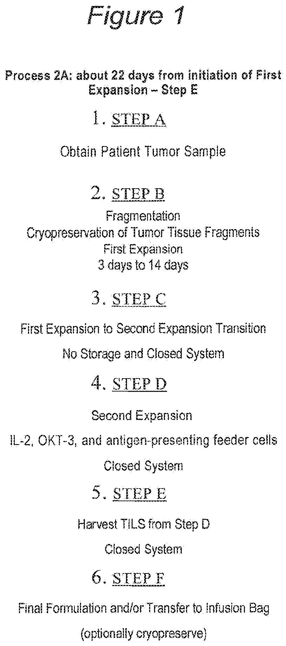

**Figure 1:** Depicts the major steps of an embodiment of the 2A process, including the optional cryopreservation of fresh tumor tissue after fragmentation in Step B. The total time from the initiation of the first expansion culture to the end of Step E is about 22 days.

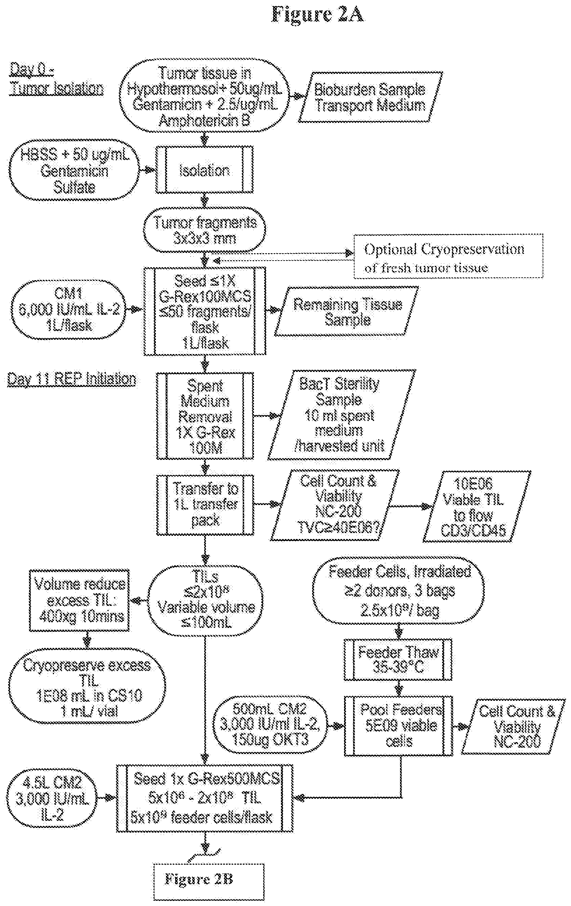

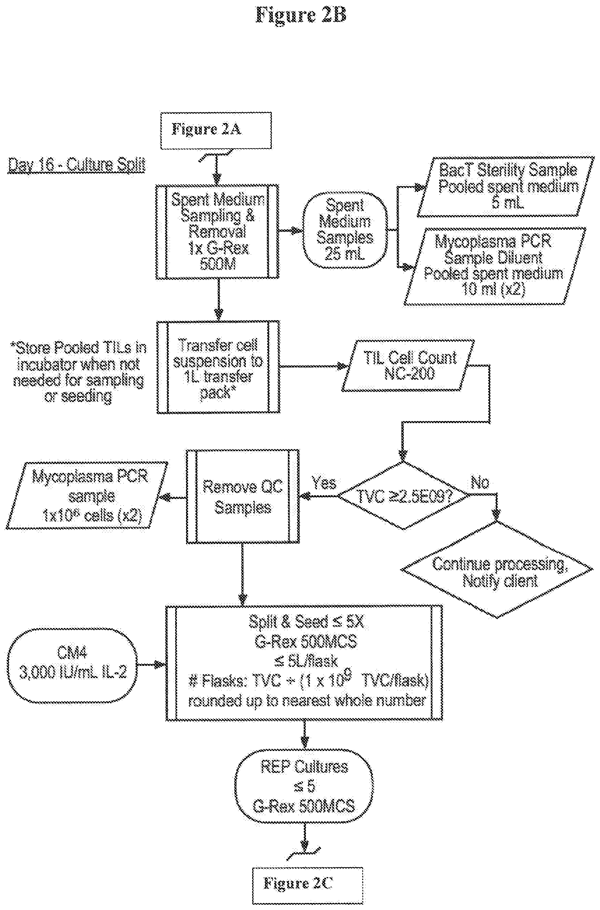

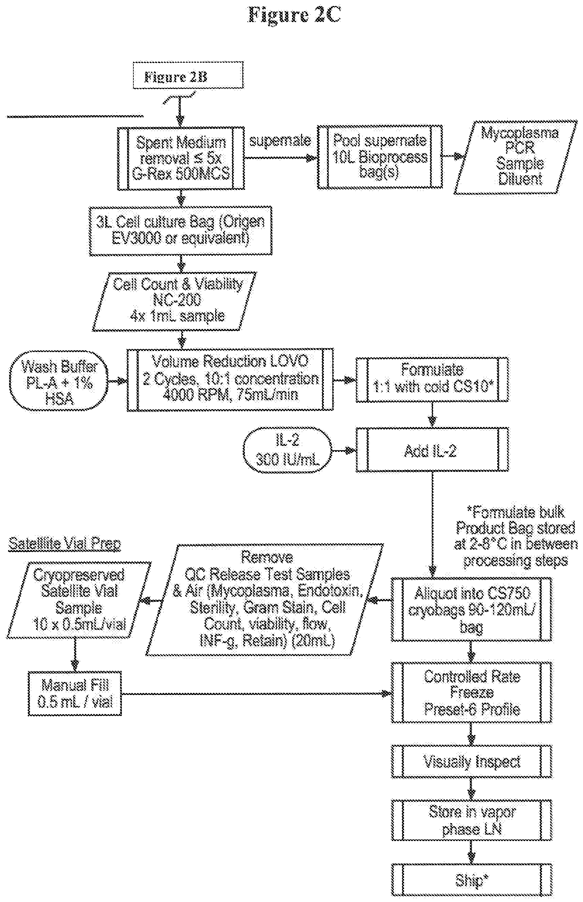

**Figure 2:** Panels A through C depict the various steps of an embodiment of the TIL manufacturing process, including cryopreservation of fresh tumor fragments allowing later initiation of preREP culture.

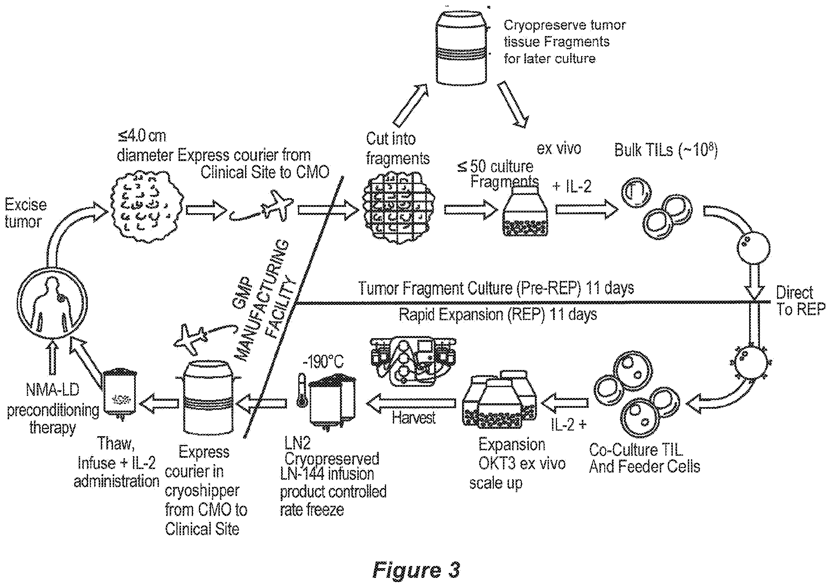

**Figure 3:** Depicts an embodiment of TIL manufacturing, including the cryopreservation of fresh tumor fragments that are then later used to initiate a preREP culture.

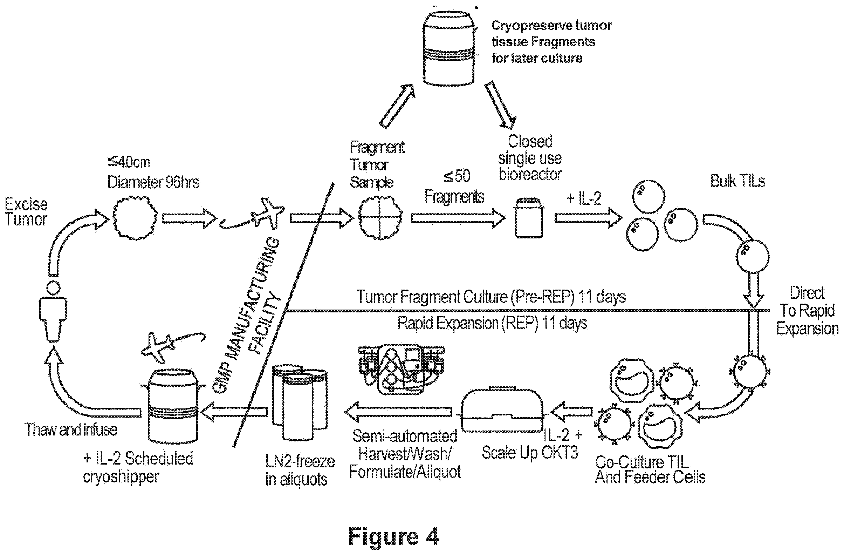

**Figure 4:** Depicts an embodiment of TIL manufacturing, including the cryopreservation of fresh tumor fragments that are then later used to initiate a pre-REP culture.

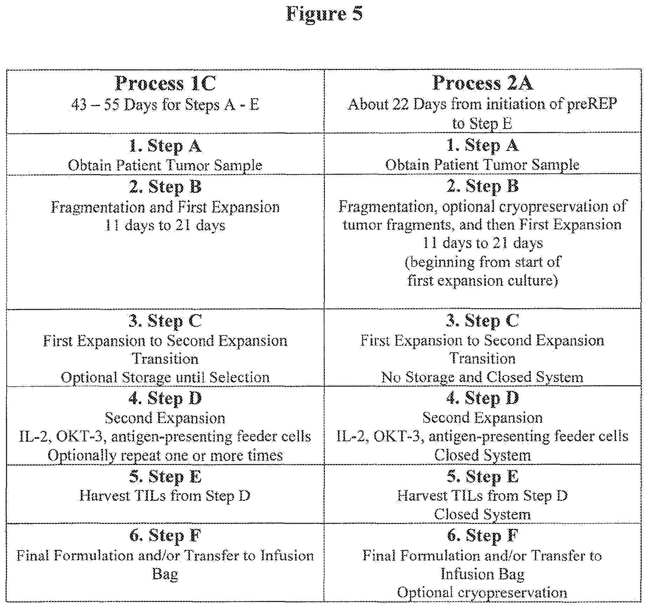

**Figure 5:** Generally compares an embodiment of Process 1C to an exemplary embodiment of Process 2A. Process 2A contemplates initiation of pre-REP cultures from either fresh tumor tissue fragments or thawed cryopreserved tumor tissue fragments.

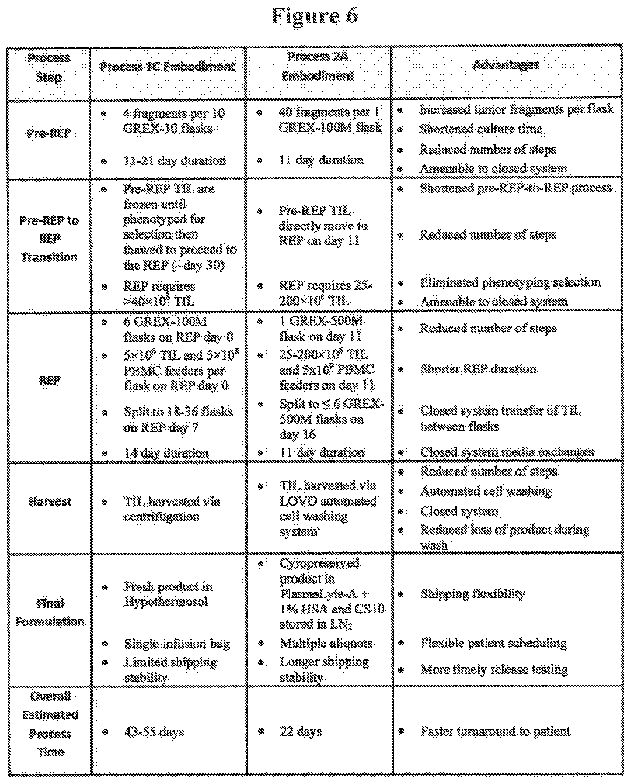

**Figure 6:** Further compares embodiments of Process 1C to embodiments of Process 2A.

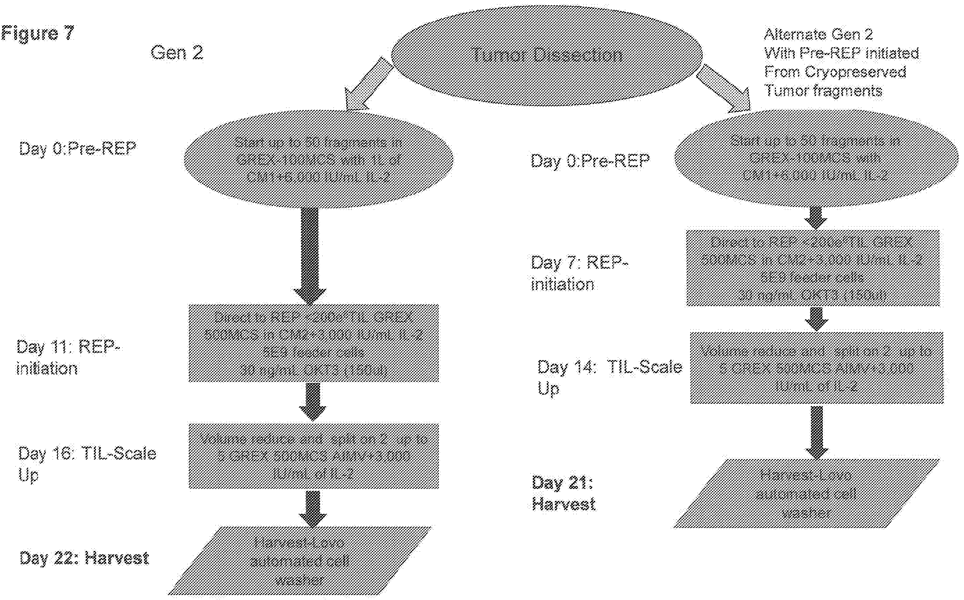

**Figure 7:** Depicts an embodiment of the 2A process wherein the REP is initiated "early."

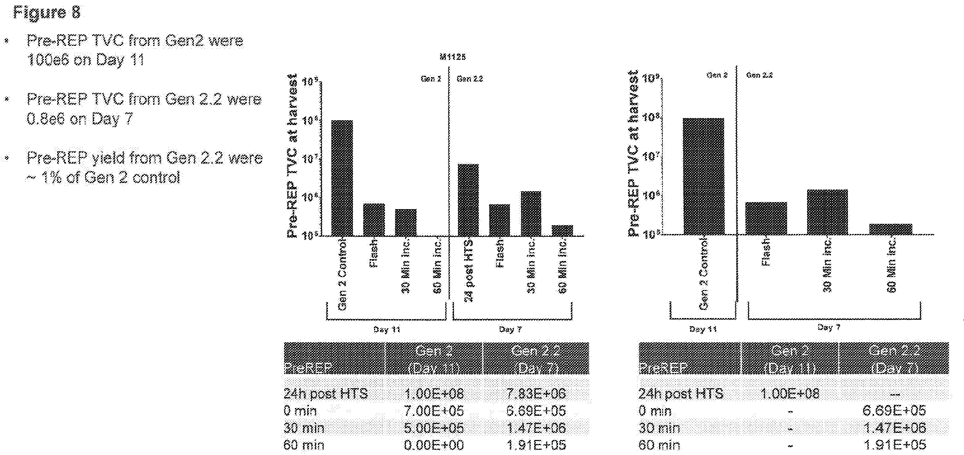

**Figure 8:** Compares the total viable cells derived from a Gen 2A process and a modified Gen 2A process, noted as "Gen 2.2" wherein the pre-REP cultures are initiated with fresh, flash frozen, or frozen following 30 minute or 60 minute incubations in storage medium at 2°C to 8°C. Cell counts are at the end of the pre-REP culture.

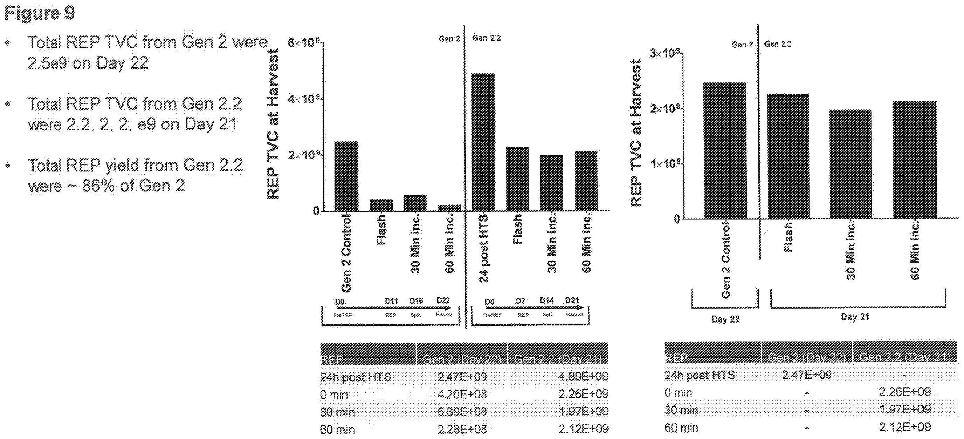

**Figure 9:** Compares the total viable cells derived from a Gen 2A process and a modified Gen 2A process, noted as "Gen 2.2" wherein the pre-REP cultures are initiated with fresh, flash frozen, or frozen following 30 minute or 60 minute incubations in storage medium at 2°C to 8°C. Cell counts at the end of the REP culture.

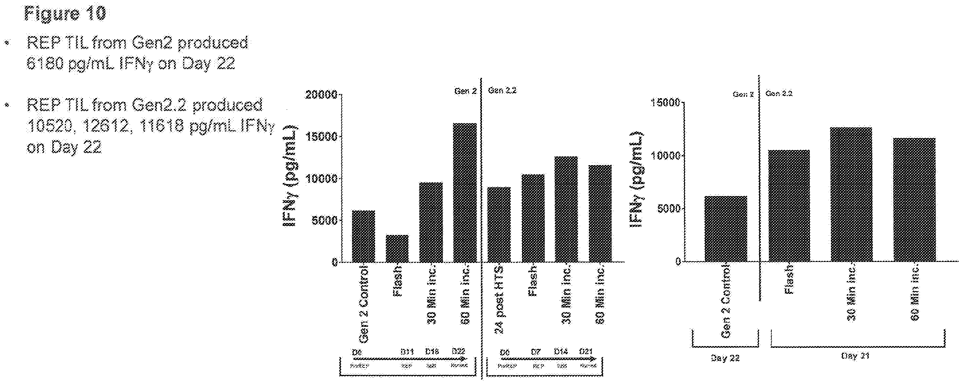

**Figure 10:** Compares IFN-$\gamma$ produced by cells derived from a Gen 2A process and a modified Gen 2A process, noted as "Gen 2.2" wherein the pre-REP cultures are initiated with fresh, flash frozen, or frozen following 30 minute or 60 minute incubations in storage medium at 2°C to 8°C.

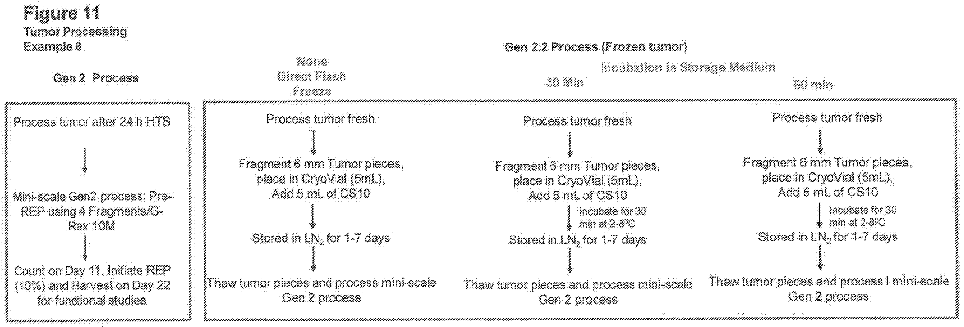

**Figure 11:** Depicts the major steps of a Gen 2 embodiment compared to the major steps of a Gen 2.2 embodiment.

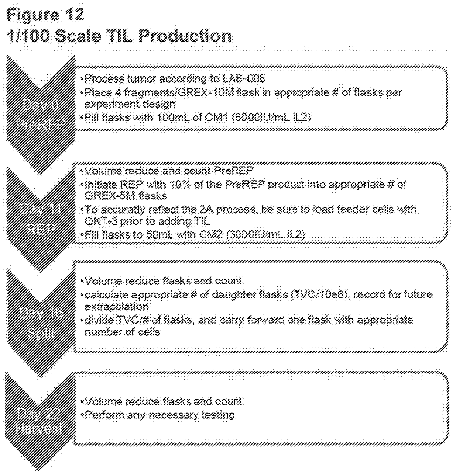

**Figure 12:** Depicts the major steps of the 1/100th scale TIL production process.

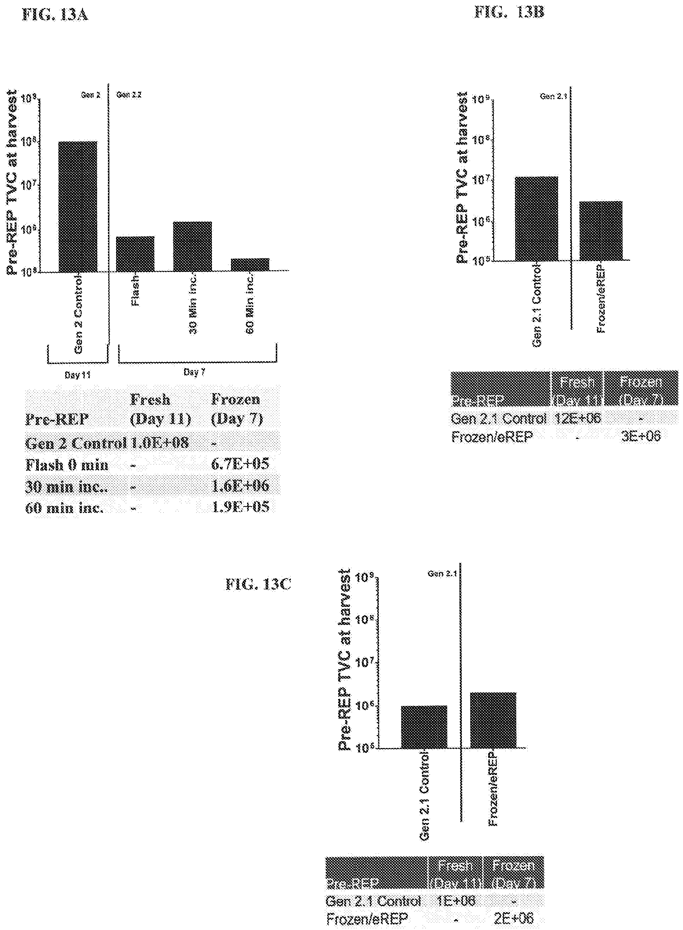

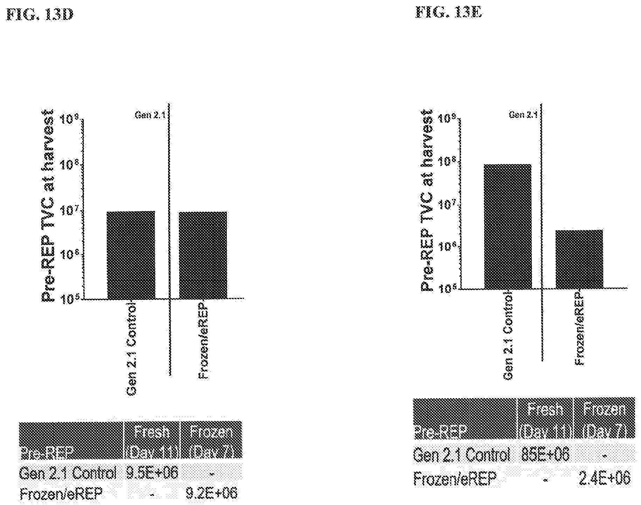

**Figure 13:** Figures 13A-13E depict total viable cells (TVC) for tumor samples processed from 5 different patients and collected during pre-REP. TVC was measured using fresh (Gen2 control) and frozen tumor samples. TVC was recorded on day 11 for fresh tumor samples or day 7 for frozen tumor samples. Figure 13A (patient M1125) shows the only patient whose tumor samples were subjected to different freezing methods: flash freezing in the vapor phase of liquid nitrogen, and incubation at 2-8° Celsius for either 30 or 60 minutes prior to flash freezing in the vapor phase of liquid nitrogen. For the remaining patients S 13018, OV8022, T6042, and O8026 (FIGS. 13B-13E, respectively), the tumor samples were incubated at 2-8° for 60 minutes prior to flash freezing. For each patient, tumor samples were sectioned in 3mm diameter fragments and 4-6 fragments were used for culture. Each bar on the graph represents TVC extrapolated to full scale (multiplied by 10). In FIG 13A, frozen, early pre-REP culture yielded about 1% TVC as compared to fresh, FIG. 13B, about 25% TVC as compared to fresh, FIG. 13C, about 200% as compared to fresh, FIG 13D, about 96% as compared to fresh, and FIG. 13E, about 3% as compared with fresh. The data indicate that TVC from frozen tumor samples on Day 7 is comparable to TVC from fresh tumor samples on Day 11.

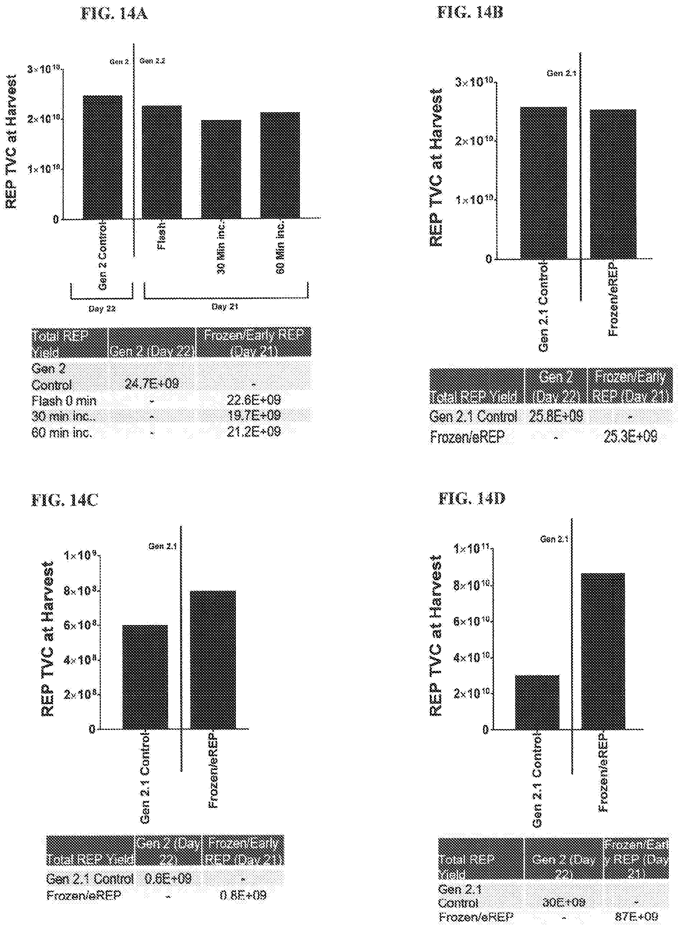

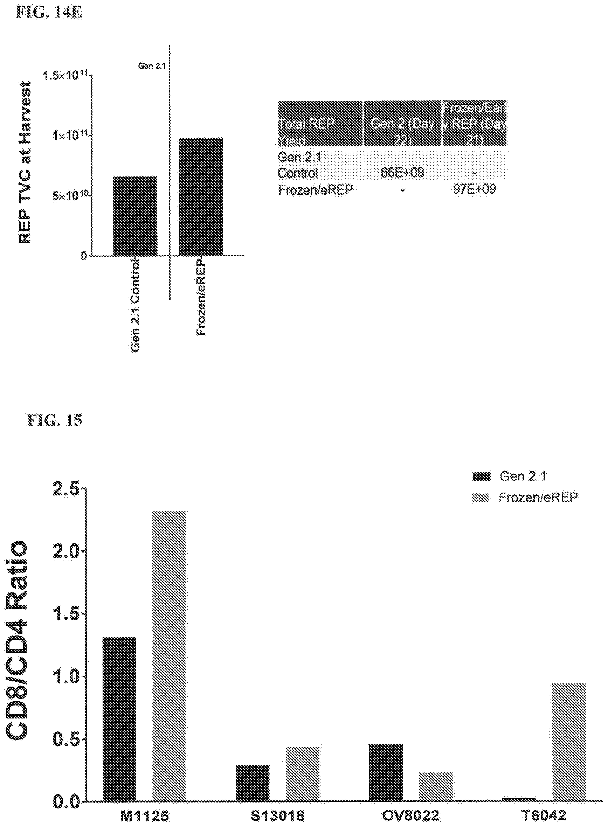

**Figure 14:** Figures 14A-14E depict TVC for tumor samples from the same 5 patients as Figure 13, collected during early REP. Cells were harvested on Day 11 (fresh) and Day 7 (frozen) as described in Figure 13. In FIG 14A, frozen, early REP culture yielded about 86% TVC as compared to fresh, FIG. 14B, about 98% TVC as compared to fresh, FIG. 14C, about 124% as compared to fresh, FIG 14D, about 286% as compared to fresh, and FIG. 14E, about 167% as compared with fresh. The data indicate that overall yield (measured as TVC) from frozen tumor samples on Day 7 is comparable to TVC from fresh tumor samples on Day 11 during early REP.

**Figure 15:** Figure 15 depicts the CD8/CD4 ratio in fresh and frozen tumor samples from 4 patients. Fresh or frozen REP TIL samples were stained with flow cytometry antibodies (CD3-BUV395, CD62LBV421, CD57-PB, CD11c-BV711, CD28-BB515, CD19-BUV563, CCR7-PE, CD123-BV605, CD27PE-CF594, CD14-BV-650, TCRg/d-APC, CD45-PerCP-Cy5.5, CD45RA-A700, CD56-BUV737, CD8-BV786, CD4-PE-Cy7AND CD16-APC-Vio770). The identity of TIL were determined based on TCR a/b+ or g/d +, monocytes (CD14+), NK (CD56+, CD16+, CD56+/CD16+ and B cells (CD19+) and purity was determined based on CD3+CD45+ cells. Patients M1125 and T6042 demonstrated high CD8 expression, but overall the CD8/CD4 ratio is comparable between fresh and frozen tumor samples.

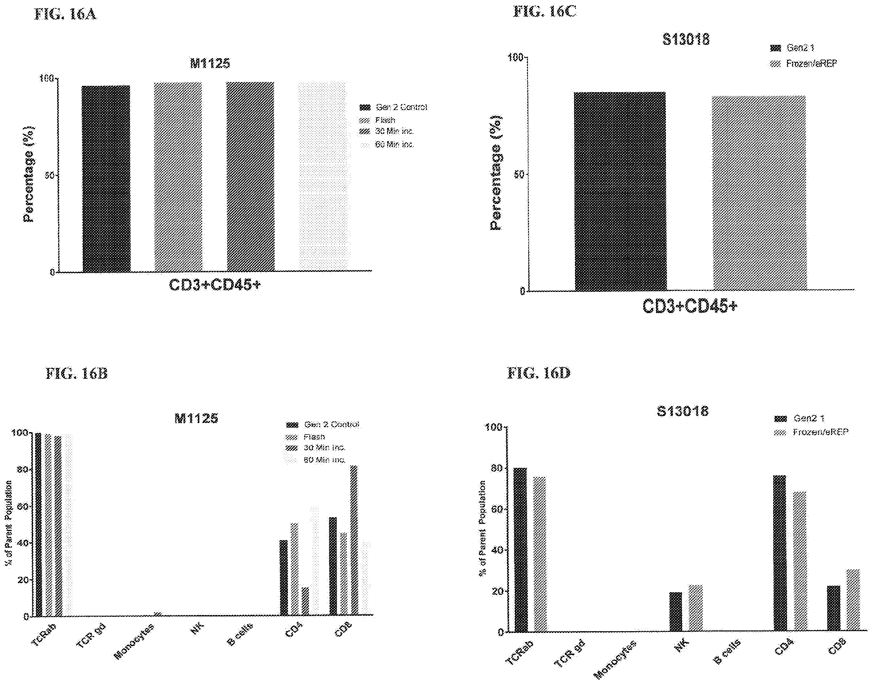

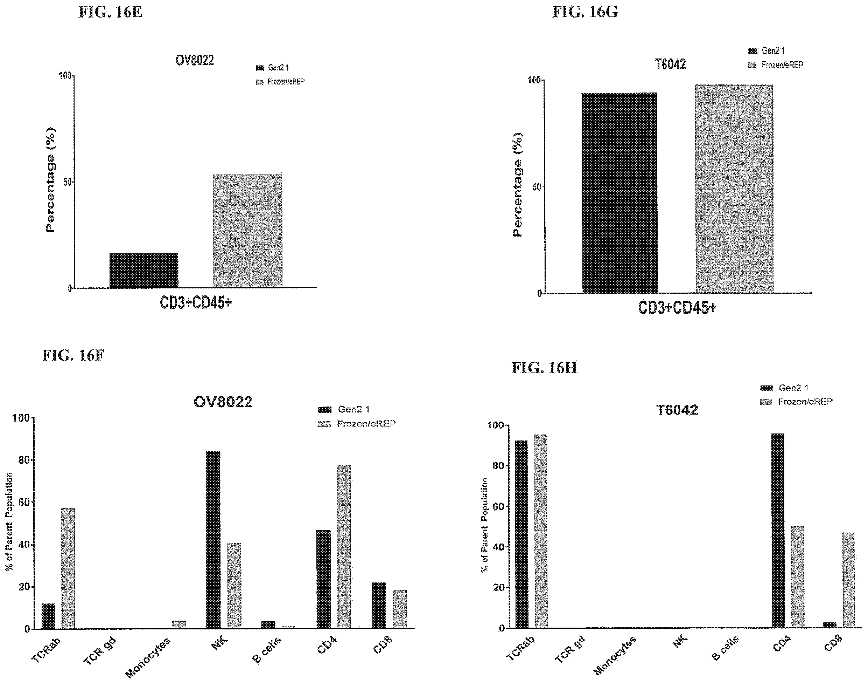

**Figure 16:** Figures 16A-16H depict CD3+CD45+ expression and other marker expression in the same 4 patients as Figure 15. Figures 16A and 16B depict data for patient M1125; Figures 16C and 16D for patient S13018; Figures 16E and 16F for patient OV8022; and Figures 16G and 16H for patient T6042. The data show that TIL purity is comparable between fresh and frozen tumor samples.

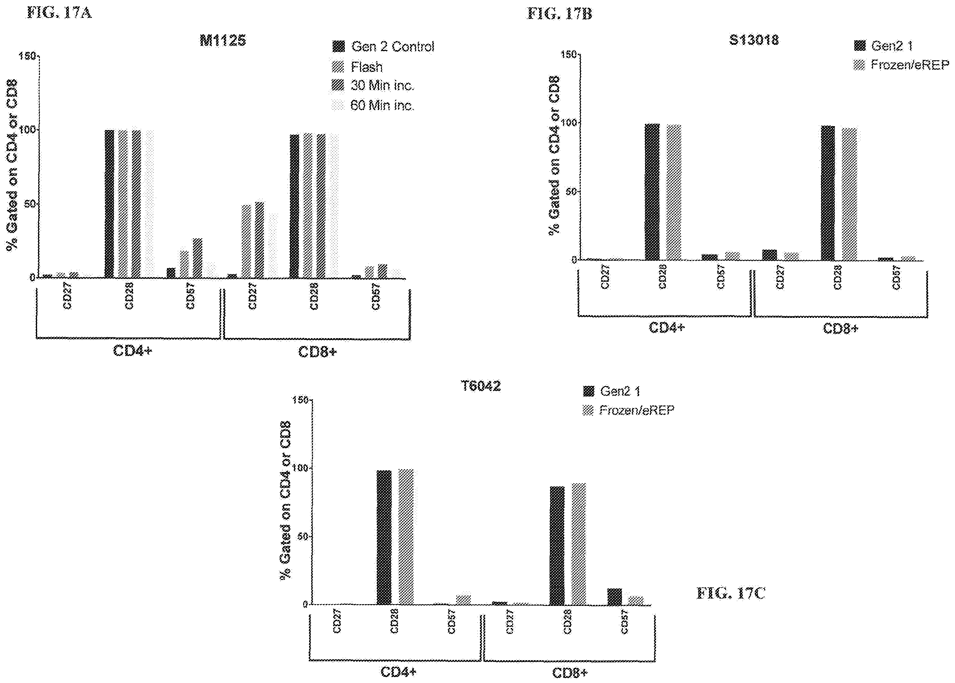

**Figure 17:** Figures 17A-17C illustrate expression of CD28, CD27, and CD57 in cells expressing CD4 or CD8 from fresh and frozen tumor samples. Figure 17A is for patient M1125; Figure 17B for patient S13018, and Figure 17C for patient T6042. Data show that the differentiation status between fresh and frozen tumor samples is comparable.

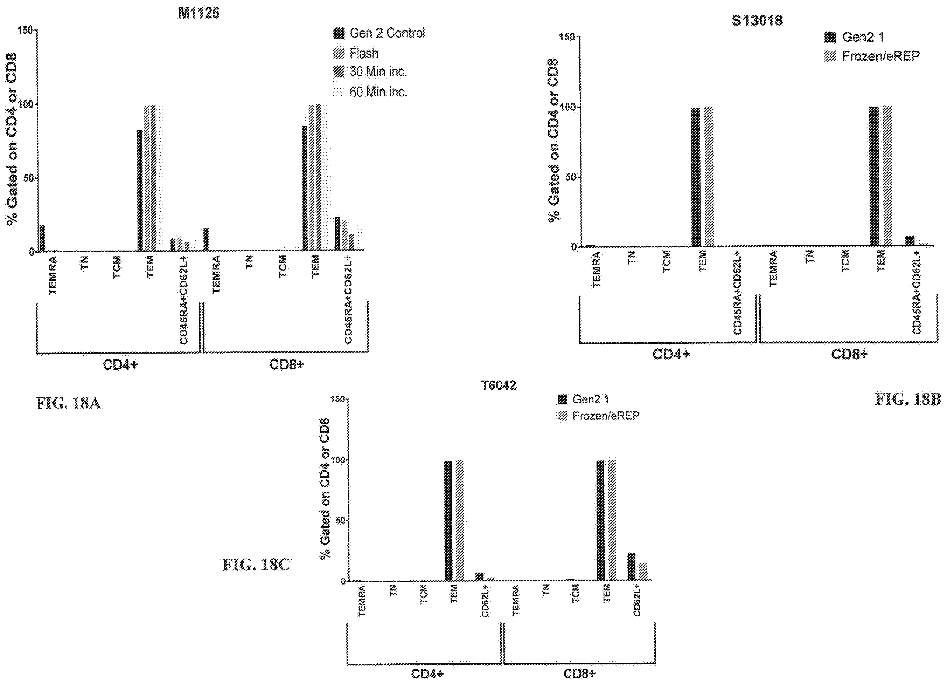

**Figure 18:** Figures 18A-18C illustrate memory status in fresh and frozen tumor samples in cells expressing CD4 or CD8. Figure 18A is for patient M1125; Figure 18B for patient S13018, and Figure 18C for patient T6042. Data show that the memory status between fresh and frozen tumor samples is comparable. TN: Naive (CD45RA+CCR7+), TCM: Central memory (CD45RA-CCR7+), TEM: Effector memory (CD45RA-CCR7-), TEMRA/TEFF: RA+ Effector memory/Effectors (CD45RA+CCR7), Central Memory/Activated cells (CD45RA+CD62L+). Bar graph presented are percentage positive CD45+/- or CCR7 +/- or CD62L +/- when gated on CD4+ or CD8+.

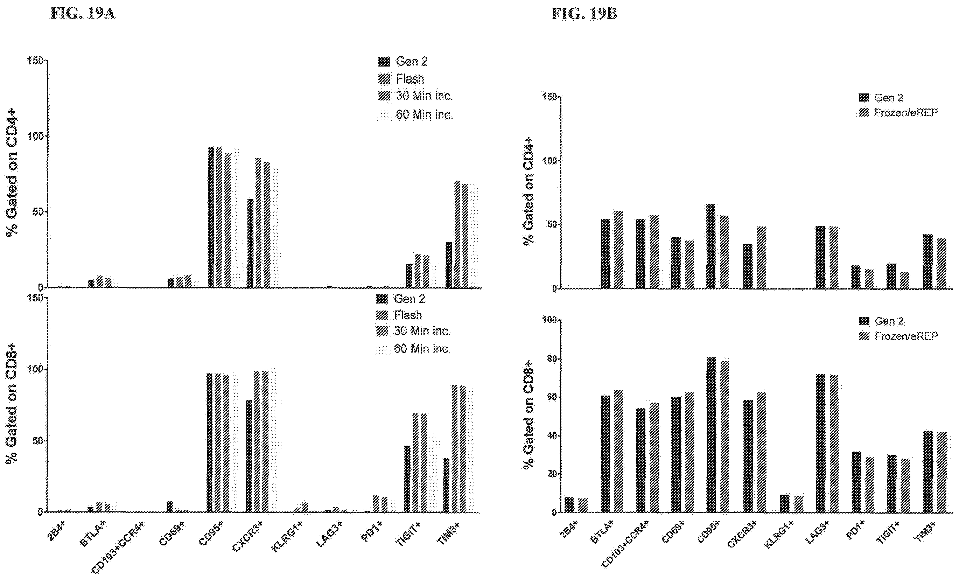

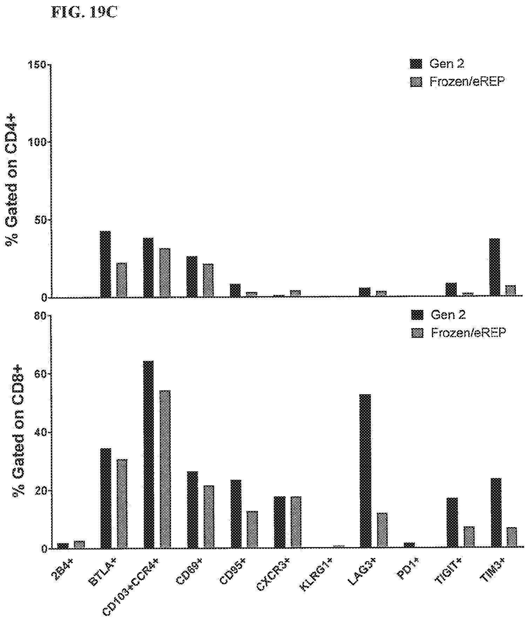

**Figure 19:** Figures 19A-19C illustrate activation/exhaustion markers in fresh and frozen tumor samples in cells expressing CD4 (top panel) or CD8 (bottom panel) in 3 patients: M1125 (FIG. 19A); S13018 (FIG. 19B); and T6042 (FIG. 19C). Activation and exhaustion of REP TIL was determined by multicolor flow cytometry. Tumor tissue was stained with antibodies (CD3-BUV395, PD-1-BV421, 2B4/CD244-PB, CD8-BB515, CD25-BUV563, BTLA-PE, KLRG1-PE-Dazzle 594, TIM-3-BV650, CD194/CCR4-APC, CD4-VioGreen, TIGIT-PerCP-eFluor 710, CD183-BV711, CD69-APC-R700, CD95-BUV737, CD127-PE-Cy7, CD103-BV786, LAG-3-APC-eFluor 780) and expression was measured using flow cytometry. Overall, TIL activation/exhaustion status is comparable between fresh and frozen tumor samples.

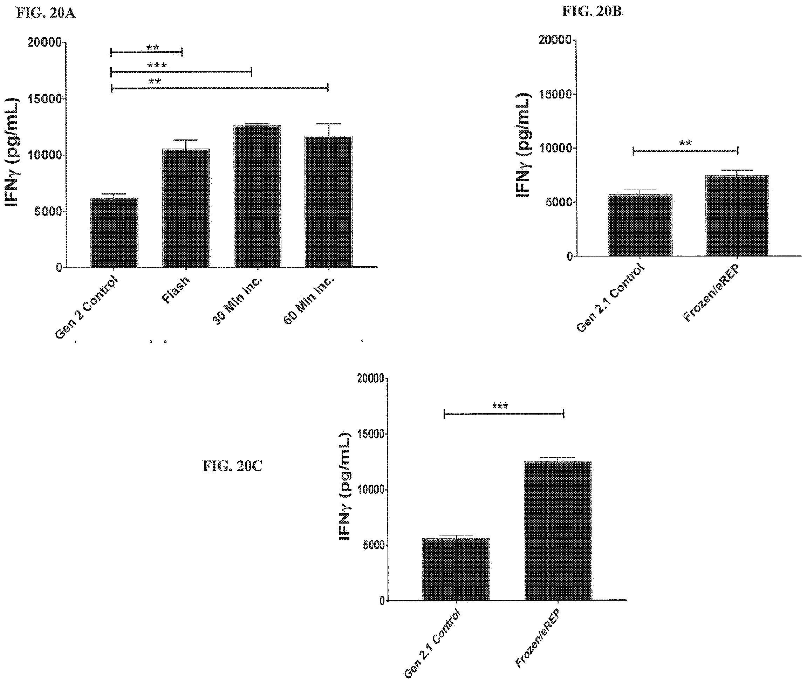

**Figure 20:** Figures 20A-20C illustrate IFN$\gamma$ function in fresh and frozen tumor samples for 3 patients: M1125 (FIG 20A); S13018 (FIG. 20B); and T6042 (FIG. 20C). $1\times10^5$ REP TIL were plated in anti-CD3 plates for 24 hours. Culture supernatants were collected and measured for IFN$\gamma$ cytokine using an IFN$\gamma$ Qantikine ELISA kit. Bar graph represent here are Mean + SD of triplicate measurement. Student 't' test was used to calculate statistical significance.

* P < 0.05, ** P < 0.01, *** P <0.001. Overall, the data demonstrate that IFNy function is higher in frozen tumor samples.

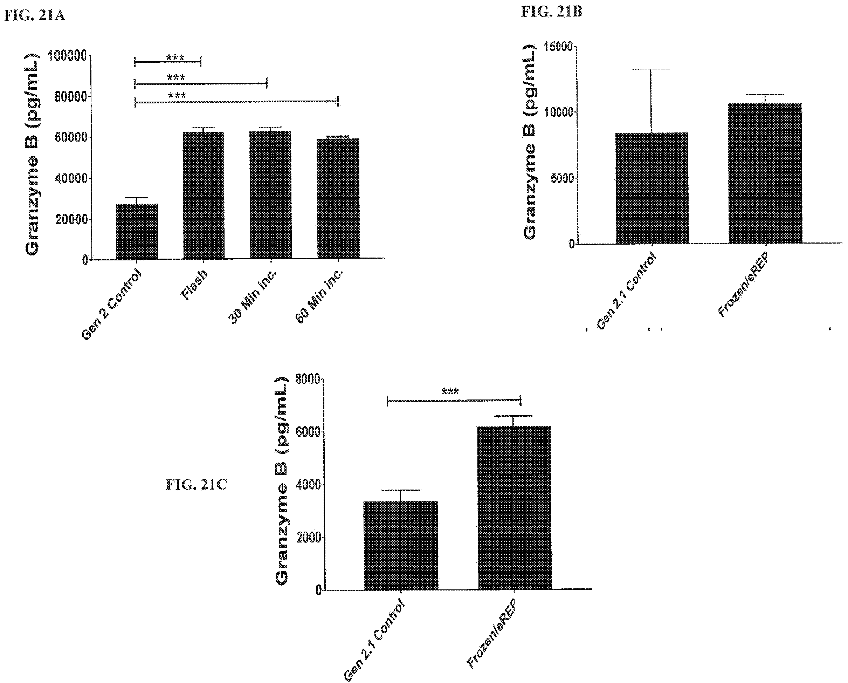

**Figure 21:** Figures 21A-21C illustrate Granzyme B (GzmB) function in fresh and frozen tumor samples for 3 patients: M1125 (FIG 21A); S13018 (FIG. 21B); and T6042 (FIG. 21C). $1 \times 10^5$ REP TIL were plated in anti-CD3 plates for 24 hours. Culture supernatants were collected and measured for Granzyme B using a Duo set ELISA kit. Bar graph represent here are Mean + SD of triplicate measurement. Student 't' test was used to calculate statistical significance. * P < 0.05, ** P < 0.01, *** P <0.001. Overall, the data demonstrate that Granzyme B (GzmB) function is comparable/higher in frozen tumor samples.

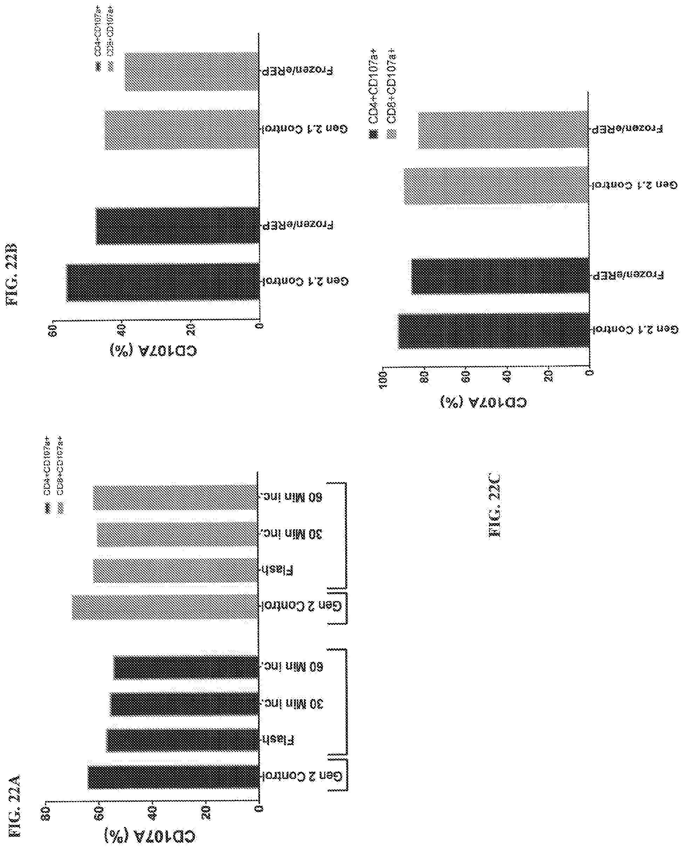

**Figure 22:** Figures 22A-22C illustrate CD107A expression in fresh and frozen tumor samples for 3 patients: M1125 (FIG 22A); S13018 (FIG. 22B); and T6042 (FIG. 22C). $2 \times 10^5$ REP TIL were stimulated with or without stimulation of PMA/IO for 2 hours, then stained for CD107A-APC700 and analyzed by flow cytometry. Bar graph represents CD107A positive cells gated on CD8 or CD4. Overall, the data demonstrate that CD107A expression is comparable between fresh and frozen tumor samples.

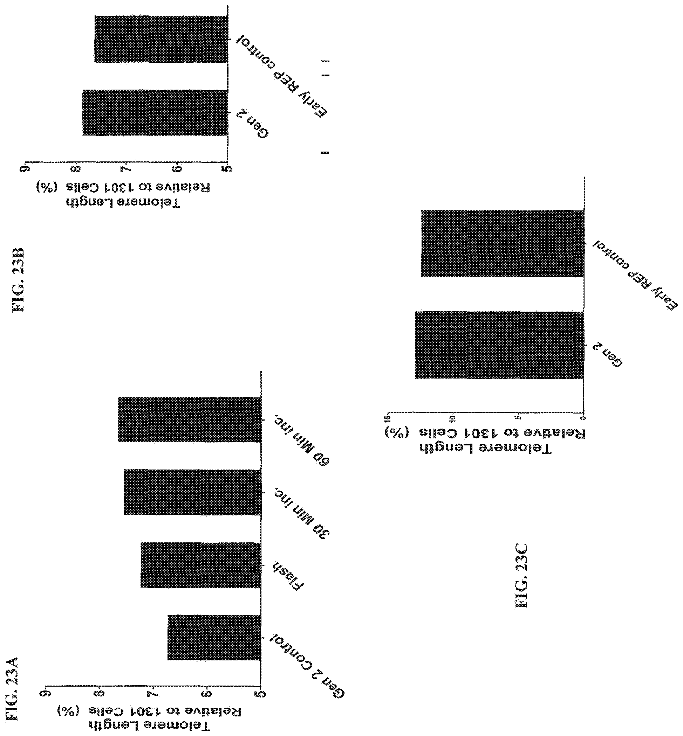

**Figure 23:** Figures 23A-23C illustrate telomere length relative to 1301 cells in fresh and frozen tumor samples for 3 patients: M1125 (FIG 23A); S13018 (FIG. 23B); and T6042 (FIG. 23C). Flow-FISH was performed using Dako/Agilent Pathology Solutions (Telomere PNA Kit/FITC for Flow Cytometry) kit and the manufacturer's instructions were followed to measure the average length of the Telomere repeat. 1301 T-cell leukemia cell line (SigmaAldrich, St. Louis, MO)) was used as an internal reference standard in each assay. Individual TIL were counted and mixed with 1301 cells at a 1:1 cell ratio. $2 \times 10^6$ TIL were mixed with $2 \times 10^6$ 1301 cells, in situ hybridization was performed using FITC-conjugated Telomere PNA probe (FITC-00-CCCTAA-CCC-TAA-CCC-TAA) and analyzed using Flow cytometry (BD canto II). Telomere fluorescence of the test sample was expressed as a percentage of the geometric mean fluorescence (fl) of the 1301 cells per the following formula: Relative telomere length = [(mean FITC fl test cells w/ probe-mean FITC fl test cells w/o probe) $\times$ DNA index of 1301 cells $\times$ 100] / [(mean FITC fl 1301 cells w/probe - mean FITC fl 1301 cells w/o probe) $\times$ DNA index of test cells. The data indicate that telomere length is comparable or better in frozen tumor samples than fresh tumor samples.

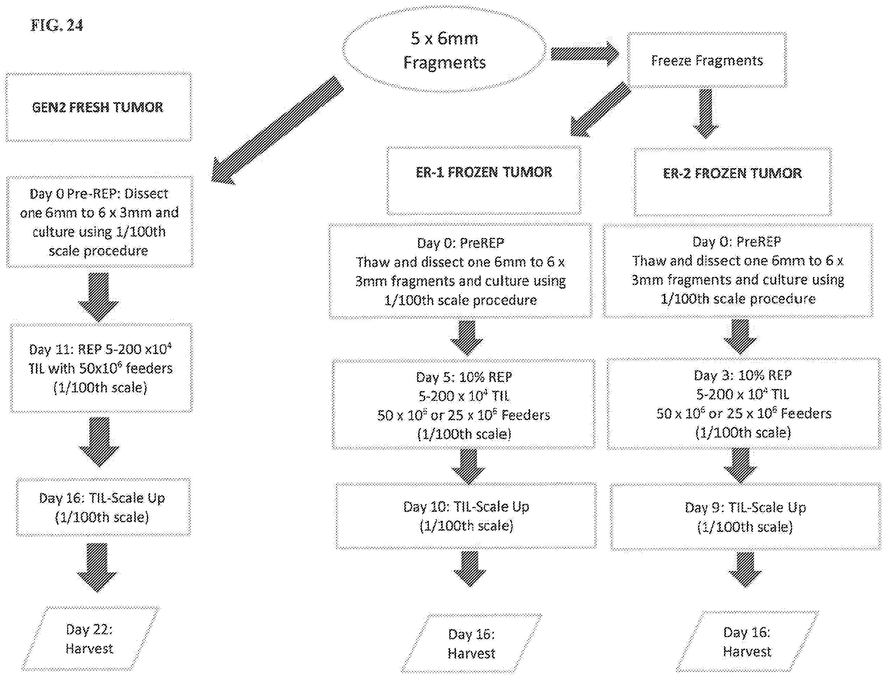

**Figure 24:** Figure 24 is a flow chart illustrating certain embodiments of the expansion process for TILs from fresh and frozen tumor fragments: fresh method ("GEN2"), early-REP method 1 ("ER-1"), and early-REP method 2 ("ER-2").

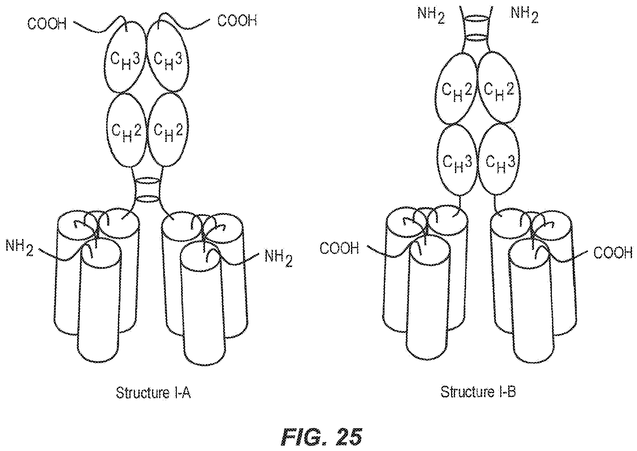

**Figure 25:** Figure 25 illustrates structures I-A and I-B as comprising three linearly-linked TNFRSF binding domains derived from e.g., 4-1BBL or an antibody that binds 4-1BB, which fold to form a trivalent protein, which is then linked to a second trivalent protein through IgG1-Fc (including CH3 and CH2 domains), and is then used to link two of the trivalent proteins together through disulfide bonds (small elongated ovals), stabilizing the structure and providing an agonist capable of bringing together the intracellular signaling domains of the six receptors and signaling proteins to form a signaling complex.

BRIEF DESCRIPTION OF THE SEQUENCE LISTING

SEQ ID NO:1 is the amino acid sequence of the heavy chain of muromonab.

SEQ ID NO:2 is the amino acid sequence of the light chain of muromonab.

SEQ ID NO:3 is the amino acid sequence of a recombinant human IL-2 protein.

SEQ ID NO:4 is the amino acid sequence of aldesleukin.

SEQ ID NO:5 is the amino acid sequence of a recombinant human IL-4 protein.

SEQ ID NO:6 is the amino acid sequence of a recombinant human IL-7 protein.

SEQ ID NO:7 is the amino acid sequence of a recombinant human IL-15 protein.

SEQ ID NO: 8 is the amino acid sequence of a recombinant human IL-21 protein.

SEQ ID NO:9 is the amino acid sequence of human 4-1BB.

SEQ ID NO:10 is the amino acid sequence of murine 4-1BB.

SEQ ID NO:11 is the heavy chain for the 4-1BB agonist monoclonal antibody utomilumab (PF-05082566).

SEQ ID NO:12 is the light chain for the 4-1BB agonist monoclonal antibody utomilumab (PF-05082566).

SEQ ID NO:13 is the heavy chain variable region ($V_H$) for the 4-1BB agonist monoclonal antibody utomilumab (PF-05082566).

SEQ ID NO:14 is the light chain variable region ($V_L$) for the 4-1BB agonist monoclonal antibody utomilumab (PF-05082566).

SEQ ID NO:15 is the heavy chain CDR1 for the 4-1BB agonist monoclonal antibody utomilumab (PF-05082566).

SEQ ID NO:16 is the heavy chain CDR2 for the 4-1BB agonist monoclonal antibody utomilumab (PF-05082566).

SEQ ID NO:17 is the heavy chain CDR3 for the 4-1BB agonist monoclonal antibody utomilumab (PF-05082566).

SEQ ID NO:18 is the light chain CDR1 for the 4-1BB agonist monoclonal antibody utomilumab (PF-05082566).

SEQ ID NO:19 is the light chain CDR2 for the 4-1BB agonist monoclonal antibody utomilumab (PF-05082566).

SEQ ID NO:20 is the light chain CDR3 for the 4-1BB agonist monoclonal antibody utomilumab (PF-05082566).

SEQ ID NO:21 is the heavy chain for the 4-1BB agonist monoclonal antibody urelumab (BMS-663513).

SEQ ID NO:22 is the light chain for the 4-1BB agonist monoclonal antibody urelumab (BMS-663513).

SEQ ID NO:23 is the heavy chain variable region ($V_H$) for the 4-1BB agonist monoclonal antibody urelumab (BMS-663513).

SEQ ID NO:24 is the light chain variable region ($V_L$) for the 4-1BB agonist monoclonal antibody urelumab (BMS-663513).

SEQ ID NO:25 is the heavy chain CDR1 for the 4-1BB agonist monoclonal antibody urelumab (BMS-663513).

SEQ ID NO:26 is the heavy chain CDR2 for the 4-1BB agonist monoclonal antibody urelumab (BMS-663513).

SEQ ID NO:27 is the heavy chain CDR3 for the 4-1BB agonist monoclonal antibody urelumab (BMS-663513).

SEQ ID NO:28 is the light chain CDR1 for the 4-1BB agonist monoclonal antibody urelumab (BMS-663513).

SEQ ID NO:29 is the light chain CDR2 for the 4-1BB agonist monoclonal antibody urelumab (BMS-663513).

SEQ ID NO:30 is the light chain CDR3 for the 4-1BB agonist monoclonal antibody urelumab (BMS-663513).

SEQ ID NO:31 is an Fc domain for a TNFRSF agonist fusion protein.

SEQ ID NO:32 is a linker for a TNFRSF agonist fusion protein.

SEQ ID NO:33 is a linker for a TNFRSF agonist fusion protein.

SEQ ID NO:34 is a linker for a TNFRSF agonist fusion protein.

SEQ ID NO:35 is a linker for a TNFRSF agonist fusion protein.

SEQ ID NO:36 is a linker for a TNFRSF agonist fusion protein.

SEQ ID NO:37 is a linker for a TNFRSF agonist fusion protein.

SEQ ID NO:38 is a linker for a TNFRSF agonist fusion protein.

SEQ ID NO:39 is a linker for a TNFRSF agonist fusion protein.

SEQ ID NO:40 is a linker for a TNFRSF agonist fusion protein.

SEQ ID NO:41 is a linker for a TNFRSF agonist fusion protein.

SEQ ID NO:42 is an Fc domain for a TNFRSF agonist fusion protein.

SEQ ID NO:43 is a linker for a TNFRSF agonist fusion protein.

SEQ ID NO:44 is a linker for a TNFRSF agonist fusion protein.

SEQ ID NO:45 is a linker for a TNFRSF agonist fusion protein.

SEQ ID NO:46 is a 4-1BB ligand (4-1BBL) amino acid sequence.

SEQ ID NO:47 is a soluble portion of 4-1BBL polypeptide.

SEQ ID NO:48 is a heavy chain variable region ($V_H$) for the 4-1BB agonist antibody 4B4-1-1 version 1.

SEQ ID NO:49 is a light chain variable region ($V_L$) for the 4-1BB agonist antibody 4B4-1-1 version 1.

SEQ ID NO:50 is a heavy chain variable region ($V_H$) for the 4-1BB agonist antibody 4B4-1-1 version 2.

SEQ ID NO:51 is a light chain variable region ($V_L$) for the 4-1BB agonist antibody 4B4-1-1 version 2.

SEQ ID NO:52 is a heavy chain variable region ($V_H$) for the 4-1BB agonist antibody H39E3-2.

SEQ ID NO:53 is a light chain variable region ($V_L$) for the 4-1BB agonist antibody H39E3-2.

SEQ ID NO:54 is the amino acid sequence of human OX40.

SEQ ID NO:55 is the amino acid sequence of murine OX40.

SEQ ID NO:56 is the heavy chain for the OX40 agonist monoclonal antibody tavolixizumab (MEDI-0562).

SEQ ID NO:57 is the light chain for the OX40 agonist monoclonal antibody tavolixizumab (MEDI-0562).

SEQ ID NO:58 is the heavy chain variable region ($V_H$) for the OX40 agonist monoclonal antibody tavolixizumab (MEDI-0562).

SEQ ID NO:59 is the light chain variable region ($V_L$) for the OX40 agonist monoclonal antibody tavolixizumab (MEDI-0562).

SEQ ID NO:60 is the heavy chain CDR1 for the OX40 agonist monoclonal antibody tavolixizumab (MEDI-0562).

SEQ ID NO:61 is the heavy chain CDR2 for the OX40 agonist monoclonal antibody tavolixizumab (MEDI-0562).

SEQ ID NO:62 is the heavy chain CDR3 for the OX40 agonist monoclonal antibody tavolixizumab (MEDI-0562).

SEQ ID NO:63 is the light chain CDR1 for the OX40 agonist monoclonal antibody tavolixizumab (MEDI-0562).

EP 3 852 524 B1

SEQ ID NO:64 is the light chain CDR2 for the OX40 agonist monoclonal antibody tavolixizumab (MEDI-0562).

SEQ ID NO:65 is the light chain CDR3 for the OX40 agonist monoclonal antibody tavolixizumab (MEDI-0562).

SEQ ID NO:66 is the heavy chain for the OX40 agonist monoclonal antibody 11D4.

SEQ ID NO:67 is the light chain for the OX40 agonist monoclonal antibody 11D4.

SEQ ID NO:68 is the heavy chain variable region ($V_H$) for the OX40 agonist monoclonal antibody 11D4.

SEQ ID NO:69 is the light chain variable region ($V_L$) for the OX40 agonist monoclonal antibody 11D4.

SEQ ID NO:70 is the heavy chain CDR1 for the OX40 agonist monoclonal antibody 11D4.

SEQ ID NO:71 is the heavy chain CDR2 for the OX40 agonist monoclonal antibody 11D4.

SEQ ID NO:72 is the heavy chain CDR3 for the OX40 agonist monoclonal antibody 11D4.

SEQ ID NO:73 is the light chain CDR1 for the OX40 agonist monoclonal antibody 11D4.

SEQ ID NO:74 is the light chain CDR2 for the OX40 agonist monoclonal antibody 11D4.

SEQ ID NO:75 is the light chain CDR3 for the OX40 agonist monoclonal antibody 11D4.

SEQ ID NO:76 is the heavy chain for the OX40 agonist monoclonal antibody 18D8.

SEQ ID NO:77 is the light chain for the OX40 agonist monoclonal antibody 18D8.

SEQ ID NO:78 is the heavy chain variable region ($V_H$) for the OX40 agonist monoclonal antibody 18D8.

SEQ ID NO:79 is the light chain variable region ($V_L$) for the OX40 agonist monoclonal antibody 18D8.

SEQ ID NO:80 is the heavy chain CDR1 for the OX40 agonist monoclonal antibody 18D8.

SEQ ID NO:81 is the heavy chain CDR2 for the OX40 agonist monoclonal antibody 18D8.

SEQ ID NO:82 is the heavy chain CDR3 for the OX40 agonist monoclonal antibody 18D8.

SEQ ID NO:83 is the light chain CDR1 for the OX40 agonist monoclonal antibody 18D8.

SEQ ID NO:84 is the light chain CDR2 for the OX40 agonist monoclonal antibody 18D8.

SEQ ID NO:85 is the light chain CDR3 for the OX40 agonist monoclonal antibody 18D8.

SEQ ID NO:86 is the heavy chain variable region ($V_H$) for the OX40 agonist monoclonal antibody Hu119-122.

SEQ ID NO:87 is the light chain variable region ($V_L$) for the OX40 agonist monoclonal antibody Hu119-122.

SEQ ID NO:88 is the heavy chain CDR1 for the OX40 agonist monoclonal antibody Hu 119-122.

SEQ ID NO:89 is the heavy chain CDR2 for the OX40 agonist monoclonal antibody Hu 119-122.

SEQ ID NO:90 is the heavy chain CDR3 for the OX40 agonist monoclonal antibody Hu 119-122.

SEQ ID NO:91 is the light chain CDR1 for the OX40 agonist monoclonal antibody Hu 119-122.

SEQ ID NO:92 is the light chain CDR2 for the OX40 agonist monoclonal antibody Hu 119-122.

13

SEQ ID NO:93 is the light chain CDR3 for the OX40 agonist monoclonal antibody Hu 119-122.

SEQ ID NO:94 is the heavy chain variable region ($V_H$) for the OX40 agonist monoclonal antibody Hu106-222.

SEQ ID NO:95 is the light chain variable region ($V_L$) for the OX40 agonist monoclonal antibody Hu106-222.

SEQ ID NO:96 is the heavy chain CDR1 for the OX40 agonist monoclonal antibody Hu 106-222.

SEQ ID NO:97 is the heavy chain CDR2 for the OX40 agonist monoclonal antibody Hu 106-222.

SEQ ID NO:98 is the heavy chain CDR3 for the OX40 agonist monoclonal antibody Hu 106-222.

SEQ ID NO:99 is the light chain CDR1 for the OX40 agonist monoclonal antibody Hu 106-222.

SEQ ID NO: 100 is the light chain CDR2 for the OX40 agonist monoclonal antibody Hu 106-222.

SEQ ID NO: 101 is the light chain CDR3 for the OX40 agonist monoclonal antibody Hu 106-222.

SEQ ID NO: 102 is an OX40 ligand (OX40L) amino acid sequence.

SEQ ID NO: 103 is a soluble portion of OX40L polypeptide.

SEQ ID NO: 104 is an alternative soluble portion of OX40L polypeptide.

SEQ ID NO: 105 is the heavy chain variable region ($V_H$) for the OX40 agonist monoclonal antibody 008.

SEQ ID NO:106 is the light chain variable region ($V_L$) for the OX40 agonist monoclonal antibody 008.

SEQ ID NO:107 is the heavy chain variable region ($V_H$) for the OX40 agonist monoclonal antibody 011.

SEQ ID NO:108 is the light chain variable region ($V_L$) for the OX40 agonist monoclonal antibody 011.

SEQ ID NO:109 is the heavy chain variable region ($V_H$) for the OX40 agonist monoclonal antibody 021.

SEQ ID NO:110 is the light chain variable region ($V_L$) for the OX40 agonist monoclonal antibody 021.

SEQ ID NO:111 is the heavy chain variable region ($V_H$) for the OX40 agonist monoclonal antibody 023.

SEQ ID NO:112 is the light chain variable region ($V_L$) for the OX40 agonist monoclonal antibody 023.

SEQ ID NO:113 is the heavy chain variable region ($V_H$) for an OX40 agonist monoclonal antibody.

SEQ ID NO:114 is the light chain variable region ($V_L$) for an OX40 agonist monoclonal antibody.

SEQ ID NO:115 is the heavy chain variable region ($V_H$) for an OX40 agonist monoclonal antibody.

SEQ ID NO:116 is the light chain variable region ($V_L$) for an OX40 agonist monoclonal antibody.

SEQ ID NO:117 is the heavy chain variable region ($V_H$) for a humanized OX40 agonist monoclonal antibody.

SEQ ID NO:118 is the heavy chain variable region ($V_H$) for a humanized OX40 agonist monoclonal antibody.

SEQ ID NO:119 is the light chain variable region ($V_L$) for a humanized OX40 agonist monoclonal antibody.

SEQ ID NO:120 is the light chain variable region ($V_L$) for a humanized OX40 agonist monoclonal antibody.

SEQ ID NO:121 is the heavy chain variable region ($V_H$) for a humanized OX40 agonist monoclonal antibody.

SEQ ID NO:122 is the heavy chain variable region (V$_H$) for a humanized OX40 agonist monoclonal antibody.

SEQ ID NO:123 is the light chain variable region (V$_L$) for a humanized OX40 agonist monoclonal antibody.

SEQ ID NO:124 is the light chain variable region (V$_L$) for a humanized OX40 agonist monoclonal antibody.

SEQ ID NO:125 is the heavy chain variable region (V$_H$) for an OX40 agonist monoclonal antibody.

SEQ ID NO:126 is the light chain variable region (V$_L$) for an OX40 agonist monoclonal antibody.

SEQ ID NO:127 is the heavy chain amino acid sequence of the PD-1 inhibitor nivolumab.

SEQ ID NO:128 is the light chain amino acid sequence of the PD-1 inhibitor nivolumab.

SEQ ID NO:129 is the heavy chain variable region (V$_H$) amino acid sequence of the PD-1 inhibitor nivolumab.

SEQ ID NO:130 is the light chain variable region (V$_L$) amino acid sequence of the PD-1 inhibitor nivolumab.

SEQ ID NO:131 is the heavy chain CDR1 amino acid sequence of the PD-1 inhibitor nivolumab.

SEQ ID NO:132 is the heavy chain CDR2 amino acid sequence of the PD-1 inhibitor nivolumab.

SEQ ID NO:133 is the heavy chain CDR3 amino acid sequence of the PD-1 inhibitor nivolumab.

SEQ ID NO:134 is the light chain CDR1 amino acid sequence of the PD-1 inhibitor nivolumab.

SEQ ID NO:135 is the light chain CDR2 amino acid sequence of the PD-1 inhibitor nivolumab.

SEQ ID NO:136 is the light chain CDR3 amino acid sequence of the PD-1 inhibitor nivolumab.

SEQ ID NO:137 is the heavy chain amino acid sequence of the PD-1 inhibitor pembrolizumab.

SEQ ID NO:138 is the light chain amino acid sequence of the PD-1 inhibitor pembrolizumab.

SEQ ID NO:139 is the heavy chain variable region (V$_H$) amino acid sequence of the PD-1 inhibitor pembrolizumab.

SEQ ID NO:140 is the light chain variable region (V$_L$) amino acid sequence of the PD-1 inhibitor pembrolizumab.

SEQ ID NO:141 is the heavy chain CDR1 amino acid sequence of the PD-1 inhibitor pembrolizumab.

SEQ ID NO:142 is the heavy chain CDR2 amino acid sequence of the PD-1 inhibitor pembrolizumab.

SEQ ID NO:143 is the heavy chain CDR3 amino acid sequence of the PD-1 inhibitor pembrolizumab.

SEQ ID NO:144 is the light chain CDR1 amino acid sequence of the PD-1 inhibitor pembrolizumab.

SEQ ID NO:145 is the light chain CDR2 amino acid sequence of the PD-1 inhibitor pembrolizumab.

SEQ ID NO:146 is the light chain CDR3 amino acid sequence of the PD-1 inhibitor pembrolizumab.

SEQ ID NO:147 is the heavy chain amino acid sequence of the PD-L1 inhibitor durvalumab.

SEQ ID NO:148 is the light chain amino acid sequence of the PD-L1 inhibitor durvalumab.

SEQ ID NO:149 is the heavy chain variable region (V$_H$) amino acid sequence of the PD-L1 inhibitor durvalumab.

SEQ ID NO:150 is the light chain variable region (V$_L$) amino acid sequence of the PD-L1 inhibitor durvalumab.

SEQ ID NO:151 is the heavy chain CDR1 amino acid sequence of the PD-L1 inhibitor durvalumab.

SEQ ID NO:152 is the heavy chain CDR2 amino acid sequence of the PD-L1 inhibitor durvalumab.

SEQ ID NO:153 is the heavy chain CDR3 amino acid sequence of the PD-L1 inhibitor durvalumab.

SEQ ID NO:154 is the light chain CDR1 amino acid sequence of the PD-L1 inhibitor durvalumab.

SEQ ID NO:155 is the light chain CDR2 amino acid sequence of the PD-L1 inhibitor durvalumab.

SEQ ID NO:156 is the light chain CDR3 amino acid sequence of the PD-L1 inhibitor durvalumab.

SEQ ID NO:157 is the heavy chain amino acid sequence of the PD-L1 inhibitor avelumab.

SEQ ID NO:158 is the light chain amino acid sequence of the PD-L1 inhibitor avelumab.

SEQ ID NO:159 is the heavy chain variable region ($V_H$) amino acid sequence of the PD-L1 inhibitor avelumab.

SEQ ID NO:160 is the light chain variable region ($V_L$) amino acid sequence of the PD-L1 inhibitor avelumab.

SEQ ID NO:161 is the heavy chain CDR1 amino acid sequence of the PD-L1 inhibitor avelumab.

SEQ ID NO:162 is the heavy chain CDR2 amino acid sequence of the PD-L1 inhibitor avelumab.

SEQ ID NO:163 is the heavy chain CDR3 amino acid sequence of the PD-L1 inhibitor avelumab.

SEQ ID NO:164 is the light chain CDR1 amino acid sequence of the PD-L1 inhibitor avelumab.

SEQ ID NO:165 is the light chain CDR2 amino acid sequence of the PD-L1 inhibitor avelumab.

SEQ ID NO:166 is the light chain CDR3 amino acid sequence of the PD-L1 inhibitor avelumab.

SEQ ID NO:167 is the heavy chain amino acid sequence of the PD-L1 inhibitor atezolizumab.

SEQ ID NO:168 is the light chain amino acid sequence of the PD-L1 inhibitor atezolizumab.

SEQ ID NO:169 is the heavy chain variable region ($V_H$) amino acid sequence of the PD-L1 inhibitor atezolizumab.

SEQ ID N0:170 is the light chain variable region ($V_L$) amino acid sequence of the PD-L1 inhibitor atezolizumab.

SEQ ID NO:171 is the heavy chain CDR1 amino acid sequence of the PD-L1 inhibitor atezolizumab.

SEQ ID NO:172 is the heavy chain CDR2 amino acid sequence of the PD-L1 inhibitor atezolizumab.

SEQ ID NO:173 is the heavy chain CDR3 amino acid sequence of the PD-L1 inhibitor atezolizumab.

SEQ ID NO:174 is the light chain CDR1 amino acid sequence of the PD-L1 inhibitor atezolizumab.

SEQ ID NO:175 is the light chain CDR2 amino acid sequence of the PD-L1 inhibitor atezolizumab.

SEQ ID NO:176 is the light chain CDR3 amino acid sequence of the PD-L1 inhibitor atezolizumab.

DETAILED DESCRIPTION OF THE INVENTION

Unless defined otherwise, all technical and scientific terms used herein have the same meaning as is commonly understood by one of skill in the art to which this invention belongs.

Definitions

The term "frozen" means that the tissue, cellular composition, or other composition, has become hard or rigid as the result of exposure to cold. The term as used herein encompasses the concept of cryopreservation, which is a process of storing cells, tissue, etc., at very low temperatures wherein the stored cells, tissue, etc., maintain their viability.

The term "cryostorage" means the keeping of already frozen compositions at very low temperatures wherein the stored cells, tissue, etc., maintain their viability.

The terms "flash freezing," "snap freezing," "flash frozen," and "snap frozen" mean causing to become frozen extremely rapidly, for non-limiting example, in less than about two minutes.

The term "LOVO cell processing system" and "LOVO" refers to the cell processing system manufactured by Fresenius Kabi USA, LLC. These two terms also refer to any instrument or device manufactured by any vendor that can pump a solution comprising cells through a membrane or filter such as a spinning membrane or spinning filter in a sterile and/or closed system environment, allowing for continuous flow and cell processing to remove supernatant or cell culture media without pelletization. In some embodiments, such a cell harvester and/or cell processing system can perform cell separation, washing, fluid-exchange, concentration, and/or other cell processing steps in a closed, sterile system.

The term "fungin" refers to the antifungal reagent Fungin™ sold by InvitroGen, San Diego, CA, USA, (catalog numbers ant-fn-1 and ant-fn-2). Fungin is a soluble formulation of pimaricin, CAS 7681-93-8. As used herein, "fungin" encompasses any commercial formulation of pimaricin or natamycin.

The term "fungizone" is a trademark of E. R. Squibb and Sons, LLC, and referes to the antimycotic amphotericin B, CAS 1397-89-3. Amphotericin B is commercially available, for example from SIGMA-Aldrich, St. Louis, MO, USA, (catalog number A2942, as a 250 $\mu$g/mL solution in deionized water). As used herein, "fungizone" encompasses any commercial formulation of amphotericin B.

The term "physiologically buffered isotonic saline solution" means any one of the many such salt solutions known to the skilled artisan wherein the solution is made to a physiological pH and isotonic salt concentration. In the art, these are commonly referred to as balanced salt solutions. Without limitation such physiologically buffered isotonic saline solution may comprise Hank's Balanced Salt Solution ("HBSS"), Tris-buffered saline ("TBS"), Phosphate buffered Saline ("PBS"), or Dulbecco's Phosphate Buffered Saline ("DPBS" or "dPBS").

The term "in vivo" refers to an event that takes place in a subject's body.

The term "in vitro" refers to an event that takes places outside of a subject's body. In vitro assays encompass cell-based assays in which cells alive or dead are employed and may also encompass a cell-free assay in which no intact cells are employed.

The term "ex vivo" refers to an event which involves treating or performing a procedure on a cell, tissue and/or organ which has been removed from a subject's body. Aptly, the cell, tissue and/or organ may be returned to the subject's body in a method of surgery or treatment.

The term "rapid expansion" means an increase in the number of antigen-specific TILs of at least about 3-fold (or 4-, 5-, 6-, 7-, 8-, or 9-fold) over a period of a week, more preferably at least about 10-fold (or 20-, 30-, 40-, 50-, 60-, 70-, 80-, or 90-fold) over a period of a week, or most preferably at least about 100-fold over a period of a week. A number of rapid expansion protocols are outlined below.

By "tumor infiltrating lymphocytes" or "TILs" herein is meant a population of cells originally obtained as white blood cells that have left the bloodstream of a subject and migrated into a tumor. TILs include, but are not limited to, CD8$^+$ cytotoxic T cells (lymphocytes), Th1 and Th17 CD4$^+$ T cells, natural killer cells, dendritic cells and M1 macrophages. TILs include both primary and secondary TILs. "Primary TILs" are those that are obtained from patient tissue samples as outlined herein (sometimes referred to as "freshly harvested"), and "secondary TILs" are any TIL cell populations that have been expanded or proliferated as discussed herein, including, but not limited to bulk TILs and expanded TILs ("REP TILs" or "post-REP TILs"). TIL cell populations can include genetically modified TILs.

By "population of cells" (including TILs) herein is meant a number of cells that share common traits. In general, populations generally range from $1 \times 10^6$ to $1 \times 10^{10}$ in number, with different TIL populations comprising different numbers. For example, initial growth of primary TILs in the presence of IL-2 results in a population of bulk TILs of roughly $1 \times 10^8$ cells. REP expansion is generally done to provide populations of $1.5 \times 10^9$ to $1.5 \times 10^{10}$ cells for infusion.

By "cryopreserved TILs" herein is meant that TILs, either primary, bulk, or expanded (REP TILs), are treated and stored at a temperature in the range of about -150°C to -60°C. General methods for cryopreservation are also described elsewhere herein, including in the Examples. For clarity and the avoidance of doubt, "cryopreserved TILs" are distinguishable from frozen tissue samples which may be used as a source of primary TILs.

By "thawed cryopreserved TILs" herein is meant a population of TILs that was previously cryopreserved and then treated to return to room temperature or higher, including but not limited to cell culture temperatures or temperatures wherein TILs may be administered to a patient.

TILs can generally be defined either biochemically, using cell surface markers, or functionally, by their ability to infiltrate tumors and effect treatment. TILs can be generally categorized by expressing one or more of the following biomarkers: CD4, CD8, TCR αβ, CD27, CD28, CD56, CCR7, CD45Ra, CD95, PD-1, and CD25. Additionally, and alternatively, TILs can be functionally defined by their ability to infiltrate solid tumors upon reintroduction into a patient.

The term "cryopreservation media" or "cryopreservation medium" refers to any medium that can be used for cryopreservation of cells. Such media can include media comprising 5% v/v DMSO to 10% v/v DMSO; such media can also include media comprising 7% v/v DMSO to 10% v/v DMSO. Exemplary media include CryoStor CS10, Hyperthermasol, as well as combinations thereof. The term "CS10" refers to a cryopreservation medium which is obtained from Stemcell Technologies or from Biolife Solutions. The CS10 medium may be referred to by the trade name "CryoStor® CS10". The CS10 medium is a serum-free, animal component-free medium which comprises DMSO.

The term "central memory T cell" refers to a subset of T cells that in the human are CD45R0+ and constitutively express CCR7 (CCR7$^{hi}$) and CD62L (CD62$^{hi}$). The surface phenotype of central memory T cells also includes TCR, CD3, CD127 (IL-7R), and IL-15R. Transcription factors for central memory T cells include BCL-6, BCL-6B, MBD2, and BMI1. Central memory T cells primarily secrete IL-2 and CD40L as effector molecules after TCR triggering. Central memory T cells are predominant in the CD4 compartment in blood, and in the human are proportionally enriched in lymph nodes and tonsils.

The term "effector memory T cell" refers to a subset of human or mammalian T cells that, like central memory T cells, are CD45R0$^+$, but have lost the constitutive expression of CCR7 (CCR7$^{lo}$) and are heterogeneous or low for CD62L expression (CD62L$^{lo}$). The surface phenotype of central memory T cells also includes TCR, CD3, CD127 (IL-7R), and IL-15R. Transcription factors for central memory T cells include BLIMP1. Effector memory T cells rapidly secret high levels of inflammatory cytokines following antigenic stimulation, including interferon-γ, IL-4, and IL-5. Effector memory T cells are predominant in the CD8 compartment in blood, and in the human are proportionally enriched in the lung, liver, and gut. CD8$^+$ effector memory T cells carry large amounts of perforin.

The term "closed system" refers to a system that is closed to the outside environment. Any closed system appropriate for cell culture methods can be employed with the methods of the present invention. Closed systems include, for example, but are not limited to closed G-containers. Once a tumor fragment is added to the closed system, the system is not opened to the outside environment until the TILs are ready to be administered to the patient.

The terms "fragmenting," "fragment," and "fragmented," as used herein to describe processes for disrupting a tumor, includes mechanical fragmentation methods such as crushing, slicing, dividing, and morcellating tumor tissue as well as any other method for disrupting the physical structure of tumor tissue.

The terms "peripheral blood mononuclear cells" and "PBMCs" refers to a peripheral blood cell having a round nucleus, including lymphocytes (T cells, B cells, NK cells) and monocytes. Preferably, the peripheral blood mononuclear cells are irradiated allogeneic peripheral blood mononuclear cells. PBMCs are a type of antigen-presenting cell.

The term "anti-CD3 antibody" refers to an antibody or variant thereof, *e.g.,* a monoclonal antibody and including human, humanized, chimeric or murine antibodies which are directed against the CD3 receptor in the T cell antigen receptor of mature T cells. Anti-CD3 antibodies include OKT-3, also known as muromonab. Anti-CD3 antibodies also include the UHCT1 clone, also known as T3 and CD3ε. Other anti-CD3 antibodies include, for example, otelixizumab, teplizumab, and visilizumab.

The term "OKT-3" (also referred to herein as "OKT3") refers to a monoclonal antibody or biosimilar or variant thereof, including human, humanized, chimeric, or murine antibodies, directed against the CD3 receptor in the T cell antigen receptor of mature T cells, and includes commercially-available forms such as OKT-3 (30 ng/mL, MACS GMP CD3 pure, Miltenyi Biotech, Inc., San Diego, CA, USA) and muromonab or variants, conservative amino acid substitutions, glycoforms, or biosimilars thereof. The amino acid sequences of the heavy and light chains of muromonab are given in Table 1 (SEQ ID NO:1 and SEQ ID NO:2). A hybridoma capable of producing OKT-3 is deposited with the American Type Culture Collection and assigned the ATCC accession number CRL 8001. A hybridoma capable of producing OKT-3 is also deposited with European Collection of Authenticated Cell Cultures (ECACC) and assigned Catalogue No. 86022706.

TABLE 1. Amino acid sequences of muromonab.

| Identifier | Sequence (One-Letter Amino Acid Symbols) | |
|---|---|---|
| SEQ ID NO:1 Muromonab heavy chain | QVQLQQSGAE LARPGASVKM SCKASGYTFT RYTMHWVKQR PGQGLEWIGY INPSRGYTNY | 60 |
| | NQKFKDKATL TTDKSSSTAY MQLSSLTSED SAVYYCARYY DDHYCLDYWG QGTTLTVSSA | 120 |
| | KTTAPSVYPL APVCGGTTGS SVTLGCLVKG YFPEPVTLTW NSGSLSSGVH TFPAVLQSDL | 180 |
| | YTLSSSVTVT SSTWPSQSIT CNVAHPASST KVDKKIEPRP KSCDKTHTCP PCPAPELLGG | 240 |
| | PSVFLFPPKP KDTLMISRTP EVTCVVVDVS HEDPEVKFNW YVDGVEVHNA KTKPREEQYN | 300 |
| | STYRVVSVLT VLHQDWLNGK EYKCKVSNKA LPAPIEKTIS KAKGQPREPQ VYTLPPSRDE | 360 |
| | LTKNQVSLTC LVKGFYPSDI AVEWESNGQP ENNYKTTPPV LDSDGSFFLY SKLTVDKSRW | 420 |
| | QQGNVFSCSV MHEALHNHYT QKSLSLSPGK | 450 |

18

(continued)

| Identifier | Sequence (One-Letter Amino Acid Symbols) | |
|---|---|---|
| SEQ ID NO:2 Muromonab light chain | QIVLTQSPAI MSASPGEKVT MTCSASSSVS YMNWYQQKSG TSPKRWIYDT SKLASGVPAH<br>FRGSGSGTSY SLTISGMEAE DAATYYCQQW SSNPFTFGSG TKLEINRADT APTVSIFPPS<br>SEQLTSGGAS VVCFLNNFYP KDINVKWKID GSERQNGVLN SWTDQDSKDS TYSMSSTLTL<br>TKDEYERHNS YTCEATHKTS TSPIVKSFNR NEC | 60<br>120<br>180<br>213 |

TABLE 2. Amino acid sequences of interleukins.

| Identifier | Sequence (One-Letter Amino Acid Symbols) | |
|---|---|---|
| SEQ ID NO:3 recombinant human IL-2 (rhIL-2) | MAPTSSSTKK TQLQLEHLLL DLQMILNGIN NYKNPKLTRM LTFKFYMPKK ATELKHLQCL<br>EEELKPLEEV LNLAQSKNFH LRPRDLISNI NVIVLELKGS ETTFMCEYAD ETATIVEFLN<br>RWITFCQSII STLT | 60<br>120<br>134 |
| SEQ ID NO:4 Aldesleukin | PTSSSTKKTQ LQLEHLLLDL QMILNGINNY KNPKLTRMLT FKFYMPKKAT ELKHLQCLEE<br>ELKPLEEVLN LAQSKNFHLR PRDLISNINV IVLELKGSET TFMCEYADET ATIVEFLNRW<br>ITFSQSIIST LT | 60<br>120<br>132 |
| SEQ ID NO:5 recombinant human IL-4 (rhIL-4) | MHKCDITLQE IIKTLNSLTE QKTLCTELTV TDIFAASKNT TEKETFCRAA TVLRQFYSHH<br>EKDTRCLGAT AQQFHRHKQL IRFLKRLDRN LWGLAGLNSC PVKEANQSTL ENFLERLKTI<br>MREKYSKCSS | 60<br>120<br>130 |
| SEQ ID NO:6 recombinant human IL-7 (rhIL-7) | MDCDIEGKDG KQYESVLMVS IDQLLDSMKE IGSNCLNNEF NFFKRHICDA NKEGMFLFRA<br>ARKLRQFLKM NSTGDFDLHL LKVSEGTTIL LNCTGQVKGR KPAALGEAQP TKSLEENKSL<br>KEQKKLNDLC FLKRLLQEIK TCWNKILMGT KEH | 60<br>120<br>153 |
| SEQ ID NO:7 recombinant human IL-15 (rhIL-15) | MNWVNVISDL KKIEDLIQSM HIDATLYTES DVHPSCKVTA MKCFLLELQV ISLESGDASI<br>HDTVENLIIL ANNSLSSNGN VTESGCKECE ELEEKNIKEF LQSFVHIVQM FINTS | 60<br>115 |
| SEQ ID NO:8 recombinant human IL-21 (rhIL-21) | MQDRHMIRMR QLIDIVDQLK NYVNDLVPEF LPAPEDVETN CEWSAFSCFQ KAQLKSANTG<br>NNERIINVSI KKLKRKPPST NAGRRQKHRL TCPSCDSYEK KPPKEFLERF KSLLQKMIHQ<br>HLSSRTHGSE DS | 60<br>120<br>132 |

The term "IL-2" (also referred to herein as "IL2") refers to the T cell growth factor known as interleukin-2, and includes all forms of IL-2 including human and mammalian forms, conservative amino acid substitutions, glycoforms, biosimilars, and variants thereof. IL-2 is described, *e.g.,* in Nelson, J. Immunol. 2004, 172, 3983-88 and Malek, Annu. Rev. Immunol. 2008, 26, 453-79. The amino acid sequence of recombinant human IL-2 suitable for use in the invention is given in Table 2 (SEQ ID NO:3). For example, the term IL-2 encompasses human, recombinant forms of IL-2 such as aldesleukin (PROLEUKIN, available commercially from multiple suppliers in 22 million IU per single use vials), as well as the form of recombinant IL-2 commercially supplied by CellGenix, Inc., Portsmouth, NH, USA (CELLGRO GMP) or ProSpec-Tany TechnoGene Ltd., East Brunswick, NJ, USA (Cat. No. CYT-209-b) and other commercial equivalents from other vendors. Aldesleukin (des-alanyl-1, serine-125 human IL-2) is a nonglycosylated human recombinant form of IL-2 with a molecular weight of approximately 15 kDa. The amino acid sequence of aldesleukin suitable for use in the invention is given in Table 2 (SEQ ID NO:4). The term IL-2 also encompasses pegylated forms of IL-2, as described herein, including the pegylated IL2 prodrug NKTR-214, available from Nektar Therapeutics, South San Francisco, CA, USA. NKTR-214 and pegylated IL-2 suitable for use in the invention is described in U.S. Patent Application Publication No. US 2014/0328791 A1 and International Patent Application Publication No. WO 2012/065086 A1. Alternative forms of conjugated IL-2 suitable for use in the invention are described in U.S. Patent Nos. 4,766,106, 5,206,344, 5,089,261 and 4902,502. Formulations of IL-2 suitable for use in the invention are described in U.S. Patent No. 6,706,289.

The term "IL-4" (also referred to herein as "IL4") refers to the cytokine known as interleukin 4, which is produced by Th2 T cells and by eosinophils, basophils, and mast cells. IL-4 regulates the differentiation of naive helper T cells (Th0 cells) to Th2 T cells. Steinke and Borish, Respir. Res. 2001, 2, 66-70. Upon activation by IL-4, Th2 T cells subsequently produce additional IL-4 in a positive feedback loop. IL-4 also stimulates B cell proliferation and class II MHC expression, and induces class switching to IgE and IgG1 expression from B cells. Recombinant human IL-4 suitable for use in the invention is commercially available from multiple suppliers, including ProSpec-Tany TechnoGene Ltd., East Brunswick, NJ, USA (Cat. No. CYT-211) and ThermoFisher Scientific, Inc., Waltham, MA, USA (human IL-15 recombinant protein, Cat. No. Gibco CTP0043). The amino acid sequence of recombinant human IL-4 suitable for use in the invention is given in Table 2 (SEQ ID NO:5).

The term "IL-7" (also referred to herein as "IL7") refers to a glycosylated tissue-derived cytokine known as interleukin 7, which may be obtained from stromal and epithelial cells, as well as from dendritic cells. Fry and Mackall, Blood 2002, 99, 3892-904. IL-7 can stimulate the development of T cells. IL-7 binds to the IL-7 receptor, a heterodimer consisting of IL-7 receptor alpha and common gamma chain receptor, which in a series of signals important for T cell development within the thymus and survival within the periphery. Recombinant human IL-7 suitable for use in the invention is commercially available from multiple suppliers, including ProSpec-Tany TechnoGene Ltd., East Brunswick, NJ, USA (Cat. No. CYT-254) and ThermoFisher Scientific, Inc., Waltham, MA, USA (human IL-15 recombinant protein, Cat. No. Gibco PHC0071). The amino acid sequence of recombinant human IL-7 suitable for use in the invention is given in Table 2 (SEQ ID NO:6).

The term "IL-15" (also referred to herein as "IL15") refers to the T cell growth factor known as interleukin-15, and includes all forms of IL-2 including human and mammalian forms, conservative amino acid substitutions, glycoforms, biosimilars, and variants thereof. IL-15 is described, e.g., in Fehniger and Caligiuri, Blood 2001, 97, 14-32. IL-15 shares β and γ signaling receptor subunits with IL-2. Recombinant human IL-15 is a single, non-glycosylated polypeptide chain containing 114 amino acids (and an N-terminal methionine) with a molecular mass of 12.8 kDa. Recombinant human IL-15 is commercially available from multiple suppliers, including ProSpec-Tany TechnoGene Ltd., East Brunswick, NJ, USA (Cat. No. CYT-230-b) and ThermoFisher Scientific, Inc., Waltham, MA, USA (human IL-15 recombinant protein, Cat. No. 34-8159-82). The amino acid sequence of recombinant human IL-15 suitable for use in the invention is given in Table 2 (SEQ ID NO:7).

The term "IL-21" (also referred to herein as "IL21") refers to the pleiotropic cytokine protein known as interleukin-21, and includes all forms of IL-21 including human and mammalian forms, conservative amino acid substitutions, glycoforms, biosimilars, and variants thereof. IL-21 is described, e.g., in Spolski and Leonard, Nat. Rev. Drug. Disc., 13:379-95 (2014). IL-21 is primarily produced by natural killer T cells and activated human CD4[+] T cells. Recombinant human IL-21 is a single, non-glycosylated polypeptide chain containing 132 amino acids with a molecular mass of 15.4 kDa. Recombinant human IL-21 is commercially available from multiple suppliers, including ProSpec-Tany TechnoGene Ltd., East Brunswick, NJ, USA (Cat. No. CYT-408-b) and ThermoFisher Scientific, Inc., Waltham, MA, USA (human IL-21 recombinant protein, Cat. No. 14-8219-80). The amino acid sequence of recombinant human IL-21 suitable for use in the invention is given in Table 2 (SEQ ID NO:8).

The term "effective amount" or "therapeutically effective amount" refers to that amount of a compound or combination of compounds as described herein that is sufficient to effect the intended application including, but not limited to, disease treatment. A therapeutically effective amount may vary depending upon the intended application (in vitro, in vivo, and ex vivo), or the subject and disease condition being treated (e.g., the weight, age and gender of the subject), the severity of the disease condition, the manner of administration, etc. which can readily be determined by one of ordinary skill in the art. The term also applies to a dose that will induce a particular response in target cells (e.g., the reduction of platelet adhesion and/or cell migration). The specific dose will vary depending on the particular compounds chosen, the dosing regimen to be followed, whether the compound is administered in combination with other compounds, timing of administration, the tissue to which it is administered, and the physical delivery system in which the compound is carried.

A "therapeutic effect" as that term is used herein, encompasses a therapeutic benefit and/or a prophylactic benefit. A prophylactic effect includes delaying or eliminating the appearance of a disease or condition, delaying or eliminating the onset of symptoms of a disease or condition, slowing, halting, or reversing the progression of a disease or condition, or any combination thereof.

"Pharmaceutically acceptable carrier" or "pharmaceutically acceptable excipient" is intended to include any and all solvents, dispersion media, coatings, antibacterial and antifungal agents, isotonic and absorption delaying agents, and inert ingredients. The use of such pharmaceutically acceptable carriers or pharmaceutically acceptable excipients for active pharmaceutical ingredients is well known in the art. Except insofar as any conventional pharmaceutically acceptable carrier or pharmaceutically acceptable excipient is incompatible with the active pharmaceutical ingredient, its use in the therapeutic compositions of the invention is contemplated. Additional active pharmaceutical ingredients, such as other drugs, can also be incorporated into the described compositions and methods.

When ranges are used herein to describe, for example, physical or chemical properties such as molecular weight or chemical formulae, all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, preferably from 0% to 10%, more preferably from 0% to 5% of the stated number or numerical range. The term "comprising" (and related terms such as "comprise" or "comprises" or "having" or "including") includes those embodiments such as, for example, an embodiment of any composition of matter, method or process that "consist of" or "consist essentially of" the described features.

Compounds of the invention also include antibodies. The terms "antibody" and its plural form "antibodies" refer to whole immunoglobulins and any antigen-binding fragment ("antigen-binding portion") or single chains thereof. An "antibody" further refers to a glycoprotein comprising at least two heavy (H) chains and two light (L) chains inter-connected by disulfide bonds, or an antigen-binding portion thereof. Each heavy chain is comprised of a heavy chain variable region (abbreviated herein as $V_H$) and a heavy chain constant region. The heavy chain constant region is comprised of three domains, CH1, CH2 and CH3. Each light chain is comprised of a light chain variable region (abbreviated herein as $V_L$) and a light chain constant region. The light chain constant region is comprised of one domain, $C_L$. The $V_H$ and $V_L$ regions of an antibody may be further subdivided into regions of hypervariability, which are referred to as complementarity determining regions (CDR) or hypervariable regions (HVR), and which can be interspersed with regions that are more conserved, termed framework regions (FR). Each $V_H$ and $V_L$ is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen epitope or epitopes. The constant regions of the antibodies may mediate the binding of the immunoglobulin to host tissues or factors, including various cells of the immune system (e.g., effector cells) and the first component (Clq) of the classical complement system.

The terms "antigen-binding portion" or "antigen-binding fragment" of an antibody (or simply "antibody portion"), as used herein, refers to one or more fragments of an antibody that retain the ability to specifically bind to an antigen (e.g., IL-33, ST2, CD20, PD-1, PD-L1, or PD-L2). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the $V_L$, $V_H$, $C_L$ and CH1 domains; (ii) a F(ab')2 fragment, a bivalent fragment comprising two Fab fragments linked by a disulfide bridge at the hinge region; (iii) a Fd fragment consisting of the $V_H$ and CH1 domains; (iv) a Fv fragment consisting of the $V_L$ and $V_H$ domains of a single arm of an antibody, (v) a domain antibody (dAb) fragment (Ward et al., Nature, 1989, 341, 544-546), which may consist of a $V_H$ or a $V_L$ domain; and (vi) an isolated complementarity determining region (CDR). Furthermore, although the two domains of the Fv fragment, $V_L$ and $V_H$, are coded for by separate genes, they can be joined, using recombinant methods, by a synthetic linker that enables them to be made as a single protein chain in which the $V_L$ and $V_H$ regions pair to form monovalent molecules known as single chain Fv (scFv); see, e.g., Bird et al., Science 1988, 242, 423-426; and Huston et al., Proc. Natl. Acad. Sci. USA 1988, 85, 5879-5883). Such scFv antibodies are also intended to be encompassed within the terms "antigen-binding portion" or "antigen-binding fragment" of an antibody. These antibody fragments are obtained using conventional techniques known to those with skill in the art, and the fragments are screened for utility in the same manner as are intact antibodies.

The term "human antibody," as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo). The term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, e.g., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the $V_H$ and $V_L$ regions of the recombinant antibodies are sequences that, while derived from and related to human germline $V_H$ and $V_L$ sequences, may not naturally exist within the human antibody germline repertoire in vivo.

As used herein, "isotype" refers to the antibody class (*e.g.*, IgM or IgG1) that is encoded by the heavy chain constant region genes. In mammals, there are five antibody isotypes: IgA, IgD, IgG, IgM and IgE. In humans, there are four subclasses of the IgG isotype: IgG1, IgG2, IgG3 and IgG4, and two subclasses of the IgA isotype: IgA1 and IgA2.

The phrases "an antibody recognizing an antigen" and "an antibody specific for an antigen" are used interchangeably herein with the term "an antibody which binds specifically to an antigen."

The term "human antibody derivatives" refers to any modified form of the human antibody, *e.g.,* a conjugate of the antibody and another active pharmaceutical ingredient or antibody. The terms "conjugate," "antibody-drug conjugate", "ADC," or "immunoconjugate" refers to an antibody, or a fragment thereof, conjugated to a therapeutic moiety, such as a bacterial toxin, a cytotoxic drug or a radionuclide-containing toxin. Toxic moieties can be conjugated to antibodies of the invention using methods available in the art.

The terms "humanized antibody," "humanized antibodies," and "humanized" are intended to refer to antibodies in which CDR sequences derived from the germline of another mammalian species, such as a mouse, have been grafted onto human framework sequences. Additional framework region modifications may be made within the human framework sequences. Humanized forms of non-human (for example, murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a 15 hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 1986, 321, 522-525; Riechmann et al., Nature 1988, 332, 323-329; and Presta, Curr. Op. Struct. Biol. 1992, 2, 593-596.

The term "chimeric antibody" is intended to refer to antibodies in which the variable region sequences are derived from one species and the constant region sequences are derived from another species, such as an antibody in which the variable region sequences are derived from a mouse antibody and the constant region sequences are derived from a human antibody.

A "diabody" is a small antibody fragment with two antigen-binding sites. The fragments comprises a heavy chain variable domain ($V_H$) connected to a light chain variable domain ($V_L$) in the same polypeptide chain ($V_H$-$V_L$ or $V_L$-$V_H$). By using a linker that is too short to allow pairing between the two domains on the same chain, the domains are forced to pair with the complementary domains of another chain and create two antigen-binding sites. Diabodies are described more fully in, *e.g.*, European Patent No. EP 404,097, International Patent Publication No. WO 93/11161; and Bolliger et al., Proc. Natl. Acad. Sci. USA 1993, 90, 6444-6448.

The term "glycosylation" refers to a modified derivative of an antibody. An aglycoslated antibody lacks glycosylation. Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Aglycosylation may increase the affinity of the antibody for antigen, as described in U.S. Patent Nos. 5,714,350 and 6,350,861. Additionally, or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation. For example, the cell lines Ms704, Ms705, and Ms709 lack the fucosyltransferase gene, FUT8 (alpha (1,6) fucosyltransferase), such that antibodies expressed in the Ms704, Ms705, and Ms709 cell lines lack fucose on their carbohydrates. The Ms704, Ms705, and Ms709 FUT8$^{-/-}$ cell lines were created by the targeted disruption of the FUT8 gene in CHO/DG44 cells using two replacement vectors (*see e.g.* U.S. Patent Publication No. 2004/0110704 or Yamane-Ohnuki, et al. Biotechnol. Bioeng., 2004, 87, 614-622). As another example, European Patent No. EP 1,176,195 describes a cell line with a functionally disrupted FUT8 gene, which encodes a fucosyl transferase, such that antibodies expressed in such a cell line exhibit hypofucosylation by reducing or eliminating the alpha 1,6 bond-related enzyme, and also describes cell lines which have a low enzyme activity for adding fucose to the N-acetylglucosamine that binds to the Fc region of the antibody or does not have the enzyme activity, for example the rat myeloma cell line YB2/0 (ATCC CRL 1662). International Patent Publication WO 03/035835 describes a variant CHO cell line, Lec 13 cells, with reduced ability to attach fucose to Asn(297)-linked carbohydrates, also resulting in hypofucosylation of antibodies expressed in that host cell (see also Shields, et al., J. Biol. Chem. 2002, 277, 26733-26740. International Patent Publication WO 99/54342 describes cell lines engineered to express glycoprotein-modifying glycosyl transferases (e.g., beta(1,4)-N-acetylglucosaminyltransferase III (GnTIII)) such that antibodies expressed in the engineered cell lines exhibit increased bisecting GlcNac structures which results in increased ADCC activity of the antibodies (see also Umana, et al., Nat. Biotech. 1999, 17, 176-180). Alternatively, the fucose residues of the antibody may be cleaved off using a fucosidase enzyme. For example, the fucosidase alpha-L-fucosidase removes fucosyl residues from antibodies as described in Tarentino, et al., Biochem. 1975, 14, 5516-5523.

"Pegylation" refers to a modified antibody, or a fragment thereof, that typically is reacted with polyethylene glycol (PEG), such as a reactive ester or aldehyde derivative of PEG, under conditions in which one or more PEG groups become attached to the antibody or antibody fragment. Pegylation may, for example, increase the biological (e.g., serum) half life of the antibody. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono ($C_1$-$C_{10}$) alkoxy- or aryloxy-polyethylene glycol or polyethylene glycol-maleimide. The antibody to be pegylated may be an aglycosylated antibody. Methods for pegylation are known in the art and can be applied to the antibodies of the invention, as described for example in European Patent Nos. EP 0154316 and EP 0401384.

The term "biosimilar" means a biological product that is highly similar to a U.S. licensed reference biological product notwithstanding minor differences in clinically inactive components, and for which there are no clinically meaningful differences between the biological product and the reference product in terms of the safety, purity, and potency of the product. Furthermore, a similar biological or "biosimilar" medicine is a biological medicine that is similar to another biological medicine that has already been authorized for use by the European Medicines Agency. The term "biosimilar" is also used synonymously by other national and regional regulatory agencies. Biological products or biological medicines are medicines that are made by or derived from a biological source, such as a bacterium or yeast. They can consist of relatively small molecules such as human insulin or erythropoietin, or complex molecules such as monoclonal antibodies. For example, if the reference anti-CD20 monoclonal antibody is rituximab, an anti-CD20 biosimilar monoclonal antibody approved by drug regulatory authorities with reference to rituximab is a "biosimilar to" rituximab or is a "biosimilar thereof' of rituximab. In Europe, a similar biological or "biosimilar" medicine is a biological medicine that is similar to another biological medicine that has already been authorized for use by the European Medicines Agency (EMA). The relevant legal basis for similar biological applications in Europe is Article 6 of Regulation (EC) No 726/2004 and Article 10(4) of Directive 2001/83/EC, as amended and therefore in Europe, the biosimilar may be authorised, approved for authorisation or subject of an application for authorisation under Article 6 of Regulation (EC) No 726/2004 and Article 10(4) of Directive 2001/83/EC. The already authorized original biological medicinal product may be referred to as a "reference medicinal product" in Europe. Some of the requirements for a product to be considered a biosimilar are outlined in the CHMP Guideline on Similar Biological Medicinal Products. In addition, product specific guidelines, including guidelines relating to monoclonal antibody biosimilars, are provided on a product-by-product basis by the EMA and published on its website. A biosimilar as described herein may be similar to the reference medicinal product by way of quality characteristics, biological activity, mechanism of action, safety profiles and/or efficacy. In addition, the biosimilar may be used or be intended for use to treat the same conditions as the reference medicinal product. Thus, a biosimilar as described herein may be deemed to have similar or highly similar quality characteristics to a reference medicinal product. Alternatively, or in addition, a biosimilar as described herein may be deemed to have similar or highly similar biological activity to a reference medicinal product. Alternatively, or in addition, a biosimilar as described herein may be deemed to have a similar or highly similar safety profile to a reference medicinal product. Alternatively, or in addition, a biosimilar as described herein may be deemed to have similar or highly similar efficacy to a reference medicinal product. As described herein, a biosimilar in Europe is compared to a reference medicinal product which has been authorised by the EMA. However, in some instances, the biosimilar may be compared to a biological medicinal product which has been authorised outside the European Economic Area (a non-EEA authorised "comparator") in certain studies. Such studies include for example certain clinical and in vivo non-clinical studies. As used herein, the term "biosimilar" also relates to a biological medicinal product which has been or may be compared to a non-EEA authorised comparator. Certain biosimilars are proteins such as antibodies, antibody fragments (for example, antigen binding portions) and fusion proteins. A protein biosimilar may have an amino acid sequence that has minor modifications in the amino acid structure (including for example deletions, additions, and/or substitutions of amino acids) which do not significantly affect the function of the polypeptide. The biosimilar may comprise an amino acid sequence having a sequence identity of 97% or greater to the amino acid sequence of its reference medicinal product, e.g., 97%, 98%, 99% or 100%. The biosimilar may comprise one or more post-translational modifications, for example, although not limited to, glycosylation, oxidation, deamidation, and/or truncation which is/are different to the post-translational modifications of the reference medicinal product, provided that the differences do not result in a change in safety and/or efficacy of the medicinal product. The biosimilar may have an identical or different glycosylation pattern to the reference medicinal product. Particularly, although not exclusively, the biosimilar may have a different glycosylation pattern if the differences address or are intended to address safety concerns associated with the reference medicinal product. Additionally, the biosimilar may deviate from the reference medicinal product in for example its strength, pharmaceutical form, formulation, excipients and/or presentation, providing safety and efficacy of the medicinal product is not compromised. The biosimilar may comprise differences in for example pharmacokinetic (PK) and/or pharmacodynamic (PD) profiles as compared to the reference medicinal product but is still deemed sufficiently similar to the reference medicinal product as to be authorised or considered suitable for authorisation. In certain circumstances, the biosimilar exhibits different binding characteristics as compared to the reference medicinal product, wherein the different binding characteristics are considered by a Regulatory Authority such as the EMA not to be a barrier for authorisation as a similar biological product. The term "biosimilar" is also used synonymously by other national and regional regulatory agencies.

The term "hematological malignancy" refers to mammalian cancers and tumors of the hematopoietic and lymphoid tissues, including but not limited to tissues of the blood, bone marrow, lymph nodes, and lymphatic system. Hematological malignancies are also referred to as "liquid tumors." Hematological malignancies include, but are not limited to, ALL, CLL, SLL, acute myelogenous leukemia (AML), chronic myelogenous leukemia (CML), acute monocytic leukemia (AMoL), Hodgkin's lymphoma, and non-Hodgkin's lymphomas. The term "B cell hematological malignancy" refers to hematological malignancies that affect B cells.

The term "solid tumor" refers to an abnormal mass of tissue that usually does not contain cysts or liquid areas. Solid tumors may be benign or malignant. The term "solid tumor cancer" refers to malignant, neoplastic, or cancerous solid tumors. Solid tumor cancers include, but are not limited to, sarcomas, carcinomas, and lymphomas, such as cancers of the lung, breast, prostate, colon, rectum, and bladder. The tissue structure of solid tumors includes interdependent tissue compartments including the parenchyma (cancer cells) and the supporting stromal cells in which the cancer cells are dispersed and which may provide a supporting microenvironment.

The term "liquid tumor" refers to an abnormal mass of cells that is fluid in nature. Liquid tumor cancers include, but are not limited to, leukemias, myelomas, and lymphomas, as well as other hematological malignancies. TILs obtained from liquid tumors may also be referred to herein as marrow infiltrating lymphocytes (MILs).

The term "microenvironment," as used herein, may refer to the solid or hematological tumor microenvironment as a whole or to an individual subset of cells within the microenvironment. The tumor microenvironment, as used herein, refers to a complex mixture of "cells, soluble factors, signaling molecules, extracellular matrices, and mechanical cues that promote neoplastic transformation, support tumor growth and invasion, protect the tumor from host immunity, foster therapeutic resistance, and provide niches for dominant metastases to thrive," as described in Swartz, et al., Cancer Res., 2012, 72, 2473. Although tumors express antigens that should be recognized by T cells, tumor clearance by the immune system is rare because of immune suppression by the microenvironment.

Disclosed herein but not claimed is a method of treating a cancer with a population of TILs, wherein a patient is pre-treated with non-myeloablative chemotherapy prior to an infusion of TILs according to the invention. In some embodiments, the population of TILs may be provided wherein a patient is pre-treated with nonmyeloablative chemotherapy prior to an infusion of TILs according to the present invention. In an embodiment, the non-myeloablative chemotherapy is cyclophosphamide 60 mg/kg/d for 2 days (days 27 and 26 prior to TIL infusion) and fludarabine 25 mg/m2/d for 5 days (days 27 to 23 prior to TIL infusion). Disclosed herein but not claimed is a method wherein, after non-myeloablative chemotherapy and TIL infusion (at day 0) according to the invention, the patient receives an intravenous infusion of IL-2 intravenously at 720,000 IU/kg every 8 hours to physiologic tolerance.

Experimental findings indicate that lymphodepletion prior to adoptive transfer of tumor-specific T lymphocytes plays a key role in enhancing treatment efficacy by eliminating regulatory T cells and competing elements of the immune system ("cytokine sinks"). Disclosed herein but not claimed are methods utilizing a lymphodepletion step (sometimes also referred to as "immunosuppressive conditioning") on the patient prior to the introduction of the rTILs of the invention

The terms "sequence identity," "percent identity," and "sequence percent identity" in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. Suitable programs to determine percent sequence identity include for example the BLAST suite of programs available from the U.S. Government's National Center for Biotechnology Information BLAST web site. Comparisons between two sequences can be carried using either the BLASTN or BLASTP algorithm. BLASTN is used to compare nucleic acid sequences, while BLASTP is used to compare amino acid sequences. ALIGN, ALIGN-2 (Genentech, South San Francisco, California) or MegAlign, available from DNASTAR, are additional publicly available software programs that can be used to align sequences. One skilled in the art can determine appropriate parameters for maximal alignment by particular alignment software. In certain embodiments, the default parameters of the alignment software are used.

Certain embodiments of the present invention comprise a variant of an antibody, *e.g.*, an anti-IL-33 or anti-ST2 antibody and/or an anti-CD20 antibody and/or an anti-PD-1 antibody, anti-PD-L1 and/or an anti-PD-L2 antibody. As used herein, the term "variant" encompasses but is not limited to antibodies which comprise an amino acid sequence which differs from the amino acid sequence of a reference antibody by way of one or more substitutions, deletions and/or additions at certain positions within or adjacent to the amino acid sequence of the reference antibody. The variant may comprise one or more conservative substitutions in its amino acid sequence as compared to the amino acid sequence of a reference antibody. Conservative substitutions may involve, *e.g.*, the substitution of similarly charged or uncharged amino acids. The variant retains the ability to specifically bind to the antigen of the reference antibody.

.

Tumor Cryopreservation Methods

Disclosed herein but not claimed is a method for cryopreserving tumor tissue for the manufacture of tumor infiltrating lymphocytes (TILs) comprising:

(i) fragmenting the tumor tissue;

(ii) incubating the fragments in a cryopreservation medium; and,

(iii) freezing the fragments wherein the freezing is flash freezing using the vapor phase of liquid nitrogen.

In an embodiment, the tumor tissue is fragmented into approximately spherical fragments having a diameter of about 1.5 mm to about 6 mm. In a preferred embodiment, the approximately spherical fragments have a diameter of about 6 mm. In an embodiment, the approximately spherical fragments have a diameter of about 3 mm.

In some embodiments, the tumor tissue is fragmented into generally rectangular fragments having a shortest edge length of at least 1.5 mm and a longest edge length of about 6 mm. In an embodiment, the tumor tissue is fragmented into generally cubical fragments having edge lengths of between about 1.5 mm and 6 mm. In an embodiment, the generally cubical fragments have edge lengths of about 6 mm. In an embodiment, the generally cubical fragments have edge lengths of about 3 mm.

In some embodiments, the tissue sample is trimmed to separate non-tumor tissue from tumor tissue.

In some embodiments, the tumor tissue is from a dissected tumor. In an embodiment, the tumor tissue is from a tumor biopsy. In some embodiments the tumor tissue is from an incisional biopsy. In some embodiments the tumor tissue is from an excisional biopsy. In some embodiments the tumor tissues may be from one or more core needle biopsies.

In some embodiments, the fresh tumor tissue is trimmed into fragments with a cross section of about 1.5 mm × 1.5 mm, about 2 mm × 2 mm, about 2.5 mm × 2.5 mm, about 3 mm × 3 mm, about 3.5 mm × 3.5 mm, about 4 mm × 4 mm, about 4.5 mm × 4.5 mm, about 5 mm × 5 mm, about 5.5 mm × about 5.5 mm, or about 6 mm × about 6 mm.

In an embodiment, the tumor tissue is less than twelve hours old. In an embodiment, the tumor tissue is less than eight hours old. In an embodiment, the tumor tissue is less than three, less than two, or less than one-hour old.

In some embodiments, the cryopreservation medium comprises 2 to 12 % v/v (volume:volume) dimethylsulfoxide (DMSO). In some embodiments the cryopreservation medium comprises 5% v/v DMSO. In some embodiments the cryopreservation medium comprises 10% v/v DMSO. In some embodiments, the cryopreservation medium comprises between 5 and 10 % v/v DMSO. In some embodiments the cryopreservation medium comprises a percentage of DMSO selected from the group consisting of 1% v/v, 2% v/v, 3% v/v , 4% v/v , 5% v/v , 6% v/v, 7% v/v, 8% v/v, 9% v/v, 10% v/v, 11% v/v, 12% v/v, 13% v/v, 14% v/v, and 15% v/v. In some of the foregoing embodiments, the remainder of the cryopreservation medium is an aqueous medium.

In some embodiments, the cryopreservation medium comprises 2 to 12 % w/w (weight:weight) dimethylsulfoxide (DMSO). In some embodiments the cryopreservation medium comprises 5% w/w DMSO. In some embodiments the cryopreservation medium comprises 10% w/w DMSO. In some embodiments, the cryopreservation medium comprises between 5 and 10 % w/w DMSO. In some embodiments the cryopreservation medium comprises a percentage of DMSO selected from the group consisting of 1% w/w, 2% w/w, 3% w/w , 4% w/w , 5% w/w , 6% w/w, 7% w/w, 8% w/w, 9% w/w, 10% w/w, 11% w/w, 12% w/w, 13% w/w, 14% w/w, and 15% w/w. In some of the foregoing embodiments, the remainder of the cryopreservation medium is an aqueous medium.

In an embodiment, the cryopreservation medium comprises at least one antimicrobial agent. In an embodiment, the at least one antimicrobial agent is gentamicin at a concentration of at least 50 μg/mL. In some embodiments, the at least one antimicriobial agent is penicillin; in some embodiments the at least one antimicriobial agent is streptomycin. In some embodiments the at least one antimicrobial agent is an antifungal agent. In some embodiments the antifungal agent is amphotericin B; in some embodiments the antifungal agent is Fungin™. In some embodiments combinations of antimicriobial agents are used. In some embodiments at least one antifungal agent is used in combination with one or more antibacterial agents.

. In some embodiments, the tumor fragments are incubated in cryopreservation medium for about 30 minutes to about 60 minutes. In some embodiments the incubation is at least 30 minutes; at least 35 minutes; at least 40 minutes; at least 45 minutes; at least 50 minutes; at least 55 minutes; at least 60 minutes; or at least 70 minutes. In some embodiments the incubation is about 30 minutes; about 35 minutes; about 40 minutes; about 45 minutes; about 50 minutes; about 55 minutes; about 60 minutes; or about 70 minutes. In some embodiments the incubation is less than 30 minutes; less than 35 minutes; less than 40 minutes; less than 45 minutes; less than 50 minutes; less than 55 minutes; less than 60 minutes; or less than 70 minutes. In some embodiments the incubation time is proportional to tumor fragment density. In some embodiments the incubation time is proportional to tumor fragment surface to volume ratio.

In each embodiment, the tumor fragments are incubated in a cryopreservation medium at a temperature from about 2°C to about 8°C.

In some embodiments, the tumor tissue is washed in a physiologically buffered isotonic saline solution. In some embodiments, the washing comprises three serial washes of at least three minutes each, with the physiologically buffered isotonic saline solution replaced after each serial wash. In some embodiments, the physiologically buffered isotonic saline solution comprises Hank's Balance Salt Solution (HBSS). In some embodiments, the physiologically buffered isotonic saline solution comprises tris-buffered saline (TBS). In some embodiments, the physiologically buffered isotonic saline solution comprises phosphate buffered saline (PBS). In some embodiments, the physiologically buffered isotonic saline solution comprises Dulbecco's phosphate-buffered saline (DPBS). In some embodiments, the physiologically buffered isotonic saline solution in one serial wash may be a different physiologically buffered isotonic saline solution than used in one or more of the other serial washes.

In an embodiment, the freezing takes place at a temperature in the range from about -125°C to about -196°C. In an embodiment, the freezing takes place at a temperature in the range of about -140°C to about -185°C. In an embodiment, the freezing takes place at a temperature in the range of about -140°C to about -175°C. In an embodiment, the freezing takes place at a temperature of about -145°C. In some embodiments, the freezing takes place in the vapor phase of liquid nitrogen.

A problem well-known in the art is to cryopreserve cells or tissues without damaging them during the freezing process. Without being bound by theory, one source of damage during freezing is intracellular ice nucleation resulting in cellular rupture. Muldrew and McGann outline a quantitative theory for this well known and widely recognized difficulty with cellular and, in particular, whole tissue cryopreservation in "The osmotic rupture hypothesis of intracellular freezing injury", Biophysical Journal, 66:532-41 (1994). Acker and McGann further develop the fundamental mechanisms of intracellular ice formation and cellular damage in a later article, "Membrane damage occurs during the formation of intracellular ice," Cryo Letter, 22:241-54 (2001).

Damage during freezing is detected wherein upon thawing, the tissues have substantially lost their physiological structures or the cells comprising the tissue have substantially lost their viability. Viability may be determined by the fraction of cells introduced into a culture medium compared to the number of such cells that grow or show markers of normal cellular function. Numerous methods are known in the art to identify the fraction of viable cells, for example, and without limitation, dye exclusion tests, such as trypan blue exclusion. *See,* for example, Strober, Curr. Protoc. Immunol., 2001, Appendix 3B, available at https://dx.doi.org/10.1002/0471142735.ima03bs21. Without limitation, viability may also be determined by metabolic activity assays, such as the MTT assay, wherein MTT, 3-(4,5-dimethylthiazol-2-yl)-2,5-diphenyltetrazolium bromide, is metabolized by cellular enzymes into formazan. This enzymatic reaction converts the yellow MTT into purple formazan. *See,* for example, Berridge et al., Tetrazolium dyes as tools in cell biology: new insights into their cellular reduction. Biotechnology Annual Review, 11: 127-152 (2005); Mosmann, "Rapid colorimetric assay for cellular growth and survival: application to proliferation and cytotoxicity assays," J. Immunol. Methods 65 (1-2): 55-63 (1983).

Without being bound by theory, slow cooling is hypothesized to produce innocuous intracellular ice, *see* Acker and McGann, "Protective effect of intracellular ice during freezing?" Cryobiology, 46(2): 197-202 (2003). A conventional approach to achieve freezing without excessive cellular damage or to achieve freezing without significantly reducing cellular viability has been to employ slow freezing rates. Watson et al., U.S. Patent 5,891,617, emphasize this approach, for example claim one, step (c), teaches a very slow rate of cooling: "about -0.3° C. per minute or less." Similarly, Comhaire et al., U.S. Patent 9,938,495, teach "high cell viability after thawing were obtained by slow freezing using a DMSO-free cryopreservation medium" for stem cells.

Based on these exemplary teachings, the methods and products of the present disclosure are surprising and unexpected. Further the present disclosure, including the examples and the data therein, demonstrate a technical solution to the problem of rapidly and efficiently cryoprotecting tumor tissues, tumor fragments, or tumor specimens, for the use in the manufacture of tumor infiltrating lymphocytes for therapeutic use.

In some embodiments, the method of cryopreserving tumor tissue for the manufacture of tumor infiltrating lymphocytes (TILs), further comprises a step (iv) storing the frozen fragments at a temperature below at least -130°C. In some embodiments, the frozen fragments are stored in the vapor phase of liquid nitrogen. In some embodiments, the frozen fragments are stored submerged in liquid nitrogen. In some embodiments, the cryopreserved fragments are stored for later manufacture of TILs for autologous therapeutic use.

Disclosed herein but not claimed is a method for expanding tumor infiltrating lymphocytes (TILs) from frozen tumor tissue comprising the steps of:

(a) storing the tumor tissue in a frozen state, the method of storing the tumor tissue comprising incubating the tumor tissue for a period of about 20 minutes to about 70 minutes in a storage medium at a temperature from about 2°C to about 8°C before freezing;

(b) thawing the tumor tissue;

(c) treating the tumor tissue in a gas permeable container with a first cell culture medium comprising interleukin 2 (IL-2) and optionally OKT-3 antibody to provide TILs;

(d) removing at least a plurality of the TILs; and

(e) expanding the TILs with a second cell culture medium comprising cell culture media, irradiated feeder cells, OKT-3 antibody, and IL-2 in a gas permeable container to provide an expanded number of TILs.

Disclosed herein but not claimed is a method for expanding tumor infiltrating lymphocytes (TILs) from frozen tumor tissue comprising the steps of:

(a) fragmenting a tumor sample;

(b) incubating the tumor fragments in a storage medium;

(c) storing the tumor fragments in a frozen state;

(d) thawing the tumor fragments;

(e) treating the tumor fragments in a gas permeable container with a first cell culture medium comprising interleukin 2 (IL-2) and optionally OKT-3 antibody to provide TILs;

(f) removing at least a plurality of the TILs; and

(g) expanding the TILs with a second cell culture medium comprising cell culture media, irradiated feeder cells, OKT-3 antibody, and IL-2 in a gas permeable container to provide an expanded number of TILs.

In such methods, the storage medium may comprise 2 to 12 % v/v (volume:volume or volume-to-volume) dimethylsulfoxide (DMSO). In such methods, the storage medium may comprise 5% v/v DMSO. In such methods, the storage medium may comprise 10% v/v DMSO. In such methods, the storage medium may comprise between 5 and 10 % v/v DMSO. In such methods the storage medium may comprise a percentage of DMSO selected from the group consisting of 1% v/v, 2% v/v, 3% v/v , 4% v/v , 5% v/v , 6% v/v, 7% v/v, 8% v/v, 9% v/v, 10% v/v, 11% v/v, 12% v/v, 13% v/v, 14% v/v, and 15% v/v. In such methods, the remainder of the storage medium may be an aqueous medium.

In such methods, the storage medium may comprise 2 to 12 % w/w (weight weight or weight-to-weight) dimethylsulfoxide (DMSO). In such methods, the storage medium may comprise 5% w/w DMSO. In such methods, the storage medium may comprise 10% w/w DMSO. In such methods, the storage medium may comprise between 5 and 10 % w/w DMSO. In such methods, the storage medium may comprise a percentage of DMSO selected from the group consisting of 1% w/w, 2% w/w, 3% w/w , 4% w/w , 5% w/w , 6% w/w, 7% w/w, 8% w/w, 9% w/w, 10% w/w, 11% w/w, 12% w/w, 13% w/w, 14% w/w, and 15% w/w. In such methods, the remainder of the storage medium may be an aqueous medium.

In such methods, the tumor tissue may be incubated for between about 20 minutes and about 70 minutes in a storage medium at a temperature from about 2°C to about 8°C before freezing. In such methods, the tumor tissue may be incubated for at least 30 minutes in a storage medium at a temperature from about 2°C to about 8°C before freezing. In such methods, the tumor tissue may be incubated for between 30 minutes and about 60 minutes in a storage medium at a temperature from about 2°C to about 8°C before freezing. In such methods, the tumor tissue may be incubated for about 15 minutes in a storage medium at a temperature in the range of about 2°C to about 8°C before freezing. In such methods, the tumor tissue may be incubated for about 30 minutes in a storage medium at a temperature in the range of about 2°C to about 8°C before freezing. In such methods, the tumor tissue may be incubated for about 45 minutes in a storage medium at a temperature in the range of about 2°C to about 8°C before freezing. In such methods, the tumor tissue may be incubated for about 60 minutes in a storage medium at a temperature in the range of about 2°C to about 8°C before freezing. In such methods, the tumor tissue may be incubated for about 75 minutes in a storage medium at a temperature in the range of about 2°C to about 8°C before freezing. In such methods, the tumor tissue may be incubated for about 60 minutes in a storage medium at a temperature in the range of about 2°C to about 8°C before freezing. In such methods, the tumor tissue may be incubated for 60 minutes in a storage medium at a temperature from about 2°C to about 8°C before freezing.

In such methods, the storage medium may comprise about 5% v/v DMSO and the tumor tissue may be incubated for about 60 minutes in a storage medium at a temperature from about 2°C to about 8°C before freezing. In such methods, the storage medium may comprise about 10% v/v DMSO and the tumor tissue may be incubated for at least 30 minutes to about 60 minutes in a storage medium at a temperature in the range of about 2°C to about 8°C before freezing. In such methods, the incubation time may be proportional to tumor fragment density. In such methods, the incubation time may be proportional to tumor fragment surface to volume ratio.

Cryopreserved Tumor Fragments

Disclosed herein but not claimed is a cryopreserved tumor fragment for the manufacture of tumor infiltrating lymphocytes (TILs), prepared by a process comprising the steps of:

(i) fragmenting a tumor sample;

(ii) incubating the fragments in a cryopreservation medium; and,

(iii) freezing the fragments wherein the freezing is flash freezing using the vapor phase of liquid nitrogen.

Disclosed herein but not claimed is a cryopreserved tumor fragment for the manufacture of tumor infiltrating lymphocytes (TILs), prepared by a process comprising the steps of:

(i) fragmenting a tumor sample;

(ii) incubating the fragments for about 20 minutes to about 70 minutes at a temperature in the range of about 2°C to about 8°C in a cryopreservation medium comprising 10% v/v DMSO; and,

(iii) freezing the fragments wherein the freezing is flash freezing using the vapor phase of liquid nitrogen.

In an embodiment, the tumor sample is fragmented into approximately spherical fragments having a diameter of about 1.5 mm to 6 mm. In an embodiment, the approximately spherical fragments have a diameter of about 6 mm. In an embodiment, the approximately spherical fragments have a diameter of about 3 mm. In an embodiment, the approximately spherical fragments have a diameter selected from the group consisting of about 20 mm, about 19 mm, about 18 mm, about 17 mm, about 16 mm, about 15 mm, about 14 mm, about 13 mm, about 12 mm, about 11 mm, about 10 mm, about 9 mm, about 8 mm, about 7 mm, about 6 mm, about 5 mm, about 4 mm, about 3 mm, about 2 mm, and about 1 mm.

In some embodiments, the tumor sample is fragmented into generally rectangular fragments having a shortest edge length of at least 1.5 mm and a longest edge of about 6 mm. In an embodiment, the tumor sample is fragmented into generally cubical fragments having edges with lengths of between about 1.5 mm and 6 mm. In an embodiment, the generally cubical fragments have edges with lengths of about 6 mm. In an embodiment, the generally cubical fragments have edges with lengths of about 3 mm. In an embodiment, the generally cubical fragments have an edge length selected from the group consisting of about 20 mm, about 19 mm, about 18 mm, about 17 mm, about 16 mm, about 15 mm, about 14 mm, about 13 mm, about 12 mm, about 11 mm, about 10 mm, about 9 mm, about 8 mm, about 7 mm, about 6 mm, about 5 mm, about 4 mm, about 3 mm, about 2 mm, and about 1 mm.

In an embodiment, the tumor sample is fragmented into approximately spherical fragments having a volume of about 2 mm$^3$ to about 200 mm$^3$. In an embodiment, the tumor sample is fragmented into approximately spherical fragments having a volume of about 5 mm$^3$ to about 150 mm$^3$. In an embodiment, the tumor sample is fragmented into approximately spherical fragments having a volume of about 25 mm$^3$ to about 150 mm$^3$. In an embodiment, the tumor sample is fragmented into approximately spherical fragments having a volume of about 50 mm$^3$ to about 150 mm$^3$. In an embodiment, the tumor sample is fragmented into approximately spherical fragments having a volume of about 100 mm$^3$ to about 125 mm$^3$. In an embodiment, the tumor sample is fragmented into approximately spherical fragments having a volume of about 50 mm$^3$ to about 75 mm$^3$. In an embodiment, the tumor sample is fragmented into approximately spherical fragments having a volume of about 75 mm$^3$ to about 100 mm$^3$. In an embodiment, the tumor sample is fragmented into approximately spherical fragments having a volume of about 200 mm$^3$ to about 1000 mm$^3$. In an embodiment, the tumor sample is fragmented into approximately spherical fragments having a volume of about 500 mm$^3$ to about 800 mm$^3$. In an embodiment, the approximately spherical fragments have a diameter selected from the group consisting of about 20 mm, about 19 mm, about 18 mm, about 17 mm, about 16 mm, about 15 mm, about 14 mm, about 13 mm, about 12 mm, about 11 mm, about 10 mm, about 9 mm, about 8 mm, about 7 mm, about 6 mm, about 5 mm, about 4 mm, about 3 mm, about 2 mm, and about 1 mm.

In some embodiments, the fresh tumor tissue is trimmed into fragments, wherein a fragment has a cross section of about 1.5 mm × 1.5 mm, about 2 mm × 2 mm, about 2.5 mm × 2.5 mm, about 3 mm × 3 mm, about 3.5 mm × 3.5 mm, about 4 mm × 4mm, about 4.5 mm × 4.5 mm, about 5 mm × 5 mm, about 5.5 mm × about 5.5 mm, about 6 mm × 6 mm, about 6.5 mm × 6.5 mm, about 7 mm × 7 mm, about 7.5 mm × 7.5 mm, about 8 mm × 8 mm, about 8.5 mm × 8.5 mm, about 9 mm × 9 mm, about 9.5 mm × 9.5 mm, about 10 mm × 10 mm, about 10.5 mm × 10.5 mm, about 11 mm × 11 mm, about 11.5 mm × 11.5 mm, about 12 mm × 12 mm.

In some embodiments, the fresh tumor tissue is trimmed into fragments, wherein a fragment has a cross-sectional area of about 2 mm$^2$ to about 3 mm$^2$, about 3 mm$^2$ to about 4 mm$^2$, about 4 mm$^2$ to about 5 mm$^2$, about 5 mm$^2$ to about 6 mm$^2$, about 6 mm$^2$ to about 7 mm$^2$, about 7 mm$^2$ to about 8 mm$^2$, about 8 mm$^2$ to about 9 mm$^2$, about 9 mm$^2$ to about 10 mm$^2$, about 10 mm$^2$ to about 11 mm$^2$, about 11 mm$^2$ to about 12 mm$^2$, about 12 mm$^2$ to about 20 mm$^2$, about 20 mm$^2$ to about 50 mm$^2$, about 50 mm$^2$ to about 100 mm$^2$, or about 100 mm$^2$ to about 500 mm$^2$. In some embodiments, the fresh tumor tissue is trimmed into fragments, wherein a fragment has a cross-sectional area of about 1.5 mm$^2$, about 2 mm$^2$, about 2.5 mm$^2$, about 3 mm$^2$, about 3.5 mm$^2$, about 4 mm$^2$, about 4.5 mm$^2$, about 5 mm$^2$, about 5.5 mm$^2$, about 6 mm$^2$, about 6.5 mm$^2$, about 7 mm$^2$, about 7.5 mm$^2$, about 8 mm$^2$, about 8.5 mm$^2$, about 9 mm$^2$, about 9.5 mm$^2$, about 10 mm$^2$, about 10.5 mm$^2$, about 11 mm$^2$, about 11.5 mm$^2$, about 12 mm$^2$, about 20 mm$^2$, about 25 mm$^2$, about 30 mm$^2$, about 40 mm$^2$, about 50 mm$^2$, about 100 mm$^2$, about 200 mm$^2$, about 300 mm$^2$, about 400 mm$^2$, about 500 mm$^2$, about 750 mm$^2$, or about 1000 mm$^2$.

In some embodiments, the fresh tumor tissue is trimmed into fragments, wherein a fragment has a volume of about 2 mm$^3$ to about 3 mm$^3$, about 3 mm$^3$ to about 4 mm$^3$, about 4 mm$^3$ to about 5 mm$^3$, about 5 mm$^3$ to about 6 mm$^3$, about 6 mm$^3$ to about 7 mm$^3$, about 7 mm$^3$ to about 8 mm$^3$, about 8 mm$^3$ to about 9 mm$^3$, about 9 mm$^3$ to about 10 mm$^3$, about 10 mm$^3$ to about 11 mm$^3$, about 11 mm$^3$ to about 12 mm$^3$, and about 12 mm$^3$ to about 20 mm$^3$. In some embodiments, the fresh tumor tissue is trimmed into fragments, wherein a fragment has a volume of about 1.5 mm$^3$, about 2 mm$^3$, about 2.5 mm$^3$, about 3 mm$^3$, about 3.5 mm$^3$, about 4 mm$^3$, about 4.5 mm$^3$, about 5 mm$^3$, about 5.5 mm$^3$, about 6 mm$^3$, about 6.5 mm$^3$, about 7 mm$^3$, about 7.5 mm$^3$, about 8 mm$^3$, about 8.5 mm$^3$, about 9 mm$^3$, about 9.5 mm$^3$, about 10 mm$^3$, about 10.5 mm$^3$, about 11 mm$^3$, about 11.5 mm$^3$, about 12 mm$^3$, about 20 mm$^3$, about 25 mm$^3$, about 30 mm$^3$, about 40 mm$^3$, about 50 mm$^3$, about 100 mm$^3$, about 200 mm$^3$, about 300 mm$^3$, about 400 mm$^3$, about 500 mm$^3$, about 750 mm$^3$, or about 1000 mm$^3$.

In some embodiments, the tumor sample is from a dissected tumor. In an embodiment, the tumor sample is from a tumor biopsy. In some embodiments the tumor sample is from an incisional biopsy. In some embodiments the tumor sample is from an excisional biopsy. In some embodiments the tumor samples may be from one or more core needle biopsies.

In an embodiment, the tumor sample is less than twelve hours old prior to freezing. In an embodiment, the tumor sample is less than eight hours old prior to freezing. In an embodiment, the tumor sample is less than three, less than two, or less than one-hour old prior to freezing.

In some embodiments the cryopreservation medium comprises 10% v/v DMSO. In some of the foregoing embodiments, the remainder of the cryopreservation medium is an aqueous medium.

In an embodiment, the cryopreservation medium comprises at least one antimicrobial agent. In an embodiment, the at least one antimicrobial agent is gentamicin at a concentration of at least 20 μg/mL. In some embodiments, the at least one antimicrobial agent is gentamicin at a concentration of at least 50 μg/mL. In some embodiments, the at least one antimicriobial agent is penicillin; in some embodiments the at least one antimicriobial agent is streptomycin. In some embodiments the at least one antimicrobial agent is an antifungal agent. In some embodiments the antifungal agent is amphotericin B; in some embodiments the antifungal agent is Fungin™. In some embodiments combinations of antimicriobial agents are used. In some embodiments at least one antifungal agent is used in combination with one or more antibacterial agents.

In some embodiments, the tumor fragments are incubated in cryopreservation medium for about 30 minutes to about 60 minutes. In some embodiments the incubation is at at least 30 minutes; about 35 minutes; about 40 minutes; about 45 minutes; about 50 minutes; about 55 minutes; or about 60 minutes. In some embodiments the incubation time is proportional to tumor fragment density. In some embodiments the incubation time is proportional to tumor fragment surface to volume ratio.

In each embodiment, the tumor fragments are incubated in cryopreservation medium at a temperature from about 2°C to about 8°C.

In some embodiments, the tumor sample is washed in a physiologically buffered isotonic saline solution. In some embodiments, the washing comprises three serial washes of at least three minutes each, with the physiologically buffered isotonic saline solution replaced after each serial wash. In some embodiments, the physiologically buffered isotonic saline solution comprises Hank's Balance Salt Solution (HBSS). In some embodiments, the physiologically buffered isotonic saline solution comprises tris-buffered saline (TBS). In some embodiments, the physiologically buffered isotonic saline solution comprises phosphate buffered saline (PBS). In some embodiments, the physiologically buffered isotonic saline solution comprises Dulbecco's phosphate-buffered saline (DPBS). In some embodiments, the physiologically buffered isotonic saline solution in one serial wash may be a different physiologically buffered isotonic saline solution than used in one or more of the other serial washes.

In an embodiment, the freezing takes place at a temperature in the range from about -125°C to about -180°C. In an embodiment, the freezing takes place at a temperature in the range of about -140°C to about -175°C. In an embodiment, the freezing takes place at a temperature of about -145°C. In some embodiments, the freezing takes place in the vapor phase of liquid nitrogen.

In an embodiment, the number of tumor fragments used according to any of the embodiments above or herein is one. In an embodiment, the number of tumor fragments used according to any of the embodiments above or herein is two. In an embodiment, the number of tumor fragments used according to any of the embodiments above or herein is three. In an embodiment, the number of tumor fragments used according to any of the embodiments above or herein is four. In an embodiment, the number of tumor fragments used according to any of the embodiments above or herein is five. In an embodiment, the number of tumor fragments used according to any of the embodiments above or herein is six. In an embodiment, the number of tumor fragments used according to any of the embodiments above or herein is seven. In an embodiment, the number of tumor fragments used according to any of the embodiments above or herein is eight. In an embodiment, the number of tumor fragments used according to any of the embodiments above or herein is nine. In an embodiment, the number of tumor fragments used according to any of the embodiments above or herein is ten.

Manufacturing TILs from Cryopreserved Tumor Fragments

Disclosed herein but not claimed is a method for expanding tumor infiltrating lymphocytes (TILs) from a cryopreserved tumor fragment into a therapeutic population of TILs, the method comprising the following steps:

(i) obtaining a first population of TILs from a cryopreserved tumor fragment;

(ii) performing a first expansion by culturing the first population of TILs in a cell culture medium comprising IL-2 and optionally OKT-3 to produce a second population of TILs; and

(iii) performing a second expansion by supplementing the cell culture medium of the second population of TILs with additional IL-2, OKT-3, and antigen presenting cells (APCs), to produce a third population of TILs, wherein the third population of TILs is at least 50-fold or 100-fold greater in number than the second population of TILs, and wherein the second expansion is performed for at least 14 days in order to obtain the third population of TILs, wherein the third population of TILs is a therapeutic population of TILs which comprises an increased subpopulation of effector T cells and/or central memory T cells relative to the second population of TILs.

The invention provides a method for preparing tumor infiltrating lymphocytes (TILs) for adoptive T-cell therapy, the method comprising:

(a) culturing a cryopreserved tumor fragment in a first cell culture medium comprising interleukin 2 (IL-2) and optionally OKT-3 antibody to provide TILs;

(b) expanding the TILs in a second cell culture medium comprising irradiated feeder cells, OKT-3 antibody, and IL-2 to provide an expanded number of TILs; and,

(c) optionally cryopreserving the expanded number of TILs.

Disclosed herein but not claimed is a method for expanding tumor infiltrating lymphocytes (TILs) from frozen tumor tissue comprising the steps of:

(a) storing the tumor tissue in a frozen state, the method of storing the tumor tissue comprising incubating the tumor tissue for about 30 minutes in a storage medium at a temperature in the range of about 2°C to about 8°C before freezing;

(b) thawing the tumor tissue;

(c) treating the tumor tissue in a gas permeable container with a first cell culture medium and interleukin 2 (IL-2) to provide TILs;

(d) removing at least a plurality of the TILs; and

(e) expanding the TILs with a second cell culture medium comprising cell culture media, irradiated feeder cells, OKT-3 antibody, and IL-2 in a gas permeable container to provide an expanded number of TILs.

Disclosed herein but not claimed is a method for expanding tumor infiltrating lymphocytes (TILs) from frozen tumor tissue comprising the steps of:

(a) fragmenting a tumor sample;

(b) incubating the tumor fragments in a storage medium for about 30 minutes at a temperature in the range of about 2°C to about 8°C;

(c) storing the tumor fragments in a frozen state;

(d) thawing the tumor fragments;

(e) treating the tumor fragments in a gas permeable container with a first cell culture medium and interleukin 2 (IL-2) to provide TILs;

(f) removing at least a plurality of the TILs; and

(g) expanding the TILs with a second cell culture medium comprising cell culture media, irradiated feeder cells, OKT-3 antibody, and IL-2 in a gas permeable container to provide an expanded number of TILs.

In some embodiments, the storage medium comprises 2% v/v DMSO to 12 % v/v dimethylsulfonoxide (DMSO). In some embodiments the storage medium comprises 5% v/v DMSO. In some embodiments the storage medium comprises 10% v/v DMSO. In some embodiments, the storage medium comprises between about 5% v/v DMSO and 10 % v/v DMSO.

In some embodiments, the tumor tissue is incubated for at least 30 minutes in a storage medium at a temperature from about 2°C to about 8°C before freezing. In some embodiments, the tumor tissue is incubated for between 30 minutes and about 60 minutes in a storage medium at a temperature in the range of about 2°C to about 8°C before freezing. In some embodiments, the tumor tissue is incubated for about 45 minutes in a storage medium at a temperature in the range of about 2°C to about 8°C before freezing. In some embodiments, the tumor tissue is incubated for about 60 minutes in a storage medium at a temperature in the range of about 2°C to about 8°C before freezing. In some embodiments, the tumor tissue is incubated for about 60 minutes in a storage medium at a temperature in the range of about 2°C to about 8°C before freezing.

In some embodiments, the tumor tissue is incubated in cryopreservation medium for about 20 minutes to about 70 minutes. In some embodiments, the tumor tissue is incubated in cryopreservation medium for about 30 minutes to about 60 minutes. In some embodiments the incubation is at least 30 minutes; about 35 minutes; about 40 minutes; about 45 minutes; about 50 minutes; about 55 minutes; or about 60 minutes. In some embodiments the incubation time is proportional to tumor fragment density. In some embodiments the incubation time is proportional to tumor fragment surface to volume ratio.

In some embodiments, a tumor fragment with a cross section of about 1.5 mm × 1.5 mm is incubated in cryopreservation medium for about 30 minutes, for about 35 minutes, for about 40 minutes, or for less than about 60 minutes. In some embodiments, a tumor fragment with a cross section of about 2 mm × 2 mm is incubated in cryopreservation medium for about 30 minutes, for about 35 minutes, for about 40 minutes, for about 45 minutes, for about 50 minutes, or for about 60 minutes. In some embodiments, a tumor fragment with a cross section of about 3 mm × 3 mm is incubated in cryopreservation medium for about 30 minutes, for about 35 minutes, for about 40 minutes, for about 45 minutes, for about 50 minutes, or for about 60 minutes. In some embodiments, a tumor fragment with a cross section of about 4 mm × 4 mm is incubated in cryopreservation medium for about 30 minutes, for about 35 minutes, for about 40 minutes, for about 45 minutes, for about 50 minutes, for about 60 minutes, or for about 70 minutes. In some embodiments, a tumor fragment with a cross section of about 5 mm × 5 mm is incubated in cryopreservation medium for about 30 minutes, for about 35 minutes, for about 40 minutes, for about 45 minutes, for about 50 minutes, for about 60 minutes, or for about 70 minutes. In some embodiments, a tumor fragment with a cross section of about 6 mm × 6 mm is incubated in cryopreservation medium for at least 20 minutes, for about 30 minutes, for about 40 minutes, for about 45 minutes, for about 50 minutes, for about 60 minutes, or for about 70 minutes.

In some embodiments, the storage medium comprises about 5% v/v DMSO and the tumor tissue is incubated for about 60 minutes in a storage medium at a temperature in the range from about 2°C to about 8°C before freezing. In some embodiments, the storage medium comprises about 10% v/v DMSO and the tumor tissue is incubated for at least about 30 minutes to about 60 minutes in a storage medium at a temperature in the range from about 2°C to about 8°C before freezing.

In some embodiments, a method for expanding TILs from frozen tumor tissue comprises incubating cryopreserved tumor fragments in a culture medium comprising IL-2, wherein the number of TILs are expanded. In some embodiments, the number of TILs is expanded 100-fold. In some embodiments, the number of TILs is expanded 150-fold. In some embodiments, the number of TILs is expanded 200-fold. In some embodiments, the number of TILs is expanded 250-fold. In some embodiments, the number of TILs is expanded about 300-fold. In some embodiments, the number of TILs is expanded about 350-fold. In some embodiments, the number of TILs is expanded about 400-fold. In some embodiments, the number of TILs is expanded about 450-fold. In some embodiments, the number of TILs is expanded about 500-fold. In some embodiments, the number of TILs is expanded about 550-fold. In some embodiments, the number of TILs is expanded about 600-fold. In some embodiments, the number of TILs is expanded about 700-fold. In some embodiments, the number of TILs is expanded about 750-fold. In some embodiments, the number of TILs is expanded about 800-fold. In some embodiments, the number of TILs is expanded about 850-fold. In some embodiments, the number of TILs is expanded about 900-fold. In some embodiments, the number of TILs is expanded about 1000-fold. In some embodiments, the number of TILs is expanded about 1200-fold. In some embodiments, the number of TILs is expanded about 1500-fold. In some embodiments, the number of TILs is expanded about 1600-fold. In some embodiments, the number of TILs is expanded about 2000-fold. In some embodiments, the number of TILs is expanded about 2100-fold. In some embodiments, the number of TILs is expanded about 2200-fold. In some embodiments, the number of TILs is expanded about 2500-fold. In some embodiments, the number of TILs is expanded greater than 2500-fold.

Disclosed herein but not claimed is a method for manufacturing TILs from frozen tumor tissue comprising:

> (a) storing the tumor tissue in a frozen state, the method of storing the tumor tissue comprising:

>> (i) trimming the tumor tissue to remove excess non-tumor tissue;

>> (ii) placing the tumor tissue in a closable vessel containing a storage medium;

>> (iii) incubating the vessel, which contains the tumor tissue and the storage medium, at a temperature in the range of about 2°C to about 8°C for about 20 minutes to about 70 minutes;

>> (iv) freezing the vessel; and

>> (v) storing the vessel such that the vessel remains frozen;

> (b) thawing the vessel;

> (c) treating the tumor tissue in a gas permeable container with a first cell culture medium and interleukin 2 (IL-2) to provide TILs;

> (d) removing at least a plurality of the TILs; and

> (e) expanding the TILs with a second cell culture medium comprising cell culture media, irradiated feeder cells, OKT-3, and IL-2 in a gas permeable container to provide an expanded number of TILs.

Disclosed herein but not claimed is a method for manufacturing TILs from frozen tumor tissue comprising:

> (a) obtaining tumor tissue from a patient;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range of about 2°C to about 8°C for about 20 minutes to about 70 minutes;

(e) freezing the vessel;

(f) storing the vessel such that the vessel remains frozen;

(g) thawing the vessel;

(h) treating the tumor tissue in a gas permeable container with a first cell culture medium and interleukin 2 (IL-2) to provide TILs;

(i) removing at least a plurality of the TILs; and

(j) expanding the TILs with a second cell culture medium comprising cell culture media, irradiated feeder cells, OKT-3, and IL-2 in a gas permeable container to provide an expanded number of TILs.

In some embodiments, the method of manufacturing TILs from a cryopreserved tumor fragment, further comprises addition to OKT-3 to the first culture medium.

In some embodiments, the fresh tumor tissue is trimmed to between 1.5 mm and 6 mm in diameter and between 1.5 mm and 6 mm in thickness. In yet other embodiments, the fresh tumor tissue is trimmed to about 6 mm × 6 mm × 6 mm. In some embodiments, the fresh tumor tissue is trimmed into fragments with a cross section of about 1.5 mm × 1.5 mm, about 2 mm × 2 mm, about 2.5 mm × 2.5 mm, about 3 mm × 3 mm, about 3.5 mm × 3.5 mm, about 4 mm × 4 mm, about 4.5 mm × 4.5 mm, about 5 mm × 5 mm, about 5.5 mm × about 5.5 mm, or about 6 mm × 6 mm.

In some embodiments, the storage medium further comprises an antibacterial agent. In some embodiments the antibacterial agent is gentamicin. In some embodiments the storage medium comprises gentamicin at a concentration of at least 20 μg/mL. In some embodiments the storage medium comprises gentamicin at a concentration of at least 50 μg/mL. In some embodiments the storage medium comprises an antibacterial agent, an antifungal agent, and combinations thereof. In some embodiments, the antifungal agent is amphotericin B and is present from about 0.25 μg/mL to about 2.5 μg/mL. In some embodiments, the antifungal agent is Fungin™ and is present from about 5 μg/mL to about 50 μg/mL.

In some embodiments, the tumor tissue is washed in a physiologically buffered isotonic saline solution. In some embodiments, the washing comprises three serial washes of at least three minutes each, with the physiologically buffered isotonic saline solution replaced after each serial wash. In some embodiments, the physiologically buffered isotonic saline solution comprises Hank's Balance Salt Solution (HBSS). In some embodiments, the physiologically buffered isotonic saline solution comprises tris-buffered saline (TBS). In some embodiments, the physiologically buffered isotonic saline solution comprises phosphate buffered saline (PBS). In some embodiments, the physiologically buffered isotonic saline solution comprises Dulbecco's phosphate-buffered saline (DPBS). In some embodiments, the physiologically buffered isotonic saline solution in one serial wash may be a different physiologically buffered isotonic saline solution than used in one or more of the other serial washes.

In some embodiments the thawing step comprises immersing the vessel in a 37°C water bath for about 5 minutes.

In some embodiments, the freezing step comprises freezing the vessel at about -125°C to about -195°C. In some embodiments, the vessel is frozen at a temperature from about -125°C to about -150°C; in some embodiments the vessel is frozen at a temperature from about -125°C to about -145°C; in yet further embodiments, the vessel is frozen at a temperature from about - 135°C.

In some embodiments, the method is performed with a fresh tumor sample from a human subject.

In some embodiments, the tumor tissue is selected from the group consisting of melanoma tumor tissue, head and neck tumor tissue, breast tumor tissue, renal tumor tissue, pancreatic tumor tissue, glioblastoma tumor tissue, lung tumor tissue, colorectal tumor tissue, sarcoma tumor tissue, triple negative breast tumor tissue, cervical tumor tissue, endometrial tumor tissue, thyroid tumor tissue, ovarian tumor tissue, head and neck squamous cell carcinoma (HNSCC) tissue, and HPV-positive tumor tissue.

In some embodiments, the method further comprises a split between the first expansion (or first culture) step and the second expansion (or second culture) step. In an embodiment, the split occurs on about day 16. In some embodiments, the first culture step is complete in about 11 days. In other embodiments, the first expansion (or first culture) step is complete in about 7 days. In yet other embodiments, the first expansion (or first culture) step is complete in about 7 days and the second expansion (or second culture) step is complete in about 14 days. In some embodiments, steps (c) through (e) are complete in about 22 days. In yet other embodiments, steps (c) through (e) or steps (h) through (j), as applicable, are complete in about 21 days; in some embodiments steps (c) through (e) or steps (h) through (j), as applicable, are complete in about 20 days; and in some embodiments steps (c) through (e) or steps (h) through (j), as applicable, are complete in about 16 days.

In some embodiments, the first culture medium comprises OKT-3.

Disclosed herein but not claimed are methods of treating cancer for a human subject in need thereof, such methods comprising:

> (a) storing the tumor tissue in a frozen state, the method of storing the tumor tissue comprising:
>
>> (i) trimming the tumor tissue to remove excess non-tumor tissue;
>>
>> (ii) placing the tumor tissue in a closable vessel containing a storage medium;
>>
>> (iii) incubating the vessel, which contains the tumor tissue and the storage medium, at a temperature in the range from about 2°C to about 8°C for about 30 minutes;
>>
>> (iv) freezing the vessel using the vapor phase of liquid nitrogen; and
>>
>> (v) storing the vessel at the vapor phase of liquid nitrogen temperature;
>
> (b) thawing the tumor tissue;
>
> (c) adding the tumor tissue into a closed system;
>
> (d) performing a first expansion by culturing a first population of TILs from the tumor tissue in a cell culture medium comprising IL-2 to produce a second population of TILs, wherein the first expansion is performed in a closed container providing a first gas-permeable surface area, wherein the first expansion is performed for about 3-14 days to obtain the second population of TILs, wherein the second population of TILs is at least 50-fold greater in number than the first population of TILs, and wherein the transition from step (c) to step (d) occurs without opening the system;
>
> (e) performing a second expansion by supplementing the cell culture medium of the second population of TILs with additional IL-2, OKT-3, and antigen presenting cells (APCs), to produce a third population of TILs, wherein the second expansion is performed for about 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (d) to step (e) occurs without opening the system;
>
> (f) harvesting the therapeutic population of TILs obtained from step (f), wherein the transition from step (e) to step (f) occurs without opening the system;
>
> (g) transferring the harvested TIL population from step (f) to an infusion bag, wherein the transfer from step (f) to (g) occurs without opening the system;
>
> (h) cryopreserving the infusion bag comprising the harvested TIL population from step (g) using a cryopreservation process; and
>
> (i) administering a therapeutically effective dosage of the third population of TILs from the infusion bag in step (g) to the subject.

Disclosed herein but not claimed are methods of treating cancer for a human subject in need thereof are provided, such methods comprising administering expanded tumor infiltrating lymphocytes (TILs) comprising:

> (a) obtaining tumor tissue from the subject;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range from about 2°C to about 8°C for about 30 minutes;

(e) freezing the vessel using the vapor phase of liquid nitrogen;

(f) storing the vessel at the vapor phase of liquid nitrogen temperature;

(g) thawing the tumor tissue;

(h) adding the tumor tissue into a closed system;

(i) performing in the closed system a first expansion by culturing a first population of TILs from the tumor tissue in a cell culture medium comprising IL-2 to produce a second population of TILs, wherein the first expansion is performed in a closed container providing a first gas-permeable surface area, wherein the first expansion is performed for about 3-14 days to obtain the second population of TILs, wherein the second population of TILs is at least 50-fold greater in number than the first population of TILs, and wherein the transition from step (h) to step (i) occurs without opening the system;

(j) performing a second expansion by supplementing the cell culture medium of the second population of TILs with additional IL-2, OKT-3, and antigen presenting cells (APCs), to produce a third population of TILs, wherein the second expansion is performed for about 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (i) to step (j) occurs without opening the system;

(k) harvesting the therapeutic population of TILs obtained from step (j), wherein the transition from step (j) to step (k) occurs without opening the system;

(l) transferring the therapeutic of TILs harvested in step (k) to an infusion bag, wherein the transfer from step (k) to (1) occurs without opening the system;

(m) cryopreserving the infusion bag comprising the therapeutic population of TILs from step (1) using a cryopreservation process;

(n) thawing the therapeutic population of TILs in the infusion bag from step (m); and

(o) administering a therapeutically effective dosage of the therapeutic population of TILs from the infusion bag in step (n) to the subject.

In some embodiments, the cancer is selected from cervical cancer, head and neck cancer (including, for example, head and neck squamous cell carcinoma (HNSCC)), glioblastoma, endometrial cancer, thyroid cancer, colorectal cancer, ovarian cancer, sarcoma, pancreatic cancer, bladder cancer, breast cancer, triple negative breast cancer, melanoma, refractory melanoma, metastatic melanoma, and non-small cell lung carcinoma. The tissue structure of solid tumors includes interdependent tissue compartments including the parenchyma (cancer cells) and the supporting stromal cells in which the cancer cells are dispersed and which may provide a supporting microenvironment.

Disclosed herein but not claimed is a method for expanding tumor infiltrating lymphocytes (TILs) from frozen tumor tissue comprising:

(a) obtaining tumor tissue from a patient, wherein the tumor tissue comprises TILs;

(b) storing the tumor tissue in a frozen state, the method of storing the tumor tissue comprising incubating the tumor tissue for 30 minutes in a storage medium at 2°C - 8°C before freezing;

(c) thawing the tumor tissue;

(d) treating the tumor tissue in a gas permeable container with a first cell culture medium and interleukin 2 (IL-2) to provide TILs;

(e) removing at least a plurality of the TILs; and

(f) expanding the TILs with a second cell culture medium comprising cell culture media, irradiated feeder cells, OKT-3 antibody, and IL-2 in a gas permeable container to provide an expanded number of TILs.

Disclosed herein but not claimed is a method for expanding tumor infiltrating lymphocytes (TILs) from frozen tumor tissue comprising:

(a) obtaining tumor tissue from a patient, wherein the tumor tissue comprises TILs;

(b) incubating the tumor tissue for 30 minutes in a storage medium at 2°C - 8°C;

(c) storing the tumor tissue in a frozen state;

(d) thawing the tumor tissue;

(e) treating the tumor tissue in a gas permeable container with a first cell culture medium and interleukin 2 (IL-2) to provide TILs;

(f) removing at least a plurality of the TILs; and

(g) expanding the TILs with a second cell culture medium comprising cell culture media, irradiated feeder cells, OKT-3 antibody, and IL-2 in a gas permeable container to provide an expanded number of TILs.

Disclosed herein but not claimed is a method for expanding TILs from frozen tumor tissue comprising:

(a) obtaining tumor tissue from a patient, wherein the tumor tissue comprises TILs;

(b) storing the tumor tissue in a frozen state, the method of storing the tumor tissue comprising:

(i) trimming the tumor tissue to remove excess non-tumor tissue;

(ii) placing the tumor tissue in a closable vessel containing a storage medium;

(iii) incubating the vessel, which contains the tumor tissue and the storage medium, at a temperature in the range from about 2°C to about 8°C for about 30 minutes;

(iv) freezing the vessel; and

(v) storing the vessel such that the vessel remains frozen;

(c) thawing the vessel;

(d) treating the tumor tissue in a gas permeable container with a first cell culture medium and interleukin 2 (IL-2) to provide TILs;

(e) removing at least a plurality of the TILs; and

(f) expanding the TILs with a second cell culture medium comprising cell culture media, irradiated feeder cells, OKT-3, and IL-2 in a gas permeable container to provide an expanded number of TILs.

Disclosed herein but not claimed is a method for expanding TILs from frozen tumor tissue comprising:

(a) obtaining tumor tissue from a patient, wherein the tumor tissue comprises TILs;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range from about 2°C to about 8°C for about 30 minutes;

(e) freezing the vessel;

(f) storing the vessel such that the vessel remains frozen;

(g) thawing the vessel;

(h) treating the tumor tissue in a gas permeable container with a first cell culture medium and interleukin 2 (IL-2) to provide TILs;

(i) removing at least a plurality of the TILs; and

(j) expanding the TILs with a second cell culture medium comprising cell culture media, irradiated feeder cells, OKT-3, and IL-2 in a gas permeable container to provide an expanded number of TILs.

In some embodiments, the tumor tissue is washed in a physiologically buffered isotonic saline solution. In some embodiments, the washing comprises three serial washes of at least three minutes each, with the physiologically buffered isotonic saline solution replaced after each serial wash. In some embodiments, the physiologically buffered isotonic saline solution comprises Hank's Balance Salt Solution (HBSS). In some embodiments, the physiologically buffered isotonic saline solution comprises tris-buffered saline (TBS). In some embodiments, the physiologically buffered isotonic saline solution comprises phosphate buffered saline (PBS). In some embodiments, the physiologically buffered isotonic saline solution comprises Dulbecco's phosphate-buffered saline (DPBS). In some embodiments, the physiologically buffered isotonic saline solution in one serial wash may be a different physiologically buffered isotonic saline solution than used in one or more of the other serial washes.

In some embodiments the thawing step comprises immersing the vessel in a 37°C water bath for about 5 minutes.

In some embodiments, the freezing step comprises freezing the vessel at about -125°C to about -195°C. In some embodiments, the vessel is frozen at a temperature from about -125°C to about -150°C; in some embodiments the vessel is frozen at a temperature from about -125°C to about -145°C; in yet further embodiments, the vessel is frozen at a temperature of about - 135°C.

In some embodiments, the tumor tissue is selected from the group consisting of melanoma tumor tissue, head and neck tumor tissue, breast tumor tissue, renal tumor tissue, pancreatic tumor tissue, glioblastoma tumor tissue, lung tumor tissue, colorectal tumor tissue, sarcoma tumor tissue, triple negative breast tumor tissue, cervical tumor tissue, endometrial tumor tissue, thyroid tumor tissue, ovarian tumor tissue, head and neck squamous cell carcinoma (HNSCC) tissue, and HPV-positive tumor tissue.

In some embodiments, the method further comprises a split between the first expansion (or first culture) step and the second expansion (or second culture) step. In an embodiment, the split occurs on about day 16. In some embodiments, the first culture step is complete in about 11 days. In other embodiments, the first expansion (or first culture) step is complete in about 7 days. In yet other embodiments, the first expansion (or first culture) step is complete in about 7 days and the second expansion (or second culture) step is complete in about 14 days. In some embodiments, steps (d) through (f) or steps (h) through (j), as applicable, are complete in about 22 days. In yet other embodiments, steps (d) through (f) or steps (h) through (j), as applicable, are complete in about 21 days; in some embodiments steps (d) through (f) or steps (h) through (j), as applicable, are complete in about 20 days; and in some embodiments steps (d) through (f) or steps (h) through (j), as applicable, are complete in about 16 days.

Disclosed herein but not claimed are methods of treating cancer for a human subject in need thereof, such methods comprising:

(a) obtaining tumor tissue from the subject, wherein the tumor tissue comprises TILs;

(b) storing the tumor tissue in a frozen state, the method of storing the tumor tissue comprising:

(i) trimming the tumor tissue to remove excess non-tumor tissue;

(ii) placing the tumor tissue in a closable vessel containing a storage medium;

(iii) incubating the vessel, which contains the tumor tissue and the storage medium, at a temperature in the range of about 2°C to about 8°C for about 20 to about 70 minutes;

(iv) freezing the vessel using the vapor phase of liquid nitrogen; and

(v) storing the vessel at the vapor phase of liquid nitrogen temperature;

(c) thawing the tumor tissue;

(d) adding the tumor tissue into a closed system;

(e) performing a first expansion by culturing a first population of TILs from the tumor tissue in a cell culture medium comprising IL-2 to produce a second population of TILs, wherein the first expansion is performed in a closed container providing a first gas-permeable surface area, wherein the first expansion is performed for about 3-14 days to obtain the second population of TILs, wherein the second population of TILs is at least 50-fold greater in number than the first population of TILs, and wherein the transition from step (d) to step (e) occurs without opening the system;

(f) performing a second expansion by supplementing the cell culture medium of the second population of TILs with additional IL-2, OKT-3, and antigen presenting cells (APCs), to produce a third population of TILs, wherein the second expansion is performed for about 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (e) to step (f) occurs without opening the system;

(g) harvesting the therapeutic population of TILs obtained from step (f), wherein the transition from step (f) to step (g) occurs without opening the system;

(h) transferring the harvested TIL population from step (g) to an infusion bag, wherein the transfer from step (g) to (h) occurs without opening the system;

(i) cryopreserving the infusion bag comprising the harvested TIL population from step (h) using a cryopreservation process; and

(j) administering a therapeutically effective dosage of the third population of TILs from the infusion bag in step (i) to the subject.

Disclosed herein but not claimed are methods of treating cancer for a human subject in need thereof, such methods comprising:

(a) obtaining tumor tissue from the subject, wherein the tumor tissue comprises TILs;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range of about 2°C to about 8°C for about 20 to about 70 minutes;

(e) freezing the vessel using the vapor phase of liquid nitrogen;

(f) storing the vessel at the vapor phase of liquid nitrogen temperature;

(g) thawing the tumor tissue;

(h) adding the tumor tissue into a closed system;

(i) performing in the closed system a first expansion by culturing a first population of TILs from the tumor tissue in a cell culture medium comprising IL-2 to produce a second population of TILs, wherein the first expansion is performed in a closed container providing a first gas-permeable surface area, wherein the first expansion is performed for about 3-14 days to obtain the second population of TILs, wherein the second population of TILs is at least 50-fold greater in number than the first population of TILs, and wherein the transition from step (h) to step (i) occurs without opening the system;

(j) performing a second expansion by supplementing the cell culture medium of the second population of TILs with additional IL-2, OKT-3, and antigen presenting cells (APCs), to produce a third population of TILs, wherein the second expansion is performed for about 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (i) to step (j) occurs without opening the system;

(k) harvesting the therapeutic population of TILs obtained from step (j), wherein the transition from step (j) to step (k) occurs without opening the system;

(l) transferring the therapeutic population of TILs harvested in step (k) to an infusion bag, wherein the transfer from step (k) to (1) occurs without opening the system;

(m) cryopreserving the infusion bag comprising the therapeutic population of TILs from step (1) using a cryopreservation process;

(o) thawing the therapeutic population of TILs in the infusion bag from step (m); and

(p) administering a therapeutically effective dosage of the therapeutic population of TILs from the infusion bag in step (o) to the subject.

Disclosed herein but not claimed is a method for expanding TILs from frozen tumor tissue comprising:

(a) storing the tumor tissue in a frozen state, the method of storing the tumor tissue comprising:

(i) trimming the tumor tissue to remove excess non-tumor tissue;

(ii) placing the tumor tissue in a closable vessel containing a storage medium;

(iii)incubating the vessel, which contains the tumor tissue and the storage medium, at a temperature in the range from about 2°C to about 8°C for about 30 minutes;

(iv) freezing the vessel; and

(v) storing the vessel such that the vessel remains frozen;

(b) thawing the vessel;

(c) treating the tumor tissue in a gas permeable container with a first cell culture medium and interleukin 2 (IL-2) to provide TILs;

(d) removing at least a plurality of the TILs; and(e) expanding the TILs with a second cell culture medium comprising cell culture media, irradiated feeder cells, OKT-3, and IL-2 in a gas permeable container to provide an expanded number of TILs.

Disclosed herein but not claimed is a method for expanding TILs from frozen tumor tissue comprising:

(a) obtaining tumor tissue from a patient;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range from about 2°C to about 8°C for about 30 minutes;

(e) freezing the vessel;

(f) storing the vessel such that the vessel remains frozen;

(g) thawing the vessel;

(h) treating the tumor tissue in a gas permeable container with a first cell culture medium and interleukin 2 (IL-2) to provide TILs;

(i) removing at least a plurality of the TILs; and

(j) expanding the TILs with a second cell culture medium comprising cell culture media, irradiated feeder cells, OKT-3, and IL-2 in a gas permeable container to provide an expanded number of TILs.

In some embodiments, the fresh tumor tissue is trimmed to between 1.5 mm and 6 mm in diameter and between about 1.5 mm and about 6 mm in thickness. In yet other embodiments, the fresh tumor tissue is trimmed to about 6 mm $\times$ 6 mm $\times$ 6 mm.

In some embodiments, the storage medium further comprises an antibacterial agent. In some embodiments the antibacterial agent is gentamicin. In some embodiments the storage medium comprises gentamicin at a concentration of at least 20 $\mu$g/mL. In some embodiments the storage medium comprises gentamicin at a concentration of at least 50 $\mu$g/mL. In some embodiments the storage medium comprises an antibacterial agent, an antifungal agent, and combinations thereof. In some embodiments, the antifungal agent is amphotericin B and is present from about 0.25 $\mu$g/mL to about 2.5 $\mu$g/mL. In some embodiments, the antifungal agent is fungin and is present from about 5 $\mu$g/mL to about 50 $\mu$g/mL.

In some embodiments, the fresh tumor tissue is first washed in Hank's Balanced Salt Solution (HBSS) before performing the trimming step of the method. In some embodiments, the washing comprises at least three serial washes of at least three minutes each, wherein the HBSS is replaced after each wash.

In some embodiments, thawing step comprises immersing the vessel in a 37°C water bath for about 5 minutes.

In some embodiments, the freezing step comprises freezing the vessel at about -125°C to about -195°C. In some embodiments, the vessel is frozen at about -125°C to about -150°C; in some embodiments the vessel is frozen at about -125°C to about -145°C; in yet further embodiments, the vessel is frozen at about -135°C.

In some embodiments, the method is performed with a fresh tumor sample from a human subject.

In some embodiments, the tumor tissue is selected from the group consisting of melanoma tumor tissue, head and neck tumor tissue, breast tumor tissue, renal tumor tissue, pancreatic tumor tissue, glioblastoma tumor tissue, lung tumor tissue, colorectal tumor tissue, sarcoma tumor tissue, triple negative breast tumor tissue, cervical tumor tissue, endometrial tumor tissue, thyroid tumor tissue, ovarian tumor tissue, head and neck squamous cell carcinoma (HNSCC), and HPV-positive tumor tissue.

In some embodiments, the method further comprises a split between the first expansion (or first culture) step and the second expansion (or second culture) step. In an embodiment, the split occurs on day 16. In some embodiments, the first culture step is complete in about 11 days. In other embodiments, the first expansion (or first culture) step is complete in about 7 days. In yet other embodiments, the first expansion (or first culture) step is complete in about 7 days and the second expansion (or second culture) step is complete in about 14 days. In some embodiments, steps (c) through (e) or steps (h) through (j), as applicable, are complete in about 22 days. In yet other embodiments, steps (c) through (e) or steps (h) through (j), as applicable, are complete in 21 days; in some embodiments steps (c) through (e) or steps (h) through (j), as applicable, are complete in about 20 days; and in some embodiments steps (c) through (e) or steps (h) through (j), as applicable, are complete in about 16 days.

Disclosed herein but not claimed are methods of treating cancer for a human subject in need thereof, such methods comprising:

(a) storing the tumor tissue in a frozen state, the method of storing the tumor tissue comprising:

(i) trimming the tumor tissue to remove excess non-tumor tissue;

(ii) placing the tumor tissue in a closable vessel containing a storage medium;

(iii) incubating the vessel, which contains the tumor tissue and the storage medium, at a temperature in the range from about 2°C to about 8°C for about 20 to about 70 minutes;

(iv) freezing the vessel using the vapor phase of liquid nitrogen; and

(v) storing the vessel at the vapor phase of liquid nitrogen temperature;

(b) thawing the tumor tissue;

(c) adding the tumor fragments into a closed system;

(d) performing a first expansion by culturing the first population of TILs in a cell culture medium comprising IL-2 to produce a second population of TILs, wherein the first expansion is performed in a closed container providing a first gas-permeable surface area, wherein the first expansion is performed for about 3-14 days to obtain the second population of TILs, wherein the second population of TILs is at least 50-fold greater in number than the first population of TILs, and wherein the transition from step (c) to step (d) occurs without opening the system;

(e) performing a second expansion by supplementing the cell culture medium of the second population of TILs with additional IL-2, OKT-3, and antigen presenting cells (APCs), to produce a third population of TILs, wherein the second expansion is performed for about 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (d) to step (e) occurs without opening the system;

(f) harvesting the therapeutic population of TILs obtained from step (f), wherein the transition from step (e) to step (f) occurs without opening the system;

(g) transferring the harvested TIL population from step (f) to an infusion bag, wherein the transfer from step (f) to (g) occurs without opening the system;

(h) cryopreserving the infusion bag comprising the harvested TIL population from step (g) using a cryopreservation process; and

(i) administering a therapeutically effective dosage of the third population of TILs from the infusion bag in step (g) to the subject.

Disclosed herein but not claimed are methods of treating cancer for a human subject in need thereof, such methods comprising:

(a) obtaining tumor tissue from the subject;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range from about 2°C to about 8°C for about 20 to about 70 minutes;

(e) freezing the vessel using the vapor phase of liquid nitrogen;

(f) storing the vessel at the vapor phase of liquid nitrogen temperature;

(g) thawing the tumor tissue;

(h) adding the tumor tissue into a closed system;

(i) performing a first expansion by culturing a first population of TILs from the tumor tissue in a cell culture medium comprising IL-2 to produce a second population of TILs, wherein the first expansion is performed in a closed container providing a first gas-permeable surface area, wherein the first expansion is performed for about 3-14 days to obtain the second population of TILs, wherein the second population of TILs is at least 50-fold greater in number than the first population of TILs, and wherein the transition from step (h) to step (i) occurs without opening the system;

(j) performing a second expansion by supplementing the cell culture medium of the second population of TILs with additional IL-2, OKT-3, and antigen presenting cells (APCs), to produce a third population of TILs, wherein the second expansion is performed for about 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (i) to step (j) occurs without opening the system;

(k) harvesting the therapeutic population of TILs obtained from step (j), wherein the transition from step (j) to step (k) occurs without opening the system;

(l) transferring the therapeutic population of TILs harvested in step (k) to an infusion bag, wherein the transfer from step (k) to (1) occurs without opening the system;

(m) cryopreserving the infusion bag comprising the therapeutic population of TILs from step (1) using a cryopreservation process;

(n) thawing the therapeutic population of TILs in infusion bag from step (m); and

(o) administering a therapeutically effective dosage of the therapeutic population of TILs from the infusion bag in step (n) to the subject.

In some embodiments, the cancer is selected from cervical cancer, head and neck cancer (including, for example, head and neck squamous cell carcinoma (HNSCC) glioblastoma, endometrial cancer, thyroid cancer, colorectal cancer, ovarian cancer, sarcoma, pancreatic cancer, bladder cancer, breast cancer, triple negative breast cancer, melanoma, refractory melanoma, metastatic melanoma, and non-small cell lung carcinoma. The tissue structure of solid tumors includes interdependent tissue compartments including the parenchyma (cancer cells) and the supporting stromal cells in which the cancer cells are dispersed and which may provide a supporting microenvironment.

Disclosed herein but not claimed is a method for expanding tumor infiltrating lymphocytes (TILs) from frozen tumor tissue comprising:

(a) obtaining tumor tissue from a patient, wherein the tumor tissue comprises TILs;

(b) storing the tumor tissue in a frozen state, the method of storing the tumor tissue comprising incubating the tumor tissue for about 20 to about 70 minutes in a storage medium at a temperature in the range from about 2°C to about 8°C before freezing;

(c) thawing the tumor tissue;

(d) treating the tumor tissue in a gas permeable container with a first cell culture medium and interleukin 2 (IL-2) to provide TILs;

(e) removing at least a plurality of the TILs; and

(f) expanding the TILs with a second cell culture medium comprising cell culture media, irradiated feeder cells, OKT-3 antibody, and IL-2 in a gas permeable container to provide an expanded number of TILs.

Disclosed herein but not claimed is a method for expanding tumor infiltrating lymphocytes (TILs) from frozen tumor tissue comprising:

(a) obtaining tumor tissue from a patient, wherein the tumor tissue comprises TILs;

(b) incubating the tumor tissue for about 20 to about 70 minutes in a storage medium at a temperature in the range from about 2°C to about 8°C;

(c) storing the tumor tissue in a frozen state;

(d) thawing the tumor tissue;

(e) treating the tumor tissue in a gas permeable container with a first cell culture medium and interleukin 2 (IL-2) to provide TILs;

(f) removing at least a plurality of the TILs; and

(g) expanding the TILs with a second cell culture medium comprising cell culture media, irradiated feeder cells, OKT-3 antibody, and IL-2 in a gas permeable container to provide an expanded number of TILs.

Disclosed herein but not claimed is a method for expanding TILs from frozen tumor tissue comprising:

(a) obtaining tumor tissue from a patient, wherein the tumor tissue comprises TILs;

(b) storing the tumor tissue in a frozen state, the method of storing the tumor tissue comprising:

(i) trimming the tumor tissue to remove excess non-tumor tissue;

(ii) placing the tumor tissue in a closable vessel containing a storage medium;

(iii) incubating the vessel, which contains the tumor tissue and the storage medium, at a temperature in the range from about 2°C to about 8°C for about 30 minutes;

(iv) freezing the vessel; and

(v) storing the vessel such that the vessel remains frozen;

(c) thawing the vessel;

(d) treating the tumor tissue in a gas permeable container with a first cell culture medium and interleukin 2 (IL-2) to provide TILs;

(e) removing at least a plurality of the TILs; and

(f) expanding the TILs with a second cell culture medium comprising cell culture media, irradiated feeder cells, OKT-3, and IL-2 in a gas permeable container to provide an expanded number of TILs.

In some embodiments, the fresh tumor tissue is first washed in Hank's Balanced Salt Solution (HBSS) before performing the tissue trimming step. In some embodiments, the washing comprises at least three serial washes of at least three minutes each, wherein the HBSS is replaced after each wash.

In some embodiments the thawing step comprises immersing the vessel in a 37°C water bath for about 5 minutes.

In some embodiments, the freezing step comprises freezing the vessel at a temperature between about -125°C to about -195°C. In some embodiments, the vessel is frozen at a temperature between about -125°C to about -150°C; in some embodiments the vessel is frozen at a temperature between about -125°C to about -145°C; in yet further embodiments, the vessel is frozen at a temperature of about -135°C.

In some embodiments, the tumor tissue is selected from the group consisting of melanoma tumor tissue, head and neck tumor tissue, breast tumor tissue, renal tumor tissue, pancreatic tumor tissue, glioblastoma tumor tissue, lung tumor tissue, colorectal tumor tissue, sarcoma tumor tissue, triple negative breast tumor tissue, cervical tumor tissue, endometrial tumor tissue, thyroid tumor tissue, ovarian tumor tissue, head and neck squamous cell carcinoma (HNSCC), and HPV-positive tumor tissue.

In some embodiments, the method further comprises a split between the first expansion (or first culture) step and the second expansion (or second culture) step. In an embodiment, the split occurs on day 16. In some embodiments, the first culture step is complete in about 11 days. In other embodiments, the first expansion (or first culture) step is complete in about 7 days. In yet other embodiments, the first expansion (or first culture) step is complete in about 7 days and the second expansion (or second culture) step is complete in about 14 days. In some embodiments, steps (d) through (f) or steps (e) through (g), as applicable, are complete in about 22 days. In yet other embodiments, steps (d) through (f) or steps (e) through (g), as applicable, are complete in about 21 days; in some embodiments steps (d) through (f) or steps (e) through (g), as applicable, are complete in about 20 days; and in some embodiments steps (d) through (f) or steps (e) through (g), as applicable, are complete in about 16 days.

Disclosed herein but not claimed are methods of treating cancer for a human subject in need thereof, such methods comprising:

(a) obtaining tumor tissue from the subject, wherein the tumor tissue comprises TILs;

(b) storing the tumor tissue in a frozen state, the method of storing the tumor tissue comprising:

(i) trimming the tumor tissue to remove excess non-tumor tissue;

(ii) placing the tumor tissue in a closable vessel containing a storage medium;

(iii) incubating the vessel, which contains the tumor tissue and the storage medium, at a temperature in the range from about 2°C to about 8°C for about 20 minutes to about 70 minutes;

(iv) freezing the vessel using the vapor phase of liquid nitrogen; and

(v) storing the vessel at the vapor phase of liquid nitrogen temperature;

(c) thawing the tumor tissue;

(d) adding the tumor tissue into a closed system;

(e) performing a first expansion by culturing a first population of TILs from the tumor tissue in a cell culture medium comprising IL-2 to produce a second population of TILs, wherein the first expansion is performed in a closed container providing a first gas-permeable surface area, wherein the first expansion is performed for about 3-14 days to obtain the second population of TILs, wherein the second population of TILs is at least 50-fold greater in number than the first population of TILs, and wherein the transition from step (d) to step (e) occurs without opening the system;

(f) performing a second expansion by supplementing the cell culture medium of the second population of TILs with additional IL-2, OKT-3, and antigen presenting cells (APCs), to produce a third population of TILs, wherein the second expansion is performed for about 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (e) to step (f) occurs without opening the system;

(g) harvesting the therapeutic population of TILs obtained from step (f), wherein the transition from step (f) to step (g) occurs without opening the system;

(h) transferring the harvested TIL population from step (g) to an infusion bag, wherein the transfer from step (g) to (h) occurs without opening the system;

(i) cryopreserving the infusion bag comprising the harvested TIL population from step (h) using a cryopreservation process; and

(j) administering a therapeutically effective dosage of the third population of TILs from the infusion bag in step (i) to the subject.

In some embodiments, the fresh tumor tissue is first washed in Hank's Balanced Salt Solution (HBSS) before performing step (i) of the tumor tissue storing method. In some embodiments, the washing comprises at least three serial washes of at least three minutes each, wherein the HBSS is replaced after each wash.

In some embodiments step (c) comprises immersing the vessel in a 37°C water bath for about 5 minutes.

In some embodiments, step (iv) of the tumor tissue storing method comprises freezing the vessel at a temperature between about -125°C to about -195°C. In some embodiments, the vessel is frozen at a temperature between about -125°C to about -150°C; in some embodiments the vessel is frozen at a temperature between about -125°C to about -145°C; in yet further embodiments, the vessel is frozen at a temperature of about -135°C.

In some embodiments, the tumor tissue is selected from the group consisting of melanoma tumor tissue, head and neck tumor tissue, breast tumor tissue, renal tumor tissue, pancreatic tumor tissue, glioblastoma tumor tissue, lung tumor tissue, colorectal tumor tissue, sarcoma tumor tissue, triple negative breast tumor tissue, cervical tumor tissue, endometrial tumor tissue, thyroid tumor tissue, ovarian tumor tissue, head and neck squamous cell carcinoma (HNSCC), and HPV-positive tumor tissue.

In some embodiments, the method further comprises a split between the first expansion (or first culture) step and the second expansion (or second culture) step. In an embodiment, the split occurs on day 16. In some embodiments, the first culture step is complete in about 11 days. In other embodiments, the first expansion (or first culture) step is complete in about 7 days. In yet other embodiments, the first expansion (or first culture) step is complete in about 7 days and the second expansion (or second culture) step is complete in about 14 days. In some embodiments, steps (e) through (g) are complete in about 22 days. In yet other embodiments, steps (e) through (g) are complete in about 21 days; in some embodiments steps (e) through (g) are complete in about 20 days; and in some embodiments steps (e) through (g) are complete in about 16 days.

In some embodiments, the first expansion (or first culture) step is complete in about 5 days. In yet other embodiments, the first expansion (or first culture) step is complete in about 3 days. In yet other embodiments, the first expansion (or first culture) step is complete in about 5 days and the second expansion (or second culture) step is complete in about 11 days. In yet other embodiments, the first expansion (or first culture) step is complete in about 3 days and the second expansion (or second culture) step is complete in about 13 days. Optionally, the second expansion (or second culture) can be split into two or more cultures on or about the fifth day or sixth day of the second expansion (or second culture).

In some embodiments of the above methods to manufacture or expand TILs, OKT-3 is present in the culture medium beginning at day 0.

Disclosed herein but not claimed are methods of treating cancer in a subject modified as appropriate such that in the step of the first expansion (or first culture) the first expansion (or first culture) is complete in about 5 days.

Disclosed herein but not claimed are methods of treating cancer in a subject modified as appropriate such that in the step of the first expansion (or first culture) the first expansion (or first culture) is complete in about 3 days.

Disclosed herein but not claimed are methods of treating cancer in a subject modified as appropriate such that in the step of the first expansion (or first culture) the first expansion (or first culture) is complete in about 5 days and the second expansion (or second culture) is complete in about 11 days.

Disclosed herein but not claimed are methods of treating cancer in a subject modified as appropriate such that in the step of the first expansion (or first culture) the first expansion (or first culture) is complete in about 5 days, the second expansion (or second culture) is complete in about 11 days, and the second expansion (or second culture) is split into two or more cultures on or about the fifth day of the second expansion (or second culture).

Disclosed herein but not claimed are methods of expanding tumor infiltrating lymphocytes (TILs) from frozen tumor tissue modified as appropriate such that in the step of the first expansion (or first culture) the first expansion (or first culture) is complete in about 3 days and the second expansion (or second culture) is complete in about 13 days.

Disclosed herein but not claimed are methods of expanding tumor infiltrating lymphocytes (TILs) from frozen tumor tissue modified as appropriate such that in the step of the first expansion (or first culture) the first expansion (or first culture) is complete in about 3 days, the second expansion (or second culture) is complete in about 13 days, and the second expansion (or second culture) is split into two or more cultures on or about the sixth day of the second expansion (or second culture).

Disclosed herein but not claimed are methods of expanding tumor infiltrating lymphocytes (TILs) from frozen tumor tissue modified as appropriate such that in the step of the first expansion (or first culture) the first expansion (or first culture) is complete in about 5 days.

Disclosed herein but not claimed are methods of expanding tumor infiltrating lymphocytes (TILs) from frozen tumor tissue modified as appropriate such that in the step of the first expansion (or first culture) the first expansion (or first culture) is complete in about 3 days.

Disclosed herein but not claimed are methods of expanding tumor infiltrating lymphocytes (TILs) from frozen tumor tissue modified as appropriate such that in the step of the first expansion (or first culture) the first expansion (or first culture) is complete in about 5 days and the second expansion (or second culture) is complete in about 11 days.

Disclosed herein but not claimed are methods of expanding tumor infiltrating lymphocytes (TILs) from frozen tumor tissue modified as appropriate such that in the step of the first expansion (or first culture) the first expansion (or first culture) is complete in about 5 days, the second expansion (or second culture) is complete in about 11 days, and the second expansion (or second culture) is split into two or more cultures on or about the fifth day of the second expansion (or second culture).

Disclosed herein but not claimed are methods of expanding tumor infiltrating lymphocytes (TILs) from frozen tumor tissue modified as appropriate such that in the step of the first expansion (or first culture) the first expansion (or first culture) is complete in about 3 days and the second expansion (or second culture) is complete in about 13 days.

Disclosed herein but not claimed are methods of expanding tumor infiltrating lymphocytes (TILs) from frozen tumor tissue modified as appropriate such that in the step of the first expansion (or first culture) the first expansion (or first culture) is complete in about 3 days, the second expansion (or second culture) is complete in about 13 days, and the second expansion (or second culture) is split into two or more cultures on or about the sixth day of the second expansion (or second culture).

Disclosed herein but not claimed are methods for preparing tumor infiltrating lymphocytes (TILs) for adoptive T-cell therapy modified as appropriate such that modified as appropriate such that in the step of the first expansion (or first culture) the first expansion (or first culture) is complete in about 3 days and the second expansion (or second culture) is complete in about 13 days.

Disclosed herein but not claimed are methods of preparing tumor infiltrating lymphocytes (TILs) for adoptive T-cell therapy modified as appropriate such that in the step of the first expansion (or first culture) the first expansion (or first culture) is complete in about 3 days, the second expansion (or second culture) is complete in about 13 days, and the second expansion (or second culture) is split into two or more cultures on or about the sixth day of the second expansion (or second culture).

Disclosed herein but not claimed are methods of preparing tumor infiltrating lymphocytes (TILs) for adoptive T-cell therapy modified as appropriate such that in the step of the first expansion (or first culture) the first expansion (or first culture) is complete in about 5 days.

Disclosed herein but not claimed are methods of preparing tumor infiltrating lymphocytes (TILs) for adoptive T-cell therapy modified as appropriate such that in the step of the first expansion (or first culture) the first expansion (or first culture) is complete in about 3 days.

Disclosed herein but not claimed are methods of preparing tumor infiltrating lymphocytes (TILs) for adoptive T-cell therapy modified as appropriate such that in the step of the first expansion (or first culture) the first expansion (or first culture) is complete in about 5 days and the second expansion (or second culture) is complete in about 11 days.

Disclosed herein but not claimed are methods of preparing tumor infiltrating lymphocytes (TILs) for adoptive T-cell therapy modified as appropriate such that in the step of the first expansion (or first culture) the first expansion (or first culture) is complete in about 5 days, the second expansion (or second culture) is complete in about 11 days, and the second expansion (or second culture) is split into two or more cultures on or about the fifth day of the second expansion (or second culture).

Disclosed herein but not claimed are methods of preparing tumor infiltrating lymphocytes (TILs) for adoptive T-cell therapy modified as appropriate such that in the step of the first expansion (or first culture) the first expansion (or first culture) is complete in about 3 days and the second expansion (or second culture) is complete in about 13 days.

Disclosed herein but not claimed are methods of preparing tumor infiltrating lymphocytes (TILs) for adoptive T-cell therapy modified as appropriate such that in the step of the first expansion (or first culture) the first expansion (or first culture) is complete in about 3 days, the second expansion (or second culture) is complete in about 13 days, and the second expansion (or second culture) is split into two or more cultures on or about the sixth day of the second expansion (or second culture).

Disclosed herein but not claimed are methods modified as appropriate such that the first expansion (or first culture) is performed in a defined medium.

Disclosed herein but not claimed are methods modified as appropriate such that the second expansion (or second culture) is performed in a defind medium.

Disclosed herein but not claimed are methods modified as appropriate such that the first expansion (or first culture) and the second expansion (or second culture) are performed in defined media that are the same or different.

TIL Manufacturing Processes

There are various methods to expand TILs known to one skilled in the art. For example, Jin et al., J. Immunother. 35(3): 283-292 (2012), "Simplified Method of the Growth of Human Tumor Infiltrating Lymphocytes in Gas-permeable Flasks to Numbers Needed for Patient Treatment," teaches simplified methods of producing TILs for clinical use. Jin *et al.* teaches a first TIL culture followed by a rapid expansion (REP) protocol, which combined, enables one skilled in the art to produce clinically useful quantities of TILs. In some embodiments, the invention provides a method of manufacturing TILs comprising the step of cryopreserving a tumor, thawing a tumor, and performing the process described in Jin *et al.* Briefly, this process involves the following process. TILs may initially be cultured from enzymatic tumor digests and tumor fragments (about 1 to 8 mm$^3$) produced by sharp dissection. Tumor digests are produced by incubation in enzyme media (RPMI 1640, 2 mM GlutaMAX, 10 mg/mL gentamicin, 30U/mL DNase, and 1.0 mg/mL collagenase) followed by mechanical dissociation (GentleMACS, Miltenyi Biotec, Auburn, CA). Immediately after placing the tumor in enzyme media, it is mechanically dissociated for approximately 1 minute. The material was then incubated for 30 minutes at 37°C in 5% $CO_2$ and is then mechanically disrupted again for approximately 1 minute and incubated again for 30 minutes at 37°C in 5% $CO_2$. The tumor is then mechanically disrupted a third time for approximately 1 minute. If after the third mechanical disruption, large pieces of tissue were present, 1 or 2 additional mechanical dissociations may be applied to the sample, with or without 30 additional minutes of incubation at 37°C in 5% $CO_2$. At the end of the final incubation, if the cell suspension contained a large number of red blood cells or dead cells, a density gradient separation using Ficoll may be performed to remove these cells. When TIL cultures are initiated in 24-well plates (Costar 24-well cell culture cluster, flat bottom; Corning Incorporated, Corning, NY), each well is seeded with $1 \times 10^6$ tumor digest cells or one tumor fragment approximately about 1 to 8 mm$^3$ in size in 2 mL of complete medium (CM) with IL-2 (6000 IU/mL; Chiron Corp., Emeryville, CA). CM comprised RPMI 1640 with GlutaMAX, supplemented with 10% human AB serum, 25mM Hepes, and about 10 $\mu$g/mL gentamicin. When cultures were initiated in gas-permeable flasks with a 40 mL capacity and a 10 cm$^2$ gas-permeable silicon bottom (G-Rex10; Wilson Wolf Manufacturing, New Brighton, MN), each flask is loaded with 10 to $40 \times 10^6$ viable tumor digest cells or 5 to 30 tumor fragments in 10 to 40 mL of CM with IL-2. Both the G-Rex10 and 24-well plates are incubated in a humidified incubator at 37°C in 5% $CO_2$ and 5 days after culture initiation, half the media is removed and replaced with fresh CM and IL-2 and after day 5, half the media is changed every 2-3 days. REP of TIL is performed using T-175 flasks and gas-permeable bags or gas-permeable G-Rex flasks. For TIL REP in T-175 flasks, $1 \times 10^6$ TILs suspended in 150 mL of media was added to each T-175 flask. The TIL were cultured with irradiated (50 Gy) allogeneic PBMC as "feeder" cells at a ratio of 1 to 100 and the cells are cultured in a 1 to 1 mixture of CM and AIM-V medium (50/50 medium), supplemented with 3000 IU/mL of IL-2 and 30 ng/mL of anti-CD3. The T-175 flasks are incubated at 37°C in 5% $CO_2$. Half the media is changed on day 5 using 50/50 medium with 3000 IU/mL of IL-2. On day 7, cells from 2 T-175 flasks are combined in a 3L bag and 300 mL of AIM-V with 5% human AB serum and 3000 IU/mL of IL-2 is added to the 300 mL of TIL suspension. The number of cells in each bag was counted every day or 2 and fresh media was added to keep the cell count between 0.5 and $2.0 \times 10^6$ cells/mL. For TIL REP in 500mL capacity flasks with 100 cm$^2$ gas-permeable silicon bottoms (G-Rex100, Wilson Wolf), $5 \times 10^6$ to $10 \times 10^6$ TIL are cultured with irradiated allogeneic PBMC at a ratio of 1 to 100 in 400 mL of 50/50 medium, supplemented with 3000 IU/mL of IL-2 and 30 ng/mL of anti-CD3. G-Rex100 flasks are incubated at 37°C in 5% $CO_2$. On day 5, 250 mL of supernatant is removed and placed into centrifuge bottles and centrifuged at 1500 rpm (491 g) for 10 minutes. TIL pellets are resuspended with 150 mL of fresh 50/50 medium with 3000 IU/mL of IL-2 and added back to the original G-Rex100 flasks. When TIL are expanded serially in G-Rex100 flasks, on day 7 the TIL in each G-Rex100 were suspended in the 300 mL of media present in each flask and the cell suspension was divided into three 100 mL aliquots that were used to seed 3 G-Rex100 flasks. 150 mL of AIM-V with 5% human AB serum and 3000 IU/mL of IL-2 is added to each flask. The G-Rex100 flasks are incubated at 37°C in 5% $CO_2$ and after 4 days 150 mL of AIM-V with 3000 IU/mL of IL-2 was added to each G-Rex100 flask. The cells are harvested on day 14 of culture.

In some embodiments, the expansion methods according to the present invention comprises preparing expansion of cells from frozen tumor. Tumor samples are collected from patients and cryopreserved according to methods disclosed herein. When ready to expand the cells, for a process performed at the 1/100th scale, about six 2-3 mm diameter frozen tumor fragments are thawed using a 37°C water bath for 5 + 1 minutes and washed in sterile Hanks Balanced Salt Solution (HBSS) supplemented with gentamicin. The fragments are then placed into a G-Rex 10M flask (Wilson Wolf Mfg., New Brighton, MN) or other gas permeable container with CM1 media or a serum-free or defined media containing about 6,000 IU/mL of rhIL-2. These pre-REP (or first expansion) cultures are incubated for 5 days in a 37°C incubator. On day 5, the pre-REP cells are harvested, and the REP (or second expansion) is initiated by co-culturing 10% of the pre-REP TILs with either $25 \times 10^6$ or $50 \times 10^6$ PBMC feeder cells in CM2 media or a serum-free or defined media containing 3,000 IU/mL rhIL-2 and 30 ng/mL of OKT-3 in a G-Rex 5M flask (Wilson Wolf Mfg., New Brighton, MN) or other gas permeable container. On day 10, the culture is split into G-Rex 5M flasks or other gas permeable container containing no more than $10 \times 10^6$ cells. The split cultures are incubated for 6 additional days in CM4 media or other serum-free or defined media containing 3,000 IU/mL rhIL-2. On day 16, the cells are harvested and frozen using CryoStorIO (Biolife, USA). This process is referred to herein as the "Early REP Method 1" process or "ER-1."

In some embodiments, the expansion methods according to the present invention comprises preparing expansion of cells from frozen tumor. Tumor samples are collected from patients and cryopreserved according to methods disclosed herein. When ready to expand the cells, for a process performed at the 1/100th scale, about six 2-3 mm diameter frozen tumor fragments are thawed using a 37°C water bath for 5 + 1 minutes and washed in sterile Hanks Balanced Salt Solution (HBSS) supplemented with gentamicin. The fragments are then placed into a G-Rex 10M flask (Wilson Wolf Mfg., New Brighton, MN) or other gas permeable container with CM1 media or a serum-free or defined media containing about 6,000 IU/mL of rhIL-2. These pre-REP (or first expansion) cultures are incubated for 3 days in a 37°C incubator. On day 3, the pre-REP cells are harvested, and the REP (or second expansion) is initiated by co-culturing 10% of the pre-REP TILs with either $25 \times 10^6$ or $50 \times 10^6$ PBMC feeder cells in CM2 media or a serum-free or defined media containing 3,000 IU/mL rhIL-2 and 30 ng/mL of OKT-3 in a G-Rex 5M flask (Wilson Wolf Mfg., New Brighton, MN) or other gas permeable container. On day 9, the culture is split into G-Rex 5M flasks or other gas permeable container containing no more than $10 \times 10^6$ cells. The split cultures are incubated for 7 additional days in CM4 media or other serum-free or defined media containing 3,000 IU/mL rhIL-2. On day 16, the cells are harvested and frozen using CryoStor10 (Biolife, USA). This process is referred to herein as the "Early REP Method 2" process or "ER-2."

In some embodiments, the invention provides a method of manufacturing TILs comprising the step of cryopreserving a tumor, thawing a tumor, and performing the following process. TILs can be produced by rapid expansion using stimulation of peripheral blood mononuclear cells (PBMC) *in vitro* with an antigen (one or more, including antigenic portions thereof, such as epitope(s), or a cell) of the cancer, which can be optionally expressed from a vector, such as an HLA-A2 binding peptide, e.g., 0.3 µM MART-1:26-35 (27L) or gp100:209-217 (210M), in the presence of a T-cell growth factor, such as 300 IU/mL IL-2 or IL-15, with IL-2 being preferred. The *in vitro*-induced TILs are rapidly expanded by re-stimulation with the same antigen(s) of the cancer pulsed onto HLA-A2-expressing antigen-presenting cells. Alternatively, the TILs can be re-stimulated with irradiated, autologous lymphocytes or with irradiated HLA-A2⁺ allogeneic lymphocytes and IL-2, for example. TILs can be selected for highly avid recognition of any of the unique antigens produced as a result of the estimated 10,000 genetic mutations encoded by each tumor cell genome. The antigen, however, need not be unique. T-cells can be selected for highly avid recognition of one or more antigens of a cancer, including an antigenic portion of one or more antigens, such as an epitope, or a cell of the cancer. An "antigen of a cancer" and an "antigen of the cancer" are intended to encompass all of the aforementioned antigens. If the cancer is melanoma, such as metastatic melanoma, preferably the TILs are selected for highly avid recognition of MART-1 (such as MART-1 :26-35 (27L)), gp100 (such as gp100:209-217 (210M)), or a "unique" or patient-specific antigen derived from a tumor encoded mutation. Other suitable melanoma antigens for which highly avid recognition by TILs can be selected include, but are not limited to, tyrosinase, tyrosinase related protein (TRP)1, TRP2, and MAGE. Antigens, such as NY-ESO-1, telomerase, p53, HER2/neu, carcinoembryonic antigen, or prostate-specific antigen, can be used to select for highly avid recognition by TILs for treatment of lung carcinoma, breast cancer, colon cancer, prostate cancer, and the like TILs can be selected include, but are not limited to, tyrosinase, tyrosinase related protein.

IL-2-based TIL expansion followed by a "rapid expansion process" (REP) has become a preferred method for TIL expansion because of its speed and efficiency. Dudley, et al., Science 2002, 298, 850-54; Dudley, et al., J. Clin. Oncol. 2005, 23, 2346-57; Dudley, et al., J. Clin. Oncol. 2008, 26, 5233-39; Riddell, et al., Science 1992, 257, 238-41; Dudley, et al., J. Immunother. 2003, 26, 332-42. REP can result in a 1,000-fold expansion of TILs over a 14-day period, although it requires a large excess (*e.g.*, 200-fold) of irradiated allogeneic peripheral blood mononuclear cells (PBMCs), often from multiple donors, as feeder cells, as well as anti-CD3 antibody (OKT3) and high doses of IL-2. Dudley, et al., J. Immunother. 2003, 26, 332-42. The cryopreserved tumor fragments are suitable starting points for initial or first cultures to manufacture TILs for therapeutic or other purposes.

An exemplary TIL process known as process 2A containing some of these features is depicted in Figure 1. Another exemplary TIL process, known as process 1C, is described and compared to process 2A in Figures 5 and 6. An embodiment of process 2A is shown Figure 1.

As discussed herein, the present invention can include a step relating to the restimulation of cryopreserved TILs to increase their metabolic activity and thus relative health prior to transplant into a patient, and methods of testing said metabolic health. As generally outlined herein, TILs are generally taken from a patient sample and manipulated to expand their number prior to transplant into a patient. In some embodiments, the TILs may be optionally genetically manipulated as discussed below.

Disclosed herein but not claimed are methods wherein the TILs may be cryopreserved and thawed for administration to a patient. Once thawed, they may also be restimulated to increase their metabolism prior to infusion into a patient.

In some embodiments, the first expansion (including processes referred to as the preREP as well as processes shown in Figure 1 as Step A) is shortened to 3 to 14 days and the second expansion (including processes referred to as the REP as well as processes shown in Figure 1 as Step B) is shorted to 7 to 14 days, as discussed in detail below as well as in the examples and figures. In some embodiments, the first expansion (for example, an expansion described as Step B in Figure 1) is shortened to 11 days and the second expansion (for example, an expansion as described in Step D in Figure 1) is shortened to 11 days. In some embodiments, the combination of the first expansion and second expansion (for example, expansions described as Step B and Step D in Figure 1) is shortened to 22 days, as discussed in detail below and in the examples and figures.

The "Step" Designations A, B, C, *etc.,* below are in reference to Figure 1 and in reference to certain embodiments described herein. The ordering of the Steps below and in Figure 1 is exemplary and any combination or order of steps, as well as additional steps, repetition of steps, and/or omission of steps is contemplated by the present application and the methods disclosed herein.

*Step A. Obtain Patient Tumor Sample*

In general, TILs are initially obtained from a patient tumor sample ("primary TILs") and then expanded into a larger population for further manipulation as described herein, optionally cryopreserved, restimulated as outlined herein and optionally evaluated for phenotype and metabolic parameters as an indication of TIL health.

A patient tumor sample may be obtained using methods known in the art, generally via surgical resection, needle biopsy or other means for obtaining a sample that contains a mixture of tumor and TIL cells. In general, the tumor sample may be from any solid tumor, including primary tumors, invasive tumors or metastatic tumors. The tumor sample may also be a liquid tumor, such as a tumor obtained from a hematological malignancy. The solid tumor may be of any cancer type, including, but not limited to, breast, pancreatic, prostate, colorectal, lung, brain, renal, stomach, and skin (including but not limited to squamous cell carcinoma, basal cell carcinoma, and melanoma). In some embodiments, useful TILs are obtained from malignant melanoma tumors, as these have been reported to have particularly high levels of TILs.

The term "solid tumor" refers to an abnormal mass of tissue that usually does not contain cysts or liquid areas. Solid tumors may be benign or malignant. The term "solid tumor cancer" refers to malignant, neoplastic, or cancerous solid tumors. Solid tumor cancers include, but are not limited to, sarcomas, carcinomas, and lymphomas, such as cancers of the lung, breast, triple negative breast cancer, prostate, colon, rectum, and bladder. In some embodiments, the cancer is selected from cervical cancer, head and neck cancer (including, for example, head and neck squamous cell carcinoma (HNSCC) glioblastoma, ovarian cancer, sarcoma, pancreatic cancer, bladder cancer, breast cancer, triple negative breast cancer, and non-small cell lung carcinoma. The tissue structure of solid tumors includes interdependent tissue compartments including the parenchyma (cancer cells) and the supporting stromal cells in which the cancer cells are dispersed and which may provide a supporting microenvironment.

The term "hematological malignancy" refers to mammalian cancers and tumors of the hematopoietic and lymphoid tissues, including but not limited to tissues of the blood, bone marrow, lymph nodes, and lymphatic system. Hematological malignancies are also referred to as "liquid tumors." Hematological malignancies include, but are not limited to, acute lymphoblastic leukemia (ALL), chronic lymphocytic lymphoma (CLL), small lymphocytic lymphoma (SLL), acute myelogenous leukemia (AML), chronic myelogenous leukemia (CML), acute monocytic leukemia (AMoL), Hodgkin's lymphoma, and non-Hodgkin's lymphomas. The term "B cell hematological malignancy" refers to hematological malignancies that affect B cells.

Once obtained, the tumor sample is generally fragmented using sharp dissection into small pieces of between 1 to about 8 mm3, with from about 2-3 mm$^3$ being particularly useful. The TILs are cultured from these fragments using enzymatic tumor digests. Such tumor digests may be produced by incubation in enzymatic media (*e.g.*, Roswell Park Memorial Institute (RPMI) 1640 buffer, 2 mM glutamate, 10 mcg/mL gentamicin, 30 units/mL of DNase and 1.0 mg/mL of collagenase) followed by mechanical dissociation (*e.g.*, using a tissue dissociator). Tumor digests may be produced by placing the tumor in enzymatic media and mechanically dissociating the tumor for approximately 1 minute, followed by incubation for 30 minutes at 37 °C in 5% $CO_2$, followed by repeated cycles of mechanical dissociation and incubation under the foregoing conditions until only small tissue pieces are present. At the end of this process, if the cell suspension contains a large number of red blood cells or dead cells, a density gradient separation using FICOLL branched hydrophilic polysaccharide may be performed to remove these cells. Alternative methods known in the art may be used, such as those described in U.S. Patent Application Publication No. 2012/0244133 A1.

In general, the harvested cell suspension is called a "primary cell population" or a "freshly harvested" cell population.

In some embodiments, fragmentation includes physical fragmentation, including for example, dissection as well as digestion. In some embodiments, the fragmentation is physical fragmentation. In some embodiments, the fragmentation is dissection. In some embodiments, the fragmentation is by digestion. In some embodiments, TILs can be initially cultured from enzymatic tumor digests and tumor fragments obtained from patients. In an embodiment, TILs can be initially cultured from enzymatic tumor digests and tumor fragments obtained from patients.

In some embodiments, where the tumor is a solid tumor, the tumor undergoes physical fragmentation after the tumor sample is obtained in, for example, Step A (as provided in Figure 1). In some embodiments, the fragmentation occurs before cryopreservation. In some embodiments, the fragmentation occurs after cryopreservation. In some embodiments, the fragmentation occurs after the tumor is obtained and in the absence of any cryopreservation. In some embodiments, the tumor is fragmented and 10, 20, 30, 40 or more fragments or pieces are placed in each container for the first expansion. In some embodiments, the tumor is fragmented and 30 or 40 fragments or pieces are placed in each container for the first expansion. In some embodiments, the tumor is fragmented and 40 fragments or pieces are placed in each container for the first expansion. In some embodiments, the multiple fragments comprise about 4 to about 50 fragments, wherein each fragment has a volume of about 27 mm$^3$. In some embodiments, the multiple fragments comprise about 30 to about 60 fragments with a total volume of about 1300 mm$^3$ to about 1500 mm$^3$. In some embodiments, the multiple fragments comprise about 50 fragments with a total volume of about 1350 mm$^3$. In some embodiments, the multiple fragments comprise about 50 fragments with a total mass of about 1 gram to about 1.5 grams. In some embodiments, the multiple fragments comprise about 4 fragments.

In some embodiments, the TILs are obtained from tumor fragments. In some embodiments, the tumor fragment is obtained by sharp dissection. In some embodiments, the tumor fragment is between about 1 mm$^3$ and 10 mm$^3$. In some embodiments, the tumor fragment is between about 1 mm$^3$ and 8 mm$^3$. In some embodiments, the tumor fragment is about 1 mm$^3$. In some embodiments, the tumor fragment is about 2 mm$^3$. In some embodiments, the tumor fragment is about 3 mm$^3$. In some embodiments, the tumor fragment is about 4 mm$^3$. In some embodiments, the tumor fragment is about 5 mm$^3$. In some embodiments, the tumor fragment is about 6 mm$^3$. In some embodiments, the tumor fragment is about 7 mm$^3$. In some embodiments, the tumor fragment is about 8 mm$^3$. In some embodiments, the tumor fragment is about 9 mm$^3$. In some embodiments, the tumor fragment is about 10 mm$^3$. In some embodiments, the tumors are 1-4 mm $\times$ 1-4 mm $\times$ 1-4 mm. In some embodiments, the tumors are 1 mm $\times$ 1 mm $\times$ 1 mm. In some embodiments, the tumors are 2 mm $\times$ 2 mm $\times$ 2 mm. In some embodiments, the tumors are 3 mm $\times$ 3 mm $\times$ 3 mm. In some embodiments, the tumors are 4 mm $\times$ 4 mm $\times$ 4 mm. In embodiments wherein the tumor tissue is first frozen before initiating a culture, tumors are about 6 mm $\times$ 6 mm $\times$ 6 mm.

Disclosed herein but not claimed are methods wherein the tumors are resected in order to minimize the amount of hemorrhagic, necrotic, and/or fatty tissues on each piece. Disclosed herein but not claimed are methods wherein the tumors are resected in order to minimize the amount of hemorrhagic tissue on each piece. Disclosed herein but not claimed are methods wherein the tumors are resected in order to minimize the amount of necrotic tissue on each piece. Disclosed herein but not claimed are methods wherein the tumors are resected in order to minimize the amount of fatty tissue on each piece.

In some embodiments, the tumor fragmentation is performed in order to maintain the tumor internal structure. In some embodiments, the tumor fragmentation is performed without preforming a sawing motion with a scalpel. In some embodiments, the TILs are obtained from tumor digests. In some embodiments, tumor digests were generated by incubation in enzyme media, for example but not limited to RPMI 1640, 2 mM GlutaMAX, 10 μg/mL gentamicin, 30 U/mL DNase, and 1.0 mg/mL collagenase, followed by mechanical dissociation (GentleMACS, Miltenyi Biotec, Auburn, CA). After placing the tumor in enzyme media, the tumor can be mechanically dissociated for approximately 1 minute. The solution can then be incubated for 30 minutes at 37 °C in 5% $CO_2$ and it then mechanically disrupted again for approximately 1 minute. After being incubated again for 30 minutes at 37 °C in 5% $CO_2$, the tumor can be mechanically disrupted a third time for approximately 1 minute. In some embodiments, after the third mechanical disruption if large pieces of tissue were present, 1 or 2 additional mechanical dissociations were applied to the sample, with or without 30 additional minutes of incubation at 37 °C in 5% $CO_2$. In some embodiments, at the end of the final incubation if the cell suspension contained a large number of red blood cells or dead cells, a density gradient separation using Ficoll can be performed to remove these cells.

In some embodiments, the harvested cell suspension prior to the first expansion step is called a "primary cell population" or a "freshly harvested" cell population.

In some embodiments, cells can be optionally frozen after sample harvest and stored frozen prior to entry into the expansion described in Step B, which is described in further detail below, as well as exemplified in Figure 1.

*Step B: First Expansion*

In some embodiments, the present methods provide for obtaining young TILs, which are capable of increased replication cycles upon administration to a subject/patient and as such may provide additional therapeutic benefits over older TILs (for example "older TILs" have further undergone more rounds of ex vivo replication prior to administration to a subject/patient). Features of young TILs have been described in the literature, for example Donia, et al., Scandinavian Journal of Immunology, 75:157-167 (2012); Dudley et al., Clin Cancer Res, 16:6122-6131 (2010); Huang et al., J Immunother, 28(3):258-267 (2005); Besser et al., Clin Cancer Res, 19(17):OF1-OF9 (2013); Besser et al., J Immunother, 32:415-423 (2009); Robbins, et al., J Immunol, 2004; 173:7125-7130; Shen et al., J Immunother, 30:123-129 (2007); Zhou, et al., J Immunother, 28:53-62 (2005); and Tran, et al., J Immunother, 31:742-751 (2008).

The diverse antigen receptors of T and B lymphocytes are produced by somatic recombination of a limited, but large number of gene segments. These gene segments: V (variable), D (diversity), J (joining), and C (constant), determine the binding specificity and downstream applications of immunoglobulins and T-cell receptors (TCRs). The present invention provides a method for generating TILs which exhibit and increase the T-cell repertoire diversity. In some embodiments, the TILs obtained by the present method exhibit an increase in the T-cell repertoire diversity. In some embodiments, the TILs obtained by the present method exhibit an increase in the T-cell repertoire diversity as compared to freshly harvested TILs and/or TILs prepared using other methods than those provide herein including for example, methods other than those embodied in Figure 1. In some embodiments, the TILs obtained by the present method exhibit an increase in the T-cell repertoire diversity as compared to freshly harvested TILs and/or TILs prepared using methods referred to as process 1C, as exemplified in Figure 5 and/or Figure 6. In some embodiments, the TILs obtained in the first expansion exhibit an increase in the T-cell repertoire diversity. In some embodiments, the increase in diversity is an increase in the immunoglobulin diversity and/or the T-cell receptor diversity. In some embodiments, the diversity is in the immunoglobulin is in the immunoglobulin heavy chain. In some embodiments, the diversity is in the immunoglobulin is in the immunoglobulin light chain. In some embodiments, the diversity is in the T-cell receptor. In some embodiments, the diversity is in one of the T-cell receptors selected from the group consisting of alpha, beta, gamma, and delta receptors. In some embodiments, there is an increase in the expression of T-cell receptor (TCR) alpha and/or beta. In some embodiments, there is an increase in the expression of T-cell receptor (TCR) alpha. In some embodiments, there is an increase in the expression of T-cell receptor (TCR) beta. In some embodiments, there is an increase in the expression of TCRab (*i.e.,* TCRα/β).

After dissection or digestion of tumor fragments, for example such as described in Step A of Figure 1, the resulting cells are cultured in serum containing IL-2 under conditions that favor the growth of TILs over tumor and other cells. In some embodiments, the tumor digests are incubated in 2 mL wells in media comprising inactivated human AB serum with 6000 IU/mL of IL-2. This primary cell population is cultured for a period of days, generally from 3 to 14 days, resulting in a bulk TIL population, generally about $1 \times 10^8$ bulk TIL cells. In some embodiments, this primary cell population is cultured for a period of 7 to 14 days, resulting in a bulk TIL population, generally about $1 \times 10^8$ bulk TIL cells. In some embodiments, this primary cell population is cultured for a period of 10 to 14 days, resulting in a bulk TIL population, generally about $1 \times 10^8$ bulk TIL cells. In some embodiments, this primary cell population is cultured for a period of about 11 days, resulting in a bulk TIL population, generally about $1 \times 10^8$ bulk TIL cells.

In a preferred embodiment, expansion of TILs may be performed using an initial bulk TIL expansion step (for example such as those described in Step B of Figure 1, which can include processes referred to as pre-REP) as described below and herein, followed by a second expansion (Step D, including processes referred to as rapid expansion protocol (REP) steps) as described below under Step D and herein, followed by optional cryopreservation, and followed by a second Step D (including processes referred to as restimulation REP steps) as described below and herein. The TILs obtained from this process may be optionally characterized for phenotypic characteristics and metabolic parameters as described herein.

In embodiments where TIL cultures are initiated in 24-well plates, for example, using Costar 24-well cell culture cluster, flat bottom (Corning Incorporated, Corning, NY, each well can be seeded with $1 \times 10^6$ tumor digest cells or one tumor fragment in 2 mL of complete medium (CM) with IL-2 (6000 IU/mL; Chiron Corp., Emeryville, CA). In some embodiments, the tumor fragment is between about 1 mm$^3$ and 10 mm$^3$.

In some embodiments, the first expansion culture medium is referred to as "CM", an abbreviation for culture media. In some embodiments, CM for Step B consists of RPMI 1640 with GlutaMAX, supplemented with 10% human AB serum, 25 mM Hepes, and 10 μg/mL gentamicin. In embodiments where cultures are initiated in gas-permeable flasks with a 40 mL capacity and a 10 cm$^2$ gas-permeable silicon bottom (for example, G-Rex10; Wilson Wolf Manufacturing, New Brighton, MN) (Fig. 1), each flask was loaded with $10 \times 10^6$ to $40 \times 10^6$ viable tumor digest cells or 5 to 30 tumor fragments in 10 to 40 mL of CM with IL-2. Both the G-Rex10 and 24-well plates were incubated in a humidified incubator at 37°C in 5% CO$_2$ and 5 days after culture initiation, half the media was removed and replaced with fresh CM and IL-2 and after day 5, half the media was changed every 2 to 3 days.

After preparation of the tumor fragments, the resulting cells (*i.e.,* fragments) are cultured in serum containing IL-2 under conditions that favor the growth of TILs over tumor and other cells. In some embodiments, the tumor digests are incubated in 2 mL wells in media comprising inactivated human AB serum (or, in some cases, as outlined herein, in the presence of aAPC cell population) with 6000 IU/mL of IL-2. This primary cell population is cultured for a period of days, generally from 10 to 14 days, resulting in a bulk TIL population, generally about $1 \times 10^8$ bulk TIL cells. In some embodiments, the growth media during the first expansion comprises IL-2 or a variant thereof. In some embodiments, the IL is recombinant human IL-2 (rhIL-2). In some embodiments the IL-2 stock solution has a specific activity of $20\text{-}30 \times 10^6$ IU/mg for a 1 mg vial. In some embodiments the IL-2 stock solution has a specific activity of $20 \times 10^6$ IU/mg for a 1 mg vial. In some embodiments the IL-2 stock solution has a specific activity of $25 \times 10^6$ IU/mg for a 1 mg vial. In some embodiments the IL-2 stock solution has a specific activity of $30 \times 10^6$ IU/mg for a 1 mg vial. In some embodiments, the IL-2 stock solution has a final concentration of $4\text{-}8 \times 10^6$ IU/mg of IL-2. In some embodiments, the IL-2 stock solution has a final concentration of $5\text{-}7 \times 10^6$ IU/mg of IL-2. In some embodiments, the IL-2 stock solution has a final concentration of $6 \times 10^6$ IU/mg of IL-2. In some embodiments, the IL-2 stock solution is prepare as described in Example E. In some embodiments, the first expansion culture media comprises about 10,000 IU/mL of IL-2, about 9,000 IU/mL of IL-2, about 8,000 IU/mL of IL-2, about 7,000 IU/mL of IL-2, about 6000 IU/mL of IL-2 or about 5,000 IU/mL of IL-2. In some embodiments, the first expansion culture media comprises about 9,000 IU/mL of IL-2 to about 5,000 IU/mL of IL-2. In some embodiments, the first expansion culture media comprises about 8,000 IU/mL of IL-2 to about 6,000 IU/mL of IL-2. In some embodiments, the first expansion culture media comprises about 7,000 IU/mL of IL-2 to about 6,000 IU/mL of IL-2. In some embodiments, the first expansion culture media comprises about 6,000 IU/mL of IL-2. In an embodiment, the cell culture medium further comprises IL-2. In some embodiments, the cell culture medium comprises about 3000 IU/mL of IL-2. In an embodiment, the cell culture medium further comprises IL-2. In a preferred embodiment, the cell culture medium comprises about 3000 IU/mL of IL-2. In an embodiment, the cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL of IL-2. In an embodiment, the cell culture medium comprises between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or about 8000 IU/mL of IL-2.

In some embodiments, first expansion culture media comprises about 500 IU/mL of IL-15, about 400 IU/mL of IL-15, about 300 IU/mL of IL-15, about 200 IU/mL of IL-15, about 180 IU/mL of IL-15, about 160 IU/mL of IL-15, about 140 IU/mL of IL-15, about 120 IU/mL of IL-15, or about 100 IU/mL of IL-15. In some embodiments, the first expansion culture media comprises about 500 IU/mL of IL-15 to about 100 IU/mL of IL-15. In some embodiments, the first expansion culture media comprises about 400 IU/mL of IL-15 to about 100 IU/mL of IL-15. In some embodiments, the first expansion culture media comprises about 300 IU/mL of IL-15 to about 100 IU/mL of IL-15. In some embodiments, the first expansion culture media comprises about 200 IU/mL of IL-15. In some embodiments, the cell culture medium comprises about 180 IU/mL of IL-15. In an embodiment, the cell culture medium further comprises IL-15. In a preferred embodiment, the cell culture medium comprises about 180 IU/mL of IL-15.

In some embodiments, first expansion culture media comprises about 20 IU/mL of IL-21, about 15 IU/mL of IL-21, about 12 IU/mL of IL-21, about 10 IU/mL of IL-21, about 5 IU/mL of IL-21, about 4 IU/mL of IL-21, about 3 IU/mL of IL-21, about 2 IU/mL of IL-21, about 1 IU/mL of IL-21, or about 0.5 IU/mL of IL-21. In some embodiments, the first expansion culture media comprises about 20 IU/mL of IL-21 to about 0.5 IU/mL of IL-21. In some embodiments, the first expansion culture media comprises about 15 IU/mL of IL-21 to about 0.5 IU/mL of IL-21. In some embodiments, the first expansion culture media comprises about 12 IU/mL of IL-21 to about 0.5 IU/mL of IL-21. In some embodiments, the first expansion culture media comprises about 10 IU/mL of IL-21 to about 0.5 IU/mL of IL-21. In some embodiments, the first expansion culture media comprises about 5 IU/mL of IL-21 to about 1 IU/mL of IL-21. In some embodiments, the first expansion culture media comprises about 2 IU/mL of IL-21. In some embodiments, the cell culture medium comprises about 1 IU/mL of IL-21. In some embodiments, the cell culture medium comprises about 0.5 IU/mL of IL-21. In an embodiment, the cell culture medium further comprises IL-21. In a preferred embodiment, the cell culture medium comprises about 1 IU/mL of IL-21.

In an embodiment, the cell culture medium comprises OKT-3 antibody. In some embodiments, the cell culture medium comprises about 30 ng/mL of OKT-3 antibody. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 µg/mL of OKT-3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT-3 antibody. In some embodiments, the cell culture medium does not comprise OKT-3 antibody. In some embodiments, the OKT-3 antibody is muromonab.

In some embodiments, the cell culture medium comprises one or more TNFRSF agonists in a cell culture medium. In some embodiments, the TNFRSF agonist comprises a 4-1BB agonist. In some embodiments, the TNFRSF agonist is a 4-1BB agonist, and the 4-1BB agonist is selected from the group consisting of urelumab, utomilumab, EU-101, a fusion protein, and fragments, derivatives, variants, biosimilars, and combinations thereof. In some embodiments, the TNFRSF agonist is added at a concentration sufficient to achieve a concentration in the cell culture medium of between 0.1 µg/mL and 100 µg/mL. In some embodiments, the TNFRSF agonist is added at a concentration sufficient to achieve a concentration in the cell culture medium of between 20 µg/mL and 40 µg/mL.

In some embodiments, in addition to one or more TNFRSF agonists, the cell culture medium further comprises IL-2 at an initial concentration of about 3000 IU/mL and OKT-3 antibody at an initial concentration of about 30 ng/mL, and wherein the one or more TNFRSF agonists comprises a 4-1BB agonist.

In some embodiments, the first expansion culture medium is referred to as "CM", an abbreviation for culture media. In some embodiments, it is referred to as CM1 (culture medium 1). In some embodiments, CM consists of RPMI 1640 with GlutaMAX, supplemented with 10% human AB serum, 25 mM Hepes, and 10 mg/mL gentamicin. In embodiments where cultures are initiated in gas-permeable flasks with a 40 mL capacity and a 10cm$^2$ gas-permeable silicon bottom (for example, G-Rex10; Wilson Wolf Manufacturing, New Brighton, MN) (Fig. 1), each flask was loaded with 10 × 10$^6$ to 40 × 10$^6$ viable tumor digest cells or 5-30 tumor fragments in 10 to 40mL of CM with IL-2. Both the G-Rex10 and 24-well plates were incubated in a humidified incubator at 37°C in 5% $CO_2$ and 5 days after culture initiation, half the media was removed and replaced with fresh CM and IL-2 and after day 5, half the media was changed every 2 to 3 days. In some embodiments, the CM is the CM1 described in the Examples, see, Example 1. In some embodiments, the first expansion occurs in an initial cell culture medium or a first cell culture medium. In some embodiments, the initial cell culture medium or the first cell culture medium comprises IL-2.

In some embodiments, the first expansion (including processes such as for example those described in Step B of Figure 1, which can include those sometimes referred to as the pre-REP) process is shortened to 3 to 14 days, as discussed in the examples and figures. In some embodiments, the first expansion (including processes such as for example those described in Step B of Figure 1, which can include those sometimes referred to as the pre-REP) is shortened to 7 to 14 days, as discussed in the Examples and shown in Figures 4 and 5, as well as including for example, an expansion as described in Step B of Figure 1. In some embodiments, the first expansion of Step B is shortened to 10-14 days. In some embodiments, the first expansion is shortened to 11 days, as discussed in, for example, an expansion as described in Step B of Figure 1.

In some embodiments, the first TIL expansion can proceed for 1 day, 2 days, 3 days, 4 days, 5 days, 6 days, 7 days, 8 days, 9 days, 10 days, 11 days, 12 days, 13 days, or 14 days. In some embodiments, the first TIL expansion can proceed for 1 day to 14 days. In some embodiments, the first TIL expansion can proceed for 2 days to 14 days. In some embodiments, the first TIL expansion can proceed for 3 days to 14 days. In some embodiments, the first TIL expansion can proceed for 4 days to 14 days. In some embodiments, the first TIL expansion can proceed for 5 days to 14 days. In some embodiments, the first TIL expansion can proceed for 6 days to 14 days. In some embodiments, the first TIL expansion can proceed for 7 days to 14 days. In some embodiments, the first TIL expansion can proceed for 8 days to 14 days. In some embodiments, the first TIL expansion can proceed for 9 days to 14 days. In some embodiments, the first TIL expansion can proceed for 10 days to 14 days. In some embodiments, the first TIL expansion can proceed for 11 days to 14 days. In some embodiments, the first TIL expansion can proceed for 12 days to 14 days. In some embodiments, the first TIL expansion can proceed for 13 days to 14 days. In some embodiments, the first TIL expansion can proceed for 14 days. In some embodiments, the first TIL expansion can proceed for 1 day to 11 days. In some embodiments, the first TIL expansion can proceed for 2 days to 11 days. In some embodiments, the first TIL expansion can proceed for 3 days to 11 days. In some embodiments, the first TIL expansion can proceed for 4 days to 11 days. In some embodiments, the first TIL expansion can proceed for 5 days to 11 days. In some embodiments, the first TIL expansion can proceed for 6 days to 11 days. In some embodiments, the first TIL expansion can proceed for 7 days to 11 days. In some embodiments, the first TIL expansion can proceed for 8 days to 11 days. In some embodiments, the first TIL expansion can proceed for 9 days to 11 days. In some embodiments, the first TIL expansion can proceed for 10 days to 11 days. In some embodiments, the first TIL expansion can proceed for 11 days.

In some embodiments, a combination of IL-2, IL-7, IL-15, and/or IL-21 are employed as a combination during the first expansion. In some embodiments, IL-2, IL-7, IL-15, and/or IL-21 as well as any combinations thereof can be included during the first expansion, including for example during a Step B processes according to Figure 1, as well as described herein. In some embodiments, a combination of IL-2, IL-15, and IL-21 are employed as a combination during the first expansion. In some embodiments, IL-2, IL-15, and IL-21 as well as any combinations thereof can be included during Step B processes according to Figure 1 and as described herein.

In some embodiments, the first expansion (including processes referred to as the pre-REP; for example, Step B according to Figure 1) process is shortened to 3 to 14 days, as discussed in the examples and figures. In some embodiments, the first expansion of Step B is shortened to 7 to 14 days. In some embodiments, the first expansion of Step B is shortened to 10 to 14 days. In some embodiments, the first expansion is shortened to 11 days.

In some embodiments, the first expansion, for example, Step B according to Figure 1, is performed in a closed system bioreactor. In some embodiments, a closed system is employed for the TIL expansion, as described herein. In some embodiments, a single bioreactor is employed. In some embodiments, the single bioreactor employed is for example a G-REX -10 or a G-REX - 100. In some embodiments, the closed system bioreactor is a single bioreactor.

*Step C: First Expansion to Second Expansion Transition*

In some cases, the bulk TIL population obtained from the first expansion, including for example the TIL population obtained from for example, Step B as indicated in Figure 1, can be cryopreserved immediately, using the protocols discussed herein below. Alternatively, the TIL population obtained from the first expansion, referred to as the second TIL population, can be subjected to a second expansion (which can include expansions sometimes referred to as REP) and then cryopreserved as discussed below. Similarly, in the case where genetically modified TILs will be used in therapy, the first TIL population (sometimes referred to as the bulk TIL population) or the second TIL population (which can in some embodiments include populations referred to as the REP TIL populations) can be subjected to genetic modifications for suitable treatments prior to expansion or after the first expansion and prior to the second expansion.

In some embodiments, the TILs obtained from the first expansion (for example, from Step B as indicated in Figure 1) are stored until phenotyped for selection. In some embodiments, the TILs obtained from the first expansion (for example, from Step B as indicated in Figure 1) are not stored and proceed directly to the second expansion. In some embodiments, the TILs obtained from the first expansion are not cryopreserved after the first expansion and prior to the second expansion. In some embodiments, the transition from the first expansion to the second expansion occurs at about 3 days, 4, days, 5 days, 6 days, 7 days, 8 days, 9 days, 10 days, 11 days, 12 days, 13 days, or 14 days from when fragmentation occurs. In some embodiments, the transition from the first expansion to the second expansion occurs at about 3 days to 14 days from when fragmentation occurs. In some embodiments, the transition from the first expansion to the second expansion occurs at about 4 days to 14 days from when fragmentation occurs. In some embodiments, the transition from the first expansion to the second expansion occurs at about 4 days to 10 days from when fragmentation occurs. In some embodiments, the transition from the first expansion to the second expansion occurs at about 7 days to 14 days from when fragmentation occurs. In some embodiments, the transition from the first expansion to the second expansion occurs at about 14 days from when fragmentation occurs.

In some embodiments, the transition from the first expansion to the second expansion occurs at 1 day, 2 days, 3 days, 4 days, 5 days, 6 days, 7 days, 8 days, 9 days, 10 days, 11 days, 12 days, 13 days, or 14 days from when fragmentation occurs. In some embodiments, the transition from the first expansion to the second expansion occurs 1 day to 14 days from when fragmentation occurs. In some embodiments, the first TIL expansion can proceed for 2 days to 14 days. In some embodiments, the transition from the first expansion to the second expansion occurs 3 days to 14 days from when fragmentation occurs. In some embodiments, the transition from the first expansion to the second expansion occurs 4 days to 14 days from when fragmentation occurs. In some embodiments, the transition from the first expansion to the second expansion occurs 5 days to 14 days from when fragmentation occurs. In some embodiments, the transition from the first expansion to the second expansion occurs 6 days to 14 days from when fragmentation occurs. In some embodiments, the transition from the first expansion to the second expansion occurs 7 days to 14 days from when fragmentation occurs. In some embodiments, the transition from the first expansion to the second expansion occurs 8 days to 14 days from when fragmentation occurs. In some embodiments, the transition from the first expansion to the second expansion occurs 9 days to 14 days from when fragmentation occurs. In some embodiments, the transition from the first expansion to the second expansion occurs 10 days to 14 days from when fragmentation occurs. In some embodiments, the transition from the first expansion to the second expansion occurs 11 days to 14 days from when fragmentation occurs. In some embodiments, the transition from the first expansion to the second expansion occurs 12 days to 14 days from when fragmentation occurs. In some embodiments, the transition from the first expansion to the second expansion occurs 13 days to 14 days from when fragmentation occurs. In some embodiments, the transition from the first expansion to the second expansion occurs 14 days from when fragmentation occurs. In some embodiments, the transition from the first expansion to the second expansion occurs 1 day to 11 days from when fragmentation occurs. In some embodiments, the transition from the first expansion to the second expansion occurs 2 days to 11 days from when fragmentation occurs. In some embodiments, the transition from the first expansion to the second expansion occurs 3 days to 11 days from when fragmentation occurs. In some embodiments, the transition from the first expansion to the second expansion occurs 4 days to 11 days from when fragmentation occurs. In some embodiments, the transition from the first expansion to the second expansion occurs 5 days to 11 days from when fragmentation occurs. In some embodiments, the transition from the first expansion to the second expansion occurs 6 days to 11 days from when fragmentation occurs. In some embodiments, the transition from the first expansion to the second expansion occurs 7 days to 11 days from when fragmentation occurs. In some embodiments, the transition from the first expansion to the second expansion occurs 8 days to 11 days from when fragmentation occurs. In some embodiments, the transition from the first expansion to the second expansion occurs 9 days to 11 days from when fragmentation occurs. In some embodiments, the transition from the first expansion to the second expansion occurs 10 days to 11 days from when fragmentation occurs. In some embodiments, the transition from the first expansion to the second expansion occurs 11 days from when fragmentation occurs.

In some embodiments, the TILs are not stored after the first expansion and prior to the second expansion, and the TILs proceed directly to the second expansion (for example, in some embodiments, there is no storage during the transition from Step B to Step D as shown in Figure 1). In some embodiments, the transition occurs in closed system, as described herein. In some embodiments, the TILs from the first expansion, the second population of TILs, proceeds directly into the second expansion with no transition period.

In some embodiments, the transition from the first expansion to the second expansion, for example, Step C according to Figure 1, is performed in a closed system bioreactor. In some embodiments, a closed system is employed for the TIL expansion, as described herein. In some embodiments, a single bioreactor is employed. In some embodiments, the single bioreactor employed is for example a G-REX-10 or a G-REX-100. In some embodiments, the closed system bioreactor is a single bioreactor.

*Cytokines*

The expansion methods described herein generally use culture media with high doses of a cytokine, in particular IL-2, as is known in the art.

Alternatively, using combinations of cytokines for the rapid expansion and or second expansion of TILs is additionally possible, with combinations of two or more of IL-2, IL-15 and IL-21 as is generally outlined in International Publication No. WO 2015/189356 and W International Publication No. WO 2015/189357. Thus, possible combinations include IL-2 and IL-15, IL-2 and IL-21, IL-15 and IL-21 and IL-2, IL-15 and IL-21, with the latter finding particular use in many embodiments. The use of combinations of cytokines specifically favors the generation of lymphocytes, and in particular T-cells as described therein.

*Step D: Second Expansion*

In some embodiments, the TIL cell population is expanded in number after harvest and initial bulk processing for example, after Step A and Step B, and the transition referred to as Step C, as indicated in Figure 1). This further expansion is referred to herein as the second expansion, which can include expansion processes generally referred to in the art as a rapid expansion process (REP; as well as processes as indicated in Step D of Figure 1). The second expansion is generally accomplished using a culture media comprising a number of components, including feeder cells, a cytokine source, and an anti-CD3 antibody, in a gas-permeable container.

In some embodiments, the second expansion or second TIL expansion (which can include expansions sometimes referred to as REP; as well as processes as indicated in Step D of Figure 1) of TIL can be performed using any TIL flasks or containers known by those of skill in the art. In some embodiments, the second TIL expansion can proceed for 7 days, 8 days, 9 days, 10 days, 11 days, 12 days, 13 days, or 14 days. In some embodiments, the second TIL expansion can proceed for about 7 days to about 14 days. In some embodiments, the second TIL expansion can proceed for about 8 days to about 14 days. In some embodiments, the second TIL expansion can proceed for about 9 days to about 14 days. In some embodiments, the second TIL expansion can proceed for about 10 days to about 14 days. In some embodiments, the second TIL expansion can proceed for about 11 days to about 14 days. In some embodiments, the second TIL expansion can proceed for about 12 days to about 14 days. In some embodiments, the second TIL expansion can proceed for about 13 days to about 14 days. In some embodiments, the second TIL expansion can proceed for about 14 days.

In an embodiment, the second expansion can be performed in a gas permeable container using the methods of the present disclosure (including for example, expansions referred to as REP; as well as processes as indicated in Step D of Figure 1). For example, TILs can be rapidly expanded using non-specific T-cell receptor stimulation in the presence of interleukin-2 (IL-2) or interleukin-15 (IL-15). The non-specific T-cell receptor stimulus can include, for example, an anti-CD3 antibody, such as about 30 ng/ml of OKT3, a mouse monoclonal anti-CD3 antibody (commercially available from Ortho-McNeil, Raritan, NJ or Miltenyi Biotech, Auburn, CA) or UHCT-1 (commercially available from BioLegend, San Diego, CA, USA). TILs can be expanded to induce further stimulation of the TILs in vitro by including one or more antigens during the second expansion, including antigenic portions thereof, such as epitope(s), of the cancer, which can be optionally expressed from a vector, such as a human leukocyte antigen A2 (HLA-A2) binding peptide, *e.g.,* 0.3 $\mu$M MART-1 :26-35 (27 L) or gpl 00:209-217 (210M), optionally in the presence of a T-cell growth factor, such as 300 IU/mL IL-2 or IL-15. Other suitable antigens may include, *e.g.,* NY-ESO-1, TRP-1, TRP-2, tyrosinase cancer antigen, MAGE-A3, SSX-2, and VEGFR2, or antigenic portions thereof. TIL may also be rapidly expanded by re-stimulation with the same antigen(s) of the cancer pulsed onto HLA-A2-expressing antigen-presenting cells. Alternatively, the TILs can be further re-stimulated with, *e.g.,* example, irradiated, autologous lymphocytes or with irradiated HLA-A2$^+$ allogeneic lymphocytes and IL-2. In some embodiments, the re-stimulation occurs as part of the second expansion. In some embodiments, the second expansion occurs in the presence of irradiated, autologous lymphocytes or with irradiated HLA-A2$^+$ allogeneic lymphocytes and IL-2.

In an embodiment, the cell culture medium further comprises IL-2. In some embodiments, the cell culture medium comprises about 3000 IU/mL of IL-2. In an embodiment, the cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL of IL-2. In an embodiment, the cell culture medium comprises between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or between 8000 IU/mL of IL-2.

In an embodiment, the cell culture medium comprises OKT-3 antibody. In some embodiments, the cell culture medium comprises about 30 ng/mL of OKT-3 antibody. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 $\mu$g/mL of OKT-3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT-3 antibody. In some embodiments, the cell culture medium does not comprise OKT-3 antibody. In some embodiments, the OKT-3 antibody is muromonab.

In some embodiments, the cell culture medium comprises one or more TNFRSF agonists in a cell culture medium. In some embodiments, the TNFRSF agonist comprises a 4-1BB agonist. In some embodiments, the TNFRSF agonist is a 4-1BB agonist, and the 4-1BB agonist is selected from the group consisting of urelumab, utomilumab, EU-101, a fusion protein, and fragments, derivatives, variants, biosimilars, and combinations thereof. In some embodiments, the TNFRSF agonist is added at a concentration sufficient to achieve a concentration in the cell culture medium of between 0.1 µg/mL and 100 µg/mL. In some embodiments, the TNFRSF agonist is added at a concentration sufficient to achieve a concentration in the cell culture medium of between 20 µg/mL and 40 µg/mL.

In some embodiments, in addition to one or more TNFRSF agonists, the cell culture medium further comprises IL-2 at an initial concentration of about 3000 IU/mL and OKT-3 antibody at an initial concentration of about 30 ng/mL, and wherein the one or more TNFRSF agonists comprises a 4-1BB agonist.

In some embodiments, a combination of IL-2, IL-7, IL-15, and/or IL-21 are employed as a combination during the second expansion. In some embodiments, IL-2, IL-7, IL-15, and/or IL-21 as well as any combinations thereof can be included during the second expansion, including for example during a Step D processes according to Figure 1, as well as described herein. In some embodiments, a combination of IL-2, IL-15, and IL-21 are employed as a combination during the second expansion. In some embodiments, IL-2, IL-15, and IL-21 as well as any combinations thereof can be included during Step D processes according to Figure 1 and as described herein.

In some embodiments, the second expansion can be conducted in a supplemented cell culture medium comprising IL-2, OKT-3, antigen-presenting feeder cells, and optionally a TNFRSF agonist. In some embodiments, the second expansion occurs in a supplemented cell culture medium. In some embodiments, the supplemented cell culture medium comprises IL-2, OKT-3, and antigen-presenting feeder cells. In some embodiments, the second cell culture medium comprises IL-2, OKT-3, and antigen-presenting cells (APCs; also referred to as antigen-presenting feeder cells). In some embodiments, the second expansion occurs in a cell culture medium comprising IL-2, OKT-3, and antigen-presenting feeder cells (*i.e.,* antigen presenting cells).

In some embodiments, the second expansion culture media comprises about 500 IU/mL of IL-15, about 400 IU/mL of IL-15, about 300 IU/mL of IL-15, about 200 IU/mL of IL-15, about 180 IU/mL of IL-15, about 160 IU/mL of IL-15, about 140 IU/mL of IL-15, about 120 IU/mL of IL-15, or about 100 IU/mL of IL-15. In some embodiments, the second expansion culture media comprises about 500 IU/mL of IL-15 to about 100 IU/mL of IL-15. In some embodiments, the second expansion culture media comprises about 400 IU/mL of IL-15 to about 100 IU/mL of IL-15. In some embodiments, the second expansion culture media comprises about 300 IU/mL of IL-15 to about 100 IU/mL of IL-15. In some embodiments, the second expansion culture media comprises about 200 IU/mL of IL-15. In some embodiments, the cell culture medium comprises about 180 IU/mL of IL-15. In an embodiment, the cell culture medium further comprises IL-15. In a preferred embodiment, the cell culture medium comprises about 180 IU/mL of IL-15.

In some embodiments, the second expansion culture media comprises about 20 IU/mL of IL-21, about 15 IU/mL of IL-21, about 12 IU/mL of IL-21, about 10 IU/mL of IL-21, about 5 IU/mL of IL-21, about 4 IU/mL of IL-21, about 3 IU/mL of IL-21, about 2 IU/mL of IL-21, about 1 IU/mL of IL-21, or about 0.5 IU/mL of IL-21. In some embodiments, the second expansion culture media comprises about 20 IU/mL of IL-21 to about 0.5 IU/mL of IL-21. In some embodiments, the second expansion culture media comprises about 15 IU/mL of IL-21 to about 0.5 IU/mL of IL-21. In some embodiments, the second expansion culture media comprises about 12 IU/mL of IL-21 to about 0.5 IU/mL of IL-21. In some embodiments, the second expansion culture media comprises about 10 IU/mL of IL-21 to about 0.5 IU/mL of IL-21. In some embodiments, the second expansion culture media comprises about 5 IU/mL of IL-21 to about 1 IU/mL of IL-21. In some embodiments, the second expansion culture media comprises about 2 IU/mL of IL-21. In some embodiments, the cell culture medium comprises about 1 IU/mL of IL-21. In some embodiments, the cell culture medium comprises about 0.5 IU/mL of IL-21. In an embodiment, the cell culture medium further comprises IL-21. In a preferred embodiment, the cell culture medium comprises about 1 IU/mL of IL-21.

In some embodiments the antigen-presenting feeder cells (APCs) are PBMCs. In an embodiment, the ratio of TILs to PBMCs and/or antigen-presenting cells in the rapid expansion and/or the second expansion is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500. In an embodiment, the ratio of TILs to PBMCs in the rapid expansion and/or the second expansion is between 1 to 50 and 1 to 300. In an embodiment, the ratio of TILs to PBMCs in the rapid expansion and/or the second expansion is between 1 to 100 and 1 to 200.

In an embodiment, REP and/or the second expansion is performed in flasks with the bulk TILs being mixed with a 100- or 200-fold excess of inactivated feeder cells, 30 mg/mL OKT3 anti-CD3 antibody and 3000 IU/mL IL-2 in 150 ml media. Media replacement is done (generally 2/3 media replacement via respiration with fresh media) until the cells are transferred to an alternative growth chamber. Alternative growth chambers include G-REX flasks and gas permeable containers as more fully discussed below.

In some embodiments, the second expansion (which can include processes referred to as the REP process) is shortened to 7-14 days, as discussed in the examples and figures. In some embodiments, the second expansion is shortened to 11 days.

In an embodiment, REP and/or the second expansion may be performed using T-175 flasks and gas permeable bags as previously described (Tran, et al., J. Immunother, 2008, 31, 742-51; Dudley, et al., J. Immunother, 2003, 26, 332-42) or gas permeable cultureware (G-Rex flasks). In some embodiments, the second expansion (including expansions referred to as rapid expansions) is performed in T-175 flasks, and about $1 \times 10^6$ TILs suspended in 150 mL of media may be added to each T-175 flask. The TILs may be cultured in a 1 to 1 mixture of CM and AIM-V medium, supplemented with 3000 IU per mL of IL-2 and 30 ng per ml of anti-CD3. The T-175 flasks may be incubated at 37° C in 5% $CO_2$. Half the media may be exchanged on day 5 using 50/50 medium with 3000 IU per mL of IL-2. In some embodiments, on day 7 cells from two T-175 flasks may be combined in a 3 L bag and 300 mL of AIM V with 5% human AB serum and 3000 IU per mL of IL-2 was added to the 300 ml of TIL suspension. The number of cells in each bag was counted every day or two and fresh media was added to keep the cell count between 0.5 and $2.0 \times 10^6$ cells/mL.

In an embodiment, the second expansion (which can include expansions referred to as REP, as well as those referred to in Step D of Figure 1) may be performed in 500 mL capacity gas permeable flasks with 100 cm gas-permeable silicon bottoms (G-Rex 100, commercially available from Wilson Wolf Manufacturing Corporation, New Brighton, MN, USA), $5 \times 10^6$ or $10 \times 10^6$ TIL may be cultured with PBMCs in 400 mL of 50/50 medium, supplemented with 5% human AB serum, 3000 IU per mL of IL-2 and 30 ng per ml of anti-CD3 (OKT3). The G-Rex 100 flasks may be incubated at 37°C in 5% $CO_2$. On day 5, 250 mL of supernatant may be removed and placed into centrifuge bottles and centrifuged at 1500 rpm (491 $\times$ g) for 10 minutes. The TIL pellets may be re-suspended with 150 mL of fresh medium with 5% human AB serum, 3000 IU per mL of IL-2, and added back to the original G-Rex 100 flasks. When TIL are expanded serially in G-Rex 100 flasks, on day 7 the TIL in each G-Rex 100 may be suspended in the 300 mL of media present in each flask and the cell suspension may be divided into 3 100 mL aliquots that may be used to seed 3 G-Rex 100 flasks. Then 150 mL of AIM-V with 5% human AB serum and 3000 IU per mL of IL-2 may be added to each flask. The G-Rex 100 flasks may be incubated at 37° C in 5% $CO_2$ and after 4 days 150 mL of AIM-V with 3000 IU per mL of IL-2 may be added to each G-REX 100 flask. The cells may be harvested on day 14 of culture.

In an embodiment, the second expansion (including expansions referred to as REP) is performed in flasks with the bulk TILs being mixed with a 100- or 200-fold excess of inactivated feeder cells, 30 mg/mL OKT3 anti-CD3 antibody and 3000 IU/mL IL-2 in 150 ml media. In some embodiments, media replacement is done until the cells are transferred to an alternative growth chamber. In some embodiments, 2/3 of the media is replaced by respiration with fresh media. In some embodiments, alternative growth chambers include G-REX flasks and gas permeable containers as more fully discussed below.

In an embodiment, the second expansion (including expansions referred to as REP) is performed and further comprises a step wherein TILs are selected for superior tumor reactivity. Any selection method known in the art may be used. For example, the methods described in U.S. Patent Application Publication No. 2016/0010058 A1 may be used for selection of TILs for superior tumor reactivity.

Optionally, a cell viability assay can be performed after the second expansion (including expansions referred to as the REP expansion), using standard assays known in the art. For example, a trypan blue exclusion assay can be done on a sample of the bulk TILs, which selectively labels dead cells and allows a viability assessment. In some embodiments, TIL samples can be counted and viability determined using a Cellometer K2 automated cell counter (Nexcelom Bioscience, Lawrence, MA). In some embodiments, viability is determined according to the standard Cellometer K2 Image Cytometer Automatic Cell Counter protocol.

In some embodiments, the second expansion (including expansions referred to as REP) of TIL can be performed using T-175 flasks and gas-permeable bags as previously described (Tran KQ, Zhou J, Durflinger KH, et al., 2008, J Immunother., 31:742-751, and Dudley ME, Wunderlich JR, Shelton TE, et al. 2003, J Immunother., 26:332-342) or gas-permeable G-Rex flasks. In some embodiments, the second expansion is performed using flasks. In some embodiments, the second expansion is performed using gas-permeable G-Rex flasks. In some embodiments, the second expansion is performed in T-175 flasks, and about $1 \times 10^6$ TIL are suspended in about 150 mL of media and this is added to each T-175 flask. The TIL are cultured with irradiated (50 Gy) allogeneic PBMC as "feeder" cells at a ratio of 1 to 100 and the cells were cultured in a 1 to 1 mixture of CM and AIM-V medium (50/50 medium), supplemented with 3000 IU/mL of IL-2 and 30 ng/mL of anti-CD3. The T-175 flasks are incubated at 37°C in 5% $CO_2$. In some embodiments, half the media is changed on day 5 using 50/50 medium with 3000 IU/mL of IL-2. In some embodiments, on day 7, cells from 2 T-175 flasks are combined in a 3 L bag and 300 mL of AIM-V with 5% human AB serum and 3000 IU/mL of IL-2 is added to the 300 mL of TIL suspension. The number of cells in each bag can be counted every day or two and fresh media can be added to keep the cell count between about 0.5 and about $2.0 \times 10^6$ cells/mL.

In some embodiments, the second expansion (including expansions referred to as REP) are performed in 500 mL capacity flasks with 100 $cm^2$ gas-permeable silicon bottoms (G-Rex 100, Wilson Wolf) (Fig. 1), about $5 \times 10^6$ or $10 \times 10^6$ TIL are cultured with irradiated allogeneic PBMC at a ratio of 1 to 100 in 400 mL of 50/50 medium, supplemented with 3000 IU/mL of IL-2 and 30 ng/ mL of anti-CD3. The G-Rex 100 flasks are incubated at 37°C in 5% $CO_2$. In some

embodiments, on day 5, 250mL of supernatant is removed and placed into centrifuge bottles and centrifuged at 1500 rpm (491g) for 10 minutes. The TIL pellets can then be resuspended with 150 mL of fresh 50/50 medium with 3000 IU/mL of IL-2 and added back to the original G-Rex 100 flasks. In embodiments where TILs are expanded serially in G-Rex 100 flasks, on day 7 the TIL in each G-Rex 100 are suspended in the 300 mL of media present in each flask and the cell suspension was divided into three 100 mL aliquots that are used to seed 3 G-Rex 100 flasks. Then 150 mL of AIM-V with 5% human AB serum and 3000 IU/mL of IL-2 is added to each flask. The G-Rex 100 flasks are incubated at 37°C in 5% $CO_2$ and after 4 days 150 mL of AIM-V with 3000 IU/mL of IL-2 is added to each G-Rex 100 flask. The cells are harvested on day 14 of culture.

The diverse antigen receptors of T and B lymphocytes are produced by somatic recombination of a limited, but large number of gene segments. These gene segments: V (variable), D (diversity), J (joining), and C (constant), determine the binding specificity and downstream applications of immunoglobulins and T-cell receptors (TCRs). The present invention provides a method for generating TILs which exhibit and increase the T-cell repertoire diversity. In some embodiments, the TILs obtained by the present method exhibit an increase in the T-cell repertoire diversity. In some embodiments, the TILs obtained in the second expansion exhibit an increase in the T-cell repertoire diversity. In some embodiments, the increase in diversity is an increase in the immunoglobulin diversity and/or the T-cell receptor diversity. In some embodiments, the diversity is in the immunoglobulin is in the immunoglobulin heavy chain. In some embodiments, the diversity is in the immunoglobulin is in the immunoglobulin light chain. In some embodiments, the diversity is in the T-cell receptor. In some embodiments, the diversity is in one of the T-cell receptors selected from the group consisting of alpha, beta, gamma, and delta receptors. In some embodiments, there is an increase in the expression of T-cell receptor (TCR) alpha and/or beta. In some embodiments, there is an increase in the expression of T-cell receptor (TCR) alpha. In some embodiments, there is an increase in the expression of T-cell receptor (TCR) beta. In some embodiments, there is an increase in the expression of TCRab (*i.e.*, TCR$\alpha/\beta$).

In some embodiments, the second expansion culture medium (*e.g.*, sometimes referred to as CM2 or the second cell culture medium), comprises IL-2, OKT-3, as well as the antigen-presenting feeder cells (APCs), as discussed in more detail below.

In some embodiments, the second expansion, for example, Step D according to Figure 1, is performed in a closed system bioreactor. In some embodiments, a closed system is employed for the TIL expansion, as described herein. In some embodiments, a single bioreactor is employed. In some embodiments, the single bioreactor employed is for example a G-REX -10 or a G-REX-100. In some embodiments, the closed system bioreactor is a single bioreactor.

*Feeder Cells and Antigen Presenting Cells*

In an embodiment, the second expansion procedures described herein (for example including expansion such as those described in Step D from Figure 1, as well as those referred to as REP) require an excess of feeder cells during REP TIL expansion and/or during the second expansion. In many embodiments, the feeder cells are peripheral blood mononuclear cells (PBMCs) obtained from standard whole blood units from healthy blood donors. The PBMCs are obtained using standard methods such as Ficoll-Paque gradient separation, *see, e.g.* "Isolation of mononuclear cells: Methodology and Application", GE Life Sciences technical publication 18-1152-69-AE, available at https://us.vwr.com/assetsvc/asset/en_US/id/16286835/contents.

In general, the allogenic PBMCs are inactivated, either via irradiation or heat treatment, and used in the REP procedures, as described in the examples, which provides an exemplary protocol for evaluating the replication incompetence of irradiate allogeneic PBMCs.

In some embodiments, PBMCs are considered replication incompetent and accepted for use in the TIL expansion procedures described herein if the total number of viable cells on day 14 is less than the initial viable cell number put into culture on day 0 of the REP and/or day 0 of the second expansion (*i.e.,* the start day of the second expansion).

In some embodiments, PBMCs are considered replication incompetent and accepted for use in the TIL expansion procedures described herein if the total number of viable cells, cultured in the presence of OKT3 and IL-2, on day 7 and day 14 has not increased from the initial viable cell number put into culture on day 0 of the REP and/or day 0 of the second expansion (*i.e.*, the start day of the second expansion). In some embodiments, the PBMCs are cultured in the presence of 30 ng/ml OKT3 antibody and 3000 IU/ml IL-2.

In some embodiments, PBMCs are considered replication incompetent and accepted for use in the TIL expansion procedures described herein if the total number of viable cells, cultured in the presence of OKT3 and IL-2, on day 7 and day 14 has not increased from the initial viable cell number put into culture on day 0 of the REP and/or day 0 of the second expansion (*i.e.*, the start day of the second expansion). In some embodiments, the PBMCs are cultured in the presence of 5 to 60 ng/ml OKT3 antibody and 1000-6000 IU/ml IL-2. In some embodiments, the PBMCs are cultured in the presence of 10 to 50 ng/ml OKT3 antibody and 2000-5000 IU/ml IL-2. In some embodiments, the PBMCs are cultured in the presence of 20 to 40 ng/ml OKT3 antibody and 2000-4000 IU/ml IL-2. In some embodiments, the PBMCs are cultured in the presence of 25 to 35 ng/ml OKT3 antibody and 2500-3500 IU/ml IL-2.

In some embodiments, the antigen-presenting feeder cells are PBMCs. In some embodiments, the antigen-presenting feeder cells are artificial antigen-presenting feeder cells. In an embodiment, the ratio of TILs to antigen-presenting feeder cells in the second expansion is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500. In an embodiment, the ratio of TILs to antigen-presenting feeder cells in the second expansion is between 1 to 50 and 1 to 300. In an embodiment, the ratio of TILs to antigen-presenting feeder cells in the second expansion is between 1 to 100 and 1 to 200.

In an embodiment, the second expansion procedures described herein require a ratio of about $2.5 \times 10^9$ feeder cells to about $100 \times 10^6$ TILs. In another embodiment, the second expansion procedures described herein require a ratio of about $2.5 \times 10^9$ feeder cells to about $50 \times 10^6$ TILs. In yet another embodiment, the second expansion procedures described herein require about $2.5 \times 10^9$ feeder cells to about $25 \times 10^6$ TILs.

In an embodiment, the second expansion procedures described herein require an excess of feeder cells during the second expansion. In many embodiments, the feeder cells are peripheral blood mononuclear cells (PBMCs) obtained from standard whole blood units from healthy blood donors. The PBMCs are obtained using standard methods such as Ficoll-Paque gradient separation. In an embodiment, artificial antigen-presenting (aAPC) cells are used in place of PBMCs.

In general, the allogenic PBMCs are inactivated, either via irradiation or heat treatment, and used in the TIL expansion procedures described herein, including the exemplary procedures described in the figures and examples.

In an embodiment, artificial antigen presenting cells are used in the second expansion as a replacement for, or in combination with, PBMCs.

*Cytokines*

The TIL expansion methods described herein generally use culture media with high doses of a cytokine, in particular IL-2, as is known in the art.

Alternatively, using combinations of cytokines for the rapid expansion and or second expansion of TILs is additionally possible, with combinations of two or more of IL-2, IL-15 and IL-21 as is generally outlined in International Publication No. WO 2015/189356 and W International Publication No. WO 2015/189357. Thus, possible combinations include IL-2 and IL-15, IL-2 and IL-21, IL-15 and IL-21 and IL-2, IL-15 and IL-21, with the latter finding particular use in many embodiments. The use of combinations of cytokines specifically favors the generation of lymphocytes, and in particular T-cells as described therein.

*Step E: Harvest TILs*

After the second expansion step, cells can be harvested. In some embodiments the TILs are harvested after one, two, three, four or more expansion steps, for example as provided in Figure 1. In some embodiments the TILs are harvested after two expansion steps, for example as provided in Figure 1.

TILs can be harvested in any appropriate and sterile manner, including for example by centrifugation. Methods for TIL harvesting are well known in the art and any such know methods can be employed with the present process. In some embodiments, TILS are harvest using an automated system.

Cell harvesters and/or cell processing systems are commercially available from a variety of sources, including, for example, Fresenius Kabi, Tomtec Life Science, Perkin Elmer, and Inotech Biosystems International, Inc. Any cell based harvester can be employed with the present methods. In some embodiments, the cell harvester and/or cell processing systems is a membrane-based cell harvester. In some embodiments, cell harvesting is via a cell processing system, such as the LOVO system (manufactured by Fresenius Kabi). The term "LOVO cell processing system" also refers to any instrument or device manufactured by any vendor that can pump a solution comprising cells through a membrane or filter such as a spinning membrane or spinning filter in a sterile and/or closed system environment, allowing for continuous flow and cell processing to remove supernatant or cell culture media without pelletization. In some embodiments, the cell harvester and/or cell processing system can perform cell separation, washing, fluid-exchange, concentration, and/or other cell processing steps in a closed, sterile system.

In some embodiments, the harvest, for example, Step E according to Figure 1, is performed from a closed system bioreactor. In some embodiments, a closed system is employed for the TIL expansion, as described herein. In some embodiments, a single bioreactor is employed. In some embodiments, the single bioreactor employed is for example a G-REX -10 or a G-REX -100. In some embodiments, the closed system bioreactor is a single bioreactor.

In some embodiments, Step E according to Figure 1, is performed according to the processes described in Example 7. In some embodiments, the closed system is accessed via syringes under sterile conditions in order to maintain the sterility and closed nature of the system. In some embodiments, a closed system, as described in Example 7, is employed.

In some embodiments, TILs are harvested according to the methods described in Example 7. In some embodiments, TILs between days 1 and 11 are harvested using the methods as described herein (referred to as the Day 11 TIL harvest in Example 7). In some embodiments, TILs between days 12 and 22 are harvested using the methods as described herein (referred to as the Day 22 TIL harvest in Example 7).

*Step F: Final Formulation/ Transfer to Infusion Bag*

After Steps A through E as provided in an exemplary order in Figure 1 and as outlined in detailed above and herein are complete, cells are transferred to a container for use in administration to a patient. In some embodiments, once a therapeutically sufficient number of TILs are obtained using the expansion methods described above, they are transferred to a container for use in administration to a patient.

Disclosed herein but not claimed are methods wherein TILs expanded using APCs of the present disclosure are administered to a patient as a pharmaceutical composition. In an embodiment, the pharmaceutical composition is a suspension of TILs in a sterile buffer. Disclosed herein but not claimed are methods wherein TILs expanded using PBMCs of the present disclosure may be administered by any suitable route as known in the art. Disclosed herein but not claimed are methods wherein the T-cells are administered as a single intra-arterial or intravenous infusion, which preferably lasts approximately 30 to 60 minutes. Other suitable routes of administration include intraperitoneal, intrathecal, and intralymphatic.

Anti-CD3 Antibodies as Optional Media Components

In some embodiments, the culture media used in expansion methods described herein (including those referred to as REP, see for example, Figure 1) also includes an anti-CD3 antibody. An anti-CD3 antibody in combination with IL-2 induces T cell activation and cell division in the TIL population. This effect can be seen with full length antibodies as well as Fab and F(ab')2 fragments, with the former being generally preferred; *see, e.g.,* Tsoukas et al., J. Immunol., 1985, 135, 1719.

As will be appreciated by those in the art, there are a number of suitable anti-human CD3 antibodies that find use in the invention, including anti-human CD3 polyclonal and monoclonal antibodies from various mammals, including, but not limited to, murine, human, primate, rat, and canine antibodies. In particular embodiments, the OKT3 anti-CD3 antibody muromonab (including the embodiments shown in Table 1) is used (commercially available from Ortho-McNeil, Raritan, NJ or Miltenyi Biotech, Auburn, CA). Anti-CD3 antibodies also include the UHCT1 clone, also known as T3 and CD3ε. Other anti-CD3 antibodies include, for example, otelixizumab, teplizumab, and visilizumab.

4-1BB (CD137) Agonists as Optional Media Components

In an embodiment, the TNFRSF agonist is a 4-1BB (CD137) agonist. The 4-1BB agonist may be any 4-1BB binding molecule known in the art. The 4-1BB binding molecule may be a monoclonal antibody or fusion protein capable of binding to human or mammalian 4-1BB. The 4-1BB agonists or 4-1BB binding molecules may comprise an immunoglobulin heavy chain of any isotype (*e.g.,* IgG, IgE, IgM, IgD, IgA, and IgY), class (*e.g.,* IgG1, IgG2, IgG3, IgG4, IgA1 and IgA2) or subclass of immunoglobulin molecule. The 4-1BB agonist or 4-1BB binding molecule may have both a heavy and a light chain. As used herein, the term binding molecule also includes antibodies (including full length antibodies), monoclonal antibodies (including full length monoclonal antibodies), polyclonal antibodies, multispecific antibodies (*e.g.*, bispecific antibodies), human, humanized or chimeric antibodies, and antibody fragments, *e.g.*, Fab fragments, F(ab') fragments, fragments produced by a Fab expression library, epitope-binding fragments of any of the above, and engineered forms of antibodies, *e.g.*, scFv molecules, that bind to 4-1BB. In an embodiment, the 4-1BB agonist is an antigen binding protein that is a fully human antibody. In an embodiment, the 4-1BB agonist is an antigen binding protein that is a humanized antibody. In some embodiments, 4-1BB agonists for use in the presently disclosed methods and compositions include anti-4-1BB antibodies, human anti-4-1BB antibodies, mouse anti-4-1BB antibodies, mammalian anti-4-1BB antibodies, monoclonal anti-4-1BB antibodies, polyclonal anti-4-1BB antibodies, chimeric anti-4-1BB antibodies, anti-4-1BB adnectins, anti-4-1BB domain antibodies, single chain anti-4-1BB fragments, heavy chain anti-4-1BB fragments, light chain anti-4-1BB fragments, anti-4-1BB fusion proteins, and fragments, derivatives, conjugates, variants, or biosimilars thereof. Agonistic anti-4-1BB antibodies are known to induce strong immune responses. Lee, et al., PLOS One, 2013, 8:e69677. In a preferred embodiment, the 4-1BB agonist is an agonistic, anti-4-1BB humanized or fully human monoclonal antibody (*i.e.,* an antibody derived from a single cell line). In an embodiment, the 4-1BB agonist is EU-101 (Eutilex Co. Ltd.), utomilumab, or urelumab, or a fragment, derivative, conjugate, variant, or biosimilar thereof. In a preferred embodiment, the 4-1BB agonist is utomilumab or urelumab, or a fragment, derivative, conjugate, variant, or biosimilar thereof.

In a preferred embodiment, the 4-1BB agonist or 4-1BB binding molecule may also be a fusion protein. In a preferred embodiment, a multimeric 4-1BB agonist, such as a trimeric or hexameric 4-1BB agonist (with three or six ligand binding domains), may induce superior receptor (4-1BBL) clustering and internal cellular signaling complex formation compared to an agonistic monoclonal antibody, which typically possesses two ligand binding domains. Trimeric (trivalent) or hexameric (or hexavalent) or greater fusion proteins comprising three TNFRSF binding domains and IgG1-Fc and optionally further linking two or more of these fusion proteins are described, for example, in Gieffers, et al., Mol. Cancer Therapeutics, 2013, 12:2735-47.

Agonistic 4-1BB antibodies and fusion proteins are known to induce strong immune responses. In a preferred embodiment, the 4-1BB agonist is a monoclonal antibody or fusion protein that binds specifically to 4-1BB antigen in a manner sufficient to reduce toxicity. In some embodiments, the 4-1BB agonist is an agonistic 4-1BB monoclonal antibody or fusion protein that abrogates antibody-dependent cellular toxicity (ADCC), for example NK cell cytotoxicity. In some embodiments, the 4-1BB agonist is an agonistic 4-1BB monoclonal antibody or fusion protein that abrogates antibody-dependent cell phagocytosis (ADCP). In some embodiments, the 4-1BB agonist is an agonistic 4-1BB monoclonal antibody or fusion protein that abrogates complement-dependent cytotoxicity (CDC). In some embodiments, the 4-1BB agonist is an agonistic 4-1BB monoclonal antibody or fusion protein which abrogates Fc region functionality.

In some embodiments, the 4-1BB agonists are characterized by binding to human 4-1BB (SEQ ID NO:9) with high affinity and agonistic activity. In an embodiment, the 4-1BB agonist is a binding molecule that binds to human 4-1BB (SEQ ID NO:9). In an embodiment, the 4-1BB agonist is a binding molecule that binds to murine 4-1BB (SEQ ID NO:10). The amino acid sequences of 4-1BB antigen to which a 4-1BB agonist or binding molecule binds are summarized in Table 3.

TABLE 3. Amino acid sequences of 4-1BB antigens.

| Identifier | Sequence (One-Letter Amino Acid Symbols) | |
|---|---|---|
| SEQ ID NO:9 human 4-1BB, Tumor necrosis factor receptor superfamily, member 9 (Homo sapiens) | MGNSCYNIVA TLLLVLNFER TRSLQDPCSN CPAGTFCDNN RNQICSPCPP NSFSSAGGQR<br>TCDICRQCKG VFRTRKECSS TSNAECDCTP GFHCLGAGCS MCEQDCKQGQ ELTKKGCKDC<br>CFGTFNDQKR GICRPWTNCS LDGKSVLVNG TKERDVVCGP SPADLSPGAS SVTPPAPARE<br>PGHSPQIISF FLALTSTALL FLLFFLTLRF SVVKRGRKKL LYIFKQPFMR PVQTTQEEDG<br>CSCRFPEEEE GGCEL | 60<br>120<br>180<br>240<br>255 |
| SEQ ID NO:10 murine 4-1BB, Tumor necrosis factor receptor superfamily, member 9 (Mus musculus) | MGNNCYNVVV IVLLLVGCEK VGAVQNSCDN CQPGTFCRKY NPVCKSCPPS TFSSIGGQPN<br>CNICRVCAGY FRFKKFCSST HNAECECIEG FHCLGPQCTR CEKDCRPGQE LTKQGCKTCS<br>LGTFNDQNGT GVCRPWTNCS LDGRSVLKTG TTEKDVVCGP PVVSFSPSTT ISVTPEGGPG<br>GHSLQVLTLF LALTSALLLA LIFITLLFSV LKWIRKKFPH IFKQPFKKTT GAAQEEDACS<br>CRCPQEEEGG GGGYEL | 60<br>120<br>180<br>240<br>256 |

In some embodiments, the compositions, processes and methods described include a 4-1BB agonist that binds human or murine 4-1BB with a $K_D$ of about 100 pM or lower, binds human or murine 4-1BB with a $K_D$ of about 90 pM or lower, binds human or murine 4-1BB with a $K_D$ of about 80 pM or lower, binds human or murine 4-1BB with a $K_D$ of about 70 pM or lower, binds human or murine 4-1BB with a $K_D$ of about 60 pM or lower, binds human or murine 4-1BB with a $K_D$ of about 50 pM or lower, binds human or murine 4-1BB with a $K_D$ of about 40 pM or lower, or binds human or murine 4-1BB with a $K_D$ of about 30 pM or lower.

In some embodiments, the compositions, processes and methods described include a 4-1BB agonist that binds to human or murine 4-1BB with a $k_{assoc}$ of about $7.5 \times 10^5$ 11M. s or faster, binds to human or murine 4-1BB with a $k_{assoc}$ of about $7.5 \times 10^5$ 1/M·s or faster, binds to human or murine 4-1BB with a $k_{assoc}$ of about $8 \times 10^5$ l/M·s or faster, binds to human or murine 4-1BB with a $k_{assoc}$ of about $8.5 \times 10^5$ 1/M·s or faster, binds to human or murine 4-1BB with a $k_{assoc}$ of about $9 \times 10^5$ 1/M-s or faster, binds to human or murine 4-1BB with a $k_{assoc}$ of about $9.5 \times 10^5$ 11M. s or faster, or binds to human or murine 4-1BB with a $k_{assoc}$ of about $1 \times 10^6$ 11M. s or faster.

In some embodiments, the compositions, processes and methods described include a 4-1BB agonist that binds to human or murine 4-1BB with a $k_{dissoc}$ of about $2 \times 10^{-5}$ 1/s or slower, binds to human or murine 4-1BB with a $k_{dissoc}$ of about $2.1 \times 10^{-5}$ 1/s or slower , binds to human or murine 4-1BB with a $k_{dissoc}$ of about $2.2 \times 10^{-5}$ 1/s or slower, binds to human or murine 4-1BB with a $k_{dissoc}$ of about $2.3 \times 10^{-5}$ 1/s or slower, binds to human or murine 4-1BB with a $k_{dissoc}$ of about $2.4 \times 10^{-5}$ 1/s or slower, binds to human or murine 4-1BB with a $k_{dissoc}$ of about $2.5 \times 10^{-5}$ 1/s or slower, binds to human or murine 4-1BB with a $k_{dissoc}$ of about $2.6 \times 10^{-5}$ 1/s or slower or binds to human or murine 4-1BB with a $k_{dissoc}$ of about $2.7 \times 10^{-5}$ 1/s or slower, binds to human or murine 4-1BB with a $k_{dissoc}$ of about $2.8 \times 10^{-5}$ 1/s or slower, binds to human or murine 4-1BB with a $k_{dissoc}$ of about $2.9 \times 10^{-5}$ 1/s or slower, or binds to human or murine 4-1BB with a $k_{dissoc}$ of about $3 \times 10^{-5}$ 1/s or slower.

In some embodiments, the compositions, processes and methods described include a 4-1BB agonist that binds to human or murine 4-1BB with an $IC_{50}$ of about 10 nM or lower, binds to human or murine 4-1BB with an $IC_{50}$ of about 9 nM or lower, binds to human or murine 4-1BB with an $IC_{50}$ of about 8 nM or lower, binds to human or murine 4-1BB with an $IC_{50}$ of about 7 nM or lower, binds to human or murine 4-1BB with an $IC_{50}$ of about 6 nM or lower, binds to human or murine 4-1BB with an $IC_{50}$ of about 5 nM or lower, binds to human or murine 4-1BB with an $IC_{50}$ of about 4 nM or lower, binds to human or murine 4-1BB with an $IC_{50}$ of about 3 nM or lower, binds to human or murine 4-1BB with an $IC_{50}$ of about 2 nM or lower, or binds to human or murine 4-1BB with an $IC_{50}$ of about 1 nM or lower.

In a preferred embodiment, the 4-1BB agonist is utomilumab, also known as PF-05082566 or MOR-7480, or a fragment, derivative, variant, or biosimilar thereof. Utomilumab is available from Pfizer, Inc. Utomilumab is an immunoglobulin G2-lambda, anti-[Homo sapiens TNFRSF9 (tumor necrosis factor receptor (TNFR) superfamily member 9, 4-1BB, T cell antigen ILA, CD137)], Homo sapiens (fully human) monoclonal antibody. The amino acid sequences of utomilumab are set forth in Table 4. Utomilumab comprises glycosylation sites at Asn59 and Asn292; heavy chain intrachain disulfide bridges at positions 22-96 ($V_H$-$V_L$), 143-199 ($C_H$1-$C_L$), 256-316 ($C_H$2) and 362-420 ($C_H$3); light chain intrachain disulfide bridges at positions 22'-87' ($V_H$-$V_L$) and 136'-195' ($C_H$1-$C_L$); interchain heavy chain-heavy chain disulfide bridges at IgG2A isoform positions 218-218, 219-219, 222-222, and 225-225, at IgG2A/B isoform positions 218-130, 219-219, 222-222, and 225-225, and at IgG2B isoform positions 219-130 (2), 222-222, and 225-225; and interchain heavy chain-light chain disulfide bridges at IgG2A isoform positions 130-213' (2), IgG2A/B isoform positions 218-213' and 130-213', and at IgG2B isoform positions 218-213' (2). The preparation and properties of utomilumab and its variants and fragments are described in U.S. Patent Nos. 8,821,867; 8,337,850; and 9,468,678, and International Patent Application Publication No. WO 2012/032433 A1. Preclinical characteristics of utomilumab are described in Fisher, et al., Cancer Immunolog. & Immunother., 2012, 61:1721-33. Current clinical trials of utomilumab in a variety of hematological and solid tumor indications include U.S. National Institutes of Health clinicaltrials.gov identifiers NCT02444793, NCT01307267, NCT02315066, and NCT02554812.

In an embodiment, a 4-1BB agonist comprises a heavy chain given by SEQ ID NO:11 and a light chain given by SEQ ID NO:12. In an embodiment, a 4-1BB agonist comprises heavy and light chains having the sequences shown in SEQ ID NO:11 and SEQ ID NO:12, respectively, or antigen binding fragments, Fab fragments, single-chain variable fragments (scFv), variants, or conjugates thereof. In an embodiment, a 4-1BB agonist comprises heavy and light chains that are each at least 99% identical to the sequences shown in SEQ ID NO:11 and SEQ ID NO:12, respectively. In an embodiment, a 4-1BB agonist comprises heavy and light chains that are each at least 98% identical to the sequences shown in SEQ ID NO:11 and SEQ ID NO:12, respectively. In an embodiment, a 4-1BB agonist comprises heavy and light chains that are each at least 97% identical to the sequences shown in SEQ ID NO:11 and SEQ ID NO:12, respectively. In an embodiment, a 4-1BB agonist comprises heavy and light chains that are each at least 96% identical to the sequences shown in SEQ ID NO:11 and SEQ ID NO:12, respectively. In an embodiment, a 4-1BB agonist comprises heavy and light chains that are each at least 95% identical to the sequences shown in SEQ ID NO:11 and SEQ ID NO:12, respectively.

In an embodiment, the 4-1BB agonist comprises the heavy and light chain CDRs or variable regions (VRs) of utomilumab. In an embodiment, the 4-1BB agonist heavy chain variable region ($V_H$) comprises the sequence shown in SEQ ID NO:13, and the 4-1BB agonist light chain variable region ($V_L$) comprises the sequence shown in SEQ ID NO:14, and conservative amino acid substitutions thereof. In an embodiment, a 4-1BB agonist comprises $V_H$ and $V_L$ regions that are each at least 99% identical to the sequences shown in SEQ ID NO:13 and SEQ ID NO:14, respectively. In an embodiment, a 4-1BB agonist comprises $V_H$ and $V_L$ regions that are each at least 98% identical to the sequences shown in SEQ ID NO:13 and SEQ ID NO:14, respectively. In an embodiment, a 4-1BB agonist comprises $V_H$ and $V_L$ regions that are each at least 97% identical to the sequences shown in SEQ ID NO:13 and SEQ ID NO:14, respectively. In an embodiment, a 4-1BB agonist comprises $V_H$ and $V_L$ regions that are each at least 96% identical to the sequences shown in SEQ ID NO:13 and SEQ ID NO:14, respectively. In an embodiment, a 4-1BB agonist comprises $V_H$ and $V_L$ regions that are each at least 95% identical to the sequences shown in SEQ ID NO:13 and SEQ ID NO:14, respectively. In an embodiment, a 4-1BB agonist comprises an scFv antibody comprising $V_H$ and $V_L$ regions that are each at least 99% identical to the sequences shown in SEQ ID NO:13 and SEQ ID NO:14.

In an embodiment, a 4-1BB agonist comprises heavy chain CDR1, CDR2 and CDR3 domains having the sequences set forth in SEQ ID NO:15, SEQ ID NO:16, and SEQ ID NO:17, respectively, and conservative amino acid substitutions thereof, and light chain CDR1, CDR2 and CDR3 domains having the sequences set forth in SEQ ID NO:18, SEQ ID NO:19, and SEQ ID NO:20, respectively, and conservative amino acid substitutions thereof.

In an embodiment, the 4-1BB agonist is a 4-1BB agonist biosimilar monoclonal antibody approved by drug regulatory authorities with reference to utomilumab. In an embodiment, the biosimilar monoclonal antibody comprises an 4-1BB antibody comprising an amino acid sequence which has at least 97% sequence identity, e.g., 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is utomilumab. In some embodiments, the one or more post-translational modifications are selected from one or more of: glycosylation, oxidation, deamidation, and truncation. In some embodiments, the biosimilar is a 4-1BB agonist antibody authorized or submitted for authorization, wherein the 4-1BB agonist antibody is provided in a formulation which differs from the formulations of a reference medicinal product or reference biological product, wherein the reference medicinal product or reference biological product is utomilumab. The 4-1BB agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is utomilumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is utomilumab.

TABLE 4. Amino acid sequences for 4-1BB agonist antibodies related to utomilumab.

| Identifier | Sequence (One-Letter Amino Acid Symbols) |
|---|---|
| SEQ ID NO:11<br>heavy chain for<br>utomilumab | EVQLVQSGAE VKKPGESLRI SCKGSGYSFS TYWISWVRQM PGKGLEWMGK IYPGDSYTNY   60<br>SPSFQGQVTI SADKSISTAY LQWSSLKASD TAMYYCARGY GIFDYWGQGT LVTVSSASTK  120<br>GPSVFPLAPC SRSTSESTAA LGCLVKDYFP EPVTVSWNSG ALTSGVHTFP AVLQSSGLYS  180<br>LSSVVTVPSS NFGTQTYTCN VDHKPSNTKV DKTVERKCCV ECPPCPAPPV AGPSVFLFPP  240<br>KPKDTLMISR TPEVTCVVVD VSHEDPEVQF NWYVDGVEVH NAKTKPREEQ FNSTFRVVSV  300<br>LTVVHQDWLN GKEYKCKVSN KGLPAPIEKT ISKTKGQPRE PQVYTLPPSR EEMTKNQVSL  360<br>TCLVKGFYPS DIAVEWESNG QPENNYKTTP PMLDSDGSFF LYSKLTVDKS RWQQGNVFSC  420<br>SVMHEALHNH YTQKSLSLSP G  441 |
| SEQ ID NO:12<br>light chain for<br>utomilumab | SYELTQPPSV SVSPGQTASI TCSGDNIGDQ YAHWYQQKPG QSPVLVIYQD KNRPSGIPER   60<br>FSGSNSGNTA TLTISGTQAM DEADYYCATY TGFGSLAVFG GGTKLTVLGQ PKAAPSVTLF  120<br>PPSSEELQAN KATLVCLISD FYPGAVTVAW KADSSPVKAG VETTTPSKQS NNKYAASSYL  180<br>SLTPEQWKSH RSYSCQVTHE GSTVEKTVAP TECS  214 |
| SEQ ID NO:13<br>heavy chain<br>variable region for<br>utomilumab | EVQLVQSGAE VKKPGESLRI SCKGSGYSFS TYWISWVRQM PGKGLEWMG KIYPGDSYTN   60<br>YSPSFQGQVT ISADKSISTA YLQWSSLKAS DTAMYYCARG YGIFDYWGQ GTLVTVSS  118 |
| SEQ ID NO:14<br>light chain variable<br>region for<br>utomilumab | SYELTQPPSV SVSPGQTASI TCSGDNIGDQ YAHWYQQKPG QSPVLVIYQD KNRPSGIPER   60<br>FSGSNSGNTA TLTISGTQAM DEADYYCATY TGFGSLAVFG GGTKLTVL  108 |
| SEQ ID NO:15<br>heavy chain CDR1<br>for utomilumab | STYWIS |
| SEQ ID NO:16<br>heavy chain CDR2<br>for utomilumab | KIYPGDSYTN YSPSFQG |
| SEQ ID NO:17<br>heavy chain CDR3<br>for utomilumab | RGYGIFDY |
| SEQ ID NO:18<br>light chain CDR1<br>for utomilumab | SGDNIGDQYA H |
| SEQ ID NO:19<br>light chain CDR2<br>for utomilumab | QDKNRPS |
| SEQ ID NO:20<br>light chain CDR3<br>for utomilumab | ATYTGFGSLA V |

In a preferred embodiment, the 4-1BB agonist is the monoclonal antibody urelumab, also known as BMS-663513 and 20H4.9.h4a, or a fragment, derivative, variant, or biosimilar thereof. Urelumab is available from Bristol-Myers Squibb, Inc., and Creative Biolabs, Inc. Urelumab is an immunoglobulin G4-kappa, anti-[Homo sapiens TNFRSF9 (tumor necrosis factor receptor superfamily member 9, 4-1BB, T cell antigen ILA, CD137)], Homo sapiens (fully human) monoclonal antibody. The amino acid sequences of urelumab are set forth in Table 5. Urelumab comprises N-glycosylation sites at positions 298 (and 298"); heavy chain intrachain disulfide bridges at positions 22-95 ($V_H$-$V_L$), 148-204 ($C_H$1-$C_L$), 262-322 ($C_H$2) and 368-426 ($C_H$3) (and at positions 22"-95", 148"-204", 262"-322", and 368"-426"); light chain intrachain disulfide bridges at positions 23'-88' $V_H$-$V_L$) and 136'-196' ($C_H$1-$C_L$) (and at positions 23‴-88‴ and 136‴-196‴ ); interchain heavy chain-heavy chain disulfide bridges at positions 227-227" and 230-230"; and interchain heavy chain-light chain disulfide bridges at 135-216' and 135"-216‴. The preparation and properties of urelumab and its variants and fragments are described in U.S. Patent Nos. 7,288,638 and 8,962,804. The preclinical and clinical characteristics of urelumab are described in Segal, et al., Clin. Cancer Res., 2016, available at http:/dx.doi.org/ 10.1158/1078-0432.CCR-16-1272. Current clinical trials of urelumab in a variety of hematological and solid tumor indications include U.S. National Institutes of Health clinicaltrials.gov identifiers NCT01775631, NCT02110082, NCT02253992, and NCT01471210.

In an embodiment, a 4-1BB agonist comprises a heavy chain given by SEQ ID NO:21 and a light chain given by SEQ ID NO:22. In an embodiment, a 4-1BB agonist comprises heavy and light chains having the sequences shown in SEQ ID NO:21 and SEQ ID NO:22, respectively, or antigen binding fragments, Fab fragments, single-chain variable fragments (scFv), variants, or conjugates thereof. In an embodiment, a 4-1BB agonist comprises heavy and light chains that are each at least 99% identical to the sequences shown in SEQ ID NO:21 and SEQ ID NO:22, respectively. In an embodiment, a 4-1BB agonist comprises heavy and light chains that are each at least 98% identical to the sequences shown in SEQ ID NO:21 and SEQ ID NO:22, respectively. In an embodiment, a 4-1BB agonist comprises heavy and light chains that are each at least 97% identical to the sequences shown in SEQ ID NO:21 and SEQ ID NO:22, respectively. In an embodiment, a 4-1BB agonist comprises heavy and light chains that are each at least 96% identical to the sequences shown in SEQ ID NO:21 and SEQ ID NO:22, respectively. In an embodiment, a 4-1BB agonist comprises heavy and light chains that are each at least 95% identical to the sequences shown in SEQ ID NO:21 and SEQ ID NO:22, respectively.

In an embodiment, the 4-1BB agonist comprises the heavy and light chain CDRs or variable regions (VRs) of urelumab. In an embodiment, the 4-1BB agonist heavy chain variable region ($V_H$) comprises the sequence shown in SEQ ID NO:23, and the 4-1BB agonist light chain variable region ($V_L$) comprises the sequence shown in SEQ ID NO:24, and conservative amino acid substitutions thereof. In an embodiment, a 4-1BB agonist comprises $V_H$ and $V_L$ regions that are each at least 99% identical to the sequences shown in SEQ ID NO:23 and SEQ ID NO:24, respectively. In an embodiment, a 4-1BB agonist comprises $V_H$ and $V_L$ regions that are each at least 98% identical to the sequences shown in SEQ ID NO:23 and SEQ ID NO:24, respectively. In an embodiment, a 4-1BB agonist comprises $V_H$ and $V_L$ regions that are each at least 97% identical to the sequences shown in SEQ ID NO:23 and SEQ ID NO:24, respectively. In an embodiment, a 4-1BB agonist comprises $V_H$ and $V_L$ regions that are each at least 96% identical to the sequences shown in SEQ ID NO:23 and SEQ ID NO:24, respectively. In an embodiment, a 4-1BB agonist comprises $V_H$ and $V_L$ regions that are each at least 95% identical to the sequences shown in SEQ ID NO:23 and SEQ ID NO:24, respectively. In an embodiment, a 4-1BB agonist comprises an scFv antibody comprising $V_H$ and $V_L$ regions that are each at least 99% identical to the sequences shown in SEQ ID NO:23 and SEQ ID NO:24.

In an embodiment, a 4-1BB agonist comprises heavy chain CDR1, CDR2 and CDR3 domains having the sequences set forth in SEQ ID NO:25, SEQ ID NO:26, and SEQ ID NO:27, respectively, and conservative amino acid substitutions thereof, and light chain CDR1, CDR2 and CDR3 domains having the sequences set forth in SEQ ID NO:28, SEQ ID NO:29, and SEQ ID NO:30, respectively, and conservative amino acid substitutions thereof.

In an embodiment, the 4-1BB agonist is a 4-1BB agonist biosimilar monoclonal antibody approved by drug regulatory authorities with reference to urelumab. In an embodiment, the biosimilar monoclonal antibody comprises an 4-1BB antibody comprising an amino acid sequence which has at least 97% sequence identity, *e.g.*, 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is urelumab. In some embodiments, the one or more post-translational modifications are selected from one or more of: glycosylation, oxidation, deamidation, and truncation. In some embodiments, the biosimilar is a 4-1BB agonist antibody authorized or submitted for authorization, wherein the 4-1BB agonist antibody is provided in a formulation which differs from the formulations of a reference medicinal product or reference biological product, wherein the reference medicinal product or reference biological product is urelumab. The 4-1BB agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is urelumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is urelumab.

TABLE 5. Amino acid sequences for 4-1BB agonist antibodies related to urelumab.

| Identifier | Sequence (One-Letter Amino Acid Symbols) | |
|---|---|---|
| SEQ ID NO:21 heavy chain for urelumab | QVQLQQWGAG LLKPSETLSL TCAVYGGSFS GYYWSWIRQS PEKGLEWIGE INHGGYVTYN<br>PSLESRVTIS VDTSKNQFSL KLSSVTAADT AVYYCARDYG PGNYDWYFDL WGRGTLVTVS<br>SASTKGPSVF PLAPCSRSTS ESTAALGCLV KDYFPEPVTV SWNSGALTSG VHTFPAVLQS<br>SGLYSLSSVV TVPSSSLGTK TYTCNVDHKP SNTKVDKRVE SKYGPPCPPC PAPEFLGGPS<br>VFLFPPKPKD TLMISRTPEV TCVVVDVSQE DPEVQFNWYV DGVEVHNAKT KPREEQFNST<br>YRVVSVLTVL HQDWLNGKEY KCKVSNKGLP SSIEKTISKA KGQPREPQVY TLPPSQEEMT | 60<br>120<br>180<br>240<br>300<br>360 |
| | KNQVSLTCLV KGFYPSDIAV EWESNGQPEN NYKTTPPVLD SDGSFFLYSR LTVDKSRWQE<br>GNVFSCSVMH EALHNHYTQK SLSLSLGK | 420<br>448 |
| SEQ ID NO:22 light chain for urelumab | EIVLTQSPAT LSLSPGERAT LSCRASQSVS SYLAWYQQKP GQAPRLLIYD ASNRATGIPA<br>RFSGSGSGTD FTLTISSLEP EDFAVYYCQQ RSNWPPALTF CGGTKVEIKR TVAAPSVFIF<br>PPSDEQLKSG TASVVCLLNN FYPREAKVQW KVDNALQSGN SQESVTEQDS KDSTYSLSST<br>LTLSKADYEK HKVYACEVTH QGLSSPVTKS FNRGEC | 60<br>120<br>180<br>216 |
| SEQ ID NO:23 variable heavy chain for urelumab | MKHLWFFLLL VAAPRWVLSQ VQLQQWGAGL LKPSETLSLT CAVYGGSFSG YYWSWIRQSP<br>EKGLEWIGEI NHGGYVTYNP SLESRVTISV DTSKNQFSLK LSSVTAADTA VYYCARDYGP | 60<br>120 |
| SEQ ID NO:24 variable light chain for urelumab | MEAPAQLLFL LLLWLPDTTG EIVLTQSPAT LSLSPGERAT LSCRASQSVS SYLAWYQQKP<br>GQAPRLLIYD ASNRATGIPA RFSGSGSGTD FTLTISSLEP EDFAVYYCQQ | 60<br>110 |
| SEQ ID NO:25 heavy chain CDR1 for urelumab | GYYWS | |
| SEQ ID NO:26 heavy chain CDR2 for urelumab | EINHGGYVTY NPSLES | |
| SEQ ID NO:27 heavy chain CDR3 for urelumab | DYGPGNYDWY FDL | |
| SEQ ID NO:28 light chain CDR1 for urelumab | RASQSVSSYL A | |
| SEQ ID NO:29 light chain CDR2 for urelumab | DASNRAT | |
| SEQ ID NO:30 light chain CDR3 for urelumab | QQRSDWPPAL T | |

In an embodiment, the 4-1BB agonist is selected from the group consisting of 1D8, 3Elor, 4B4 (BioLegend 309809), H4-1BB-M127 (BD Pharmingen 552532), BBK2 (Thermo Fisher MS621PABX), 145501 (Leinco Technologies B591), the antibody produced by cell line deposited as ATCC No. HB-11248 and disclosed in U.S. Patent No. 6,974,863, 5F4 (BioLegend 31 1503), C65-485 (BD Pharmingen 559446), antibodies disclosed in U.S. Patent Application Publication No. US 2005/0095244, antibodies disclosed in U.S. Patent No. 7,288,638 (such as 20H4.9-IgGl (BMS-663031)), antibodies disclosed in U.S. Patent No. 6,887,673 (such as 4E9 or BMS-554271), antibodies disclosed in U.S. Patent No. 7,214,493, antibodies disclosed in U.S. Patent No. 6,303,121, antibodies disclosed in U.S. Patent No. 6,569,997, antibodies disclosed in U.S. Patent No. 6,905,685 (such as 4E9 or BMS-554271), antibodies disclosed in U.S. Patent No. 6,362,325 (such as 1D8 or BMS-469492; 3H3 or BMS-469497; or 3El), antibodies disclosed in U.S. Patent No. 6,974,863 (such as 53A2); antibodies disclosed in U.S. Patent No. 6,210,669 (such as 1D8, 3B8, or 3El), antibodies described in U.S. Patent No. 5,928,893, antibodies disclosed in U.S. Patent No. 6,303,121, antibodies disclosed in U.S. Patent No. 6,569,997, antibodies disclosed in International Patent Application Publication Nos. WO 2012/177788, WO 2015/119923, and WO 2010/042433, and fragments, derivatives, conjugates, variants, or biosimilars thereof.

In an embodiment, the 4-1BB agonist is a 4-1BB agonistic fusion protein described in International Patent Application Publication Nos. WO 2008/025516 A1, WO 2009/007120 A1, WO 2010/003766 A1, WO 2010/010051 A1, and WO 2010/078966 A1; U.S. Patent Application Publication Nos. US 2011/0027218 A1, US 2015/0126709 A1, US 2011/0111494 A1, US 2015/0110734 A1, and US 2015/0126710 A1; and U.S. Patent Nos. 9,359,420, 9,340,599, 8,921,519, and 8,450,460.

In an embodiment, the 4-1BB agonist is a 4-1BB agonistic fusion protein as depicted in Figure 25 as Structure I-A (C-terminal Fc-antibody fragment fusion protein) or Structure I-B (N-terminal Fc-antibody fragment fusion protein), or a fragment, derivative, conjugate, variant, or biosimilar thereof:

As shown in Figure 25 in structures I-A and I-B, the cylinders refer to individual polypeptide binding domains. Structures I-A and I-B comprise three linearly-linked TNFRSF binding domains derived from e.g., 4-1BBL or an antibody that binds 4-1BB, which fold to form a trivalent protein, which is then linked to a second trivalent protein through IgG1-Fc (including $C_H3$ and $C_H2$ domains) and is then used to link two of the trivalent proteins together through disulfide bonds (small elongated ovals), stabilizing the structure and providing an agonist capable of bringing together the intracellular signaling domains of the six receptors and signaling proteins to form a signaling complex. The TNFRSF binding domains denoted as cylinders may be scFv domains comprising, e.g., a $V_H$ and a $V_L$ chain connected by a linker that may comprise hydrophilic residues and Gly and Ser sequences for flexibility, as well as Glu and Lys for solubility. Any scFv domain design may be used, such as those described in de Marco, Microbial Cell Factories, 2011, 10, 44; Ahmad, et al., Clin. & Dev. Immunol., 2012, 980250; Monnier, et al., Antibodies, 2013, 2, 193-208. Fusion protein structures of this form are described in U.S. Patent Nos. 9,359,420, 9,340,599, 8,921,519, and 8,450,460.

Amino acid sequences for the other polypeptide domains of structure I-A are given in Table 6. The Fc domain preferably comprises a complete constant domain (amino acids 17-230 of SEQ ID NO:31) the complete hinge domain (amino acids 1-16 of SEQ ID NO:31) or a portion of the hinge domain (e.g., amino acids 4-16 of SEQ ID NO:31). Preferred linkers for connecting a C-terminal Fc-antibody may be selected from the embodiments given in SEQ ID NO:32 to SEQ ID NO:41, including linkers suitable for fusion of additional polypeptides.

TABLE 6. Amino acid sequences for TNFRSF fusion proteins, including 4-1BB fusion proteins, with C-terminal Fc-antibody fragment fusion protein design (structure I-A).

| Identifier | Sequence (One-Letter Amino Acid Symbols) |
|---|---|
| SEQ ID NO:31 Fc domain | KSCDKTHTCP PCPAPELLGG PSVFLFPPKP KDTLMISRTP EVTCVVVDVS HEDPEVKFNW    60<br>YVDGVEVHNA KTKPREEQYN STYRVVSVLT VLHQDWLNGK EYKCKVSNKA LPAPIEKTIS  120<br>KAKGQPREPQ VYTLPPSREE MTKNQVSLTC LVKGFYPSDI AVEWESNGQP ENNYKTTPPV  180<br>LDSDGSFFLY SKLTVDKSRW QQGNVFSCSV MHEALHNHYT QKSLSLSPGK  230 |
| SEQ ID NO:32 linker | GGPGSSKSCD KTHTCPPCPA PE |
| SEQ ID NO:33 linker | GGSGSSKSCD KTHTCPPCPA PE |
| SEQ ID NO:34 linker | GGPGSSSSSS SKSCDKTHTC PPCPAPE |
| SEQ ID NO:35 linker | GGSGSSSSSS SKSCDKTHTC PPCPAPE |
| SEQ ID NO:36 linker | GGPGSSSSSS SSSKSCDKTH TCPPCPAPE |
| SEQ ID NO:37 linker | GGSGSSSSSS SSSKSCDKTH TCPPCPAPE |
| SEQ ID NO:38 linker | GGPGSSGSGS SDKTHTCPPC PAPE |
| SEQ ID NO:39 linker | GGPGSSGSGS DKTHTCPPCP APE |
| SEQ ID NO:40 linker | GGPSSSGSDK THTCPPCPAP E |
| SEQ ID NO:41 linker | GGSSSSSSSS GSDKTHTCPP CPAPE |

Amino acid sequences for the other polypeptide domains of structure I-B are given in Table 7. If an Fc antibody fragment is fused to the N-terminus of an TNRFSF fusion protein as in structure I-B, the sequence of the Fc module is preferably that shown in SEQ ID NO:42, and the linker sequences are preferably selected from those embodiments set forth in SED ID NO:43 to SEQ ID NO:45.

TABLE 7. Amino acid sequences for TNFRSF fusion proteins, including 4-1BB fusion proteins, with N-terminal Fc-antibody fragment fusion protein design (structure I-B).

| Identifier | Sequence (One-Letter Amino Acid Symbols) |
|---|---|
| SEQ ID NO:42 Fc domain | METDTLLLWV LLLWVPAGNG DKTHTCPPCP APELLGGPSV FLFPPKPKDT LMISRTPEVT 60<br>CVVVDVSHED PEVKFNWYVD GVEVHNAKTK PREEQYNSTY RVVSVLTVLH QDWLNGKEYK 120<br>CKVSNKALPA PIEKTISKAK GQPREPQVYT LPPSREEMTK NQVSLTCLVK GFYPSDIAVE 180<br>WESNGQPENN YKTTPPVLDS DGSFFLYSKL TVDKSRWQQG NVFSCSVMHE ALHNHYTQKS 240<br>LSLSPG 246 |
| SEQ ID NO:43 linker | SGSGSGSGSG S |
| SEQ ID NO:44 linker | SSSSSSGSGS GS |
| SEQ ID NO:45 linker | SSSSSSGSGS GSGSGS |

In an embodiment, a 4-1BB agonist fusion protein according to structures I-A or I-B comprises one or more 4-1BB binding domains selected from the group consisting of a variable heavy chain and variable light chain of utomilumab, a variable heavy chain and variable light chain of urelumab, a variable heavy chain and variable light chain of utomilumab, a variable heavy chain and variable light chain selected from the variable heavy chains and variable light chains described in Table 4 or Table 5, any combination of a variable heavy chain and variable light chain of the foregoing, and fragments, derivatives, conjugates, variants, and biosimilars thereof.

In an embodiment, a 4-1BB agonist fusion protein according to structures I-A or I-B comprises one or more 4-1BB binding domains comprising a 4-1BBL sequence. In an embodiment, a 4-1BB agonist fusion protein according to structures I-A or I-B comprises one or more 4-1BB binding domains comprising a sequence according to SEQ ID NO:46. In an embodiment, a 4-1BB agonist fusion protein according to structures I-A or I-B comprises one or more 4-1BB binding domains comprising a soluble 4-1BBL sequence. In an embodiment, a 4-1BB agonist fusion protein according to structures I-A or I-B comprises one or more 4-1BB binding domains comprising a sequence according to SEQ ID NO:47.

In an embodiment, a 4-1BB agonist fusion protein according to structures I-A or I-B comprises one or more 4-1BB binding domains that is a scFv domain comprising $V_H$ and $V_L$ regions that are each at least 95% identical to the sequences shown in SEQ ID NO:13 and SEQ ID NO:14, respectively, wherein the $V_H$ and $V_L$ domains are connected by a linker. In an embodiment, a 4-1BB agonist fusion protein according to structures I-A or I-B comprises one or more 4-1BB binding domains that is a scFv domain comprising $V_H$ and $V_L$ regions that are each at least 95% identical to the sequences shown in SEQ ID NO:23 and SEQ ID NO:24, respectively, wherein the $V_H$ and $V_L$ domains are connected by a linker. In an embodiment, a 4-1BB agonist fusion protein according to structures I-A or I-B comprises one or more 4-1BB binding domains that is a scFv domain comprising $V_H$ and $V_L$ regions that are each at least 95% identical to the $V_H$ and $V_L$ sequences given in Table 8, wherein the $V_H$ and $V_L$ domains are connected by a linker.

TABLE 8. Additional polypeptide domains useful as 4-1BB binding domains in fusion proteins or as scFv 4-1BB agonist antibodies.

| Identifier | Sequence (One-Letter Amino Acid Symbols) |
|---|---|
| SEQ ID NO:46 4-1BBL | MEYASDASLD PEAPWPPAPR ARACRVLPWA LVAGLLLLLL LAAACAVFLA CPWAVSGARA 60<br>SPGSAASPRL REGPELSPDD PAGLLDLRQG MFAQLVAQNV LLIDGPLSWY SDPGLAGVSL 120<br>TGGLSYKEDT KELVVAKAGV YYVFFQLELR RVVAGEGSGS VSLALHLQPL RSAAGAAALA 180<br>LTVDLPPASS EARNSAFGFQ GRLLHLSAGQ RLGVHLHTEA RARHAWQLTQ GATVLGLFRV 240<br>TPEIPAGLPS PRSE 254 |
| SEQ ID NO:47 4-1BBL soluble domain | LRQGMFAQLV AQNVLLIDGP LSWYSDPGLA GVSLTGGLSY KEDTKELVVA KAGVYYVFFQ 60<br>LELRRVVAGE GSGSVSLALH LQPLRSAAGA AALALTVDLP PASSEARNSA FGFQGRLLHL 120<br>SAGQRLGVHL HTEARARHAW QLTQGATVLG LFRVTPEIPA GLPSPRSE 168 |

(continued)

| Identifier | Sequence (One-Letter Amino Acid Symbols) | |
|---|---|---|
| SEQ ID NO:48 variable heavy chain for 4B4-1-1 version 1 | QVQLQQPGAE LVKPGASVKL SCKASGYTFS SYWMHWVKQR PGQVLEWIGE INPGNGHTNY<br>NEKFKSKATL TVDKSSSTAY MQLSSLTSED SAVYYCARSF TTARGFAYWG QGTLVTVS | 60<br>118 |
| SEQ ID NO:49 variable light chain for 4B4-1-1 version 1 | DIVMTQSPAT QSVTPGDRVS LSCRASQTIS DYLHWYQQKS HESPRLLIKY ASQSISGIPS<br>RFSGSGSGSD FTLSINSVEP EDVGVYYCQD GHSFPPTFGG GTKLEIK | 60<br>107 |
| SEQ ID NO:50 variable heavy chain for 4B4-1-1 version 2 | QVQLQQPGAE LVKPGASVKL SCKASGYTFS SYWMHWVKQR PGQVLEWIGE INPGNGHTNY<br>NEKFKSKATL TVDKSSSTAY MQLSSLTSED SAVYYCARSF TTARGFAYWG QGTLVTVSA | 60<br>119 |
| SEQ ID NO:51 variable light chain for 4B4-1-1 version 2 | DIVMTQSPAT QSVTPGDRVS LSCRASQTIS DYLHWYQQKS HESPRLLIKY ASQSISGIPS<br>RFSGSGSGSD FTLSINSVEP EDVGVYYCQD GHSFPPTFGG GTKLEIKR | 60<br>108 |
| SEQ ID NO:52 variable heavy chain for H39E3-2 | MDWTWRILFL VAAATGAHSE VQLVESGGGL VQPGGSLRLS CAASGFTFSD YWMSWVRQAP<br>GKGLEWVADI KNDGSYTNYA PSLTNRFTIS RDNAKNSLYL QMNSLRAEDT AVYYCARELT | 60<br>120 |
| SEQ ID NO:53 variable light chain for H39E3-2 | MEAPAQLLFL LLLWLPDTTG DIVMTQSPDS LAVSLGERAT INCKSSQSLL SSGNQKNYL<br>WYQQKPGQPP KLLIYYASTR QSGVPDRFSG SGSGTDFTLT ISSLQAEDVA | 60<br>110 |

In an embodiment, the 4-1BB agonist is a 4-1BB agonistic single-chain fusion polypeptide comprising (i) a first soluble 4-1BB binding domain, (ii) a first peptide linker, (iii) a second soluble 4-1BB binding domain, (iv) a second peptide linker, and (v) a third soluble 4-1BB binding domain, further comprising an additional domain at the N-terminal and/or C-terminal end, and wherein the additional domain is a Fab or Fc fragment domain. In an embodiment, the 4-1BB agonist is a 4-1BB agonistic single-chain fusion polypeptide comprising (i) a first soluble 4-1BB binding domain, (ii) a first peptide linker, (iii) a second soluble 4-1BB binding domain, (iv) a second peptide linker, and (v) a third soluble 4-1BB binding domain, further comprising an additional domain at the N-terminal and/or C-terminal end, wherein the additional domain is a Fab or Fc fragment domain, wherein each of the soluble 4-1BB domains lacks a stalk region (which contributes to trimerisation and provides a certain distance to the cell membrane, but is not part of the 4-1BB binding domain) and the first and the second peptide linkers independently have a length of 3-8 amino acids.

In an embodiment, the 4-1BB agonist is a 4-1BB agonistic single-chain fusion polypeptide comprising (i) a first soluble tumor necrosis factor (TNF) superfamily cytokine domain, (ii) a first peptide linker, (iii) a second soluble TNF superfamily cytokine domain, (iv) a second peptide linker, and (v) a third soluble TNF superfamily cytokine domain, wherein each of the soluble TNF superfamily cytokine domains lacks a stalk region and the first and the second peptide linkers independently have a length of 3-8 amino acids, and wherein each TNF superfamily cytokine domain is a 4-1BB binding domain.

In an embodiment, the 4-1BB agonist is a 4-1BB agonistic scFv antibody comprising any of the foregoing V$_H$ domains linked to any of the foregoing V$_L$ domains.

In an embodiment, the 4-1BB agonist is BPS Bioscience 4-1BB agonist antibody catalog no. 79097-2, commercially available from BPS Bioscience, San Diego, CA, USA. In an embodiment, the 4-1BB agonist is Creative Biolabs 4-1BB agonist antibody catalog no. MOM-18179, commercially available from Creative Biolabs, Shirley, NY, USA.

OX40 (CD134) Agonists as Optional Media Components

In an embodiment, the TNFRSF agonist is an OX40 (CD134) agonist. The OX40 agonist may be any OX40 binding molecule known in the art. The OX40 binding molecule may be a monoclonal antibody or fusion protein capable of binding to human or mammalian OX40. The OX40 agonists or OX40 binding molecules may comprise an immunoglobulin heavy chain of any isotype (e.g., IgG, IgE, IgM, IgD, IgA, and IgY), class (e.g., IgG1, IgG2, IgG3, IgG4, IgA1 and IgA2) or subclass of immunoglobulin molecule. The OX40 agonist or OX40 binding molecule may have both a heavy and a light chain. As used herein, the term binding molecule also includes antibodies (including full length antibodies), monoclonal antibodies (including full length monoclonal antibodies), polyclonal antibodies, multispecific antibodies (*e.g.*, bispecific antibodies), human, humanized or chimeric antibodies, and antibody fragments, *e.g.*, Fab fragments, F(ab') fragments, fragments produced by a Fab expression library, epitope-binding fragments of any of the above, and engineered forms of antibodies, *e.g.,* scFv molecules, that bind to OX40. In an embodiment, the OX40 agonist is an antigen binding protein that is a fully human antibody. In an embodiment, the OX40 agonist is an antigen binding protein that is a humanized antibody. In some embodiments, OX40 agonists for use in the presently disclosed methods and compositions include anti-OX40 antibodies, human anti-OX40 antibodies, mouse anti-OX40 antibodies, mammalian anti-OX40 antibodies, monoclonal anti-OX40 antibodies, polyclonal anti-OX40 antibodies, chimeric anti-OX40 antibodies, anti-OX40 adnectins, anti-OX40 domain antibodies, single chain anti-OX40 fragments, heavy chain anti-OX40 fragments, light chain anti-OX40 fragments, anti-OX40 fusion proteins, and fragments, derivatives, conjugates, variants, or biosimilars thereof. In a preferred embodiment, the OX40 agonist is an agonistic, anti-OX40 humanized or fully human monoclonal antibody (*i.e.*, an antibody derived from a single cell line).

In a preferred embodiment, the OX40 agonist or OX40 binding molecule may also be a fusion protein. OX40 fusion proteins comprising an Fc domain fused to OX40L are described, for example, in Sadun, et al., J. Immunother., 2009, 182, 1481-89. In a preferred embodiment, a multimeric OX40 agonist, such as a trimeric or hexameric OX40 agonist (with three or six ligand binding domains), may induce superior receptor (OX40L) clustering and internal cellular signaling complex formation compared to an agonistic monoclonal antibody, which typically possesses two ligand binding domains. Trimeric (trivalent) or hexameric (or hexavalent) or greater fusion proteins comprising three TNFRSF binding domains and IgG1-Fc and optionally further linking two or more of these fusion proteins are described, for example, in Gieffers, et al., Mol. Cancer Therapeutics, 2013, 12, 2735-47.

Agonistic OX40 antibodies and fusion proteins are known to induce strong immune responses. Curti, et al., Cancer Res., 2013, 73, 7189-98. In a preferred embodiment, the OX40 agonist is a monoclonal antibody or fusion protein that binds specifically to OX40 antigen in a manner sufficient to reduce toxicity. In some embodiments, the OX40 agonist is an agonistic OX40 monoclonal antibody or fusion protein that abrogates antibody-dependent cellular toxicity (ADCC), for example NK cell cytotoxicity. In some embodiments, the OX40 agonist is an agonistic OX40 monoclonal antibody or fusion protein that abrogates antibody-dependent cell phagocytosis (ADCP). In some embodiments, the OX40 agonist is an agonistic OX40 monoclonal antibody or fusion protein that abrogates complement-dependent cytotoxicity (CDC). In some embodiments, the OX40 agonist is an agonistic OX40 monoclonal antibody or fusion protein which abrogates Fc region functionality.

In some embodiments, the OX40 agonists are characterized by binding to human OX40 (SEQ ID NO:54) with high affinity and agonistic activity. In an embodiment, the OX40 agonist is a binding molecule that binds to human OX40 (SEQ ID NO:54). In an embodiment, the OX40 agonist is a binding molecule that binds to murine OX40 (SEQ ID NO:55). The amino acid sequences of OX40 antigen to which an OX40 agonist or binding molecule binds are summarized in Table 9.

TABLE 9. Amino acid sequences of OX40 antigens.

| Identifier | Sequence (One-Letter Amino Acid Symbols) | |
|---|---|---|
| SEQ ID NO:54 human OX40 (Homo sapiens) | MCVGARRLGR  GPCAALLLLG  LGLSTVTGLH  CVGDTYPSND  RCCHECRPGN  GMVSRCSRSQ<br>NTVCRPCGPG  FYNDVVSSKP  CKPCTWCNLR  SGSERKQLCT  ATQDTVCRCR  AGTQPLDSYK<br>PGVDCAPCPP  GHFSPGDNQA  CKPWTNCTLA  GKHTLQPASN  SSDAICEDRD  PPATQPQETQ<br>GPPARPITVQ  PTEAWPRTSQ  GPSTRPVEVP  GGRAVAAILG  LGLVLGLLGP  LAILLALYLL<br>RRDQRLPPDA  HKPPGGGSFR  TPIQEEQADA  HSTLAKI | 60<br>120<br>180<br>240<br>277 |
| SEQ ID NO:55 murine OX40 (Mus musculus) | MYVWVQQPTA  LLLLGLTLGV  TARRLNCVKH  TYPSGHKCCR  ECQPGHGMVS  RCDHTRDTLC<br>HPCETGFYNE  AVNYDTCKQC  TQCNHRSGSE  LKQNCTPTQD  TVCRCRPGTQ  PRQDSGYKLG<br>VDCVPCPPGH  FSPGNNQACK  PWTNCTLSGK  QTRHPASDSL  DAVCEDRSLL  ATLLWETQRP<br>TFRPTTVQST  TVWPRTSELP  SPPTLVTPEG  PAFAVLLGLG  LGLLAPLTVL  LALYLLRKAW<br>RLPNTPKPCW  GNSFRTPIQE  EHTDAHFTLA  KI | 60<br>120<br>180<br>240<br>272 |

In some embodiments, the compositions, processes and methods described include a OX40 agonist that binds human or murine OX40 with a KD of about 100 pM or lower, binds human or murine OX40 with a $K_D$ of about 90 pM or lower, binds human or murine OX40 with a KD of about 80 pM or lower, binds human or murine OX40 with a $K_D$ of about 70 pM or lower, binds human or murine OX40 with a $K_D$ of about 60 pM or lower, binds human or murine OX40 with a $K_D$ of about 50 pM or lower, binds human or murine OX40 with a $K_D$ of about 40 pM or lower, or binds human or murine OX40 with a $K_D$ of about 30 pM or lower.

In some embodiments, the compositions, processes and methods described include a OX40 agonist that binds to human or murine OX40 with a $k_{assoc}$ of about $7.5 \times 10^5$ 1/M·s or faster, binds to human or murine OX40 with a $k_{assoc}$ of about $7.5 \times 10^5$ 1/M- s or faster, binds to human or murine OX40 with a $k_{assoc}$ of about $8 \times 10^5$ 1/M- s or faster, binds to human or murine OX40 with a $k_{assoc}$ of about $8.5 \times 10^5$ 1/M- s or faster, binds to human or murine OX40 with a $k_{assoc}$ of about $9 \times 10^5$ 11M. s or faster, binds to human or murine OX40 with a $k_{assoc}$ of about $9.5 \times 10^5$ 1/M- s or faster, or binds to human or murine OX40 with a $k_{assoc}$ of about $1 \times 10^6$ 1/M- s or faster.

In some embodiments, the compositions, processes and methods described include a OX40 agonist that binds to human or murine OX40 with a $k_{dissoc}$ of about $2 \times 10^{-5}$ 1/s or slower, binds to human or murine OX40 with a $k_{dissoc}$ of about $2.1 \times 10^{-5}$ 1/s or slower , binds to human or murine OX40 with a $k_{dissoc}$ of about $2.2 \times 10^{-5}$ 1/s or slower, binds to human or murine OX40 with a $k_{dissoc}$ of about $2.3 \times 10^{-5}$ 1/s or slower, binds to human or murine OX40 with a $k_{dissoc}$ of about $2.4 \times 10^{-5}$ 1/s or slower, binds to human or murine OX40 with a $k_{dissoc}$ of about $2.5 \times 10^{-5}$ 1/s or slower, binds to human or murine OX40 with a $k_{dissoc}$ of about $2.6 \times 10^{-5}$ 1/s or slower or binds to human or murine OX40 with a $k_{dissoc}$ of about $2.7 \times 10^{-5}$ 1/s or slower, binds to human or murine OX40 with a $k_{dissoc}$ of about $2.8 \times 10^{-5}$ 1/s or slower, binds to human or murine OX40 with a $k_{dissoc}$ of about $2.9 \times 10^{-5}$ 1/s or slower, or binds to human or murine OX40 with a $k_{dissoc}$ of about $3 \times 10^{-5}$ 1/s or slower.

In some embodiments, the compositions, processes and methods described include OX40 agonist that binds to human or murine OX40 with an $IC_{50}$ of about 10 nM or lower, binds to human or murine OX40 with an $IC_{50}$ of about 9 nM or lower, binds to human or murine OX40 with an $IC_{50}$ of about 8 nM or lower, binds to human or murine OX40 with an $IC_{50}$ of about 7 nM or lower, binds to human or murine OX40 with an $IC_{50}$ of about 6 nM or lower, binds to human or murine OX40 with an $IC_{50}$ of about 5 nM or lower, binds to human or murine OX40 with an $IC_{50}$ of about 4 nM or lower, binds to human or murine OX40 with an $IC_{50}$ of about 3 nM or lower, binds to human or murine OX40 with an $IC_{50}$ of about 2 nM or lower, or binds to human or murine OX40 with an $IC_{50}$ of about 1 nM or lower.

In some embodiments, the OX40 agonist is tavolixizumab, also known as MEDI0562 or MEDI-0562. Tavolixizumab is available from the MedImmune subsidary of AstraZeneca, Inc. Tavolixizumab is immunoglobulin G1-kappa, anti-[Homo sapiens TNFRSF4 (tumor necrosis factor receptor (TNFR) superfamily member 4, OX40, CD134)], humanized and chimeric monoclonal antibody. The amino acid sequences of tavolixizumab are set forth in Table 10. Tavolixizumab comprises N-glycosylation sites at positions 301 and 301", with fucosylated complex bi-antennary CHO-type glycans; heavy chain intrachain disulfide bridges at positions 22-95 ($C_H1$-$C_L$), 148-204 ($C_H1$-$C_L$), 265-325 ($C_H2$) and 371-429 ($C_H3$) (and at positions 22"-95", 148"-204", 265"-325", and 371"-429"); light chain intrachain disulfide bridges at positions 23'-88' ($V_H$-$V_L$) and 134'-194' ($C_H1$-$C_L$) (and at positions 23'''-88''' and 134'''-194'''); interchain heavy chain-heavy chain disulfide bridges at positions 230-230" and 233-233"; and interchain heavy chain-light chain disulfide bridges at 224-214' and 224"-214'''. Current clinical trials of tavolixizumab in a variety of solid tumor indications include U.S. National Institutes of Health clinicaltrials.gov identifiers NCT02318394 and NCT02705482.

In an embodiment, a OX40 agonist comprises a heavy chain given by SEQ ID NO:56 and a light chain given by SEQ ID NO:57. In an embodiment, a OX40 agonist comprises heavy and light chains having the sequences shown in SEQ ID NO:56 and SEQ ID NO:57, respectively, or antigen binding fragments, Fab fragments, single-chain variable fragments (scFv), variants, or conjugates thereof. In an embodiment, a OX40 agonist comprises heavy and light chains that are each at least 99% identical to the sequences shown in SEQ ID NO:56 and SEQ ID NO:57, respectively. In an embodiment, a OX40 agonist comprises heavy and light chains that are each at least 98% identical to the sequences shown in SEQ ID NO:56 and SEQ ID NO:57, respectively. In an embodiment, a OX40 agonist comprises heavy and light chains that are each at least 97% identical to the sequences shown in SEQ ID NO:56 and SEQ ID NO:57, respectively. In an embodiment, a OX40 agonist comprises heavy and light chains that are each at least 96% identical to the sequences shown in SEQ ID NO:56 and SEQ ID NO:57, respectively. In an embodiment, a OX40 agonist comprises heavy and light chains that are each at least 95% identical to the sequences shown in SEQ ID NO:56 and SEQ ID NO:57, respectively.

In an embodiment, the OX40 agonist comprises the heavy and light chain CDRs or variable regions (VRs) of tavolixizumab. In an embodiment, the OX40 agonist heavy chain variable region ($V_H$) comprises the sequence shown in SEQ ID NO:58, and the OX40 agonist light chain variable region ($V_L$) comprises the sequence shown in SEQ ID NO:59, and conservative amino acid substitutions thereof. In an embodiment, a OX40 agonist comprises $V_H$ and $V_L$ regions that are each at least 99% identical to the sequences shown in SEQ ID NO:58 and SEQ ID NO:59, respectively. In an embodiment, a OX40 agonist comprises $V_H$ and $V_L$ regions that are each at least 98% identical to the sequences shown in SEQ ID NO:58 and SEQ ID NO:59, respectively. In an embodiment, a OX40 agonist comprises $V_H$ and $V_L$ regions that are each at least 97% identical to the sequences shown in SEQ ID NO:58 and SEQ ID NO:59, respectively. In an embodiment, a OX40 agonist comprises $V_H$ and $V_L$ regions that are each at least 96% identical to the sequences shown in SEQ ID NO:58 and SEQ ID NO:59, respectively. In an embodiment, a OX40 agonist comprises $V_H$ and $V_L$ regions that are each at least 95% identical to the sequences shown in SEQ ID NO:58 and SEQ ID NO:59, respectively. In an embodiment, an OX40 agonist comprises an scFv antibody comprising $V_H$ and $V_L$ regions that are each at least 99% identical to the sequences shown in SEQ ID NO:58 and SEQ ID NO:59.

In an embodiment, a OX40 agonist comprises heavy chain CDR1, CDR2 and CDR3 domains having the sequences set forth in SEQ ID NO:60, SEQ ID NO:61, and SEQ ID NO:62, respectively, and conservative amino acid substitutions thereof, and light chain CDR1, CDR2 and CDR3 domains having the sequences set forth in SEQ ID NO:63, SEQ ID NO:64, and SEQ ID NO:65, respectively, and conservative amino acid substitutions thereof.

In an embodiment, the OX40 agonist is a OX40 agonist biosimilar monoclonal antibody approved by drug regulatory authorities with reference to tavolixizumab. In an embodiment, the biosimilar monoclonal antibody comprises an OX40 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is tavolixizumab. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is tavolixizumab. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is tavolixizumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is tavolixizumab.

TABLE 10. Amino acid sequences for OX40 agonist antibodies related to tavolixizumab.

| Identifier | Sequence (One-Letter Amino Acid Symbols) |
|---|---|
| SEQ ID NO:56 heavy chain for tavolixizumab | QVQLQESGPG LVKPSQTLSL TCAVYGGSFS SGYWNWIRKH PGKGLEYIGY ISYNGITYHN 60<br>PSLKSRITIN RDTSKNQYSL QLNSVTPEDT AVYYCARYKY DYDGGHAMDY WGQGTLVTVS 120<br>SASTKGPSVF PLAPSSKSTS GGTAALGCLV KDYFPEPVTV SWNSGALTSG VHTFPAVLQS 180<br>SGLYSLSSVV TVPSSSLGTQ TYICNVNHKP SNTKVDKRVE PKSCDKTHTC PPCPAPELLG 240<br>GPSVFLFPPK PKDTLMISRT PEVTCVVVDV SHEDPEVKFN WYVDGVEVHN AKTKPREEQY 300<br>NSTYRVVSVL TVLHQDWLNG KEYKCKVSNK ALPAPIEKTI SKAKGQPREP QVYTLPPSRE 360<br>EMTKNQVSLT CLVKGFYPSD IAVEWESNGQ PENNYKTTPP VLDSDGSFFL YSKLTVDKSR 420<br>WQQGNVFSCS VMHEALHNHY TQKSLSLSPG K 451 |
| SEQ ID NO:57 light chain for tavolixizumab | DIQMTQSPSS LSASVGDRVT ITCRASQDIS NYLNWYQQKP GKAPKLLIYY TSKLHSGVPS 60<br>RFSGSGSGTD YTLTISSLQP EDFATYYCQQ GSALPWTFGQ GTKVEIKRTV AAPSVFIFPP 120<br>SDEQLKSGTA SVVCLLNNFY PREAKVQWKV DNALQSGNSQ ESVTEQDSKD STYSLSSTLT 180<br>LSKADYEKHK VYACEVTHQG LSSPVTKSFN RGEC 214 |
| SEQ ID NO:58 heavy chain variable region for tavolixizumab | QVQLQESGPG LVKPSQTLSL TCAVYGGSFS SGYWNWIRKH PGKGLEYIGY ISYNGITYHN 60<br>PSLKSRITIN RDTSKNQYSL QLNSVTPEDT AVYYCARYKY DYDGGHAMDY WGQGTLVT 118 |
| SEQ ID NO:59 light chain variable region for tavolixizumab | DIQMTQSPSS LSASVGDRVT ITCRASQDIS NYLNWYQQKP GKAPKLLIYY TSKLHSGVPS 60<br>RFSGSGSGTD YTLTISSLQP EDFATYYCQQ GSALPWTFGQ GTKVEIKR 108 |
| SEQ ID NO:60 heavy chain CDR1 for tavolixizumab | GSFSSGYWN 9 |
| SEQ ID NO:61 heavy chain CDR2 for tavolixizumab | YIGYISYNGI TYH 13 |
| SEQ ID NO:62 heavy chain CDR3 for tavolixizumab | RYKYDYDGGH AMDY 14 |
| SEQ ID NO:63 light chain CDR1 for tavolixizumab | QDISNYLN 8 |

(continued)

| Identifier | Sequence (One-Letter Amino Acid Symbols) |
|---|---|
| SEQ ID NO:64 light chain CDR2 for tavolixizumab | LLIYYTSKLH S 11 |
| SEQ ID NO:65 light chain CDR3 for tavolixizumab | QQGSALPW 8 |

In some embodiments, the OX40 agonist is 11D4, which is a fully human antibody available from Pfizer, Inc. The preparation and properties of 11D4 are described in U.S. Patent Nos. 7,960,515; 8,236,930; and 9,028,824. The amino acid sequences of 11D4 are set forth in Table 11.

In an embodiment, a OX40 agonist comprises a heavy chain given by SEQ ID NO:66 and a light chain given by SEQ ID NO:67. In an embodiment, a OX40 agonist comprises heavy and light chains having the sequences shown in SEQ ID NO:66 and SEQ ID NO:67, respectively, or antigen binding fragments, Fab fragments, single-chain variable fragments (scFv), variants, or conjugates thereof. In an embodiment, a OX40 agonist comprises heavy and light chains that are each at least 99% identical to the sequences shown in SEQ ID NO:66 and SEQ ID NO:67, respectively. In an embodiment, a OX40 agonist comprises heavy and light chains that are each at least 98% identical to the sequences shown in SEQ ID NO:66 and SEQ ID NO:67, respectively. In an embodiment, a OX40 agonist comprises heavy and light chains that are each at least 97% identical to the sequences shown in SEQ ID NO:66 and SEQ ID NO:67, respectively. In an embodiment, a OX40 agonist comprises heavy and light chains that are each at least 96% identical to the sequences shown in SEQ ID NO:66 and SEQ ID NO:67, respectively. In an embodiment, a OX40 agonist comprises heavy and light chains that are each at least 95% identical to the sequences shown in SEQ ID NO:66 and SEQ ID NO:67, respectively.

In an embodiment, the OX40 agonist comprises the heavy and light chain CDRs or variable regions (VRs) of 11D4. In an embodiment, the OX40 agonist heavy chain variable region ($V_H$) comprises the sequence shown in SEQ ID NO:68, and the OX40 agonist light chain variable region ($V_L$) comprises the sequence shown in SEQ ID NO:69, and conservative amino acid substitutions thereof. In an embodiment, a OX40 agonist comprises $V_H$ and $V_L$ regions that are each at least 99% identical to the sequences shown in SEQ ID NO:68 and SEQ ID NO:69, respectively. In an embodiment, a OX40 agonist comprises $V_H$ and $V_L$ regions that are each at least 98% identical to the sequences shown in SEQ ID NO:68 and SEQ ID NO:69, respectively. In an embodiment, a OX40 agonist comprises $V_H$ and $V_L$ regions that are each at least 97% identical to the sequences shown in SEQ ID NO:68 and SEQ ID NO:69, respectively. In an embodiment, a OX40 agonist comprises $V_H$ and $V_L$ regions that are each at least 96% identical to the sequences shown in SEQ ID NO:68 and SEQ ID NO:69, respectively. In an embodiment, a OX40 agonist comprises $V_H$ and $V_L$ regions that are each at least 95% identical to the sequences shown in SEQ ID NO:68 and SEQ ID NO:69, respectively.

In an embodiment, a OX40 agonist comprises heavy chain CDR1, CDR2 and CDR3 domains having the sequences set forth in SEQ ID NO:70, SEQ ID NO:71, and SEQ ID NO:72, respectively, and conservative amino acid substitutions thereof, and light chain CDR1, CDR2 and CDR3 domains having the sequences set forth in SEQ ID NO:73, SEQ ID NO:74, and SEQ ID NO:75, respectively, and conservative amino acid substitutions thereof.

In an embodiment, the OX40 agonist is a OX40 agonist biosimilar monoclonal antibody approved by drug regulatory authorities with reference to 11D4. In an embodiment, the biosimilar monoclonal antibody comprises an OX40 antibody comprising an amino acid sequence which has at least 97% sequence identity, e.g., 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is 11D4. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is 11D4. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is 11D4. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is 11D4.

TABLE 11. Amino acid sequences for OX40 agonist antibodies related to 11D4.

| Identifier | Sequence (One-Letter Amino Acid Symbols) |
|---|---|
| SEQ ID NO:66 heavy chain for 11D4 | EVQLVESGGG LVQPGGSLRL SCAASGFTFS SYSMNWVRQA PGKGLEWVSY ISSSSSTIDY 60<br>ADSVKGRFTI SRDNAKNSLY LQMNSLRDED TAVYYCARES GWYLFDYWGQ GTLVTVSSAS 120<br>TKGPSVFPLA PCSRSTSEST AALGCLVKDY FPEPVTVSWN SGALTSGVHT FPAVLQSSGL 180<br>YSLSSVVTVP SSNFGTQTYT CNVDHKPSNT KVDKTVERKC CVECPPCPAP PVAGPSVFLF 240<br>PPKPKDTLMI SRTPEVTCVV VDVSHEDPEV QFNWYVDGVE VHNAKTKPRE EQFNSTFRVV 300<br>SVLTVVHQDW LNGKEYKCKV SNKGLPAPIE KTISKTKGQP REPQVYTLPP SREEMTKNQV 360<br>SLTCLVKGFY PSDIAVEWES NGQPENNYKT TPPMLDSDGS FFLYSKLTVD KSRWQQGNVF 420<br>SCSVMHEALH NHYTQKSLSL SPGK 444 |
| SEQ ID NO:67 light chain for 11D4 | DIQMTQSPSS LSASVGDRVT ITCRASQGIS SWLAWYQQKP EKAPKSLIYA ASSLQSGVPS 60<br>RFSGSGSGTD FTLTISSLQP EDFATYYCQQ YNSYPPTFGG GTKVEIKRTV AAPSVFIFPP 120<br>SDEQLKSGTA SVVCLLNNFY PREAKVQWKV DNALQSGNSQ ESVTEQDSKD STYSLSSTLT 180<br>LSKADYEKHK VYACEVTHQG LSSPVTKSFN RGEC 214 |
| SEQ ID NO:68 heavy chain variable region for 11D4 | EVQLVESGGG LVQPGGSLRL SCAASGFTFS SYSMNWVRQA PGKGLEWVSY ISSSSSTIDY 60<br>ADSVKGRFTI SRDNAKNSLY LQMNSLRDED TAVYYCARES GWYLFDYWGQ GTLVTVSS 118 |
| SEQ ID NO:69 light chain variable region for 11D4 | DIQMTQSPSS LSASVGDRVT ITCRASQGIS SWLAWYQQKP EKAPKSLIYA ASSLQSGVPS 60<br>RFSGSGSGTD FTLTISSLQP EDFATYYCQQ YNSYPPTFGG GTKVEIK 107 |
| SEQ ID NO:70 heavy chain CDR1 for 11D4 | SYSMN 5 |
| SEQ ID NO:71 heavy chain CDR2 for 11D4 | YISSSSSTID YADSVKG 17 |
| SEQ ID NO:72 heavy chain CDR3 for 11D4 | ESGWYLFDY 9 |
| SEQ ID NO:73 light chain CDR1 for 11D4 | RASQGISSWL A 11 |
| SEQ ID NO:74 light chain CDR2 for 11D4 | AASSLQS 7 |
| SEQ ID NO:75 light chain CDR3 for 11D4 | QQYNSYPPT 9 |

In some embodiments, the OX40 agonist is 18D8, which is a fully human antibody available from Pfizer, Inc. The preparation and properties of 18D8 are described in U.S. Patent Nos. 7,960,515; 8,236,930; and 9,028,824. The amino acid sequences of 18D8 are set forth in Table 12.

In an embodiment, a OX40 agonist comprises a heavy chain given by SEQ ID NO:76 and a light chain given by SEQ ID NO:77. In an embodiment, a OX40 agonist comprises heavy and light chains having the sequences shown in SEQ ID NO:76 and SEQ ID NO:77, respectively, or antigen binding fragments, Fab fragments, single-chain variable fragments (scFv), variants, or conjugates thereof. In an embodiment, a OX40 agonist comprises heavy and light chains that are each at least 99% identical to the sequences shown in SEQ ID NO:76 and SEQ ID NO:77, respectively. In an embodiment, a OX40 agonist comprises heavy and light chains that are each at least 98% identical to the sequences shown in SEQ ID NO:76 and SEQ ID NO:77, respectively. In an embodiment, a OX40 agonist comprises heavy and light chains that are each at least 97% identical to the sequences shown in SEQ ID NO:76 and SEQ ID NO:77, respectively. In an embodiment, a OX40 agonist comprises heavy and light chains that are each at least 96% identical to the sequences shown in SEQ ID NO:76 and SEQ ID NO:77, respectively. In an embodiment, a OX40 agonist comprises heavy and light chains that are each at least 95% identical to the sequences shown in SEQ ID NO:76 and SEQ ID NO:77, respectively.

In an embodiment, the OX40 agonist comprises the heavy and light chain CDRs or variable regions (VRs) of 18D8. In an embodiment, the OX40 agonist heavy chain variable region ($V_H$) comprises the sequence shown in SEQ ID NO:78, and the OX40 agonist light chain variable region ($V_L$) comprises the sequence shown in SEQ ID NO:79, and conservative amino acid substitutions thereof. In an embodiment, a OX40 agonist comprises $V_H$ and $V_L$ regions that are each at least 99% identical to the sequences shown in SEQ ID NO:78 and SEQ ID NO:79, respectively. In an embodiment, a OX40 agonist comprises $V_H$ and $V_L$ regions that are each at least 98% identical to the sequences shown in SEQ ID NO:78 and SEQ ID NO:79, respectively. In an embodiment, a OX40 agonist comprises $V_H$ and $V_L$ regions that are each at least 97% identical to the sequences shown in SEQ ID NO:78 and SEQ ID NO:79, respectively. In an embodiment, a OX40 agonist comprises $V_H$ and $V_L$ regions that are each at least 96% identical to the sequences shown in SEQ ID NO:78 and SEQ ID NO:79, respectively. In an embodiment, a OX40 agonist comprises $V_H$ and $V_L$ regions that are each at least 95% identical to the sequences shown in SEQ ID NO:78 and SEQ ID NO:79, respectively.

In an embodiment, a OX40 agonist comprises heavy chain CDR1, CDR2 and CDR3 domains having the sequences set forth in SEQ ID NO:80, SEQ ID NO:81, and SEQ ID NO:82, respectively, and conservative amino acid substitutions thereof, and light chain CDR1, CDR2 and CDR3 domains having the sequences set forth in SEQ ID NO:83, SEQ ID NO:84, and SEQ ID NO:85, respectively, and conservative amino acid substitutions thereof.

In an embodiment, the OX40 agonist is a OX40 agonist biosimilar monoclonal antibody approved by drug regulatory authorities with reference to 18D8. In an embodiment, the biosimilar monoclonal antibody comprises an OX40 antibody comprising an amino acid sequence which has at least 97% sequence identity, e.g., 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is 18D8. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is 18D8. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is 18D8. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is 18D8.

TABLE 12. Amino acid sequences for OX40 agonist antibodies related to 18D8.

| Identifier | Sequence (One-Letter Amino Acid Symbols) | |
|---|---|---|
| SEQ ID NO:76 heavy chain for 18D8 | EVQLVESGGG LVQPGRSLRL SCAASGFTFD DYAMHWVRQA PGKGLEWVSG ISWNSGSIGY | 60 |
| | ADSVKGRFTI SRDNAKNSLY LQMNSLRAED TALYYCAKDQ STADYYFYYG MDVWGQGTTV | 120 |
| | TVSSASTKGP SVFPLAPCSR STSESTAALG CLVKDYFPEP VTVSWNSGAL TSGVHTFPAV | 180 |
| | LQSSGLYSLS SVVTVPSSNF GTQTYTCNVD HKPSNTKVDK TVERKCCVEC PPCPAPPVAG | 240 |
| | PSVFLFPPKP KDTLMISRTP EVTCVVVDVS HEDPEVQFNW YVDGVEVHNA KTKPREEQFN | 300 |
| | STFRVVSVLT VVHQDWLNGK EYKCKVSNKG LPAPIEKTIS KTKGQPREPQ VYTLPPSREE | 360 |
| | MTKNQVSLTC LVKGFYPSDI AVEWESNGQP ENNYKTTPPM LDSDGSFFLY SKLTVDKSRW | 420 |
| | QQGNVFSCSV MHEALHNHYT QKSLSLSPGK | 450 |
| SEQ ID NO:77 light chain for 18D8 | EIVVTQSPAT LSLSPGERAT LSCRASQSVS SYLAWYQQKP GQAPRLLIYD ASNRATGIPA | 60 |
| | RFSGSGSGTD FTLTISSLEP EDFAVYYCQQ RSNWPTFGQG TKVEIKRTVA APSVFIFPPS | 120 |
| | DEQLKSGTAS VVCLLNNFYP REAKVQWKVD NALQSGNSQE SVTEQDSKDS TYSLSSTLTL | 180 |
| | SKADYEKHKV YACEVTHQGL SSPVTKSFNR GEC | 213 |

(continued)

| Identifier | Sequence (One-Letter Amino Acid Symbols) | |
|---|---|---|
| SEQ ID NO:78 heavy chain variable region for 18D8 | EVQLVESGGG LVQPGRSLRL SCAASGFTFD DYAMHWVRQA PGKGLEWVSG ISWNSGSIGY<br>ADSVKGRFTI SRDNAKNSLY LQMNSLRAED TALYYCAKDQ STADYYFYYG MDVWGQGTTV<br>TVSS | 60<br>120<br>124 |
| SEQ ID NO:79 light chain variable region for 18D8 | EIVVTQSPAT LSLSPGERAT LSCRASQSVS SYLAWYQQKP GQAPRLLIYD ASNRATGIPA<br>RFSGSGSGTD FTLTISSLEP EDFAVYYCQQ RSNWPTFGQG TKVEIK | 60<br>106 |
| SEQ ID NO:80 heavy chain CDR1 for 18D8 | DYAMH | 5 |
| SEQ ID NO:81 heavy chain CDR2 for 18D8 | GISWNSGSIG YADSVKG | 17 |
| SEQ ID NO:82 heavy chain CDR3 for 18D8 | DQSTADYYFY YGMDV | 15 |
| SEQ ID NO:83 light chain CDR1 for 18D8 | RASQSVSSYL A | 11 |
| SEQ ID NO:84 light chain CDR2 for 18D8 | DASNRAT | 7 |
| SEQ ID NO:85 light chain CDR3 for 18D8 | QQRSNWPT | 8 |

In some embodiments, the OX40 agonist is Hu119-122, which is a humanized antibody available from Glaxo-SmithKline plc. The preparation and properties of Hu119-122 are described in U.S. Patent Nos. 9,006,399 and 9,163,085, and in International Patent Publication No. WO 2012/027328. The amino acid sequences of Hu119-122 are set forth in Table 13.

In an embodiment, the OX40 agonist comprises the heavy and light chain CDRs or variable regions (VRs) of Hu119-122. In an embodiment, the OX40 agonist heavy chain variable region ($V_H$) comprises the sequence shown in SEQ ID NO:86, and the OX40 agonist light chain variable region ($V_L$) comprises the sequence shown in SEQ ID NO:87, and conservative amino acid substitutions thereof. In an embodiment, a OX40 agonist comprises $V_H$ and $V_L$ regions that are each at least 99% identical to the sequences shown in SEQ ID NO:86 and SEQ ID NO:87, respectively. In an embodiment, a OX40 agonist comprises $V_H$ and $V_L$ regions that are each at least 98% identical to the sequences shown in SEQ ID NO:86 and SEQ ID NO:87, respectively. In an embodiment, a OX40 agonist comprises $V_H$ and $V_L$ regions that are each at least 97% identical to the sequences shown in SEQ ID NO:86 and SEQ ID NO:87, respectively. In an embodiment, a OX40 agonist comprises $V_H$ and $V_L$ regions that are each at least 96% identical to the sequences shown in SEQ ID NO:86 and SEQ ID NO:87, respectively. In an embodiment, a OX40 agonist comprises $V_H$ and $V_L$ regions that are each at least 95% identical to the sequences shown in SEQ ID NO:86 and SEQ ID NO:87, respectively.

In an embodiment, a OX40 agonist comprises heavy chain CDR1, CDR2 and CDR3 domains having the sequences set forth in SEQ ID NO:88, SEQ ID NO:89, and SEQ ID NO:90, respectively, and conservative amino acid substitutions thereof, and light chain CDR1, CDR2 and CDR3 domains having the sequences set forth in SEQ ID NO:91, SEQ ID NO:92, and SEQ ID NO:93, respectively, and conservative amino acid substitutions thereof.

In an embodiment, the OX40 agonist is a OX40 agonist biosimilar monoclonal antibody approved by drug regulatory authorities with reference to Hu119-122. In an embodiment, the biosimilar monoclonal antibody comprises an OX40 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is Hu119-122. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is Hu119-122. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is Hu119-122. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is Hu119-122.

TABLE 13. Amino acid sequences for OX40 agonist antibodies related to Hu119-122.

| Identifier | Sequence (One-Letter Amino Acid Symbols) |
|---|---|
| SEQ ID NO:86 heavy chain variable region for Hu119-122 | EVQLVESGGG LVQPGGSLRL SCAASEYEFP SHDMSWVRQA PGKGLELVAA INSDGGSTYY    60<br>PDTMERRFTI SRDNAKNSLY LQMNSLRAED TAVYYCARHY DDYYAWFAYW GQGTMVTVSS   120 |
| SEQ ID NO:87 light chain variable region for Hu119-122 | EIVLTQSPAT LSLSPGERAT LSCRASKSVS TSGYSYMHWY QQKPGQAPRL LIYLASNLES    60<br>GVPARFSGSG SGTDFTLTIS SLEPEDFAVY YCQHSRELPL TFGGGTKVEI K   111 |
| SEQ ID NO:88 heavy chain CDR1 for Hu119-122 | SHDMS                                                       5 |
| SEQ ID NO:89 heavy chain CDR2 for Hu119-122 | AINSDGGSTY YPDTMER                                    17 |
| SEQ ID NO:90 heavy chain CDR3 for Hu119-122 | HYDDYYAWFA Y                                        11 |
| SEQ ID NO:91 light chain CDR1 for Hu119-122 | RASKSVSTSG YSYMH                                      15 |
| SEQ ID NO:92 light chain CDR2 for Hu119-122 | LASNLES                                             7 |

(continued)

| Identifier | Sequence (One-Letter Amino Acid Symbols) | |
|---|---|---|
| SEQ ID NO:93 light chain CDR3 for Hu119-122 | QHSRELPLT | 9 |

In some embodiments, the OX40 agonist is Hu106-222, which is a humanized antibody available from Glaxo-SmithKline plc. The preparation and properties of Hu 106-222 are described in U.S. Patent Nos. 9,006,399 and 9,163,085, and in International Patent Publication No. WO 2012/027328. The amino acid sequences of Hu106-222 are set forth in Table 14.

In an embodiment, the OX40 agonist comprises the heavy and light chain CDRs or variable regions (VRs) of Hu106-222. In an embodiment, the OX40 agonist heavy chain variable region ($V_H$) comprises the sequence shown in SEQ ID NO:94, and the OX40 agonist light chain variable region ($V_L$) comprises the sequence shown in SEQ ID NO:95, and conservative amino acid substitutions thereof. In an embodiment, a OX40 agonist comprises $V_H$ and $V_L$ regions that are each at least 99% identical to the sequences shown in SEQ ID NO:94 and SEQ ID NO:95, respectively. In an embodiment, a OX40 agonist comprises $V_H$ and $V_L$ regions that are each at least 98% identical to the sequences shown in SEQ ID NO:94 and SEQ ID NO:95, respectively. In an embodiment, a OX40 agonist comprises $V_H$ and $V_L$ regions that are each at least 97% identical to the sequences shown in SEQ ID NO:94 and SEQ ID NO:95, respectively. In an embodiment, a OX40 agonist comprises $V_H$ and $V_L$ regions that are each at least 96% identical to the sequences shown in SEQ ID NO:94 and SEQ ID NO:95, respectively. In an embodiment, a OX40 agonist comprises $V_H$ and $V_L$ regions that are each at least 95% identical to the sequences shown in SEQ ID NO:94 and SEQ ID NO:95, respectively.

In an embodiment, a OX40 agonist comprises heavy chain CDR1, CDR2 and CDR3 domains having the sequences set forth in SEQ ID NO:96, SEQ ID NO:97, and SEQ ID NO:98, respectively, and conservative amino acid substitutions thereof, and light chain CDR1, CDR2 and CDR3 domains having the sequences set forth in SEQ ID NO:99, SEQ ID NO: 100, and SEQ ID NO: 101, respectively, and conservative amino acid substitutions thereof.

In an embodiment, the OX40 agonist is a OX40 agonist biosimilar monoclonal antibody approved by drug regulatory authorities with reference to Hu 106-222. In an embodiment, the biosimilar monoclonal antibody comprises an OX40 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is Hu 106-222. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is Hu106-222. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is Hu 106-222. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is Hu 106-222.

TABLE 14. Amino acid sequences for OX40 agonist antibodies related to Hu106-222.

| Identifier | Sequence (One-Letter Amino Acid Symbols) | |
|---|---|---|
| SEQ ID NO:94 heavy chain variable region for Hu106-222 | QVQLVQSGSE LKKPGASVKV SCKASGYTFT DYSMHWVRQA PGQGLKWMGW INTETGEPTY<br>ADDFKGRFVF SLDTSVSTAY LQISSLKAED TAVYYCANPY YDYVSYYAMD YWGQGTTVTV<br>SS | 60<br>120<br>122 |

(continued)

| Identifier | Sequence (One-Letter Amino Acid Symbols) |
|---|---|
| SEQ ID NO:95 light chain variable region for Hu106-222 | DIQMTQSPSS LSASVGDRVT ITCKASQDVS TAVAWYQQKP GKAPKLLIYS ASYLYTGVPS 60<br>RFSGSGSGTD FTFTISSLQP EDIATYYCQQ HYSTPRTFGQ GTKLEIK 107 |
| SEQ ID NO:96 heavy chain CDR1 for Hul06-222 | DYSMH |
| SEQ ID NO:97 heavy chain CDR2 for Hu106-222 | WINTETGEPT YADDFKG |
| SEQ ID NO:98 heavy chain CDR3 for Hu106-222 | PYYDYVSYYA MDY |
| SEQ ID NO:99 light chain CDR1 for Hu106-222 | KASQDVSTAV A |
| SEQ ID NO:100 light chain CDR2 for Hu106-222 | SASYLYT |
| SEQ ID NO:101 light chain CDR3 for Hu106-222 | QQHYSTPRT |

In some embodiments, the OX40 agonist antibody is MEDI6469 (also referred to as 9B12). MEDI6469 is a murine monoclonal antibody. Weinberg, et al., J. Immunother., 2006, 29, 575-585. In some embodiments the OX40 agonist is an antibody produced by the 9B12 hybridoma, deposited with Biovest Inc. (Malvern, MA, USA), as described in Weinberg, et al., J. Immunother., 2006, 29, 575-585. In some embodiments, the antibody comprises the CDR sequences of MEDI6469. In some embodiments, the antibody comprises a heavy chain variable region sequence and/or a light chain variable region sequence of MEDI6469.

In an embodiment, the OX40 agonist is L106 BD (Pharmingen Product #340420). In some embodiments, the OX40 agonist comprises the CDRs of antibody L106 (BD Pharmingen Product #340420). In some embodiments, the OX40 agonist comprises a heavy chain variable region sequence and/or a light chain variable region sequence of antibody L106 (BD Pharmingen Product #340420). In an embodiment, the OX40 agonist is ACT35 (Santa Cruz Biotechnology, Catalog #20073). In some embodiments, the OX40 agonist comprises the CDRs of antibody ACT35 (Santa Cruz Biotechnology, Catalog #20073). In some embodiments, the OX40 agonist comprises a heavy chain variable region sequence and/or a light chain variable region sequence of antibody ACT35 (Santa Cruz Biotechnology, Catalog #20073). In an embodiment, the OX40 agonist is the murine monoclonal antibody anti-mCD134/mOX40 (clone OX86), commercially available from InVivoMAb, BioXcell Inc, West Lebanon, NH.

In an embodiment, the OX40 agonist is selected from the OX40 agonists described in International Patent Application Publication Nos. WO 95/12673, WO 95/21925, WO 2006/121810, WO 2012/027328, WO 2013/028231, WO 2013/038191, and WO 2014/148895; European Patent Application EP 0672141; U.S. Patent Application Publication Nos. US 2010/136030, US 2014/377284, US 2015/190506, and US 2015/132288 (including clones 20E5 and 12H3); and U.S. Patent Nos. 7,504,101, 7,550,140, 7,622,444, 7,696,175, 7,960,515, 7,961,515, 8,133,983, 9,006,399, and 9,163,085.

In an embodiment, the OX40 agonist is an OX40 agonistic fusion protein as depicted in Structure I-A (C-terminal Fc-antibody fragment fusion protein) or Structure I-B (N-terminal Fc-antibody fragment fusion protein), or a fragment, derivative, conjugate, variant, or biosimilar thereof. The properties of structures I-A and I-B are described above and in U.S. Patent Nos. 9,359,420, 9,340,599, 8,921,519, and 8,450,460. Amino acid sequences for the polypeptide domains of structure I-A are given in Table 6. The Fc domain preferably comprises a complete constant domain (amino acids 17-230 of SEQ ID NO:31) the complete hinge domain (amino acids 1-16 of SEQ ID NO:31) or a portion of the hinge domain (e.g., amino acids 4-16 of SEQ ID NO:31). Preferred linkers for connecting a C-terminal Fc-antibody may be selected from the embodiments given in SEQ ID NO:32 to SEQ ID NO:41, including linkers suitable for fusion of additional polypeptides. Likewise, amino acid sequences for the polypeptide domains of structure I-B are given in Table 7. If an Fc antibody fragment is fused to the N-terminus of an TNRFSF fusion protein as in structure I-B, the sequence of the Fc module is preferably that shown in SEQ ID NO:42, and the linker sequences are preferably selected from those embodiments set forth in SED ID NO:43 to SEQ ID NO:45.

In an embodiment, an OX40 agonist fusion protein according to structures I-A or I-B comprises one or more OX40 binding domains selected from the group consisting of a variable heavy chain and variable light chain of tavolixizumab, a variable heavy chain and variable light chain of 11D4, a variable heavy chain and variable light chain of 18D8, a variable heavy chain and variable light chain of Hu119-122, a variable heavy chain and variable light chain of Hu106-222, a variable heavy chain and variable light chain selected from the variable heavy chains and variable light chains described in Table 19, any combination of a variable heavy chain and variable light chain of the foregoing, and fragments, derivatives, conjugates, variants, and biosimilars thereof.

In an embodiment, an OX40 agonist fusion protein according to structures I-A or I-B comprises one or more OX40 binding domains comprising an OX40L sequence. In an embodiment, an OX40 agonist fusion protein according to structures I-A or I-B comprises one or more OX40 binding domains comprising a sequence according to SEQ ID NO:102. In an embodiment, an OX40 agonist fusion protein according to structures I-A or I-B comprises one or more OX40 binding domains comprising a soluble OX40L sequence. In an embodiment, a OX40 agonist fusion protein according to structures I-A or I-B comprises one or more OX40 binding domains comprising a sequence according to SEQ ID NO:103. In an embodiment, a OX40 agonist fusion protein according to structures I-A or I-B comprises one or more OX40 binding domains comprising a sequence according to SEQ ID NO: 104.

In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:58 and SEQ ID NO:59, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:68 and SEQ ID NO:69, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:78 and SEQ ID NO:79, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:86 and SEQ ID NO:87, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:94 and SEQ ID NO:95, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the $V_H$ and $V_L$ sequences given in Table 15, wherein the $V_H$ and $V_L$ domains are connected by a linker.

TABLE 15. Additional polypeptide domains useful as OX40 binding domains in fusion proteins (*e.g.*, structures I-A and I-B) or as scFv OX40 agonist antibodies.

| Identifier | Sequence (One-Letter Amino Acid Symbols) | |
|---|---|---|
| SEQ ID NO:102 OX40L | MERVQPLEEN VGNAARPRFE RNKLLLVASV IQGLGLLLCF TYICLHFSAL QVSHRYPRIQ<br>SIKVQFTEYK KEKGFILTSQ KEDEIMKVQN NSVIINCDGF YLISLKGYFS QEVNISLHYQ<br>KDEEPLFQLK KVRSVNSLMV ASLTYKDKVY LNVTTDNTSL DDFHVNGGEL ILIHQNPGEF<br>CVL | 60<br>120<br>180<br>183 |
| SEQ ID NO:103 OX40L soluble domain | SHRYPRIQSI KVQFTEYKKE KGFILTSQKE DEIMKVQNNS VIINCDGFYL ISLKGYFSQE<br>VNISLHYQKD EEPLFQLKKV RSVNSLMVAS LTYKDKVYLN VTTDNTSLDD FHVNGGELIL<br>IHQNPGEFCV L | 60<br>120<br>131 |
| SEQ ID NO:104 OX40L soluble domain (alternative) | YPRIQSIKVQ FTEYKKEKGF ILTSQKEDEI MKVQNNSVII NCDGFYLISL KGYFSQEVNI<br>SLHYQKDEEP LFQLKKVRSV NSLMVASLTY KDKVYLNVTT DNTSLDDFHV NGGELILIHQ<br>NPGEFCVL | 60<br>120<br>128 |
| SEQ ID NO:105 variable heavy chain for 008 | EVQLVESGGG LVQPGGSLRL SCAASGFTFS NYTMNWVRQA PGKGLEWVSA ISGSGGSTYY<br>ADSVKGRFTI SRDNSKNTLY LQMNSLRAED TAVYYCAKDR YSQVHYALDY WGQGTLVTVS | 60<br>120 |

(continued)

| Identifier | Sequence (One-Letter Amino Acid Symbols) | |
|---|---|---|
| SEQ ID NO:106 variable light chain for 008 | DIVMTQSPDS LPVTPGEPAS ISCRSSQSLL HSNGYNYLDW YLQKAGQSPQ LLIYLGSNRA<br>SGVPDRFSGS GSGTDFTLKI SRVEAEDVGV YYCQQYYNHP TTFGQGTK | 60<br>108 |
| SEQ ID NO:107 variable heavy chain for 011 | EVQLVESGGG VVQPGRSLRL SCAASGFTFS DYTMNWVRQA PGKGLEWVSS ISGGSTYYAD<br>SRKGRFTISR DNSKNTLYLQ MNNLRAEDTA VYYCARDRYF RQQNAFDYWG QGTLVTVSSA | 60<br>120 |
| SEQ ID NO:108 variable light chain for 011 | DIVMTQSPDS LPVTPGEPAS ISCRSSQSLL HSNGYNYLDW YLQKAGQSPQ LLIYLGSNRA<br>SGVPDRFSGS GSGTDFTLKI SRVEAEDVGV YYCQQYYNHP TTFGQGTK | 60<br>108 |
| SEQ ID NO:109 variable heavy chain for 021 | EVQLVESGGG LVQPRGSLRL SCAASGFTFS SYAMNWVRQA PGKGLEWVAV ISYDGSNKYY<br>ADSVKGRFTI SRDNSKNTLY LQMNSLRAED TAVYYCAKDR YITLPNALDY WGQGTLVTVS | 60<br>120 |
| SEQ ID NO:110 variable light chain for 021 | DIQMTQSPVS LPVTPGEPAS ISCRSSQSLL HSNGYNYLDW YLQKPGQSPQ LLIYLGSNRA<br>SGVPDRFSGS GSGTDFTLKI SRVEAEDVGV YYCQQYKSNP PTFGQGTK | 60<br>108 |
| SEQ ID NO:111 variable heavy chain for 023 | EVQLVESGGG LVHPGGSLRL SCAGSGFTFS SYAMHWVRQA PGKGLEWVSA IGTGGGTYYA<br>DSVMGRFTIS RDNSKNTLYL QMNSLRAEDT AVYYCARYDN VMGLYWFDYW GQGTLVTVSS | 60<br>120 |
| SEQ ID NO:112 variable light chain for 023 | EIVLTQSPAT LSLSPGERAT LSCRASQSVS SYLAWYQQKP GQAPRLLIYD ASNRATGIPA<br>RFSGSGSGTD FTLTISSLEP EDFAVYYCQQ RSNWPPAFGG GTKVEIKR | 60<br>108 |
| SEQ ID NO:113 heavy chain variable region | EVQLQQSGPE LVKPGASVKM SCKASGYTFT SYVMHWVKQK PGQGLEWIGY INPYNDGTKY<br>NEKFKGKATL TSDKSSSTAY MELSSLTSED SAVYYCANYY GSSLSMDYWG QGTSVTVSS | 60<br>119 |
| SEQ ID NO:114 light chain variable region | DIQMTQTTSS LSASLGDRVT ISCRASQDIS NYLNWYQQKP DGTVKLLIYY TSRLHSGVPS<br>RFSGSGSGTD YSLTISNLEQ EDIATYFCQQ GNTLPWTFGG GTKLEIKR | 60<br>108 |
| SEQ ID NO:115 heavy chain variable region | EVQLQQSGPE LVKPGASVKI SCKTSGYTFK DYTMHWVKQS HGKSLEWIGG IYPNNGGSTY<br>NQNFKDKATL TVDKSSSTAY MEFRSLTSED SAVYYCARMG YHGPHLDFDV WGAGTTVTVS<br>P | 60<br>120<br>121 |
| SEQ ID NO:116 light chain variable region | DIVMTQSHKF MSTSLGDRVS ITCKASQDVG AAVAWYQQKP GQSPKLLIYW ASTRHTGVPD<br>RFTGGGSGTD FTLTISNVQS EDLTDYFCQQ YINYPLTFGG GTKLEIKR | 60<br>108 |
| SEQ ID NO:117 heavy chain variable region of humanized antibody | QIQLVQSGPE LKKPGETVKI SCKASGYTFT DYSMHWVKQA PGKGLKWMGW INTETGEPTY<br>ADDFKGRFAF SLETSASTAY LQINNLKNED TATYFCANPY YDYVSYYAMD YWGHGTSVTV<br>SS | 60<br>120<br>122 |
| SEQ ID NO:118 heavy chain variable region of humanized antibody | QVQLVQSGSE LKKPGASVKV SCKASGYTFT DYSMHWVRQA PGQGLKWMGW INTETGEPTY<br>ADDFKGRFVF SLDTSVSTAY LQISSLKAED TAVYYCANPY YDYVSYYAMD YWGQGTTVTV<br>SS | 60<br>120<br>122 |
| SEQ ID NO:119 light chain variable region of humanized antibody | DIVMTQSHKF MSTSVRDRVS ITCKASQDVS TAVAWYQQKP GQSPKLLIYS ASYLYTGVPD<br>RFTGSGSGTD FTFTISSVQA EDLAVYYCQQ HYSTPRTFGG GTKLEIK | 60<br>107 |

(continued)

| Identifier | Sequence (One-Letter Amino Acid Symbols) | |
|---|---|---|
| SEQ ID NO:120 light chain variable region of humanized antibody | DIVMTQSHKF MSTSVRDRVS ITCKASQDVS TAVAWYQQKP GQSPKLLIYS ASYLYTGVPD<br>RFTGSGSGTD FTFTISSVQA EDLAVYYCQQ HYSTPRTFGG GTKLEIK | 60<br>107 |
| SEQ ID NO:121 heavy chain variable region of humanized antibody | EVQLVESGGG LVQPGESLKL SCESNEYEFP SHDMSWVRKT PEKRLELVAA INSDGGSTYY<br>PDTMERRFII SRDNTKKTLY LQMSSLRSED TALYYCARHY DDYYAWFAYW GQGTLVTVSA | 60<br>120 |
| SEQ ID NO:122 heavy chain variable region of humanized antibody | EVQLVESGGG LVQPGGSLRL SCAASEYEFP SHDMSWVRQA PGKGLELVAA INSDGGSTYY<br>PDTMERRFTI SRDNAKNSLY LQMNSLRAED TAVYYCARHY DDYYAWFAYW GQGTMVTVSS | 60<br>120 |
| SEQ ID NO:123 light chain variable region of humanized antibody | DIVLTQSPAS LAVSLGQRAT ISCRASKSVS TSGYSYMHWY QQKPGQPPKL LIYLASNLES<br>GVPARFSGSG SGTDFTLNIH PVEEEDAATY YCQHSRELPL TFGAGTKLEL K | 60<br>111 |
| SEQ ID NO:124 light chain variable region of humanized antibody | EIVLTQSPAT LSLSPGERAT LSCRASKSVS TSGYSYMHWY QQKPGQAPRL LIYLASNLES<br>GVPARFSGSG SGTDFTLTIS SLEPEDFAVY YCQHSRELPL TFGGGTKVEI K | 60<br>111 |
| SEQ ID NO:125 heavy chain variable region | MYLGLNYVFI VFLLNGVQSE VKLEESGGGL VQPGGSMKLS CAASGFTFSD AWMDWVRQSP<br>EKGLEWVAEI RSKANNHATY YAESVNGRFT ISRDDSKSSV YLQMNSLRAE DTGIYYCTWG<br>EVFYFDYWGQ GTTLTVSS | 60<br>120<br>138 |
| SEQ ID NO:126 light chain variable region | MRPSIQFLGL LLFWLHGAQC DIQMTQSPSS LSASLGGKVT ITCKSSQDIN KYIAWYQHKP<br>GKGPRLLIHY TSTLQPGIPS RFSGSGSGRD YSFSISNLEP EDIATYYCLQ YDNLLTFGAG<br>TKLELK | 60<br>120<br>126 |

In an embodiment, the OX40 agonist is a OX40 agonistic single-chain fusion polypeptide comprising (i) a first soluble OX40 binding domain, (ii) a first peptide linker, (iii) a second soluble OX40 binding domain, (iv) a second peptide linker, and (v) a third soluble OX40 binding domain, further comprising an additional domain at the N-terminal and/or C-terminal end, and wherein the additional domain is a Fab or Fc fragment domain. In an embodiment, the OX40 agonist is a OX40 agonistic single-chain fusion polypeptide comprising (i) a first soluble OX40 binding domain, (ii) a first peptide linker, (iii) a second soluble OX40 binding domain, (iv) a second peptide linker, and (v) a third soluble OX40 binding domain, further comprising an additional domain at the N-terminal and/or C-terminal end, wherein the additional domain is a Fab or Fc fragment domain wherein each of the soluble OX40 binding domains lacks a stalk region (which contributes to trimerisation and provides a certain distance to the cell membrane, but is not part of the OX40 binding domain) and the first and the second peptide linkers independently have a length of 3-8 amino acids.

In an embodiment, the OX40 agonist is an OX40 agonistic single-chain fusion polypeptide comprising (i) a first soluble tumor necrosis factor (TNF) superfamily cytokine domain, (ii) a first peptide linker, (iii) a second soluble TNF superfamily cytokine domain, (iv) a second peptide linker, and (v) a third soluble TNF superfamily cytokine domain, wherein each of the soluble TNF superfamily cytokine domains lacks a stalk region and the first and the second peptide linkers independently have a length of 3-8 amino acids, and wherein the TNF superfamily cytokine domain is an OX40 binding domain.

In some embodiments, the OX40 agonist is MEDI6383. MEDI6383 is an OX40 agonistic fusion protein and can be prepared as described in U.S. Patent No. 6,312,700.

In an embodiment, the OX40 agonist is an OX40 agonistic scFv antibody comprising any of the foregoing $V_H$

domains linked to any of the foregoing $V_L$ domains.

In an embodiment, the OX40 agonist is Creative Biolabs OX40 agonist monoclonal antibody MOM-18455, commercially available from Creative Biolabs, Inc., Shirley, NY, USA.

In an embodiment, the OX40 agonist is OX40 agonistic antibody clone Ber-ACT35, commercially available from BioLegend, Inc., San Diego, CA, USA.

Option Cell Viability Analyses

Optionally, a cell viability assay can be performed after the first expansion (sometimes referred to as the initial bulk expansion), using standard assays known in the art. For example, a trypan blue exclusion assay can be done on a sample of the bulk TILs, which selectively labels dead cells and allows a viability assessment. Other assays for use in testing viability can include but are not limited to the Alamar blue assay; and the MTT assay.

*Cell Counts, Viability, Flow Cytometry*

In some embodiments, cell counts and/or viability are measured. The expression of markers such as but not limited CD3, CD4, CD8, and CD56, as well as any other disclosed or described herein, can be measured by flow cytometry with antibodies, for example but not limited to those commercially available from BD Bio-sciences (BD Biosciences, San Jose, CA) using a FACSCantoTM flow cytometer (BD Biosciences). The cells can be counted manually using a disposable c-chip hemocytometer (VWR, Batavia, IL) and viability can be assessed using any method known in the art, including but not limited to trypan blue staining. The cell viability can also be assayed based on US Application 15/863,634, "Processes for Production of Tumor Infiltrating Lymphocytes and Uses of Same in Immunotherapy,".

In some cases, the bulk TIL population can be cryopreserved immediately, using the protocols discussed below. Alternatively, the bulk TIL population can be subjected to REP and then cryopreserved as discussed below. Similarly, in the case where genetically modified TILs will be used in therapy, the bulk or REP TIL populations can be subjected to genetic modifications for suitable treatments.

Disclosed herein but not claimed is a method for assaying TILs for viability and/or further use in administration to a subject. In some embodiments, the method for assay tumor infiltrating lymphocytes (TILs) comprises:

(i) obtaining a first population of TILs;

(ii) performing a first expansion by culturing the first population of TILs in a cell culture medium comprising IL-2, and optionally OKT-3, to produce a second population of TILs; and

(iii) performing a second expansion by supplementing the cell culture medium of the second population of TILs with additional IL-2, OKT-3, and antigen presenting cells (APCs), to produce a third population of TILs, wherein the third population of TILs is at least 50-fold greater in number than the second population of TILs;

(iv) harvesting, washing, and cryopreserving the third population of TILs;

(v) storing the cryopreserved TILs at a cryogenic temperature;

(vi) thawing the third population of TILs to provide a thawed third population of TILs; and

(vii) performing an additional second expansion of a portion of the thawed third population of TILs by supplementing the cell culture medium of the third population with IL-2, OKT-3, and APCs for an additional expansion period (sometimes referred to as a reREP period) of at least 3 days, wherein the third expansion is performed to obtain a fourth population of TILs, wherein the number of TILs in the fourth population of TILs is compared to the number of TILs in the third population of TILs to obtain a ratio;

(viii) determining based on the ratio in step (vii) whether the thawed population of TILs is suitable for administration to a patient;

(ix) administering a therapeutically effective dosage of the thawed third population of TILs to the patient when the ratio of the number of TILs in the fourth population of TILs to the number of TILs in the third population of TILs is determined to be greater than 5:1 in step (viii).

Disclosed herein but not claimed is a method whereinthe TILs are assayed for viability after step (vii).

Disclosed herein but not claimed are methods for assaying TILs. In some embodiments, the disclosure provides a method for assaying TILs comprising:

(i) obtaining a portion of a first population of cryopreserved TILs;
(ii) thawing the portion of the first population of cryopreserved TILs;
(iii) performing a first expansion by culturing the portion of the first population of TILs in a cell culture medium comprising IL-2, OKT-3, and antigen presenting cells (APCs) for an additional expansion period (sometimes referred to as a reREP period) of at least 3 days, to produce a second population of TILs, wherein the portion from the first population of TILs is compared to the second population of TILs to obtain a ratio of the number of TILs, wherein the ratio of the number of TILs in the second population of TILs to the number of TILs in the portion of the first population of TILs is greater than 5:1;
(iv) determining based on the ratio in step (iii) whether the first population of TILs is suitable for use in therapeutic administration to a patient;
(v) determining the first population of TILs is suitable for use in therapeutic administration when the ratio of the number of TILs in the second population of TILs to the number of TILs in the first population of TILs is determined to be greater than 5:1 in step (iv).

Disclosed herein but not claimed is a method wherein the ratio of the number of TILs in the second population of TILs to the number of TILs in the portion of the first population of TILs is greater than 50:1.

Disclosed herein but not claimed is a method further comprising performing expansion of the entire first population of cryopreserved TILs from step (i) according to the methods as described in any of the embodiments provided herein.

Disclosed herein but not claimed is a method further comprising administering the entire first population of cryopreserved TILs from step (i) to the patient.

Cell Culture Media

In an embodiment, a method for expanding TILs, including those discussed above as well as exemplified in Figure 1, may include using about 5,000 mL to about 25,000 mL of cell medium, about 5,000 mL to about 10,000 mL of cell medium, or about 5,800 mL to about 8,700 mL of cell medium. In some embodiments, the media is a serum free medium. In some embodiments, the media in the first expansion is serum free. In some embodiments, the media in the second expansion is serum free. In some embodiments, the media in the first expansion and the second are both serum free. In an embodiment, expanding the number of TILs uses no more than one type of cell culture medium. Any suitable cell culture medium may be used, *e.g.,* AIM-V cell medium (L-glutamine, 50 μM streptomycin sulfate, and 10 μM gentamicin sulfate) cell culture medium (Invitrogen, Carlsbad CA). In this regard, the inventive methods advantageously reduce the amount of medium and the number of types of medium required to expand the number of TIL. In an embodiment, expanding the number of TIL may comprise feeding the cells no more frequently than every third or fourth day. Expanding the number of cells in a gas permeable container simplifies the procedures necessary to expand the number of cells by reducing the feeding frequency necessary to expand the cells.

In some embodiments, the culture medium used in the expansion processes disclosed herein is a serum-free medium or a defined medium. In some embodiments, the serum-free or defined medium comprises a basal cell medium and a serum supplement and/or a serum replacement. In some embodiments, the serum-free or defined medium is used to prevent and/or decrease experimental variation due in part to the lot-to-lot variation of serum-containing media.

In some embodiments, the serum-free or defined medium comprises a basal cell medium and a serum supplement and/or serum replacement. In some embodiments, the basal cell medium includes, but is not limited to CTS™ OpTmizer™ T-cell Expansion Basal Medium , CTS™ OpTmizer™ T-Cell Expansion SFM, CTS™ AIM-V Medium, CTS™ AIM-V SFM, LymphoONE™ T-Cell Expansion Xeno-Free Medium, Dulbecco's Modified Eagle's Medium (DMEM), Minimal Essential Medium (MEM), Basal Medium Eagle (BME), RPMI 1640, F-10, F-12, Minimal Essential Medium (αMEM), Glasgow's Minimal Essential Medium (G-MEM), RPMI growth medium, and Iscove's Modified Dulbecco's Medium.

In some embodiments, the serum supplement or serum replacement includes, but is not limited to one or more of CTS™ OpTmizer T-Cell Expansion Serum Supplement, CTS™ Immune Cell Serum Replacement, one or more albumins or albumin substitutes, one or more amino acids, one or more vitamins, one or more transferrins or transferrin substitutes, one or more antioxidants, one or more insulins or insulin substitutes, one or more collagen precursors, one or more antibiotics, and one or more trace elements. In some embodiments, the defined medium comprises albumin and one or more ingredients selected from the group consisting of glycine, L- histidine, L-isoleucine, L-methionine, L-phenylalanine, L-proline, L- hydroxyproline, L-serine, L-threonine, L-tryptophan, L-tyrosine, L-valine, thiamine, reduced glutathione, L-ascorbic acid-2-phosphate, iron saturated transferrin, insulin, and compounds containing the trace element moieties $Ag^+$, $Al^{3+}$, $Ba^{2+}$, $Cd^{2+}$, $Co^{2+}$, $Cr^{3"}$, $Ge^{4+}$, $Se^{4+}$, Br, T, $Mn^{2+}$, P, $Si^{4+}$, $V^{5+}$, $Mo^{6+}$, $Ni^{2+}$, $Rb^+$, $Sn^{2+}$ and $Zr^{4+}$. In

some embodiments, the defined medium further comprises L-glutamine, sodium bicarbonate and/or 2-mercaptoethanol.

In some embodiments, the CTS™ OpTmizer™ T-cell Immune Cell Serum Replacement is used with conventional growth media, including but not limited to CTS™ OpTmizer™ T-cell Expansion Basal Medium, CTS™ OpTmizer™ T-cell Expansion SFM, CTS™ AIM-V Medium, CST™ AIM-V SFM, LymphoONE™ T-Cell Expansion Xeno-Free Medium, Dulbecco's Modified Eagle's Medium (DMEM), Minimal Essential Medium (MEM), Basal Medium Eagle (BME), RPMI 1640, F-10, F-12, Minimal Essential Medium ($\alpha$MEM), Glasgow's Minimal Essential Medium (G-MEM), RPMI growth medium, and Iscove's Modified Dulbecco's Medium.

In some embodiments, the total serum replacement concentration (vol%) in the serum-free or defined medium is from about 1%, 2%, 3%, 4%, 5%, 6%, 7%, 8%, 9%, 10%, 11%, 12%, 13%, 14%, 15%, 16%, 17%, 18%, 19%, or 20% by volume of the total serum-free or defined medium. In some embodiments, the total serum replacement concentration is about 3% of the total volume of the serum-free or defined medium. In some embodiments, the total serum replacement concentration is about 5% of the total volume of the serum-free or defined medium. In some embodiments, the total serum replacement concentration is about 10% of the total volume of the serum-free or defined medium.

In some embodiments, the serum-free or defined medium is CTS™ OpTmizer™ T-cell Expansion SFM (ThermoFisher Scientific). Any formulation of CTS™ OpTmizer™ is useful in the present invention. CTS™ OpTmizer™ T-cell Expansion SFM is a combination of 1L CTS™ OpTmizer™ T-cell Expansion Basal Medium and 26 mL CTS™ OpTmizer™ T-Cell Expansion Supplement, which are mixed together prior to use. In some embodiments, the CTS™ OpTmizer™ T-cell Expansion SFM is supplemented with about 3% of the CTS™ Immune Cell Serum Replacement (SR) (ThermoFisher Scientific), along with 2-mercaptoethanol at 55mM.

In some embodiments, the defined medium is CTS™ OpTmizer™ T-cell Expansion SFM (ThermoFisher Scientific). Any formulation of CTS™ OpTmizer™ is useful in the present invention. CTS™ OpTmizer™ T-cell Expansion SFM is a combination of 1L CTS™ OpTmizer™ T-cell Expansion Basal Medium and 26 mL CTS™ OpTmizer™ T-Cell Expansion Supplement, which are mixed together prior to use. In some embodiments, the CTS™ OpTmizer™ T-cell Expansion SFM is supplemented with about 3% of the CTS™ Immune Cell Serum Replacement (SR) (ThermoFisher Scientific), along with 2-mercaptoethanol at 55mM. In some embodiments, the CTS™OpTmizer™ T-cell Expansion SFM is supplemented with about 3% of the CTS™ Immune Cell Serum Replacement (SR) (ThermoFisher Scientific), 55mM of 2-mercaptoethanol, and 2mM of L-glutamine. In some embodiments, the CTS™OpTmizer™ T-cell Expansion SFM is supplemented with about 3% of the CTS™ Immune Cell Serum Replacement (SR) (ThermoFisher Scientific), 55mM of 2-mercaptoethanol, and 2mM of L-glutamine, and further comprises about 1000 IU/mL to about 8000 IU/mL of IL-2. In some embodiments, the CTS™OpTmizer™ T-cell Expansion SFM is supplemented with about 3% of the CTS™ Immune Cell Serum Replacement (SR) (ThermoFisher Scientific), 55mM of 2-mercaptoethanol, and 2mM of L-glutamine, and further comprises about 3000 IU/mL of IL-2. In some embodiments, the CTS™OpTmizer™ T-cell Expansion SFM is supplemented with about 3% of the CTS™ Immune Cell Serum Replacement (SR) (ThermoFisher Scientific), 55mM of 2-mercaptoethanol, and 2mM of L-glutamine, and further comprises about 6000 IU/mL of IL-2. In some embodiments, the CTS™OpTmizer™ T-cell Expansion SFM is supplemented with about 3% of the CTS™ Immune Cell Serum Replacement (SR) (ThermoFisher Scientific) and 55mM of 2-mercaptoethanol, and further comprises about 1000 IU/mL to about 8000 IU/mL of IL-2. In some embodiments, the CTS™OpTmizer™ T-cell Expansion SFM is supplemented with about 3% of the CTS™ Immune Cell Serum Replacement (SR) (ThermoFisher Scientific) and 55mM of 2-mercaptoethanol, and further comprises about 3000 IU/mL of IL-2. In some embodiments, the CTS™OpTmizer™ T-cell Expansion SFM is supplemented with about 3% of the CTS™ Immune Cell Serum Replacement (SR) (ThermoFisher Scientific) and 55mM of 2-mercaptoethanol, and further comprises about 1000 IU/mL to about 6000 IU/mL of IL-2. In some embodiments, the CTS™OpTmizer™ T-cell Expansion SFM is supplemented with about 3% of the CTS™ Immune Cell Serum Replacement (SR) (ThermoFisher Scientific) and about 2mM glutamine, and further comprises about 1000 IU/mL to about 8000 IU/mL of IL-2. In some embodiments, the CTS™OpTmizer™ T-cell Expansion SFM is supplemented with about 3% of the CTS™ Immune Cell Serum Replacement (SR) (ThermoFisher Scientific) and about 2mM glutamine, and further comprises about 3000 IU/mL of IL-2. In some embodiments, the CTS™OpTmizer™ T-cell Expansion SFM is supplemented with about 3% of the CTS™ Immune Cell Serum Replacement (SR) (ThermoFisher Scientific) and about 2mM glutamine, and further comprises about 6000 IU/mL of IL-2.

In some embodiments, the serum-free medium or defined medium is supplemented with glutamine (i.e., GlutaMAX®) at a concentration of from about 0.1mM to about 10mM, 0.5mM to about 9mM, 1mM to about 8mM, 2mM to about 7mM, 3mM to about 6mM, or 4mM to about 5 mM. In some embodiments, the serum-free medium or defined medium is supplemented with glutamine (i.e., GlutaMAX®) at a concentration of about 2mM.

In some embodiments, the serum-free medium or defined medium is supplemented with 2-mercaptoethanol at a concentration of from about 5mM to about 150mM, 10mM to about 140mM, 15mM to about 130mM, 20mM to about 120mM, 25mM to about 110mM, 30mM to about 100mM, 35mM to about 95mM, 40mM to about 90mM, 45mM to about 85mM, 50mM to about 80mM, 55mM to about 75mM, 60mM to about 70mM, or about 65mM. In some embodiments, the serum-free medium or defined medium is supplemented with 2-mercaptoethanol at a concentration of about 55mM.

In some embodiments, the defined media described in International PCT Publication No. WO/1998/030679 are useful in the present invention. In that publication, serum-free eukaryotic cell culture media are described. The serum-free, eukaryotic cell culture medium includes a basal cell culture medium supplemented with a serum-free supplement capable of supporting the growth of cells in serum-free culture. The serum-free eukaryotic cell culture medium supplement comprises or is obtained by combining one or more ingredients selected from the group consisting of one or more albumins or albumin substitutes, one or more amino acids, one or more vitamins, one or more transferrins or transferrin substitutes, one or more antioxidants, one or more insulins or insulin substitutes, one or more collagen precursors, one or more trace elements, and one or more antibiotics. In some embodiments, the defined medium further comprises L-glutamine, sodium bicarbonate and/or beta-mercaptoethanol. In some embodiments, the defined medium comprises an albumin or an albumin substitute and one or more ingredients selected from group consisting of one or more amino acids, one or more vitamins, one or more transferrins or transferrin substitutes, one or more antioxidants, one or more insulins or insulin substitutes, one or more collagen precursors, and one or more trace elements. In some embodiments, the defined medium comprises albumin and one or more ingredients selected from the group consisting of glycine, L-histidine, L-isoleucine, L-methionine, L-phenylalanine, L-proline, L-hydroxyproline, L-serine, L-threonine, L-tryptophan, L-tyrosine, L-valine, thiamine, reduced glutathione, L-ascorbic acid-2-phosphate, iron saturated transferrin, insulin, and compounds containing the trace element moieties $Ag^+$, $Al^{3+}$, $Ba^{2+}$, $Cd^{2+}$, $Co^{2+}$, $Cr^{3"}$, $Ge^{4+}$, $Se^{4+}$, Br, T, $Mn^{2+}$, P, $Si^{4+}$, $V^{5+}$, $Mo^{6+}$, $Ni^{2+}$, $Rb^+$, $Sn^{2+}$ and $Zr^{4+}$. In some embodiments, the basal cell media is selected from the group consisting of Dulbecco's Modified Eagle's Medium (DMEM), Minimal Essential Medium (MEM), Basal Medium Eagle (BME), RPMI 1640, F-10, F-12, Minimal Essential Medium (αMEM), Glasgow's Minimal Essential Medium (G-MEM), RPMI growth medium, and Iscove's Modified Dulbecco's Medium.

In some embodiments, the concentration of glycine in the defined medium is in the range of from about 5-200 mg/L, the concentration of L-histidine is about 5-250 mg/L, the concentration of L-isoleucine is about 5-300 mg/L, the concentration of L-methionine is about 5-200 mg/L, the concentration of L-phenylalanine is about 5-400 mg/L, the concentration of L-proline is about 1-1000 mg/L, the concentration of L-hydroxyproline is about 1-45 mg/L, the concentration of L-serine is about 1-250 mg/L, the concentration of L-threonine is about 10-500 mg/L, the concentration of L-tryptophan is about 2-110 mg/L, the concentration of L-tyrosine is about 3-175 mg/L, the concentration of L-valine is about 5-500 mg/L, the concentration of thiamine is about 1-20 mg/L, the concentration of reduced glutathione is about 1-20 mg/L, the concentration of L-ascorbic acid-2-phosphate is about 1-200 mg/L, the concentration of iron saturated transferrin is about 1-50 mg/L, the concentration of insulin is about 1-100 mg/L, the concentration of sodium selenite is about 0.000001-0.0001 mg/L, and the concentration of albumin (e.g., AlbuMAX® I) is about 5000-50,000 mg/L.

In some embodiments, the non-trace element moiety ingredients in the defined medium are present in the concentration ranges listed in the column under the heading "Concentration Range in 1X Medium" in Table A below. In other embodiments, the non-trace element moiety ingredients in the defined medium are present in the final concentrations listed in the column under the heading "A Preferred Embodiment of the 1X Medium" in Table A below. In other embodiments, the defined medium is a basal cell medium comprising a serum free supplement. In some of these embodiments, the serum free supplement comprises non-trace moiety ingredients of the type and in the concentrations listed in the column under the heading "A Preferred Embodiment in Supplement" in Table A below.

Table A: Concentrations of Non-Trace Element Moiety Ingredients

| Ingredient | A preferred embodiment in supplement (mg/L) (About) | Concentration range in 1X medium (mg/L) (About) | A preferred embodiment in 1X medium (mg/L) (About) |
|---|---|---|---|
| Glycine | 150 | 5-200 | 53 |
| L-Histidine | 940 | 5-250 | 183 |
| L-Isoleucine | 3400 | 5-300 | 615 |
| L-Methionine | 90 | 5-200 | 44 |
| L-Phenylalanine | 1800 | 5-400 | 336 |
| L-Proline | 4000 | 1-1000 | 600 |
| L-Hvdroxvproline | 100 | 1-45 | 15 |
| L-Serine | 800 | 1-250 | 162 |
| L-Threonine | 2200 | 10-500 | 425 |
| L-Tryptophan | 440 | 2-110 | 82 |
| L-Tvrosine | 77 | 3-175 | 84 |
| L-Valine | 2400 | 5-500 | 454 |

(continued)

| Ingredient | A preferred embodiment in supplement (mg/L) (About) | Concentration range in 1X medium (mg/L) (About) | A preferred embodiment in 1X medium (mg/L) (About) |
|---|---|---|---|
| Thiamine | 33 | 1-20 | 9 |
| Reduced Glutathione | 10 | 1-20 | 1.5 |
| Ascorbic Acid-2-PO$_4$ (Mg Salt) | 330 | 1-200 | 50 |
| Transferrin (iron saturated) | 55 | 1-50 | 8 |
| Insulin | 100 | 1-100 | 10 |
| Sodium Selenite | 0.07 | 0.000001-0.0001 | 0.00001 |
| AlbuMAX®I | 83,000 | 5000-50,000 | 12,500 |

In some embodiments, the osmolarity of the defined medium is between about 260 and 350 mOsmol. In some embodiments, the osmolarity is between about 280 and 310 mOsmol. In some embodiments, the defined medium is supplemented with up to about 3.7 g/L, or about 2.2 g/L sodium bicarbonate. The defined medium can be further supplemented with L-glutamine (final concentration of about 2 mM), one or more antibiotics, non-essential amino acids (NEAA; final concentration of about 100 μM), 2-mercaptoethanol (final concentration of about 100 μM).

In some embodiments, the defined media described in Smith, et al., "Ex vivo expansion of human T cells for adoptive immunotherapy using the novel Xeno-free CTS Immune Cell Serum Replacement," Clin Transl Immunology, 4(1) 2015 (doi: 10.1038/cti.2014.31) are useful in the present invention. Briefly, RPMI or CTS™ OpTmizer™ was used as the basal cell medium, and supplemented with either 0, 2%, 5%, or 10% CTS™ Immune Cell Serum Replacement.

In an embodiment, the cell medium in the first and/or second gas permeable container is unfiltered. The use of unfiltered cell medium may simplify the procedures necessary to expand the number of cells. In an embodiment, the cell medium in the first and/or second gas permeable container lacks beta-mercaptoethanol (BME or βME; also known as 2-mercaptoethanol, CAS 60-24-2).

Disclosed herein but not claimed are methods wherein the duration of the method comprising obtaining a tumor tissue sample from the mammal; culturing the tumor tissue sample in a first gas permeable container containing cell medium therein; obtaining TILs from the tumor tissue sample; expanding the number of TILs in a second gas permeable container containing cell medium for a duration of about 7 to 14 days, for example, about 11 days. In some embodiments pre-REP is about 7 to 14 days, for example, about 11 days. In some embodiments, REP is about 7 to 14 days, for example, about 11 days.

In an embodiment, TILs are expanded in gas-permeable containers. Gas-permeable containers have been used to expand TILs using PBMCs using methods, compositions, and devices known in the art, including those described in U.S. Patent Application Publication No. 2005/0106717 A1. In an embodiment, TILs are expanded in gas-permeable bags. In an embodiment, TILs are expanded using a cell expansion system that expands TILs in gas permeable bags, such as the Xuri Cell Expansion System W25 (GE Healthcare). In an embodiment, TILs are expanded using a cell expansion system that expands TILs in gas permeable bags, such as the WAVE Bioreactor System, also known as the Xuri Cell Expansion System W5 (GE Healthcare). In an embodiment, TILs are expanded using a cell expansion system that expands TILs in gas permeable bags, such as the OMNI C3® cell culture bag (Lampire Biological Laboratories). In an embodiment, TILs are expanded using a cell expansion system that expands TILs in gas permeable bags, such as the EXP-PAK™ Cell Expansion Bio-Containers (Charter Medical). In an embodiment, the cell expansion system for expanding TILs uses the same gas permeable containers for each expansion step. In an embodiment, the cell expansion system for expanding TILs uses different gas permeable containers for each expansion step. In an embodiment, the cell expansion system includes a gas permeable cell bag with a volume selected from the group consisting of about 100 mL, about 200 mL, about 300 mL, about 400 mL, about 500 mL, about 600 mL, about 700 mL, about 800 mL, about 900 mL, about 1 L, about 2 L, about 3 L, about 4 L, about 5 L, about 6 L, about 7 L, about 8 L, about 9 L, and about 10 L.

In an embodiment, TILs can be expanded in G-Rex flasks (commercially available from Wilson Wolf Manufacturing). Such embodiments allow for cell populations to expand from about $5 \times 10^5$ cells/cm$^2$ to between $10 \times 10^6$ and $30 \times 10^6$ cells/cm2. In an embodiment this is without feeding. In an embodiment, this is without feeding so long as medium resides at a height of about 10 cm in the G-Rex flask. In an embodiment this is without feeding but with the addition of one or more cytokines. In an embodiment, the cytokine can be added as a bolus without any need to mix the cytokine with the medium. Such containers, devices, and methods are known in the art and have been used to expand TILs, and include those described in U.S. Patent Application Publication No. US 2014/0377739A1, International Publication No. WO 2014/210036 A1, U.S. Patent Application Publication No. us 2013/0115617 A1, International Publication No. WO 2013/188427 A1, U.S. Patent Application Publication No. US 2011/0136228 A1, U.S. Patent No. US 8,809,050 B2, International publication No. WO 2011/072088 A2, U.S. Patent Application Publication No. US 2016/0208216 A1, U.S. Patent Application Publication No. US 2012/0244133 A1, International Publication No. WO 2012/129201 A1, U.S. Patent Application Publication No. US 2013/0102075 A1, U.S. Patent No. US 8,956,860 B2, International Publication No. WO 2013/173835 A1, U.S. Patent Application Publication No. US 2015/0175966 A1. Such processes are also described in Jin et al., J. Immunotherapy, 2012, 35:283-292.

Optional Genetic Engineering of TILs

In some embodiments, the TILs are optionally genetically engineered to include additional functionalities, including, but not limited to, a high-affinity T cell receptor (TCR), for example, a TCR targeted at a tumor-associated antigen such as MAGE-1, HER2, or NY-ESO-1, or a chimeric antigen receptor (CAR) which binds to a tumor-associated cell surface molecule (e.g., mesothelin) or lineage-restricted cell surface molecule (*e.g.*, CD19).

Closed Systems for TIL Manufacturing

The present invention provides for the use of closed systems during the TIL culturing process. Such closed systems allow for preventing and/or reducing microbial contamination, allow for the use of fewer flasks, and allow for cost reductions. In some embodiments, the closed system uses two containers.

Closed systems are known in the art and can be found, for example, at http://www.fda.gov/cber/guidelines.htm and https://www.fda.gov/BiologicsBloodVaccines/GuidanceComplianceRegulatoryInformation/Guidances/Blood/ucm076779.htm.

Sterile connecting devices (STCDs) produce sterile welds between two pieces of compatible tubing. This procedure permits sterile connection of a variety of containers and tube diameters. In some embodiments, the closed systems include luer lock and heat sealed systems as described in for example, Example 4. In some embodiments, the closed system is accessed via syringes under sterile conditions in order to maintain the sterility and closed nature of the system. In some embodiments, a closed system as described in Example 6 is employed. In some embodiments, the TILs are formulated into a final product formulation container according to the method described in Example 4, section "Final Formulation and Fill".

In some embodiments, the closed system uses one container from the time the tumor fragments are obtained until the TILs are ready for administration to the patient or cryopreserving. In some embodiments when two containers are used, the first container is a closed G-container and the population of TILs is centrifuged and transferred to an infusion bag without opening the first closed G-container. In some embodiments, when two containers are used, the infusion bag is a HypoThermosol-containing infusion bag. A closed system or closed TIL cell culture system is characterized in that once the tumor sample and/or tumor fragments have been added, the system is tightly sealed from the outside to form a closed environment free from the invasion of bacteria, fungi, and/or any other microbial contamination.

In some embodiments, the reduction in microbial contamination is between about 5% and about 100%. In some embodiments, the reduction in microbial contamination is between about 5% and about 95%. In some embodiments, the reduction in microbial contamination is between about 5% and about 90%. In some embodiments, the reduction in microbial contamination is between about 10% and about 90%. In some embodiments, the reduction in microbial contamination is between about 15% and about 85%. In some embodiments, the reduction in microbial contamination is about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 97%, about 98%, about 99%, or about 100%.

The closed system allows for TIL growth in the absence and/or with a significant reduction in microbial contamination.

Moreover, pH, carbon dioxide partial pressure and oxygen partial pressure of the TIL cell culture environment each vary as the cells are cultured. Consequently, even though a medium appropriate for cell culture is circulated, the closed environment still needs to be constantly maintained as an optimal environment for TIL proliferation. To this end, it is desirable that the physical factors of pH, carbon dioxide partial pressure and oxygen partial pressure within the culture liquid of the closed environment be monitored by means of a sensor, the signal whereof is used to control a gas exchanger installed at the inlet of the culture environment, and the that gas partial pressure of the closed environment be adjusted in real time according to changes in the culture liquid so as to optimize the cell culture environment. In some embodiments, the present invention provides a closed cell culture system which incorporates at the inlet to the closed environment a gas exchanger equipped with a monitoring device which measures the pH, carbon dioxide partial pressure and oxygen partial pressure of the closed environment, and optimizes the cell culture environment by automatically adjusting gas concentrations based on signals from the monitoring device.

In some embodiments, the pressure within the closed environment is continuously or intermittently controlled. That is, the pressure in the closed environment can be varied by means of a pressure maintenance device for example, thus ensuring that the space is suitable for growth of TILs in a positive pressure state, or promoting exudation of fluid in a negative pressure state and thus promoting cell proliferation. By applying negative pressure intermittently, moreover, it is possible to uniformly and efficiently replace the circulating liquid in the closed environment by means of a temporary shrinkage in the volume of the closed environment.

In some embodiments, optimal culture components for proliferation of the TILs can be substituted or added, and including factors such as IL-2 and/or OKT3, as well as combination, can be added.

Optional Cryopreservation of TILs

As discussed above, and exemplified in Steps A through E as provided in Figure 1, cryopreservation can occur at numerous points throughout the TIL expansion process, including at the final stage of the process after TIL harvesting for preservation of the therapeutic product. In some embodiments, the expanded population of TILs after the second expansion (as provided for example, according to Step D of Figure 1) can be cryopreserved. Cryopreservation can be generally accomplished by placing the TIL population into a freezing solution, *e.g.*, 85% complement inactivated AB serum and 15% dimethyl sulfoxide (DMSO). The cells in solution are placed into cryogenic vials and stored for 24 hours at -80°C, with optional transfer to gaseous nitrogen freezers for cryopreservation. *See* Sadeghi, et al., Acta Oncologica, 2013, 52, 978-986. In some embodiments, the TILs are cryopreserved in 5% DMSO. In some embodiments, the TILs are cryopreserved in cell culture media plus 5% DMSO. In some embodiments, the TILs are cryopreserved according to the methods provided in Examples 6 and 7.

When appropriate, the cells are removed from the freezer and thawed in a 37°C water bath until approximately 4/5 of the solution is thawed. The cells are generally resuspended in complete media and optionally washed one or more times. In some embodiments, the thawed TILs can be counted and assessed for viability as is known in the art.

Either the bulk TIL population or the expanded population of TILs can be optionally cryopreserved. In some embodiments, cryopreservation occurs on the therapeutic TIL population. In some embodiments, cryopreservation occurs on the TILs harvested after the second expansion. In some embodiments, cryopreservation occurs on the TILs in exemplary Step F of Figure 1. In some embodiments, the TILs are cryopreserved in the infusion bag. In some embodiments, the TILs are cryopreserved prior to placement in an infusion bag. In some embodiments, the TILs are cryopreserved and not placed in an infusion bag. In some embodiments, cryopreservation is performed using a cryopreservation medium. In some embodiments, the cryopreservation media contains dimethylsulfoxide (DMSO). This is generally accomplished by putting the TIL population into a freezing solution, *e.g.* 85% complement inactivated AB serum and 15% dimethyl sulfoxide (DMSO). The cells in solution are placed into cryogenic vials and stored for 24 hours at -80°C, with optional transfer to gaseous nitrogen freezers for cryopreservation. *See,* Sadeghi, et al., Acta Oncologica, 2013, 52, 978-986.

When appropriate, the cells are removed from the freezer and thawed in a 37°C water bath until approximately 4/5 of the solution is thawed. The cells are generally resuspended in complete media and optionally washed one or more times. In some embodiments, the thawed TILs can be counted and assessed for viability as is known in the art.

In a preferred embodiment, a population of TILs is cryopreserved using CS10 cryopreservation media (CryoStor 10, BioLife Solutions). In a preferred embodiment, a population of TILs is cryopreserved using a cryopreservation media containing dimethylsulfoxide (DMSO). In a preferred embodiment, a population of TILs is cryopreserved using a 1:1 (vol:vol) ratio of CS10 and cell culture media. In a preferred embodiment, a population of TILs is cryopreserved using about a 1:1 (vol:vol) ratio of CS10 and cell culture media, further comprising additional IL-2.

As discussed above in Steps A through E, cryopreservation can occur at numerous points throughout the TIL expansion process. In some embodiments, the bulk TIL population after the first expansion according to Step B or the expanded population of TILs after the one or more second expansions according to Step D can be cryopreserved. Cryopreservation can be generally accomplished by placing the TIL population into a freezing solution, *e.g.*, 85% complement inactivated AB serum and 15% dimethyl sulfoxide (DMSO). The cells in solution are placed into cryogenic vials and stored for 24 hours at -80°C, with optional transfer to gaseous nitrogen freezers for cryopreservation. *See* Sadeghi, et al., Acta Oncologica, 2013, 52, 978-986.

When appropriate, the cells are removed from the freezer and thawed in a 37°C water bath until approximately 4/5 of the solution is thawed. The cells are generally resuspended in complete media and optionally washed one or more times. In some embodiments, the thawed TILs can be counted and assessed for viability as is known in the art.

In some cases, the Step B TIL population can be cryopreserved immediately, using the protocols discussed below. Alternatively, the bulk TIL population can be subjected to Step C and Step D and then cryopreserved after Step D. Similarly, in the case where genetically modified TILs will be used in therapy, the Step B or Step D TIL populations can be subjected to genetic modifications for suitable treatments.

Pharmaceutical Compositions, Dosages, and Dosing Regimens

In methods disclosed herein but not claimed, expanded TILs are administered to a patient as a pharmaceutical composition. In an embodiment, the pharmaceutical composition is a suspension of TILs in a sterile buffer. In methods disclosed herein but not claimed, TILs expanded using PBMCs may be administered by any suitable route as known in the art. In methods disclosed herein but not claimed, the T-cells are administered as a single intra-arterial or intravenous infusion, which preferably lasts approximately 30 to 60 minutes. Other suitable routes of administration include intraperitoneal, intrathecal, and intralymphatic administration.

In methods disclosed herein but not claimed, any suitable dose of TILs can be administered, e.g., from about $2.3 \times 10^{10}$ to about $13.7 \times 10^{10}$ TILs are administered, with an average of around $7.8 \times 10^{10}$ TILs, particularly if the cancer is melanoma. In methods disclosed herein but not claimed, about $1.2 \times 10^{10}$ to about $4.3 \times 10^{10}$ of TILs are administered. In methods disclosed herein but not claimed, about $3 \times 10^{10}$ to about $12 \times 10^{10}$ TILs are administered. In methods disclosed herein but not claimed, about $4 \times 10^{10}$ to about $10 \times 10^{10}$ TILs are administered. In methods disclosed herein but not claimed, about $5 \times 10^{10}$ to about $8 \times 10^{10}$ TILs are administered. In methods disclosed herein but not claimed, about $6 \times 10^{10}$ to about $8 \times 10^{10}$ TILs are administered. In methods disclosed herein but not claimed, about $7 \times 10^{10}$ to about $8 \times 10^{10}$ TILs are administered. In some embodiments, the therapeutically effective dosage is about $2.3 \times 10^{10}$ to about $13.7 \times 10^{10}$. In some embodiments, the therapeutically effective dosage is about $7.8 \times 10^{10}$ TILs, particularly of the cancer is melanoma. In some embodiments, the therapeutically effective dosage is about $1.2 \times 10^{10}$ to about $4.3 \times 10^{10}$ of TILs. In some embodiments, the therapeutically effective dosage is about $3 \times 10^{10}$ to about $12 \times 10^{10}$ TILs. In some embodiments, the therapeutically effective dosage is about $4 \times 10^{10}$ to about $10 \times 10^{10}$ TILs. In some embodiments, the therapeutically effective dosage is about $5 \times 10^{10}$ to about $8 \times 10^{10}$ TILs. In some embodiments, the therapeutically effective dosage is about $6 \times 10^{10}$ to about $8 \times 10^{10}$ TILs. In some embodiments, the therapeutically effective dosage is about $7 \times 10^{10}$ to about $8 \times 10^{10}$ TILs.

In some embodiments, the number of the TILs provided in the pharmaceutical compositions of the invention is about $1\times10^6$, $2\times10^6$, $3\times10^6$, $4\times10^6$, $5\times10^6$, $6\times10^6$, $7\times10^6$, $8\times10^6$, $9\times10^6$, $1\times10^7$, $2\times10^7$, $3\times10^7$, $4\times10^7$, $5\times10^7$, $6\times10^7$, $7\times10^7$, $8\times10^7$, $9\times10^7$, $1\times10^8$, $2\times10^8$, $3\times10^8$, $4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$, $5\times10^{10}$, $6\times10^{10}$, $7\times10^{10}$, $8\times10^{10}$, $9\times10^{10}$, $1\times10^{11}$, $2\times10^{11}$, $3\times10^{11}$, $4\times10^{11}$, $5\times10^{11}$, $6\times10^{11}$, $7\times10^{11}$, $8\times10^{11}$, $9\times10^{11}$, $1\times10^{12}$, $2\times10^{12}$, $3\times10^{12}$, $4\times10^{12}$, $5\times10^{12}$, $6\times10^{12}$, $7\times10^{12}$, $8\times10^{12}$, $9\times10^{12}$, $1\times10^{13}$, $2\times10^{13}$, $3\times10^{13}$, $4\times10^{13}$, $5\times10^{13}$, $6\times10^{13}$, $7\times10^{13}$, $8\times10^{13}$, and $9\times10^{13}$. In an embodiment, the number of the TILs provided in the pharmaceutical compositions of the invention is in the range of $1\times10^6$ to $5\times10^6$ $5\times10^6$ to $1\times10^7$, $1\times10^7$ to $5\times10^7$, $5\times10^7$ to $1\times10^8$, $1\times10^8$ to $5\times10^8$, $5\times10^8$ to $1\times10^9$, $1\times10^9$ to $5\times10^9$, $5\times10^9$ to $1\times10^{10}$, $1\times10^{10}$ to $5\times10^{10}$, $5\times10^{10}$ to $1\times10^{11}$, $5\times10^{11}$ to $1\times10^{12}$, $1\times10^{12}$ to $5\times10^{12}$ , and $5\times10^{12}$ to $1\times10^{13}$.

In some embodiments, the concentration of the TILs provided in the pharmaceutical compositions of the invention is less than, for example, 100%, 90%, 80%, 70%, 60%, 50%, 40%, 30%, 20%, 19%, 18%, 17%, 16%, 15%, 14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v or v/v of the pharmaceutical composition.

In some embodiments, the concentration of the TILs provided in the pharmaceutical compositions of the invention is greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v, or v/v of the pharmaceutical composition.

In some embodiments, the concentration of the TILs provided in the pharmaceutical compositions of the invention is in the range from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12% or about 1% to about 10% w/w, w/v or v/v of the pharmaceutical composition.

In some embodiments, the concentration of the TILs provided in the pharmaceutical compositions of the invention is in the range from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, about 0.1% to about 0.9% w/w, w/v or v/v of the pharmaceutical composition.

In some embodiments, the amount of the TILs provided in the pharmaceutical compositions of the invention is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of the TILs provided in the pharmaceutical compositions of the invention is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5 g, 7 g, 7.5 g, 8 g, 8.5 g, 9 g, 9.5 g, or 10 g.

The TILs provided in the pharmaceutical compositions of the invention are effective over a wide dosage range. The exact dosage will depend upon the route of administration, the form in which the compound is administered, the gender and age of the subject to be treated, the body weight of the subject to be treated, and the preference and experience of the attending physician. The clinically-established dosages of the TILs may also be used if appropriate. The amounts of the pharmaceutical compositions administered using the methods herein, such as the dosages of TILs, will be dependent on the human or mammal being treated, the severity of the disorder or condition, the rate of administration, the disposition of the active pharmaceutical ingredients and the discretion of the prescribing physician.

In methods disclosed herein but not claimed, TILs may be administered in a single dose. Such administration may be by injection, e.g., intravenous injection. In methods disclosed herein but not claimed, TILs may be administered in multiple doses. Dosing may be once, twice, three times, four times, five times, six times, or more than six times per year. Dosing may be once a month, once every two weeks, once a week, or once every other day. Administration of TILs may continue as long as necessary.

In some embodiments, an effective dosage of TILs is about $1\times10^6$, $2\times10^6$, $3\times10^6$, $4\times10^6$, $5\times10^6$, $6\times10^6$, $7\times10^6$, $8\times10^6$, $9\times10^6$, $1\times10^7$, $2\times10^7$, $3\times10^7$, $4\times10^7$, $5\times10^7$, $6\times10^7$, $7\times10^7$, $8\times10^7$, $9\times10^7$, $1\times10^8$, $2\times10^8$, $3\times10^8$, $4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$, $5\times10^{10}$, $6\times10^{10}$, $7\times10^{10}$, $8\times10^{10}$, $9\times10^{10}$, $1\times10^{11}$, $2\times10^{11}$, $3\times10^{11}$, $4\times10^{11}$, $5\times10^{11}$, $6\times10^{11}$, $7\times10^{11}$, $8\times10^{11}$, $9\times10^{11}$, $1\times10^{12}$, $2\times10^{12}$, $3\times10^{12}$, $4\times10^{12}$, $5\times10^{12}$, $6\times10^{12}$, $7\times10^{12}$, $8\times10^{12}$, $9\times10^{12}$, $1\times10^{13}$, $2\times10^{13}$, $3\times10^{13}$, $4\times10^{13}$, $5\times10^{13}$, $6\times10^{13}$, $7\times10^{13}$, $8\times10^{13}$, and $9\times10^{13}$. In some embodiments, an effective dosage of TILs is in the range of $1\times10^6$ to $5\times10^6$, $5\times10^6$ to $1\times10^7$, $1\times10^7$ to $5\times10^7$, $5\times10^7$ to $1\times10^8$, $1\times10^8$ to $5\times10^8$, $5\times10^8$ to $1\times10^9$, $1\times10^9$ to $5\times10^9$, $5\times10^9$ to $1\times10^{10}$, $1\times10^{10}$ to $5\times10^{10}$, $5\times10^{10}$ to $1\times10^{11}$, $5\times10^{11}$ to $1\times10^{12}$, $1\times10^{12}$ to $5\times10^{12}$, and $5\times10^{12}$ to $1\times10^{13}$.

In some embodiments, an effective dosage of TILs is in the range of about 0.01 mg/kg to about 4.3 mg/kg, about 0.15 mg/kg to about 3.6 mg/kg, about 0.3 mg/kg to about 3.2 mg/kg, about 0.35 mg/kg to about 2.85 mg/kg, about 0.15 mg/kg to about 2.85 mg/kg, about 0.3 mg to about 2.15 mg/kg, about 0.45 mg/kg to about 1.7 mg/kg, about 0.15 mg/kg to about 1.3 mg/kg, about 0.3 mg/kg to about 1.15 mg/kg, about 0.45 mg/kg to about 1 mg/kg, about 0.55 mg/kg to about 0.85 mg/kg, about 0.65 mg/kg to about 0.8 mg/kg, about 0.7 mg/kg to about 0.75 mg/kg, about 0.7 mg/kg to about 2.15 mg/kg, about 0.85 mg/kg to about 2 mg/kg, about 1 mg/kg to about 1.85 mg/kg, about 1.15 mg/kg to about 1.7 mg/kg, about 1.3 mg/kg mg to about 1.6 mg/kg, about 1.35 mg/kg to about 1.5 mg/kg, about 2.15 mg/kg to about 3.6 mg/kg, about 2.3 mg/kg to about 3.4 mg/kg, about 2.4 mg/kg to about 3.3 mg/kg, about 2.6 mg/kg to about 3.15 mg/kg, about 2.7 mg/kg to about 3 mg/kg, about 2.8 mg/kg to about 3 mg/kg, or about 2.85 mg/kg to about 2.95 mg/kg.

In some embodiments, an effective dosage of TILs is in the range of about 1 mg to about 500 mg, about 10 mg to about 300 mg, about 20 mg to about 250 mg, about 25 mg to about 200 mg, about 1 mg to about 50 mg, about 5 mg to about 45 mg, about 10 mg to about 40 mg, about 15 mg to about 35 mg, about 20 mg to about 30 mg, about 23 mg to about 28 mg, about 50 mg to about 150 mg, about 60 mg to about 140 mg, about 70 mg to about 130 mg, about 80 mg to about 120 mg, about 90 mg to about 110 mg, or about 95 mg to about 105 mg, about 98 mg to about 102 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 207 mg.

An effective amount of the TILs may be administered in either single or multiple doses by any of the accepted modes of administration of agents having similar utilities, including intranasal and transdermal routes, by intra-arterial injection, intravenously, intraperitoneally, parenterally, intramuscularly, subcutaneously, topically, by transplantation, or by inhalation.

Methods of Treating Patients

Methods of treatment begin with the initial TIL collection and culture of TILs. Such methods have been both described in the art by, for example, Jin et al., J. Immunotherapy, 2012, 35(3):283-292. Embodiments of methods of treatment are described throughout the sections below, including the Examples.

The expanded TILs produced according the methods described herein, including for example as described in Steps A through F above or according to Steps A through F above (also as shown, for example, in Figure 1) find particular use in the treatment of patients with cancer (for example, as described in Goff, et al., J. Clinical Oncology, 2016, 34(20):2389-239, as well as the supplemental content). In some embodiments, TIL were grown from resected deposits of metastatic melanoma as previously described (see, Dudley, et al., J Immunother., 2003, 26:332-342). Fresh tumor can be dissected under sterile conditions. A representative sample can be collected for formal pathologic analysis. Single fragments of 2 mm$^3$ to 3 mm$^3$ may be used. In some embodiments, 5, 10, 15, 20, 25 or 30 samples per patient are obtained. In some embodiments, 20, 25, or 30 samples per patient are obtained. In some embodiments, 20, 22, 24, 26, or 28 samples per patient are obtained. In some embodiments, 24 samples per patient are obtained. Samples can be placed in individual wells of a 24-well plate, maintained in growth media with high-dose IL-2 (6,000 IU/mL), and monitored for destruction of tumor and/or proliferation of TIL. Any tumor with viable cells remaining after processing can be enzymatically digested into a single cell suspension and cryopreserved, as described herein.

In some embodiments, successfully grown TIL can be sampled for phenotype analysis (CD3, CD4, CD8, and CD56) and tested against autologous tumor when available. TIL can be considered reactive if overnight coculture yielded interferon-gamma (IFN-γ) levels > 200 pg/mL and twice background. (Goff, et al., J Immunother., 2010, 33:840-847). In some embodiments, cultures with evidence of autologous reactivity or sufficient growth patterns can be selected for a second expansion (for example, a second expansion as provided in according to Step D of Figure 1), including second expansions that are sometimes referred to as rapid expansion (REP). In some embodiments, expanded TILs with high autologous reactivity (for example, high proliferation during a second expansion), are selected for an additional second expansion. In some embodiments, TILs with high autologous reactivity (for example, high proliferation during second expansion as provided in Step D of Figure 1), are selected for an additional second expansion according to Step D of Figure 1.

In some embodiments, the patient is not moved directly to ACT (adoptive cell transfer), for example, in some embodiments, after tumor harvesting and/or a first expansion, the cells are not utilized immediately. In such embodiments, TILs can be cryopreserved and thawed 2 days before administration to a patient. In such embodiments, TILs can be cryopreserved and thawed 1 day before administration to a patient. In some embodiments, the TILs can be cryopreserved and thawed immediately before the administration to a patient.

Cell phenotypes of cryopreserved samples of infusion bag TIL can be analyzed by flow cytometry (e.g., FlowJo) for surface markers CD3, CD4, CD8, CCR7, and CD45RA (BD BioSciences), as well as by any of the methods described herein. Serum cytokines were measured by using standard enzyme-linked immunosorbent assay techniques. A rise in serum IFN-γ was defined as >100 pg/mL and greater than 4 3 baseline levels.

In some embodiments, the TILs produced by the methods provided herein, for example those exemplified in Figure 1, provide for a surprising improvement in clinical efficacy of the TILs. In some embodiments, the TILs produced by the methods provided herein, for example those exemplified in Figure 1, exhibit increased clinical efficacy as compared to TILs produced by methods other than those described herein, including for example, methods other than those exemplified in Figure 1. In some embodiments, the methods other than those described herein include methods referred to as process 1C and/or Generation 1 (Gen 1). In some embodiments, the increased efficacy is measured by DCR, ORR, and/or other clinical responses. In some embodiments, the TILs produced by the methods provided herein, for example those exemplified in Figure 1, exhibit a similar time to response and safety profile compared to TILs produced by methods other than those described herein, including for example, methods other than those exemplified in Figure 1, for example the Gen 1 process.

In some embodiments, IFN-gamma (IFN-γ) is indicative of treatment efficacy and/or increased clinical efficacy. In some embodiments, IFN-γ in the blood of subjects treated with TILs is indicative of active TILs. In some embodiments, a potency assay for IFN-γ production is employed. IFN-γ production is another measure of cytotoxic potential. IFN-γ production can be measured by determining the levels of the cytokine IFN-γ in the blood, serum, or TILs ex vivo of a subject treated with TILs prepared by the methods of the present invention, including those as described for example in Figure 1. In some embodiments, an increase in IFN-γ is indicative of treatment efficacy in a patient treated with the TILs produced by the methods of the present invention. In some embodiments, IFN-γ is increased one-fold, two-fold, three-fold, four-fold, or five-fold or more as compared to an untreated patient and/or as compared to a patient treated with TILs prepared using other methods than those provide herein including for example, methods other than those embodied in Figure 1. In some embodiments, IFN-γ secretion is increased one-fold as compared to an untreated patient and/or as compared to a patient treated with TILs prepared using other methods than those provide herein including for example, methods other than those embodied in Figure 1. In some embodiments, IFN-γ secretion is increased two-fold as compared to an untreated patient and/or as compared to a patient treated with TILs prepared using other methods than those provide herein including for example, methods other than those embodied in Figure 1. In some embodiments, IFN-γ secretion is increased three-fold as compared to an untreated patient and/or as compared to a patient treated with TILs prepared using other methods than those provide herein including for example, methods other than those embodied in Figure 1. In some embodiments, IFN-γ secretion is increased four-fold as compared to an untreated patient and/or as compared to a patient treated with TILs prepared using other methods than those provide herein including for example, methods other than those embodied in Figure 1. In some embodiments, IFN-γ secretion is increased five-fold as compared to an untreated patient and/or as compared to a patient treated with TILs prepared using other methods than those provide herein including for example, methods other than those embodied in Figure 1. In some embodiments, IFN-γ is measured using a Quantikine ELISA kit. In some embodiments, IFN-γ is measured in TILs *ex vivo* of a subject treated with TILs prepared by the methods of the present invention, including those as described for example in Figure 1. In some embodiments, IFN-γ is measured in blood of a subject treated with TILs prepared by the methods of the present invention, including those as described for example in Figure 1. In some embodiments, IFN-γ is measured in TILs serum of a subject treated with TILs prepared by the methods of the present invention, including those as described for example in Figure 1.

In some embodiments, the TILs prepared by the methods of the present invention, including those as described for example in Figure 1, exhibit increased polyclonality as compared to TILs produced by other methods, including those not exemplified in Figure 1, such as for example, methods referred to as process 1C methods. In some embodiments, significantly improved polyclonality and/or increased polyclonality is indicative of treatment efficacy and/or increased clinical efficacy. In some embodiments, polyclonality refers to the T-cell repertoire diversity. In some embodiments, an increase in polyclonality can be indicative of treatment efficacy with regard to administration of the TILs produced by the methods of the present invention. In some embodiments, polyclonality is increased one-fold, two-fold, ten-fold, 100-fold, 500-fold, or 1000-fold as compared to TILs prepared using methods than those provide herein including for example, methods other than those embodied in Figure 1. In some embodiments, polyclonality is increased one-fold as compared to an untreated patient and/or as compared to a patient treated with TILs prepared using other methods than those provide herein including for example, methods other than those embodied in Figure 1. In some embodiments, polyclonality is increased two-fold as compared to an untreated patient and/or as compared to a patient treated with TILs prepared using other methods than those provide herein including for example, methods other than those embodied in Figure 1. In some embodiments, polyclonality is increased ten-fold as compared to an untreated patient and/or as compared to a patient treated with TILs prepared using other methods than those provide herein including for example, methods other than those embodied in Figure 1. In some embodiments, polyclonality is increased 100-fold as compared to an untreated patient and/or as compared to a patient treated with TILs prepared using other methods than those provide herein including for example, methods other than those embodied in Figure 1. In some embodiments, polyclonality is increased 500-fold as compared to an untreated patient and/or as compared to a patient treated with TILs prepared using other methods than those provide herein including for example, methods other than those embodied in Figure 1. In some embodiments, polyclonality is increased 1000-fold as compared to an untreated patient and/or as compared to a patient treated with TILs prepared using other methods than those provide herein including for example, methods other than those embodied in Figure 1.

Measures of efficacy can include the disease control rate (DCR) as well as overall response rate (ORR), as known in the art as well as described herein.

*Methods of Treating Cancer and Other Diseases*

The compositions described herein can be used in a method for treating diseases. In an embodiment, they are for use in treating hyperproliferative disorders. They may also be used in treating other disorders as described herein and in the following paragraphs.

In some embodiments, the hyperproliferative disorder is cancer. In some embodiments, the hyperproliferative disorder is a solid tumor cancer. In some embodiments, the solid tumor cancer is selected from the group consisting of melanoma, ovarian cancer, endometrial cancer, thyroid cancer, colorectal cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), renal cancer, and renal cell carcinoma. In some embodiments, the hyperproliferative disorder is a hematological malignancy. In some embodiments, the solid tumor cancer is selected from the group consisting of chronic lymphocytic leukemia, acute lymphoblastic leukemia, diffuse large B cell lymphoma, non-Hodgkin's lymphoma, Hodgkin's lymphoma, follicular lymphoma, and mantle cell lymphoma.

Disclosed herein but not claimed is a method of treating a cancer with a population of TILs, wherein a patient is pre-treated with non-myeloablative chemotherapy prior to an infusion of TILs according to the present disclosure. Disclosed herein but not claimed is a method wherein the non-myeloablative chemotherapy is cyclophosphamide 60 mg/kg/d for 2 days (days 27 and 26 prior to TIL infusion) and fludarabine 25 mg/m$^2$/d for 5 days (days 27 to 23 prior to TIL infusion). Disclosed herein but not claimed is a method wherein, after non-myeloablative chemotherapy and TIL infusion (at day 0) according to the present disclosure, the patient receives an intravenous infusion of IL-2 intravenously at 720,000 IU/kg every 8 hours to physiologic tolerance.

Efficacy of the compounds and combinations of compounds described herein in treating, preventing and/or managing the indicated diseases or disorders can be tested using various models known in the art, which provide guidance for treatment of human disease. For example, models for determining efficacy of treatments for ovarian cancer are described, e.g., in Mullany, et al., Endocrinology 2012, 153, 1585-92; and Fong, et al., J. Ovarian Res. 2009, 2, 12. Models for determining efficacy of treatments for pancreatic cancer are described in Herreros-Villanueva, et al., World J. Gastroenterol. 2012, 18, 1286-1294. Models for determining efficacy of treatments for breast cancer are described, e.g., in Fantozzi, Breast Cancer Res. 2006, 8, 212. Models for determining efficacy of treatments for melanoma are described, e.g., in Damsky, et al., Pigment Cell & Melanoma Res. 2010, 23, 853-859. Models for determining efficacy of treatments for lung cancer are described, e.g., in Meuwissen, et al., Genes & Development, 2005, 19, 643-664. Models for determining efficacy of treatments for lung cancer are described, e.g., in Kim, Clin. Exp. Otorhinolaryngol. 2009, 2, 55-60; and Sano, Head Neck Oncol. 2009, 1, 32.

In some embodiments, IFN-gamma (IFN-$\gamma$) is indicative of treatment efficacy for hyperproliferative disorder treatment. In some embodiments, IFN-$\gamma$ in the blood of subjects treated with TILs is indicative of active TILs. In some embodiments, a potency assay for IFN-$\gamma$ production is employed. IFN-$\gamma$ production is another measure of cytotoxic potential. IFN-$\gamma$ production can be measured by determining the levels of the cytokine IFN-$\gamma$ in the blood of a subject treated with TILs prepared by the methods of the present invention, including those as described for example in Figure 1. In some embodiments, the TILs obtained by the present method provide for increased IFN-$\gamma$ in the blood of subjects treated with the TILs of the present method as compared subjects treated with TILs prepared using methods referred to as process 1C, as exemplified in Figure 5 and/or Figure 6. In some embodiments, an increase in IFN-$\gamma$ is indicative of treatment efficacy in a patient treated with the TILs produced by the methods of the present invention. In some embodiments, IFN-$\gamma$ is increased one-fold, two-fold, three-fold, four-fold, or five-fold or more as compared to an untreated patient and/or as compared to a patient treated with TILs prepared using other methods than those provide herein including for example, methods other than those embodied in Figure 1. In some embodiments, IFN-y secretion is increased one-fold as compared to an untreated patient and/or as compared to a patient treated with TILs prepared using other methods than those provide herein including for example, methods other than those embodied in Figure 1. In some embodiments, IFN-$\gamma$ secretion is increased two-fold as compared to an untreated patient and/or as compared to a patient treated with TILs prepared using other methods than those provide herein including for example, methods other than those embodied in Figure 1. In some embodiments, IFN-$\gamma$ secretion is increased three-fold as compared to an untreated patient and/or as compared to a patient treated with TILs prepared using other methods than those provide herein including for example, methods other than those embodied in Figure 1. In some embodiments, IFN-$\gamma$ secretion is increased four-fold as compared to an untreated patient and/or as compared to a patient treated with TILs prepared using other methods than those provide herein including for example, methods other than those embodied in Figure 1. In some embodiments, IFN-$\gamma$ secretion is increased five-fold as compared to an untreated patient and/or as compared to a patient treated with TILs prepared using other methods than those provide herein including for example, methods other than those embodied in Figure 1. In some embodiments, IFN-$\gamma$ is measured using a Quantikine ELISA kit. In some embodiments, IFN-$\gamma$ is measured using a Quantikine ELISA kit. In some embodiments, IFN-$\gamma$ is measured in TILs ex vivo from a patient treated with the TILs produced by the methods of the present invention. In some embodiments, IFN-$\gamma$ is measured in blood in a patient treated with the TILs produced by the methods of the present invention. In some embodiments, IFN-$\gamma$ is measured in serum in a patient treated with the TILs produced by the methods of the present invention.

In some embodiments, the TILs prepared by the methods of the present invention, including those as described for example in Figure 1, exhibit increased polyclonality as compared to TILs produced by other methods, including those not exemplified in Figure 1, such as for example, methods referred to as process 1C methods. In some embodiments, significantly improved polyclonality and/or increased polyclonality is indicative of treatment efficacy and/or increased clinical efficacy for cancer treatment. In some embodiments, polyclonality refers to the T-cell repertoire diversity. In some embodiments, an increase in polyclonality can be indicative of treatment efficacy with regard to administration of the TILs produced by the methods of the present invention. In some embodiments, polyclonality is increased one-fold, two-fold, ten-fold, 100-fold, 500-fold, or 1000-fold as compared to TILs prepared using methods than those provide herein including for example, methods other than those embodied in Figure 1. In some embodiments, polyclonality is increased one-fold as compared to an untreated patient and/or as compared to a patient treated with TILs prepared using other methods than those provide herein including for example, methods other than those embodied in Figure 1. In some embodiments, polyclonality is increased two-fold as compared to an untreated patient and/or as compared to a patient treated with TILs prepared using other methods than those provide herein including for example, methods other than those embodied in Figure 1. In some embodiments, polyclonality is increased ten-fold as compared to an untreated patient and/or as compared to a patient treated with TILs prepared using other methods than those provide herein including for example, methods other than those embodied in Figure 1. In some embodiments, polyclonality is increased 100-fold as compared to an untreated patient and/or as compared to a patient treated with TILs prepared using other methods than those provide herein including for example, methods other than those embodied in Figure 1. In some embodiments, polyclonality is increased 500-fold as compared to an untreated patient and/or as compared to a patient treated with TILs prepared using other methods than those provide herein including for example, methods other than those embodied in Figure 1. In some embodiments, polyclonality is increased 1000-fold as compared to an untreated patient and/or as compared to a patient treated with TILs prepared using other methods than those provided herein including for example, methods other than those embodied in Figure 1.

Disclosed herein but not claimed is a method of treating cancer in a patient comprising administering to the patient a therapeutically effective dosage of TILs prepared using any of the foregoing methods of expanding TILs from frozen tumor tissue. In some embodiments, the cancer is a solid tumor cancer. In some embodiments, the solid tumor cancer is selected from the group consisting of melanoma, ovarian cancer, endometrial cancer, thyroid cancer, colorectal cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), renal cancer, and renal cell carcinoma. In some embodiments, the cancer is a hematological malignancy. In some embodiments, the hematological malignancy is selected from the group consisting of chronic lymphocytic leukemia, acute lymphoblastic leukemia, diffuse large B cell lymphoma, non-Hodgkin's lymphoma, Hodgkin's lymphoma, follicular lymphoma, and mantle cell lymphoma.

Disclosed herein but not claimed is a pharmaceutical composition comprising TILs prepared using any of the foregoing methods of expanding TILs from frozen tumor tissue and a pharmaceutically acceptable carrier.

Disclosed herein but not claimed, a pharmaceutical composition comprising TILs prepared using any of the foregoing methods of expanding TILs from frozen tumor tissue and a pharmaceutically acceptable carrier for use in a method of treating cancer in a patient, the method comprising administering to the patient a therapeutically effective dosage of the pharmaceutical composition. In some embodiments, the cancer is a solid tumor cancer. In some embodiments, the solid tumor cancer is selected from the group consisting of melanoma, ovarian cancer, endometrial cancer, thyroid cancer, colorectal cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), renal cancer, and renal cell carcinoma. In some embodiments, the cancer is a hematological malignancy. In some embodiments, the hematological malignancy is selected from the group consisting of chronic lymphocytic leukemia, acute lymphoblastic leukemia, diffuse large B cell lymphoma, non-Hodgkin's lymphoma, Hodgkin's lymphoma, follicular lymphoma, and mantle cell lymphoma.

Disclosed herein but not claimed is a pharmaceutical composition comprising TILs prepared using any of the foregoing methods of expanding TILs from frozen tumor tissue and a pharmaceutically acceptable carrier in a method of treating cancer in a patient, the method comprising administering to the patient a therapeutically effective dosage of the pharmaceutical composition. In some embodiments, the cancer is a solid tumor cancer. In some embodiments, the solid tumor cancer is selected from the group consisting of melanoma, ovarian cancer, endometrial cancer, thyroid cancer, colorectal cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), renal cancer, and renal cell carcinoma. In some embodiments, the cancer is a hematological malignancy. In some embodiments, the hematological malignancy is selected from the group consisting of chronic lymphocytic leukemia, acute lymphoblastic leukemia, diffuse large B cell lymphoma, non-Hodgkin's lymphoma, Hodgkin's lymphoma, follicular lymphoma, and mantle cell lymphoma.

*Methods of co-administration*

In some embodiments, the TILs produced as described herein, including for example TILs derived from a method described in Steps A through F of Figure 1, can be administered in combination with one or more immune checkpoint regulators, such as the antibodies described below. For example, antibodies that target PD-1 and which can be co-administered with the TILs of the present invention include, *e.g.,* but are not limited to nivolumab (BMS-936558, Bristol-Myers Squibb; Opdivo®), pembrolizumab (lambrolizumab, MK03475 or MK-3475, Merck; Keytruda®), humanized anti-PD-1 antibody JS001 (ShangHai JunShi), monoclonal anti-PD-1 antibody TSR-042 (Tesaro, Inc.), Pidilizumab (anti-PD-1 mAb CT-011, Medivation), anti-PD-1 monoclonal Antibody BGB-A317 (BeiGene), and/or anti-PD-1 antibody SHR-1210 (ShangHai HengRui), human monoclonal antibody REGN2810 (Regeneron), human monoclonal antibody MDX-1106 (Bristol-Myers Squibb), and/or humanized anti-PD-1 IgG4 antibody PDR001 (Novartis). In some embodiments, the PD-1 antibody is from clone: RMP1-14 (rat IgG) - BioXcell cat# BP0146. Other suitable antibodies suitable for use in co-administration methods with TILs produced according to Steps A through F as described herein are anti-PD-1 antibodies disclosed in U.S. Patent No. 8,008,449. In some embodiments, the antibody or antigen-binding portion thereof binds specifically to PD-L1 and inhibits its interaction with PD-1, thereby increasing immune activity. Any antibodies known in the art which bind to PD-L1 and disrupt the interaction between the PD-1 and PD-L1, and stimulates an anti-

tumor immune response, are suitable for use in co-administration methods with TILs produced according to Steps A through F as described herein. For example, antibodies that target PD-L1 and are in clinical trials, include BMS-936559 (Bristol-Myers Squibb) and MPDL3280A (Genentech). Other suitable antibodies that target PD-L1 are disclosed in U.S. Patent No. 7,943,743. It will be understood by one of ordinary skill that any antibody which binds to PD-1 or PD-L1, disrupts the PD-1/PD-L1 interaction, and stimulates an anti-tumor immune response, are suitable for use in co-administration methods with TILs produced according to Steps A through F as described herein. Disclosed herein but not claimed are methods wherein the subject administered the combination of TILs produced according to Steps A through F is co administered with a and anti-PD-1 antibody when the patient has a cancer type that is refractory to administration of the anti-PD-1 antibody alone. Disclosed herein but not claimed are methods whereinthe patient is administered TILs in combination with and anti-PD-1 when the patient has refractory melanoma. Disclosed herein but not claimed are methods whereinthe patient is administered TILs in combination with and anti-PD-1 when the patient has non-small-cell lung carcinoma (NSCLC).

*Optional Lymphodepletion Preconditioning of Patients*

In an embodiment, the invention includes a method of treating a cancer with a population of TILs, wherein a patient is pre-treated with non-myeloablative chemotherapy prior to an infusion of TILs according to the present disclosure. In an embodiment, the invention includes a population of TILs for use in the treatment of cancer in a patient which has been pre-treated with non-myeloablative chemotherapy. In an embodiment, the population of TILs is for administration by infusion. In an embodiment, the non-myeloablative chemotherapy is cyclophosphamide 60 mg/kg/d for 2 days (days 27 and 26 prior to TIL infusion) and fludarabine 25 mg/m$^2$/d for 5 days (days 27 to 23 prior to TIL infusion). Disclosed herein but not claimed are methods wherein, after non-myeloablative chemotherapy and TIL infusion (at day 0) according to the present disclosure, the patient receives an intravenous infusion of IL-2 (aldesleukin, commercially available as PROLEUKIN) intravenously at 720,000 IU/kg every 8 hours to physiologic tolerance. In certain embodiments, the population of TILs is for use in treating cancer in combination with IL-2, wherein the IL-2 is administered after the population of TILs.

Experimental findings indicate that lymphodepletion prior to adoptive transfer of tumor-specific T lymphocytes plays a key role in enhancing treatment efficacy by eliminating regulatory T cells and competing elements of the immune system ('cytokine sinks'). Accordingly, some embodiments of the invention utilize a lymphodepletion step (sometimes also referred to as "immunosuppressive conditioning") on the patient prior to the introduction of the TILs of the invention.

In general, lymphodepletion is achieved using administration of fludarabine or cyclophosphamide (the active form being referred to as mafosfamide) and combinations thereof. Such methods are described in Gassner, et al., Cancer Immunol. Immunother. 2011, 60, 75-85, Muranski, et al., Nat. Clin. Pract. Oncol., 2006, 3, 668-681, Dudley, et al., J. Clin. Oncol. 2008, 26, 5233-5239, and Dudley, et al., J. Clin. Oncol. 2005, 23, 2346-2357.

Disclosed herein but not claimed are methods wherein the fludarabine is administered at a concentration of 0.5 μg/mL -10 μg/mL fludarabine. Disclosed herein but not claimed are methods wherein the fludarabine is administered at a concentration of 1 μg/mL fludarabine. Disclosed herein but not claimed are methods wherein the fludarabine treatment is administered for 1 day, 2 days, 3 days, 4 days, 5 days, 6 days, or 7 days or more. Disclosed herein but not claimed are methods wherein the fludarabine is administered at a dosage of 10 mg/kg/day, 15 mg/kg/day, 20 mg/kg/day, 25 mg/kg/day, 30 mg/kg/day, 35 mg/kg/day, 40 mg/kg/day, or 45 mg/kg/day. Disclosed herein but not claimed are methods wherein the fludarabine treatment is administered for 2-7 days at 35 mg/kg/day. Disclosed herein but not claimed are methods wherein the fludarabine treatment is administered for 4-5 days at 35 mg/kg/day. Disclosed herein but not claimed are methods wherein the fludarabine treatment is administered for 4-5 days at 25 mg/kg/day.

In some embodiments, the mafosfamide, the active form of cyclophosphamide, is obtained at a concentration of 0.5 μg/mL -10 μg/mL by administration of cyclophosphamide. In some embodiments, mafosfamide, the active form of cyclophosphamide, is obtained at a concentration of 1 μg/mL by administration of cyclophosphamide. Disclosed herein but not claimed are methods wherein the cyclophosphamide treatment is administered for 1 day, 2 days, 3 days, 4 days, 5 days, 6 days, or 7 days or more. Disclosed herein but not claimed are methods wherein the cyclophosphamide is administered at a dosage of 100 mg/m$^2$/day, 150 mg/m$^2$/day, 175 mg/m$^2$/day, 200 mg/m$^2$/day, 225 mg/m$^2$/day, 250 mg/m$^2$/day, 275 mg/m$^2$/day, or 300 mg/m$^2$/day. Disclosed herein but not claimed are methods wherein the cyclophosphamide is administered intravenously (i.v.) Disclosed herein but not claimed are methods wherein the cyclophosphamide treatment is administered for 2-7 days at 35 mg/kg/day. Disclosed herein but not claimed are methods wherein, the cyclophosphamide treatment is administered for 4-5 days at 250 mg/m$^2$/day i.v. Disclosed herein but not claimed are methods wherein, the cyclophosphamide treatment is administered for 4 days at 250 mg/m$^2$/day i.v.

Disclosed herein but not claimed are methods wherein lymphodepletion is performed by administering the fludarabine and the cyclophosphamide together to a patient. Disclosed herein but not claimed are methods wherein fludarabine is administered at 25 mg/m$^2$/day i.v. and cyclophosphamide is administered at 250 mg/m$^2$/day i.v. over 4 days.

Disclosed herein but not claimed are methods wherein the lymphodepletion is performed by administration of cyclophosphamide at a dose of 60 mg/m$^2$/day for two days followed by administration of fludarabine at a dose of 25 mg/m$^2$/day for five days.

*IL-2 Regimens*

Disclosed herein but not claimed are methods wherein the IL-2 regimen comprises a high-dose IL-2 regimen, wherein the high-dose IL-2 regimen comprises aldesleukin, or a biosimilar or variant thereof, administered intravenously starting on the day after administering a therapeutically effective portion of the therapeutic population of TILs, wherein the aldesleukin or a biosimilar or variant thereof is administered at a dose of 0.037 mg/kg or 0.044 mg/kg IU/kg (patient body mass) using 15-minute bolus intravenous infusions every eight hours until tolerance, for a maximum of 14 doses. Following 9 days of rest, this schedule may be repeated for another 14 doses, for a maximum of 28 doses in total.

In an embodiment, the IL-2 regimen comprises a decrescendo IL-2 regimen. Decrescendo IL-2 regimens have been described in O'Day, et al., J. Clin. Oncol. 1999, 17, 2752-61 and Eton, et al., Cancer 2000, 88, 1703-9. Disclosed herein but not claimed are methods wherein a decrescendo IL-2 regimen comprises $18 \times 10^6$ IU/m$^2$ administered intravenously over 6 hours, followed by $18 \times 10^6$ IU/m$^2$ administered intravenously over 12 hours, followed by $18 \times 10^6$ IU/m$^2$ administered intravenously over 24 hrs, followed by $4.5 \times 10^6$ IU/m$^2$ administered intravenously over 72 hours. This treatment cycle may be repeated every 28 days for a maximum of four cycles. In an embodiment, a decrescendo IL-2 regimen comprises 18,000,000 IU/m$^2$ on day 1, 9,000,000 IU/m$^2$ on day 2, and 4,500,000 IU/m$^2$ on days 3 and 4.

Disclosed herein but not claimed are methods wherein the IL-2 regimen comprises administration of pegylated IL-2 every 1, 2, 4, 6, 7, 14 or 21 days at a dose of 0.10 mg/day to 50 mg/day.

*Adoptive Cell Transfer*

Adoptive cell transfer (ACT) is a very effective form of immunotherapy and involves the transfer of immune cells with antitumor activity into cancer patients. ACT is a treatment approach that involves the identification, *in vitro,* of lymphocytes with antitumor activity, the in vitro expansion of these cells to large numbers and their infusion into the cancer-bearing host. Lymphocytes used for adoptive transfer can be derived from the stroma of resected tumors (tumor infiltrating lymphocytes or TILs). TILs for ACT can be prepared as described in the TIL manufacturing process herein, including using a tumor cryopreservation and thawing step. In some embodiments, the TILs are prepared, for example, according to a method as described in Figure 1. They can also be derived or from blood if they are genetically engineered to express antitumor T-cell receptors (TCRs) or chimeric antigen receptors (CARs), enriched with mixed lymphocyte tumor cell cultures (MLTCs), or cloned using autologous antigen presenting cells and tumor derived peptides. ACT in which the lymphocytes originate from the cancer-bearing host to be infused is termed autologous ACT. U.S. Publication No. 2011/0052530 relates to a method for performing adoptive cell therapy to promote cancer regression, primarily for treatment of patients suffering from metastatic melanoma.. Disclosed herein but not claimed are methods wherein TILs can be administered in a single dose. Such administration may be by injection, e.g., intravenous injection. Disclosed herein but not claimed are methods wherein TILs and/or cytotoxic lymphocytes may be administered in multiple doses. Dosing may be once, twice, three times, four times, five times, six times, or more than six times per year. Dosing may be once a month, once every two weeks, once a week, or once every other day. Administration of TILs and/or cytotoxic lymphocytes may continue as long as necessary.

Combinations with PD-1 and PD-L1 Inhibitors

Programmed death 1 (PD-1) is a 288-amino acid transmembrane immunocheckpoint receptor protein expressed by T cells, B cells, natural killer (NK) T cells, activated monocytes, and dendritic cells. PD-1, which is also known as CD279, belongs to the CD28 family, and in humans is encoded by the *Pdcd1* gene on chromosome 2. PD-1 consists of one immunoglobulin (Ig) superfamily domain, a transmembrane region, and an intracellular domain containing an immunoreceptor tyrosine-based inhibitory motif (ITIM) and an immunoreceptor tyrosine-based switch motif (ITSM). PD-1 and its ligands (PD-L1 and PD-L2) are known to play a key role in immune tolerance, as described in Keir, et al., Annu. Rev. Immunol. 2008, 26:677-704. PD-1 provides inhibitory signals that negatively regulate T cell immune responses. PD-L1 (also known as B7-H1 or CD274) and PD-L2 (also known as B7-DC or CD273) are expressed on tumor cells and stromal cells, which may be encountered by activated T cells expressing PD-1, leading to immunosuppression of the T cells. PD-L1 is a 290 amino acid transmembrane protein encoded by the *Cd274* gene on human chromosome 9. Blocking the interaction between PD-1 and its ligands PD-L1 and PD-L2 by use of a PD-1 inhibitor, a PD-L1 inhibitor, and/or a PD-L2 inhibitor can overcome immune resistance, as demonstrated in recent clinical studies, such as that described in Topalian, et al., N. Eng. J. Med. 2012, 366:2443-54. PD-L1 is expressed on many tumor cell lines, while PD-L2 is expressed is expressed mostly on dendritic cells and a few tumor lines. In addition to T cells (which inducibly express PD-1 after activation), PD-1 is also expressed on B cells, natural killer cells, macrophages, activated monocytes, and dendritic cells.

The methods, compositions, and combinations of TILs and TNFRSF agonists described herein may also be further combined with programmed death-1 (PD-1), programmed death ligand 1 (PD-L1), and/or programmed death ligand 2 (PD-L2) binding antibodies, antagonists, or inhibitors (*i.e.*, blockers). PD-1, PD-L1, and/or PD-L2 inhibitors may be used in cell culture in conjunction with the TNFRSF agonists described herein during the pre-REP or REP stages of TIL expansion. PD-1, PD-L1, and/or PD-L2 inhibitors may also be used in conjunction with TNFRSF agonists prior to surgical resection of tumor, or during or after infusion of TILs. For example, suitable methods of using PD-1/PD-L1 inhibitors in conjunction with agonistic GITR antibodies and compositions comprising PD-1/PD-L1 antagonists and GITR agonists are described in International Patent Application Publication No. WO 2015/026684 A1.

In an embodiment, the PD-1 inhibitor may be any PD-1 inhibitor or PD-1 blocker known in the art. In particular, it is one of the PD-1 inhibitors or blockers described in more detail in the following paragraphs. The terms "inhibitor," "antagonist," and "blocker" are used interchangeably herein in reference to PD-1 inhibitors. For avoidance of doubt, references herein to a PD-1 inhibitor that is an antibody may refer to a compound or antigen-binding fragments, variants, conjugates, or biosimilars thereof. For avoidance of doubt, references herein to a PD-1 inhibitor may also refer to a small molecule compound or a pharmaceutically acceptable salt, ester, solvate, hydrate, cocrystal, or prodrug thereof.

In some embodiments, the compositions and methods described herein include a PD-1 inhibitor. In some embodiments, the PD-1 inhibitor is a small molecule. In a preferred embodiment, the PD-1 inhibitor is an antibody (*i.e.*, an anti-PD-1 antibody), a fragment thereof, including Fab fragments, or a single-chain variable fragment (scFv) thereof. In some embodiments the PD-1 inhibitor is a polyclonal antibody. In a preferred embodiment, the PD-1 inhibitor is a monoclonal antibody. In some embodiments, the PD-1 inhibitor competes for binding with PD-1, and/or binds to an epitope on PD-1. In an embodiment, the antibody competes for binding with PD-1, and/or binds to an epitope on PD-1.

In some embodiments, the compositions and methods described include a PD-1 inhibitor that binds human PD-1 with a $K_D$ of about 100 pM or lower, binds human PD-1 with a $K_D$ of about 90 pM or lower, binds human PD-1 with a $K_D$ of about 80 pM or lower, binds human PD-1 with a $K_D$ of about 70 pM or lower, binds human PD-1 with a $K_D$ of about 60 pM or lower, binds human PD-1 with a $K_D$ of about 50 pM or lower, binds human PD-1 with a $K_D$ of about 40 pM or lower, binds human PD-1 with a $K_D$ of about 30 pM or lower, binds human PD-1 with a $K_D$ of about 20 pM or lower, binds human PD-1 with a $K_D$ of about 10 pM or lower, or binds human PD-1 with a $K_D$ of about 1 pM or lower.

In some embodiments, the compositions and methods described include a PD-1 inhibitor that binds to human PD-1 with a $k_{assoc}$ of about $7.5 \times 10^5$ l/M·s or faster, binds to human PD-1 with a $k_{assoc}$ of about $7.5 \times 10^5$ 1/M-s or faster, binds to human PD-1 with a $k_{assoc}$ of about $8 \times 10^5$ 11M. s or faster, binds to human PD-1 with a $k_{assoc}$ of about $8.5 \times 10^5$ l/M·s or faster, binds to human PD-1 with a $k_{assoc}$ of about $9 \times 10^5$ 1/M-s or faster, binds to human PD-1 with a $k_{assoc}$ of about $9.5 \times 10^5$ l/M·s or faster, or binds to human PD-1 with a $k_{assoc}$ of about $1 \times 10^6$ 11M. s or faster.

In some embodiments, the compositions and methods described include a PD-1 inhibitor that binds to human PD-1 with a $k_{dissoc}$ of about $2 \times 10^{-5}$ 1/s or slower, binds to human PD-1 with a $k_{dissoc}$ of about $2.1 \times 10^{-5}$ 1/s or slower , binds to human PD-1 with a $k_{dissoc}$ of about $2.2 \times 10^{-5}$ 1/s or slower, binds to human PD-1 with a $k_{dissoc}$ of about $2.3 \times 10^{-5}$ 1/s or slower, binds to human PD-1 with a $k_{dissoc}$ of about $2.4 \times 10^{-5}$ 1/s or slower, binds to human PD-1 with a $k_{dissoc}$ of about $2.5 \times 10^{-5}$ 1/s or slower, binds to human PD-1 with a $k_{dissoc}$ of about $2.6 \times 10^{-5}$ 1/s or slower or binds to human PD-1 with a $k_{dissoc}$ of about $2.7 \times 10^{-5}$ 1/s or slower, binds to human PD-1 with a $k_{dissoc}$ of about $2.8 \times 10^{-5}$ 1/s or slower, binds to human PD-1 with a $k_{dissoc}$ of about $2.9 \times 10^{-5}$ 1/s or slower, or binds to human PD-1 with a $k_{dissoc}$ of about $3 \times 10^{-5}$ 1/s or slower.

In some embodiments, the compositions and methods described include a PD-1 inhibitor that blocks or inhibits binding of human PD-L1 or human PD-L2 to human PD-1 with an $IC_{50}$ of about 10 nM or lower, blocks or inhibits binding of human PD-L1 or human PD-L2 to human PD-1 with an $IC_{50}$ of about 9 nM or lower, blocks or inhibits binding of human PD-L1 or human PD-L2 to human PD-1 with an $IC_{50}$ of about 8 nM or lower, blocks or inhibits binding of human PD-L1 or human PD-L2 to human PD-1 with an $IC_{50}$ of about 7 nM or lower, blocks or inhibits binding of human PD-L1 or human PD-L2 to human PD-1 with an $IC_{50}$ of about 6 nM or lower, blocks or inhibits binding of human PD-L1 or human PD-L2 to human PD-1 with an $IC_{50}$ of about 5 nM or lower, blocks or inhibits binding of human PD-L1 or human PD-L2 to human PD-1 with an $IC_{50}$ of about 4 nM or lower, blocks or inhibits binding of human PD-L1 or human PD-L2 to human PD-1 with an $IC_{50}$ of about 3 nM or lower, blocks or inhibits binding of human PD-L1 or human PD-L2 to human PD-1 with an $IC_{50}$ of about 2 nM or lower, or blocks or inhibits binding of human PD-L1 or human PD-L2 to human PD-1 with an $IC_{50}$ of about 1 nM or lower.

In an embodiment, the PD-1 inhibitor is nivolumab (commercially available as OPDIVO from Bristol-Myers Squibb Co.), or biosimilars, antigen-binding fragments, conjugates, or variants thereof. Nivolumab is a fully human IgG4 antibody blocking the PD-1 receptor. In an embodiment, the anti-PD-1 antibody is an immunoglobulin G4 kappa, anti-(human CD274) antibody. Nivolumab is assigned Chemical Abstracts Service (CAS) registry number 946414-94-4 and is also known as 5C4, BMS-936558, MDX-1106, and ONO-4538. The preparation and properties of nivolumab are described in U.S. Patent No. 8,008,449 and International Patent Publication No. WO 2006/121168. The clinical safety and efficacy of nivolumab in various forms of cancer has been described in Wang, et al., Cancer Immunol Res. 2014, 2:846-56; Page, et al., Ann. Rev. Med., 2014, 65, 185-202; and Weber, et al., J. Clin. Oncology, 2013, 31:4311-4318. The amino acid sequences of nivolumab are set forth in Table 16. Nivolumab has intra-heavy chain disulfide linkages at 22-96,140-196, 254-314, 360-418, 22"-96", 140"-196", 254"-314", and 360"-418"; intra-light chain disulfide linkages at 23'-88', 134'-194', 23'''-88''', and 134'''-194'''; inter-heavy-light chain disulfide linkages at 127-214', 127"-214''', inter-heavy-heavy chain disulfide linkages at 219-219" and 222-222"; and N-glycosylation sites (H CH$_2$ 84.4) at 290, 290".

In an embodiment, a PD-1 inhibitor comprises a heavy chain given by SEQ ID NO:127 and a light chain given by SEQ ID NO:128. In an embodiment, a PD-1 inhibitor comprises heavy and light chains having the sequences shown in SEQ ID NO:127 and SEQ ID NO:128, respectively, or antigen binding fragments, Fab fragments, single-chain variable fragments (scFv), variants, or conjugates thereof. In an embodiment, a PD-1 inhibitor comprises heavy and light chains that are each at least 99% identical to the sequences shown in SEQ ID NO:127 and SEQ ID NO:128, respectively. In an embodiment, a PD-1 inhibitor comprises heavy and light chains that are each at least 98% identical to the sequences shown in SEQ ID NO:127 and SEQ ID NO:128, respectively. In an embodiment, a PD-1 inhibitor comprises heavy and light chains that are each at least 97% identical to the sequences shown in SEQ ID NO:127 and SEQ ID NO:128, respectively. In an embodiment, a PD-1 inhibitor comprises heavy and light chains that are each at least 96% identical to the sequences shown in SEQ ID NO:127 and SEQ ID NO:128, respectively. In an embodiment, a PD-1 inhibitor comprises heavy and light chains that are each at least 95% identical to the sequences shown in SEQ ID NO:127 and SEQ ID NO:128, respectively.

In an embodiment, the PD-1 inhibitor comprises the heavy and light chain CDRs or variable regions (VRs) of nivolumab. In an embodiment, the PD-1 inhibitor heavy chain variable region (V$_H$) comprises the sequence shown in SEQ ID NO:129, and the PD-1 inhibitor light chain variable region (V$_L$) comprises the sequence shown in SEQ ID NO:130, and conservative amino acid substitutions thereof. In an embodiment, a PD-1 inhibitor comprises V$_H$ and V$_L$ regions that are each at least 99% identical to the sequences shown in SEQ ID NO:129 and SEQ ID NO: 130, respectively. In an embodiment, a PD-1 inhibitor comprises V$_H$ and V$_L$ regions that are each at least 98% identical to the sequences shown in SEQ ID NO:129 and SEQ ID NO:130, respectively. In an embodiment, a PD-1 inhibitor comprises V$_H$ and V$_L$ regions that are each at least 97% identical to the sequences shown in SEQ ID NO:129 and SEQ ID NO:130, respectively. In an embodiment, a PD-1 inhibitor comprises V$_H$ and V$_L$ regions that are each at least 96% identical to the sequences shown in SEQ ID NO:129 and SEQ ID NO:130, respectively. In an embodiment, a PD-1 inhibitor comprises V$_H$ and V$_L$ regions that are each at least 95% identical to the sequences shown in SEQ ID NO:129 and SEQ ID NO:130, respectively.

In an embodiment, a PD-1 inhibitor comprises heavy chain CDR1, CDR2 and CDR3 domains having the sequences set forth in SEQ ID NO: 131, SEQ ID NO: 132, and SEQ ID NO:133, respectively, and conservative amino acid substitutions thereof, and light chain CDR1, CDR2 and CDR3 domains having the sequences set forth in SEQ ID NO:134, SEQ ID NO:135, and SEQ ID NO:136, respectively, and conservative amino acid substitutions thereof. In an embodiment, the antibody competes for binding with, and/or binds to the same epitope on PD-1 as any of the aforementioned antibodies.

In an embodiment, the PD-1 inhibitor is an anti-PD-1 biosimilar monoclonal antibody approved by drug regulatory authorities with reference to nivolumab. In an embodiment, the biosimilar comprises an anti-PD-1 antibody comprising an amino acid sequence which has at least 97% sequence identity, *e.g.*, 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is nivolumab. In some embodiments, the one or more post-translational modifications are selected from one or more of: glycosylation, oxidation, deamidation, and truncation. In some embodiments, the biosimilar is an anti-PD-1 antibody authorized or submitted for authorization, wherein the anti-PD-1 antibody is provided in a formulation which differs from the formulations of a reference medicinal product or reference biological product, wherein the reference medicinal product or reference biological product is nivolumab. The anti-PD-1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is nivolumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is nivolumab.

TABLE 16. Amino acid sequences for PD-1 inhibitors related to nivolumab.

| Identifier | Sequence (One-Letter Amino Acid Symbols) | |
|---|---|---|
| SEQ ID NO:127 nivolumab heavy chain | QVQLVESGGG VVQPGRSLRL DCKASGITFS NSGMHWVRQA PGKGLEWVAV IWYDGSKRYY | 60 |
| | ADSVKGRFTI SRDNSKNTLF LQMNSLRAED TAVYYCATND DYWGQGTLVT VSSASTKGPS | 120 |
| | VFPLAPCSRS TSESTAALGC LVKDYFPEPV TVSWNSGALT SGVHTFPAVL QSSGLYSLSS | 180 |
| | VVTVPSSSLG TKTYTCNVDH KPSNTKVDKR VESKYGPPCP PCPAPEFLGG PSVFLFPPKP | 240 |
| | KDTLMISRTP EVTCVVVDVS QEDPEVQFNW YVDGVEVHNA KTKPREEQFN STYRVVSVLT | 300 |
| | VLHQDWLNGK EYKCKVSNKG LPSSIEKTIS KAKGQPREPQ VYTLPPSQEE MTKNQVSLTC | 360 |
| | LVKGFYPSDI AVEWESNGQP ENNYKTTPPV LDSDGSFFLY SRLTVDKSRW QEGNVFSCSV | 420 |
| | MHEALHNHYT QKSLSLSLGK | 440 |
| SEQ ID NO:128 nivolumab light chain | EIVLTQSPAT LSLSPGERAT LSCRASQSVS SYLAWYQQKP GQAPRLLIYD ASNRATGIPA | 60 |
| | RFSGSGSGTD FTLTISSLEP EDFAVYYCQQ SSNWPRTFGQ GTKVEIKRTV AAPSVFIFPP | 120 |
| | SDEQLKSGTA SVVCLLNNFY PREAKVQWKV DNALQSGNSQ ESVTEQDSKD STYSLSSTLT | 180 |
| | LSKADYEKHK VYACEVTHQG LSSPVTKSFN RGEC | 214 |
| SEQ ID NO:129 nivolumab variable heavy chain | QVQLVESGGG VVQPGRSLRL DCKASGITFS NSGMHWVRQA PGKGLEWVAV IWYDGSKRYY | 60 |
| | ADSVKGRFTI SRDNSKNTLF LQMNSLRAED TAVYYCATND DYWGQGTLVT VSS | 113 |
| SEQ ID NO:130 nivolumab variable light chain | EIVLTQSPAT LSLSPGERAT LSCRASQSVS SYLAWYQQKP GQAPRLLIYD ASNRATGIPA | 60 |
| | RFSGSGSGTD FTLTISSLEP EDFAVYYCQQ SSNWPRTFGQ GTKVEIK | 107 |
| SEQ ID NO:131 nivolumab heavy chain CDR1 | NSGMH | |
| SEQ ID NO:132 nivolumab heavy chain CDR2 | VIWYDGSKRY YADSVKG | |
| SEQ ID NO:133 nivolumab heavy chain CDR3 | NDDY | |
| SEQ ID NO:134 nivolumab light chain CDR1 | RASQSVSSYL A | |
| SEQ ID NO:135 nivolumab light chain CDR2 | DASNRAT | |
| SEQ ID NO:136 nivolumab light chain CDR3 | QQSSNWPRT | |

In another embodiment, the PD-1 inhibitor comprises pembrolizumab (commercially available as KEYTRUDA from Merck & Co., Inc., Kenilworth, NJ, USA), or antigen-binding fragments, conjugates, or variants thereof. Pembrolizumab is assigned CAS registry number 1374853-91-4 and is also known as lambrolizumab, MK-3475, and SCH-900475. Pembrolizumab has an immunoglobulin G4, anti-(human protein PDCD1 (programmed cell death 1)) (human-Mus musculus monoclonal heavy chain), disulfide with human-Mus musculus monoclonal light chain, dimer structure. The structure of pembrolizumab may also be described as immunoglobulin G4, anti-(human programmed cell death 1); humanized mouse monoclonal [228-L-proline(H10-S>P)]γ4 heavy chain (134-218')-disulfide with humanized mouse monoclonal κ light chain dimer (226-226":229-229")-bisdisulfide. The properties, uses, and preparation of pembrolizumab are described in International Patent Publication No. WO 2008/156712 A1, U.S. Patent No. 8,354,509 and U.S. Patent Application Publication Nos. US 2010/0266617 A1, US 2013/0108651 A1, and US 2013/0109843 A2. The clinical safety and efficacy of pembrolizumab in various forms of cancer is described in Fuerst, Oncology Times, 2014, 36:35-36; Robert, et al., Lancet, 2014, 384:1109-17; and Thomas, et al., Exp. Opin. Biol. Ther., 2014, 14:1061-1064. The amino acid sequences of pembrolizumab are set forth in Table 21. Pembrolizumab includes the following disulfide bridges: 22-96, 22"-96", 23'-92', 23‴-92‴, 134-218', 134"-218‴, 138'-198', 138‴-198‴, 147-203, 147"-203", 226-226", 229-229", 261-321, 261"-321", 367-425, and 367"-425", and the following glycosylation sites (N): Asn-297 and Asn-297". Pembrolizumab is an IgG4/kappa isotype with a stabilizing S228P mutation in the Fc region; insertion of this mutation in the IgG4 hinge region prevents the formation of half molecules typically observed for IgG4 antibodies. Pembrolizumab is heterogeneously glycosylated at Asn297 within the Fc domain of each heavy chain, yielding a molecular weight of approximately 149 kDa for the intact antibody. The dominant glycoform of pembrolizumab is the fucosylated agalacto diantennary glycan form (G0F).

In an embodiment, a PD-1 inhibitor comprises a heavy chain given by SEQ ID NO:137 and a light chain given by SEQ ID NO:138. In an embodiment, a PD-1 inhibitor comprises heavy and light chains having the sequences shown in SEQ ID NO: 137 and SEQ ID NO: 138, respectively, or antigen binding fragments, Fab fragments, single-chain variable fragments (scFv), variants, or conjugates thereof. In an embodiment, a PD-1 inhibitor comprises heavy and light chains that are each at least 99% identical to the sequences shown in SEQ ID NO: 137 and SEQ ID NO: 138, respectively. In an embodiment, a PD-1 inhibitor comprises heavy and light chains that are each at least 98% identical to the sequences shown in SEQ ID NO: 137 and SEQ ID NO: 138, respectively. In an embodiment, a PD-1 inhibitor comprises heavy and light chains that are each at least 97% identical to the sequences shown in SEQ ID NO: 137 and SEQ ID NO: 138, respectively. In an embodiment, a PD-1 inhibitor comprises heavy and light chains that are each at least 96% identical to the sequences shown in SEQ ID NO: 137 and SEQ ID NO: 138, respectively. In an embodiment, a PD-1 inhibitor comprises heavy and light chains that are each at least 95% identical to the sequences shown in SEQ ID NO: 137 and SEQ ID NO: 138, respectively. Representative sequences are listed in Table 17.

In an embodiment, the PD-1 inhibitor comprises the heavy and light chain CDRs or variable regions (VRs) of pembrolizumab. In an embodiment, the PD-1 inhibitor heavy chain variable region ($V_H$) comprises the sequence shown in SEQ ID NO: 139, and the PD-1 inhibitor light chain variable region ($V_L$) comprises the sequence shown in SEQ ID NO: 140, and conservative amino acid substitutions thereof. In an embodiment, a PD-1 inhibitor comprises $V_H$ and $V_L$ regions that are each at least 99% identical to the sequences shown in SEQ ID NO: 139 and SEQ ID NO: 140, respectively. In an embodiment, a PD-1 inhibitor comprises $V_H$ and $V_L$ regions that are each at least 98% identical to the sequences shown in SEQ ID NO: 139 and SEQ ID NO: 140, respectively. In an embodiment, a PD-1 inhibitor comprises $V_H$ and $V_L$ regions that are each at least 97% identical to the sequences shown in SEQ ID NO: 139 and SEQ ID NO: 140, respectively. In an embodiment, a PD-1 inhibitor comprises $V_H$ and $V_L$ regions that are each at least 96% identical to the sequences shown in SEQ ID NO: 139 and SEQ ID NO: 140, respectively. In an embodiment, a PD-1 inhibitor comprises $V_H$ and $V_L$ regions that are each at least 95% identical to the sequences shown in SEQ ID NO: 139 and SEQ ID NO: 140, respectively.

In an embodiment, a PD-1 inhibitor comprises heavy chain CDR1, CDR2 and CDR3 domains having the sequences set forth in SEQ ID NO: 141, SEQ ID NO: 142, and SEQ ID NO: 143, respectively, and conservative amino acid substitutions thereof, and light chain CDR1, CDR2 and CDR3 domains having the sequences set forth in SEQ ID NO:144, SEQ ID NO:145, and SEQ ID NO:146, respectively, and conservative amino acid substitutions thereof. In an embodiment, the antibody competes for binding with, and/or binds to the same epitope on PD-1 as any of the aforementioned antibodies.

In an embodiment, the PD-1 inhibitor is an anti-PD-1 biosimilar monoclonal antibody approved by drug regulatory authorities with reference to pembrolizumab. In an embodiment, the biosimilar comprises an anti-PD-1 antibody comprising an amino acid sequence which has at least 97% sequence identity, *e.g.*, 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is pembrolizumab. In some embodiments, the one or more post-translational modifications are selected from one or more of: glycosylation, oxidation, deamidation, and truncation. In some embodiments, the biosimilar is an anti-PD-1 antibody authorized or submitted for authorization, wherein the anti-PD-1 antibody is provided in a formulation which differs from the formulations of a reference medicinal product or reference biological product, wherein the reference medicinal product or reference biological product is pembrolizumab. The anti-PD-1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is pembrolizumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is pembrolizumab.

TABLE 17. Amino acid sequences for PD-1 inhibitors related to pembrolizumab.

| Identifier | Sequence (One-Letter Amino Acid Symbols) | |
|---|---|---|
| SEQ ID NO:137 | QVQLVQSGVE VKKPGASVKV SCKASGYTFT NYYMYWVRQA PGQGLEWMGG INPSNGGTNF | 60 |

| Identifier | Sequence (One-Letter Amino Acid Symbols) |
|---|---|
| pembrolizumab heavy chain | NEKFKNRVTL TTDSSTTTAY MELKSLQFDD TAVYYCARRD YRFDMGFDYW GQGTTVTVSS 120<br>ASTKGPSVFP LAPCSRSTSE STAALGCLVK DYFPEPVTVS WNSGALTSGV HTFPAVLQSS 180<br>GLYSLSSVVT VPSSSLGTKT YTCNVDHKPS NTKVDKRVES KYGPPCPPCP APEFLGGPSV 240<br>FLFPPKPKDT LMISRTPEVT CVVVDVSQED PEVQFNWYVD GVEVHNAKTK PREEQFNSTY 300<br>RVVSVLTVLH QDWLNGKEYK CKVSNKGLPS SIEKTISKAK GQPREPQVYT LPPSQEEMTK 360<br>NQVSLTCLVK GFYPSDIAVE WESNGQPENN YKTTPPVLDS DGSFFLYSRL TVDKSRWQEG 420<br>NVFSCSVMHE ALHNHYTQKS LSLSLGK 447 |
| SEQ ID NO:138 pembrolizumab light chain | EIVLTQSPAT LSLSPGERAT LSCRASKGVS TSGYSYLHWY QQKPGQAPRL LIYLASYLES 60<br>GVPARFSGSG SGTDFTLTIS SLEPEDFAVY YCQHSRDLPL TFGGGTKVEI KRTVAAPSVF 120<br>IFPPSDEQLK SGTASVVCLL NNFYPREAKV QWKVDNALQS GNSQESVTEQ DSKDSTYSLS 180<br>STLTLSKADY EKHKVYACEV THQGLSSPVT KSFNRGEC 218 |
| SEQ ID NO:139 pembrolizumab variable heavy chain | QVQLVQSGVE VKKPGASVKV SCKASGYTFT NYYMYWVRQA PGQGLEWMGG INPSNGGTNF 60<br>NEKFKNRVTL TTDSSTTTAY MELKSLQFDD TAVYYCARRD YRFDMGFDYW GQGTTVTVSS 120 |
| SEQ ID NO:140 pembrolizumab variable light chain | EIVLTQSPAT LSLSPGERAT LSCRASKGVS TSGYSYLHWY QQKPGQAPRL LIYLASYLES 60<br>GVPARFSGSG SGTDFTLTIS SLEPEDFAVY YCQHSRDLPL TFGGGTKVEI K 111 |
| SEQ ID NO:141 pembrolizumab heavy chain CDR1 | NYYMY |
| SEQ ID NO:142 pembrolizumab heavy chain CDR2 | GINPSNGGTN FNEKFK |
| SEQ ID NO:143 pembrolizumab heavy chain CDR3 | RDYRFDMGFD Y |
| SEQ ID NO:144 pembrolizumab light chain CDR1 | RASKGVSTSG YSYLH |
| SEQ ID NO:145 pembrolizumab light chain CDR2 | LASYLES |
| SEQ ID NO:146 pembrolizumab light chain CDR3 | QHSRDLPLT |

In an embodiment, the PD-1 inhibitor is a commercially-available anti-PD-1 monoclonal antibody, such as anti-m-PD-1 clones J43 (Cat # BE0033-2) and RMP1-14 (Cat # BE0146) (Bio X Cell, Inc., West Lebanon, NH, USA). A number of commercially-available anti-PD-1 antibodies are known to one of ordinary skill in the art.

In an embodiment, the PD-1 inhibitor is an antibody disclosed in U.S. Patent No. 8,354,509 or U.S. Patent Application Publication Nos. 2010/0266617 A1, 2013/0108651 A1, 2013/0109843 A2. In an embodiment, the PD-1 inhibitor is an anti-PD-1 antibody described in U.S. Patent Nos. 8,287,856, 8,580,247, and 8,168,757 and U.S. Patent Application Publication Nos. 2009/0028857 A1, 2010/0285013 A1, 2013/0022600 A1, and 2011/0008369 A1. In another embodiment, the PD-1 inhibitor is an anti-PD-1 antibody disclosed in U.S. Patent No. 8,735,553 B1. In an embodiment, the PD-1 inhibitor is pidilizumab, also known as CT-011, which is described in U.S. Patent No. 8,686,119.

In an embodiment, the PD-1 inhibitor may be a small molecule or a peptide, or a peptide derivative, such as those described in U.S. Patent Nos. 8,907,053; 9,096,642; and 9,044,442 and U.S. Patent Application Publication No. US 2015/0087581; 1,2,4-oxadiazole compounds and derivatives such as those described in U.S. Patent Application Publication No. 2015/0073024; cyclic peptidomimetic compounds and derivatives such as those described in U.S. Patent Application Publication No. US 2015/0073042; cyclic compounds and derivatives such as those described in U.S. Patent Application Publication No. US 2015/0125491; 1,3,4-oxadiazole and 1,3,4-thiadiazole compounds and derivatives such as those described in International Patent Application Publication No. WO 2015/033301; peptide-based compounds and derivatives such as those described in International Patent Application Publication Nos. WO 2015/036927 and WO 2015/04490, or a macrocyclic peptide-based compounds and derivatives such as those described in U.S. Patent Application Publication No. US 2014/0294898.

In an embodiment, the PD-L1 or PD-L2 inhibitor may be any PD-L1 or PD-L2 inhibitor, antagonist, or blocker known in the art. In particular, it is one of the PD-L1 or PD-L2 inhibitors, antagonist, or blockers described in more detail in the following paragraphs. The terms "inhibitor," "antagonist," and "blocker" are used interchangeably herein in reference to PD-L1 and PD-L2 inhibitors. For avoidance of doubt, references herein to a PD-L1 or PD-L2 inhibitor that is an antibody may refer to a compound or antigen-binding fragments, variants, conjugates, or biosimilars thereof. For avoidance of doubt, references herein to a PD-L1 or PD-L2 inhibitor may refer to a compound or a pharmaceutically acceptable salt, ester, solvate, hydrate, cocrystal, or prodrug thereof.

In some embodiments, the compositions, processes and methods described herein include a PD-L1 or PD-L2 inhibitor. In some embodiments, the PD-L1 or PD-L2 inhibitor is a small molecule. In a preferred embodiment, the PD-L1 or PD-L2 inhibitor is an antibody (*i.e.*, an anti-PD-1 antibody), a fragment thereof, including Fab fragments, or a single-chain variable fragment (scFv) thereof. In some embodiments the PD-L1 or PD-L2 inhibitor is a polyclonal antibody. In a preferred embodiment, the PD-L1 or PD-L2 inhibitor is a monoclonal antibody. In some embodiments, the PD-L1 or PD-L2 inhibitor competes for binding with PD-L1 or PD-L2, and/or binds to an epitope on PD-L1 or PD-L2. In an embodiment, the antibody competes for binding with PD-L1 or PD-L2, and/or binds to an epitope on PD-L1 or PD-L2.

In some embodiments, the PD-L1 inhibitors provided herein are selective for PD-L1, in that the compounds bind or interact with PD-L1 at substantially lower concentrations than they bind or interact with other receptors, including the PD-L2 receptor. In certain embodiments, the compounds bind to the PD-L1 receptor at a binding constant that is at least about a 2-fold higher concentration, about a 3-fold higher concentration, about a 5-fold higher concentration, about a 10-fold higher concentration, about a 20-fold higher concentration, about a 30-fold higher concentration, about a 50-fold higher concentration, about a 100-fold higher concentration, about a 200-fold higher concentration, about a 300-fold higher concentration, or about a 500-fold higher concentration than to the PD-L2 receptor.

In some embodiments, the PD-L2 inhibitors provided herein are selective for PD-L2, in that the compounds bind or interact with PD-L2 at substantially lower concentrations than they bind or interact with other receptors, including the PD-L1 receptor. In certain embodiments, the compounds bind to the PD-L2 receptor at a binding constant that is at least about a 2-fold higher concentration, about a 3-fold higher concentration, about a 5-fold higher concentration, about a 10-fold higher concentration, about a 20-fold higher concentration, about a 30-fold higher concentration, about a 50-fold higher concentration, about a 100-fold higher concentration, about a 200-fold higher concentration, about a 300-fold higher concentration, or about a 500-fold higher concentration than to the PD-L1 receptor.

Without being bound by any theory, it is believed that tumor cells express PD-L1, and that T cells express PD-1. However, PD-L1 expression by tumor cells is not required for efficacy of PD-1 or PD-L1 inhibitors or blockers. In an embodiment, the tumor cells express PD-L1. In another embodiment, the tumor cells do not express PD-L1. In some embodiments, the methods and compositions described herein include a combination of a PD-1 and a PD-L1 antibody, such as those described herein, in combination with a TIL. The administration of a combination of a PD-1 and a PD-L1 antibody and a TIL may be simultaneous or sequential.

In some embodiments, the compositions and methods described include a PD-L1 and/or PD-L2 inhibitor that binds human PD-L1 and/or PD-L2 with a $K_D$ of about 100 pM or lower, binds human PD-L1 and/or PD-L2 with a $K_D$ of about 90 pM or lower, binds human PD-L1 and/or PD-L2 with a $K_D$ of about 80 pM or lower, binds human PD-L1 and/or PD-L2 with a $K_D$ of about 70 pM or lower, binds human PD-L1 and/or PD-L2 with a $K_D$ of about 60 pM or lower, a $K_D$ of about 50 pM or lower, binds human PD-L1 and/or PD-L2 with a $K_D$ of about 40 pM or lower, or binds human PD-L1 and/or PD-L2 with a KD of about 30 pM or lower,

In some embodiments, the compositions and methods described include a PD-L1 and/or PD-L2 inhibitor that binds to human PD-L1 and/or PD-L2 with a $k_{assoc}$ of about $7.5 \times 10^5$ 1/M·s or faster, binds to human PD-L1 and/or PD-L2 with a $k_{assoc}$ of about $8 \times 10^5$ 1/M-s or faster, binds to human PD-L1 and/ or PD-L2 with a $k_{assoc}$ of about $8.5 \times 10^5$ 1/M·s or faster, binds to human PD-L1 and/or PD-L2 with a $k_{assoc}$ of about $9 \times 10^5$ 1/M-s or faster, binds to human PD-L1 and/or PD-L2 with a $k_{assoc}$ of about $9.5 \times 10^5$ 1/M·s and/or faster, or binds to human PD-L1 and/or PD-L2 with a $k_{assoc}$ of about $1 \times 10^6$ 1/M·s or faster.

In some embodiments, the compositions and methods described include a PD-L1 and/or PD-L2 inhibitor that binds to human PD-L1 or PD-L2 with a $k_{dissoc}$ of about $2 \times 10^{-5}$ 1/s or slower, binds to human PD-1 with a $k_{dissoc}$ of about $2.1 \times 10^{-5}$ 1/s or slower , binds to human PD-1 with a $k_{dissoc}$ of about $2.2 \times 10^{-5}$ 1/s or slower, binds to human PD-1 with a $k_{dissoc}$ of about $2.3 \times 10^{-5}$ 1/s or slower, binds to human PD-1 with a $k_{dissoc}$ of about $2.4 \times 10^{-5}$ 1/s or slower, binds to human PD-1 with a $k_{dissoc}$ of about $2.5 \times 10^{-5}$ 1/s or slower, binds to human PD-1 with a $k_{dissoc}$ of about $2.6 \times 10^{-5}$ 1/s or slower, binds to human PD-L1 or PD-L2 with a $k_{dissoc}$ of about $2.7 \times 10^{-5}$ 1/s or slower, or binds to human PD-L1 or PD-L2 with a $k_{dissoc}$ of about $3 \times 10^{-5}$ 1/s or slower.

In some embodiments, the compositions and methods described include a PD-L1 and/or PD-L2 inhibitor that blocks or inhibits binding of human PD-L1 or human PD-L2 to human PD-1 with an $IC_{50}$ of about 10 nM or lower; blocks or inhibits binding of human PD-L1 or human PD-L2 to human PD-1 with an $IC_{50}$ of about 9 nM or lower; blocks or inhibits binding of human PD-L1 or human PD-L2 to human PD-1 with an $IC_{50}$ of about 8 nM or lower; blocks or inhibits binding of human PD-L1 or human PD-L2 to human PD-1 with an $IC_{50}$ of about 7 nM or lower; blocks or inhibits binding of human PD-L1 or human PD-L2 to human PD-1 with an $IC_{50}$ of about 6 nM or lower; blocks or inhibits binding of human PD-L1 or human PD-L2 to human PD-1 with an $IC_{50}$ of about 5 nM or lower; blocks or inhibits binding of human PD-L1 or human PD-L2 to human PD-1 with an $IC_{50}$ of about 4 nM or lower; blocks or inhibits binding of human PD-L1 or human PD-L2 to human PD-1 with an $IC_{50}$ of about 3 nM or lower; blocks or inhibits binding of human PD-L1 or human PD-L2 to human PD-1 with an $IC_{50}$ of about 2 nM or lower; or blocks human PD-1, or blocks binding of human PD-L1 or human PD-L2 to human PD-l with an $IC_{50}$ of about 1 nM or lower.

In an embodiment, the PD-L1 inhibitor is durvalumab, also known as MEDI4736 (which is commercially available from Medimmune, LLC, Gaithersburg, Maryland, a subsidiary of AstraZeneca plc.), or antigen-binding fragments, conjugates, or variants thereof. In an embodiment, the PD-L1 inhibitor is an antibody disclosed in U.S. Patent No. 8,779,108 or U.S. Patent Application Publication No. 2013/0034559. The clinical efficacy of durvalumab has been described in Page, et al., Ann. Rev. Med., 2014, 65:185-202; Brahmer, et al., J. Clin. Oncol. 2014, 32:5s (supplement, abstract 8021); and McDermott, et al., Cancer Treatment Rev., 2014, 40:1056-64. The preparation and properties of durvalumab are described in U.S. Patent No. 8,779,108. The amino acid sequences of durvalumab are set forth in Table 18. The durvalumab monoclonal antibody includes disulfide linkages at 22-96, 22"-96", 23'-89', 23‴-89‴, 135'-195', 135‴-195‴, 148-204, 148"-204", 215'-224', 215‴-224", 230-230", 233-233", 265-325, 265"-325", 371-429, and 371"-429'; and N-glycosylation sites at Asn-301 and Asn-301".

In an embodiment, a PD-L1 inhibitor comprises a heavy chain given by SEQ ID NO:147 and a light chain given by SEQ ID NO:148. In an embodiment, a PD-L1 inhibitor comprises heavy and light chains having the sequences shown in SEQ ID NO:147 and SEQ ID NO:148, respectively, or antigen binding fragments, Fab fragments, single-chain variable fragments (scFv), variants, or conjugates thereof. In an embodiment, a PD-L1 inhibitor comprises heavy and light chains that are each at least 99% identical to the sequences shown in SEQ ID NO:147 and SEQ ID NO:148, respectively. In an embodiment, a PD-L1 inhibitor comprises heavy and light chains that are each at least 98% identical to the sequences shown in SEQ ID NO:147 and SEQ ID NO:148, respectively. In an embodiment, a PD-L1 inhibitor comprises heavy and light chains that are each at least 97% identical to the sequences shown in SEQ ID NO:147 and SEQ ID NO:148, respectively. In an embodiment, a PD-L1 inhibitor comprises heavy and light chains that are each at least 96% identical to the sequences shown in SEQ ID NO:147 and SEQ ID NO:148, respectively. In an embodiment, a PD-L1 inhibitor comprises heavy and light chains that are each at least 95% identical to the sequences shown in SEQ ID NO:147 and SEQ ID NO:148, respectively.

In an embodiment, the PD-L1 inhibitor comprises the heavy and light chain CDRs or variable regions (VRs) of durvalumab. In an embodiment, the PD-L1 inhibitor heavy chain variable region ($V_H$) comprises the sequence shown in SEQ ID NO: 149, and the PD-L1 inhibitor light chain variable region ($V_L$) comprises the sequence shown in SEQ ID NO: 150, and conservative amino acid substitutions thereof. In an embodiment, a PD-L1 inhibitor comprises $V_H$ and $V_L$ regions that are each at least 99% identical to the sequences shown in SEQ ID NO: 149 and SEQ ID NO: 150, respectively. In an embodiment, a PD-L1 inhibitor comprises $V_H$ and $V_L$ regions that are each at least 98% identical to the sequences shown in SEQ ID NO: 149 and SEQ ID NO: 150, respectively. In an embodiment, a PD-L1 inhibitor comprises $V_H$ and $V_L$ regions that are each at least 97% identical to the sequences shown in SEQ ID NO: 149 and SEQ ID NO: 150, respectively. In an embodiment, a PD-L1 inhibitor comprises $V_H$ and $V_L$ regions that are each at least 96% identical to the sequences shown in SEQ ID NO: 149 and SEQ ID NO: 150, respectively. In an embodiment, a PD-L1 inhibitor comprises $V_H$ and $V_L$ regions that are each at least 95% identical to the sequences shown in SEQ ID NO: 149 and SEQ ID NO: 150, respectively.

In an embodiment, a PD-L1 inhibitor comprises heavy chain CDR1, CDR2 and CDR3 domains having the sequences set forth in SEQ ID NO: 151, SEQ ID NO: 152, and SEQ ID NO: 153, respectively, and conservative amino acid substitutions thereof, and light chain CDR1, CDR2 and CDR3 domains having the sequences set forth in SEQ ID NO: 154, SEQ ID NO: 155, and SEQ ID NO: 156, respectively, and conservative amino acid substitutions thereof. In an embodiment, the antibody competes for binding with, and/or binds to the same epitope on PD-L1 as any of the aforementioned antibodies.

In an embodiment, the PD-L1 inhibitor is an anti-PD-L1 biosimilar monoclonal antibody approved by drug regulatory authorities with reference to durvalumab. In an embodiment, the biosimilar comprises an anti-PD-L1 antibody comprising an amino acid sequence which has at least 97% sequence identity, e.g., 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is durvalumab. In some embodiments, the one or more post-translational modifications are selected from one or more of: glycosylation, oxidation, deamidation, and truncation. In some embodiments, the biosimilar is an anti-PD-L1 antibody authorized or submitted for authorization, wherein the anti-PD-L1 antibody is provided in a formulation which differs from the formulations of a reference medicinal product or reference biological product, wherein the reference medicinal product or reference biological product is durvalumab. The anti-PD-L1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is durvalumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is durvalumab.

TABLE 18. Amino acid sequences for PD-L1 inhibitors related to durvalumab.

| Identifier | Sequence (One-Letter Amino Acid Symbols) |
|---|---|
| SEQ ID NO: 147 durvalumab heavy chain | EVQLVESGGG LVQPGGSLRL SCAASGFTFS RYWMSWVRQA PGKGLEWVAN IKQDGSEKYY   60<br>VDSVKGRFTI SRDNAKNSLY LQMNSLRAED TAVYYCAREG GWFGELAFDY WGQGTLVTVS  120<br>SASTKGPSVF PLAPSSKSTS GGTAALGCLV KDYFPEPVTV SWNSGALTSG VHTFPAVLQS  180<br>SGLYSLSSVV TVPSSSLGTQ TYICNVNHKP SNTKVDKRVE PKSCDKTHTC PPCPAPEFEG  240<br>GPSVFLFPPK PKDTLMISRT PEVTCVVVDV SHEDPEVKFN WYVDGVEVHN AKTKPREEQY  300<br>NSTYRVVSVL TVLHQDWLNG KEYKCKVSNK ALPASIEKTI SKAKGQPREP QVYTLPPSRE  360<br>EMTKNQVSLT CLVKGFYPSD IAVEWESNGQ PENNYKTTPP VLDSDGSFFL YSKLTVDKSR  420<br>WQQGNVFSCS VMHEALHNHY TQKSLSLSPG K  451 |
| SEQ ID NO: 148 durvalumab light chain | EVQLVESGGG LVQPGGSLRL SCAASGFTFS RYWMSWVRQA PGKGLEWVAN EIVLTQSPGT   60<br>LSLSPGERAT LSCRASQRVS SSYLAWYQQK PGQAPRLLIY DASSRATGIP DRFSGSGSGT  120<br>DFTLTISRLE PEDFAVYYCQ QYGSLPWTFG QGTKVEIKRT VAAPSVFIFP PSDEQLKSGT  180<br>ASVVCLLNNF YPREAKVQWK VDNALQSGNS QESVTEQDSK DSTYSLSSTL TLSKADYEKH  240<br>KVYACEVTHQ GLSSPVTKSF NRGEC  265 |
| SEQ ID NO: 149 durvalumab variable heavy chain | EVQLVESGGG LVQPGGSLRL SCAASGFTFS RYWMSWVRQA PGKGLEWVAN IKQDGSEKYY   60<br>VDSVKGRFTI SRDNAKNSLY LQMNSLRAED TAVYYCAREG GWFGELAFDY WGQGTLVTVS  120<br>S  121 |
| SEQ ID NO: 150 durvalumab variable light chain | EIVLTQSPGT LSLSPGERAT LSCRASQRVS SSYLAWYQQK PGQAPRLLIY DASSRATGIP   60<br>DRFSGSGSGT DFTLTISRLE PEDFAVYYCQ QYGSLPWTFG QGTKVEIK  108 |
| SEQ ID NO: 151 durvalumab heavy chain CDR1 | RYWMS                                           5 |
| SEQ ID NO: 152 durvalumab heavy chain CDR2 | NIKQDGSEKY YVDSVKG                          17 |
| SEQ ID NO: 153 durvalumab heavy chain CDR3 | EGGWFGELAF DY                               12 |
| SEQ ID NO: 154 durvalumab light chain CDR1 | RASQRVSSSY LA                               12 |

(continued)

| Identifier | Sequence (One-Letter Amino Acid Symbols) | |
|---|---|---|
| SEQ ID NO: 155 durvalumab light chain CDR2 | DASSRAT | 7 |
| SEQ ID NO: 156 durvalumab light chain CDR3 | QQYGSLPWT | 9 |

In an embodiment, the PD-L1 inhibitor is avelumab, also known as MSB0010718C (commercially available from Merck KGaA/EMD Serono), or antigen-binding fragments, conjugates, or variants thereof. The preparation and properties of avelumab are described in U.S. Patent Application Publication No. US 2014/0341917 A1. The amino acid sequences of avelumab are set forth in Table 19. Avelumab has intra-heavy chain disulfide linkages (C23-C104) at 22-96, 147-203, 264-324, 370-428, 22"-96", 147"-203", 264"-324", and 370"-428"; intra-light chain disulfide linkages (C23-C104) at 22'-90', 138'-197', 22'''-90''', and 138'''-197'''; intra-heavy-light chain disulfide linkages (h 5-CL 126) at 223-215' and 223"-215'''; intra-heavy-heavy chain disulfide linkages (h 11, h 14) at 229-229" and 232-232"; N-glycosylation sites (H CH$_2$ N84.4) at 300, 300"; fucosylated complex bi-antennary CHO-type glycans; and H CHS K2 C-terminal lysine clipping at 450 and 450'.

In an embodiment, a PD-L1 inhibitor comprises a heavy chain given by SEQ ID NO:157 and a light chain given by SEQ ID NO:158. In an embodiment, a PD-L1 inhibitor comprises heavy and light chains having the sequences shown in SEQ ID NO:157 and SEQ ID NO:158, respectively, or antigen binding fragments, Fab fragments, single-chain variable fragments (scFv), variants, or conjugates thereof. In an embodiment, a PD-L1 inhibitor comprises heavy and light chains that are each at least 99% identical to the sequences shown in SEQ ID NO:157 and SEQ ID NO:158, respectively. In an embodiment, a PD-L1 inhibitor comprises heavy and light chains that are each at least 98% identical to the sequences shown in SEQ ID NO:157 and SEQ ID NO:158, respectively. In an embodiment, a PD-L1 inhibitor comprises heavy and light chains that are each at least 97% identical to the sequences shown in SEQ ID NO:157 and SEQ ID NO:158, respectively. In an embodiment, a PD-L1 inhibitor comprises heavy and light chains that are each at least 96% identical to the sequences shown in SEQ ID NO: 157 and SEQ ID NO: 158, respectively. In an embodiment, a PD-L1 inhibitor comprises heavy and light chains that are each at least 95% identical to the sequences shown in SEQ ID NO: 157 and SEQ ID NO: 158, respectively.

In an embodiment, the PD-L1 inhibitor comprises the heavy and light chain CDRs or variable regions (VRs) of avelumab. In an embodiment, the PD-L1 inhibitor heavy chain variable region (V$_H$) comprises the sequence shown in SEQ ID NO: 159, and the PD-L1 inhibitor light chain variable region (V$_L$) comprises the sequence shown in SEQ ID NO: 160, and conservative amino acid substitutions thereof. In an embodiment, a PD-L1 inhibitor comprises V$_H$ and V$_L$ regions that are each at least 99% identical to the sequences shown in SEQ ID NO: 159 and SEQ ID NO: 160, respectively. In an embodiment, a PD-L1 inhibitor comprises V$_H$ and V$_L$ regions that are each at least 98% identical to the sequences shown in SEQ ID NO: 160 and SEQ ID NO: 160, respectively. In an embodiment, a PD-L1 inhibitor comprises V$_H$ and V$_L$ regions that are each at least 97% identical to the sequences shown in SEQ ID NO: 159 and SEQ ID NO: 160, respectively. In an embodiment, a PD-L1 inhibitor comprises V$_H$ and V$_L$ regions that are each at least 96% identical to the sequences shown in SEQ ID NO: 159 and SEQ ID NO: 160, respectively. In an embodiment, a PD-L1 inhibitor comprises V$_H$ and V$_L$ regions that are each at least 95% identical to the sequences shown in SEQ ID NO: 159 and SEQ ID NO: 160, respectively.

In an embodiment, a PD-L1 inhibitor comprises heavy chain CDR1, CDR2 and CDR3 domains having the sequences set forth in SEQ ID NO: 161, SEQ ID NO: 162, and SEQ ID NO: 163, respectively, and conservative amino acid substitutions thereof, and light chain CDR1, CDR2 and CDR3 domains having the sequences set forth in SEQ ID NO: 164, SEQ ID NO: 165, and SEQ ID NO: 166, respectively, and conservative amino acid substitutions thereof. In an embodiment, the antibody competes for binding with, and/or binds to the same epitope on PD-L1 as any of the aforementioned antibodies.

In an embodiment, the PD-L1 inhibitor is an anti-PD-L1 biosimilar monoclonal antibody approved by drug regulatory authorities with reference to avelumab. In an embodiment, the biosimilar comprises an anti-PD-L1 antibody comprising an amino acid sequence which has at least 97% sequence identity, *e.g.*, 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is avelumab. In some embodiments, the one or more post-translational modifications are selected from one or more of: glycosylation, oxidation, deamidation, and truncation. In some embodiments, the biosimilar is an anti-PD-L1 antibody authorized or submitted for authorization, wherein the anti-PD-L1 antibody is provided in a formulation which differs from the formulations of a reference medicinal product or reference biological product, wherein the reference medicinal product or reference biological product is avelumab. The anti-PD-L1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is avelumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is avelumab.

TABLE 19. Amino acid sequences for PD-L1 inhibitors related to avelumab.

| Identifier | Sequence (One-Letter Amino Acid Symbols) | |
|---|---|---|
| SEQ ID NO:157 avelumab heavy chain | EVQLLESGGG LVQPGGSLRL SCAASGFTFS SYIMMWVRQA PGKGLEWVSS IYPSGGITFY | 60 |
| | ADTVKGRFTI SRDNSKNTLY LQMNSLRAED TAVYYCARIK LGTVTTVDYW GQGTLVTVSS | 120 |
| | ASTKGPSVFP LAPSSKSTSG GTAALGCLVK DYFPEPVTVS WNSGALTSGV HTFPAVLQSS | 180 |
| | GLYSLSSVVT VPSSSLGTQT YICNVNHKPS NTKVDKKVEP KSCDKTHTCP PCPAPELLGG | 240 |
| | PSVFLFPPKP KDTLMISRTP EVTCVVVDVS HEDPEVKFNW YVDGVEVHNA KTKPREEQYN | 300 |
| | STYRVVSVLT VLHQDWLNGK EYKCKVSNKA LPAPIEKTIS KAKGQPREPQ VYTLPPSRDE | 360 |
| | LTKNQVSLTC LVKGFYPSDI AVEWESNGQP ENNYKTTPPV LDSDGSFFLY SKLTVDKSRW | 420 |
| | QQGNVFSCSV MHEALHNHYT QKSLSLSPGK | 450 |
| SEQ ID NO:158 avelumab light chain | QSALTQPASV SGSPGQSITI SCTGTSSDVG GYNYVSWYQQ HPGKAPKLMI YDVSNRPSGV | 60 |
| | SNRFSGSKSG NTASLTISGL QAEDEADYYC SSYTSSSTRV FGTGTKVTVL GQPKANPTVT | 120 |
| | LFPPSSEELQ ANKATLVCLI SDFYPGAVTV AWKADGSPVK AGVETTKPSK QSNNKYAASS | 180 |
| | YLSLTPEQWK SHRSYSCQVT HEGSTVEKTV APTECS | 216 |
| SEQ ID NO:159 avelumab variable heavy chain | EVQLLESGGG LVQPGGSLRL SCAASGFTFS SYIMMWVRQA PGKGLEWVSS IYPSGGITFY | 60 |
| | ADTVKGRFTI SRDNSKNTLY LQMNSLRAED TAVYYCARIK LGTVTTVDYW GQGTLVTVSS | 120 |
| SEQ ID NO:160 avelumab variable light chain | QSALTQPASV SGSPGQSITI SCTGTSSDVG GYNYVSWYQQ HPGKAPKLMI YDVSNRPSGV | 60 |
| | SNRFSGSKSG NTASLTISGL QAEDEADYYC SSYTSSSTRV FGTGTKVTVL | 110 |
| SEQ ID NO:161 avelumab heavy chain CDR1 | SYIMM | |
| SEQ ID NO:162 avelumab heavy chain CDR2 | SIYPSGGITF YADTVKG | |
| SEQ ID NO:163 avelumab heavy chain CDR3 | IKLGTVTTVD Y | |
| SEQ ID NO:164 avelumab light chain CDR1 | TGTSSDVGGY NYVS | |
| SEQ ID NO:165 avelumab light chain CDR2 | DVSNRPS | |
| SEQ ID NO:166 avelumab light chain CDR3 | SSYTSSSTRV | |

In an embodiment, the PD-L1 inhibitor is atezolizumab, also known as MPDL3280A or RG7446 (commercially available as TECENTRIQ from Genentech, Inc., a subsidiary of Roche Holding AG, Basel, Switzerland), or antigen-binding fragments, conjugates, or variants thereof. In an embodiment, the PD-L1 inhibitor is an antibody disclosed in U.S. Patent No. 8,217,149. In an embodiment, the PD-L1 inhibitor is an antibody disclosed in U.S. Patent Application Publication Nos. 2010/0203056 A1, 2013/0045200 A1, 2013/0045201 A1, 2013/0045202 A1, or 2014/0065135 A1. The preparation and properties of atezolizumab are described in U.S. Patent No. 8,217,149. The amino acid sequences of atezolizumab are set forth in Table 20. Atezolizumab has intra-heavy chain disulfide linkages (C23-C104) at 22-96, 145-201, 262-322, 368-426, 22"-96", 145"-201", 262"-322", and 368"-426"; intra-light chain disulfide linkages (C23-C104) at 23'-88', 134'-194', 23'''-88''', and 134'''-194'''; intra-heavy-light chain disulfide linkages (h 5-CL 126) at 221-214' and 221"-214'''; intra-heavy-heavy chain disulfide linkages (h 11, h 14) at 227-227" and 230-230"; and N-glycosylation sites (H CH$_2$ N84.4>A) at 298 and 298'.

In an embodiment, a PD-L1 inhibitor comprises a heavy chain given by SEQ ID NO:167 and a light chain given by SEQ ID NO:168. In an embodiment, a PD-L1 inhibitor comprises heavy and light chains having the sequences shown in SEQ ID NO:167 and SEQ ID NO:168, respectively, or antigen binding fragments, Fab fragments, single-chain variable fragments (scFv), variants, or conjugates thereof. In an embodiment, a PD-L1 inhibitor comprises heavy and light chains that are each at least 99% identical to the sequences shown in SEQ ID NO: 167 and SEQ ID NO: 168, respectively. In an embodiment, a PD-L1 inhibitor comprises heavy and light chains that are each at least 98% identical to the sequences shown in SEQ ID NO: 167 and SEQ ID NO: 168, respectively. In an embodiment, a PD-L1 inhibitor comprises heavy and light chains that are each at least 97% identical to the sequences shown in SEQ ID NO: 167 and SEQ ID NO: 168, respectively. In an embodiment, a PD-L1 inhibitor comprises heavy and light chains that are each at least 96% identical to the sequences shown in SEQ ID NO: 167 and SEQ ID NO: 168, respectively. In an embodiment, a PD-L1 inhibitor comprises heavy and light chains that are each at least 95% identical to the sequences shown in SEQ ID NO: 167 and SEQ ID NO: 168, respectively.

In an embodiment, the PD-L1 inhibitor comprises the heavy and light chain CDRs or variable regions (VRs) of atezolizumab. In an embodiment, the PD-L1 inhibitor heavy chain variable region (V$_H$) comprises the sequence shown in SEQ ID NO: 169, and the PD-L1 inhibitor light chain variable region (V$_L$) comprises the sequence shown in SEQ ID NO: 170, and conservative amino acid substitutions thereof. In an embodiment, a PD-L1 inhibitor comprises V$_H$ and V$_L$ regions that are each at least 99% identical to the sequences shown in SEQ ID NO: 169 and SEQ ID NO: 170, respectively. In an embodiment, a PD-L1 inhibitor comprises V$_H$ and V$_L$ regions that are each at least 98% identical to the sequences shown in SEQ ID NO: 169 and SEQ ID NO: 170, respectively. In an embodiment, a PD-L1 inhibitor comprises V$_H$ and V$_L$ regions that are each at least 97% identical to the sequences shown in SEQ ID NO: 169 and SEQ ID NO: 170, respectively. In an embodiment, a PD-L1 inhibitor comprises V$_H$ and V$_L$ regions that are each at least 96% identical to the sequences shown in SEQ ID NO: 169 and SEQ ID NO: 170, respectively. In an embodiment, a PD-L1 inhibitor comprises V$_H$ and V$_L$ regions that are each at least 95% identical to the sequences shown in SEQ ID NO: 169 and SEQ ID NO: 170, respectively.

In an embodiment, a PD-L1 inhibitor comprises heavy chain CDR1, CDR2 and CDR3 domains having the sequences set forth in SEQ ID NO: 171, SEQ ID NO: 172, and SEQ ID NO: 173, respectively, and conservative amino acid substitutions thereof, and light chain CDR1, CDR2 and CDR3 domains having the sequences set forth in SEQ ID NO: 174, SEQ ID NO: 175, and SEQ ID NO: 176, respectively, and conservative amino acid substitutions thereof. In an embodiment, the antibody competes for binding with, and/or binds to the same epitope on PD-L1 as any of the aforementioned antibodies.

In an embodiment, the anti-PD-L1 antibody is an anti-PD-L1 biosimilar monoclonal antibody approved by drug regulatory authorities with reference to atezolizumab. In an embodiment, the biosimilar comprises an anti-PD-L1 antibody comprising an amino acid sequence which has at least 97% sequence identity, e.g., 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is atezolizumab. In some embodiments, the one or more post-translational modifications are selected from one or more of: glycosylation, oxidation, deamidation, and truncation. In some embodiments, the biosimilar is an anti-PD-L1 antibody authorized or submitted for authorization, wherein the anti-PD-L1 antibody is provided in a formulation which differs from the formulations of a reference medicinal product or reference biological product, wherein the reference medicinal product or reference biological product is atezolizumab. The anti-PD-L1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is atezolizumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is atezolizumab.

TABLE 20. Amino acid sequences for PD-L1 inhibitors related to atezolizumab.

| Identifier | Sequence (One-Letter Amino Acid Symbols) | |
|---|---|---|
| SEQ ID NO:167 atezolizumab heavy chain | EVQLVESGGG LVQPGGSLRL SCAASGFTFS DSWIHWVRQA PGKGLEWVAW ISPYGGSTYY | 60 |
| | ADSVKGRFTI SADTSKNTAY LQMNSLRAED TAVYYCARRH WPGGFDYWGQ GTLVTVSSAS | 120 |
| | TKGPSVFPLA PSSKSTSGGT AALGCLVKDY FPEPVTVSWN SGALTSGVHT FPAVLQSSGL | 180 |
| | YSLSSVVTVP SSSLGTQTYI CNVNHKPSNT KVDKKVEPKS CDKTHTCPPC PAPELLGGPS | 240 |
| | VFLFPPKPKD TLMISRTPEV TCVVVDVSHE DPEVKFNWYV DGVEVHNAKT KPREEQYAST | 300 |
| | YRVVSVLTVL HQDWLNGKEY KCKVSNKALP APIEKTISKA KGQPREPQVY TLPPSREEMT | 360 |
| | KNQVSLTCLV KGFYPSDIAV EWESNGQPEN NYKTTPPVLD SDGSFFLYSK LTVDKSRWQQ | 420 |
| | GNVFSCSVMH EALHNHYTQK SLSLSPGK | 448 |
| SEQ ID NO:168 atezolizumab light chain | DIQMTQSPSS LSASVGDRVT ITCRASQDVS TAVAWYQQKP GKAPKLLIYS ASFLYSGVPS | 60 |
| | RFSGSGSGTD FTLTISSLQP EDFATYYCQQ YLYHPATFGQ GTKVEIKRTV AAPSVFIFPP | 120 |
| | SDEQLKSGTA SVVCLLNNFY PREAKVQWKV DNALQSGNSQ ESVTEQDSKD STYSLSSTLT | 180 |
| | LSKADYEKHK VYACEVTHQG LSSPVTKSFN RGEC | 214 |
| SEQ ID NO:169 atezolizumab variable heavy chain | EVQLVESGGG LVQPGGSLRL SCAASGFTFS DSWIHWVRQA PGKGLEWVAW ISPYGGSTYY | 60 |
| | ADSVKGRFTI SADTSKNTAY LQMNSLRAED TAVYYCARRH WPGGFDYWGQ GTLVTVSA | 118 |
| SEQ ID NO:170 atezolizumab variable light chain | DIQMTQSPSS LSASVGDRVT ITCRASQDVS TAVAWYQQKP GKAPKLLIYS ASFLYSGVPS | 60 |
| | RFSGSGSGTD FTLTISSLQP EDFATYYCQQ YLYHPATFGQ GTKVEIKR | 108 |
| SEQ ID NO:171 atezolizumab heavy chain CDR1 | GFTFSDSWIH | |
| SEQ ID NO:172 atezolizumab heavy chain CDR2 | AWISPYGGST YYADSVKG | |
| SEQ ID NO:173 atezolizumab heavy chain CDR3 | RHWPGGFDY | |
| SEQ ID NO:174 atezolizumab light chain CDR1 | RASQDVSTAV A | |
| SEQ ID NO:175 atezolizumab light chain CDR2 | SASFLYS | |
| SEQ ID NO:176 atezolizumab light chain CDR3 | QQYLYHPAT | |

EXAMPLES

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

Example 1. Preparation of Media for Pre-REP and REP Processes.

This Example describes the procedure for the preparation of tissue culture media for use in protocols involving the culture of tumor infiltrating lymphocytes (TIL) derived from various tumor types including, but not limited to, metastatic melanoma, head and neck squamous cell carcinoma (HNSCC), ovarian carcinoma, triple-negative breast carcinoma, and lung adenocarcinoma. This media can be used for preparation of any of the TILs described in the present application and Examples.

*Preparation of CM1*

Removed the following reagents from cold storage and warmed them in a 37°C water bath: (RPMI1640, Human AB serum, 200 mM L-glutamine). Prepared CM1 medium according to Table 21 below by adding each of the ingredients into the top section of a 0.2 μm filter unit appropriate to the volume to be filtered. Store at 4°C.

TABLE 21. Preparation of CM1

| Ingredient | Final Concentration | Final Volume 500 ml | Final Volume 1L |
|---|---|---|---|
| RPMI1640 | NA | 450 mL | 900 mL |
| Human AB serum, heat-inactivated 10% | 50 mL | 100 mL | |
| 200mM L-glutamine | 2 mM | 5 mL | 10 mL |
| 55mM BME | 55 μM | 0.5 mL | 1 mL |
| 50mg/ml gentamicin sulfate | 50 μg/ml | 0.5 mL | 1 mL |

On the day of use, prewarmed required amount of CM1 in 37°C water bath and add 6000 IU/mL IL-2.
Additional supplementation as needed according to Table 22.

TABLE 22. Additional Supplementation of CM1, as needed.

| Supplement | Stock concentration | Dilution | Final concentration |
|---|---|---|---|
| GlutaMAX™ | 200 mM | 1:100 | 2 mM |
| Penicillin/streptomycin | 10,000 U/mL penicillin 10,000 μg/mL streptomycin | 1:100 | 100 U/mL penicillin 100 μg/mL streptomycin |
| Amphotericin B | 250 μg/mL | 1:100 | 2.5μg/mL |

*Preparation of CM2*

Removed prepared CM1 from refrigerator or prepare fresh CM1 as above. Removed AIM-V® from refrigerator and prepared the amount of CM2 needed by mixing prepared CM1 with an equal volume of AIM-V® in a sterile media bottle. Added 3000 IU/mL IL-2 to CM2 medium on the day of usage. Made sufficient amount of CM2 with 3000 IU/mL IL-2 on the day of usage. Labeled the CM2 media bottle with its name, the initials of the preparer, the date it was filtered/prepared, the two-week expiration date and store at 4°C until needed for tissue culture.

*Preparation of CM3*

Prepared CM3 on the day it was required for use. CM3 was the same as AIM-V® medium, supplemented with 3000 IU/mL IL-2 on the day of use. Prepared an amount of CM3 sufficient to experimental needs by adding IL-2 stock solution directly to the bottle or bag of AIM-V. Mixed well by gentle shaking. Label bottle with "3000 IU/mL IL-2" immediately after adding to the AIM-V. If there was excess CM3, stored it in bottles at 4°C labeled with the media name, the initials of the preparer, the date the media was prepared, and its expiration date (7 days after preparation). Discarded media supplemented with IL-2 after 7 days of storage at 4°C.

*Preparation of CM4*

CM4 was the same as CM3, with the additional supplement of 2 mM GlutaMAX™ (final concentration). For every 1L of CM3, added 10ml of 200 mM GlutaMAX™. Prepared an amount of CM4 sufficient to experimental needs by adding IL-2 stock solution and GlutaMAX™ stock solution directly to the bottle or bag of AIM-V. Mixed well by gentle shaking. Labeled bottle with "3000 IL/mL IL-2 and GlutaMAX" immediately after adding to the AIM-V. If there was excess CM4, stored it in bottles at 4°C labeled with the media name, "GlutaMAX", and its expiration date (7 days after preparation). Discarded media supplemented with IL-2 after 7-days of storage at 4°C.

Example 2. Use of IL-2, IL-15, and IL-21 Cytokine Cocktail.

This example describes the use of IL-2, IL-15, and IL-21 cytokines, which serve as additional T cell growth factors, in combination with the TIL process of any of the examples or embodiments herein, including examples and embodiments that encompass cryopreservation of tumor tissue or tumor fragments.

Using the processes described herein, TILs were grown from colorectal, melanoma, cervical, triple negative breast, lung and renal tumors in presence of IL-2 in one arm of the experiment and, in place of IL-2, a combination of IL-2, IL-15, and IL-21 in another arm at the initiation of culture. At the completion of the pre-REP, cultures were assessed for expansion, phenotype, function (CD107a$^+$ and IFN-$\gamma$) and TCR V$\beta$ repertoire. IL-15 and IL-21 are described elsewhere herein and in Gruijl, *et al.,* IL-21 promotes the expansion of CD27$^+$CD28$^+$ tumor infiltrating lymphocytes with high cytotoxic potential and low collateral expansion of regulatory T cells, Santegoets, S. J., J. Transl Med., 11:37 (2013) available at https://www.ncbi.nlm.nih.gov/pmc/articles/PMC3626797/.

The results showed that enhanced TIL expansion (>20%), in both CD4$^+$ and CD8$^+$ cells in the IL-2, IL-15, and IL-21 treated conditions were observed in multiple histologies relative to the IL-2 only conditions. There was a skewing towards a predominantly CD8$^+$ population with a skewed TCR V$\beta$ repertoire in the TILs obtained from the IL-2, IL-15, and IL-21 treated cultures relative to the IL-2 only cultures. IFN-y and CD107A were elevated in the IL-2, IL-15, and IL-21 treated TILs, in comparison to TILs treated only IL-2.

Example 3. Preparation of IL-2 Stock Solution.

This Example describes the process of dissolving purified, lyophilized recombinant human interleukin-2 into stock samples suitable for use in further tissue culture protocols, including all of those described in the present application and Examples, including those that involve using rhIL-2.

*Procedure*

Prepared 0.2% Acetic Acid solution (HAc). Transferred 29 mL sterile water to a 50 mL conical tube. Added 1 mL 1N (1 normal) acetic acid to the 50 mL conical tube. Mixed well by inverting tube 2-3 times. Sterilized the HAc solution by filtration using a Steriflip filter

Prepare 1% HSA in PBS. Added 4 mL of 25% HSA stock solution to 96 mL PBS in a 150 mL sterile filter unit. Filtered solution. Stored at 4°C. For each vial of rhIL-2 prepared, fill out forms.

Prepared rhIL-2 stock solution (6 × 10$^6$ IU/mL final concentration). Each lot of rhIL-2 was different and required information found in the manufacturer's Certificate of Analysis (COA), such as: 1) Mass of rhIL-2 per vial (mg), 2) Specific activity of rhIL-2 (IU/mg) and 3) Recommended 0.2% HAc reconstitution volume (mL).

Calculated the volume of 1% HSA required for rhIL-2 lot by using the equation below:

$$\left( \frac{Vial\ Mass\ (mg)\ x\ Biological\ Activity\ \left(\frac{IU}{mg}\right)}{6x10^6\ \frac{IU}{mL}} \right) - HAc\ vol\ (mL) = 1\%\ HSA\ vol\ (mL)$$

For example, according to CellGenix's rhIL-2 lot 10200121 COA, the specific activity for the 1 mg vial is 25 × 10$^6$ IU/mg. It recommends reconstituting the rhIL-2 in 2 mL 0.2% HAc.

$$\left( \frac{1mg\ x\ 25x10^6\ \frac{IU}{mg}}{6x10^6\ \frac{IU}{mL}} \right) - 2mL = 2.167mL\ HSA$$

Wiped rubber stopper of IL-2 vial with alcohol wipe. Using a 16G needle attached to a 3 mL syringe, injected recommended volume of 0.2% HAc into vial. Took care to not dislodge the stopper as the needle is withdrawn. Inverted vial 3 times and swirled until all powder is dissolved. Carefully removed the stopper and set aside on an alcohol wipe. Added the calculated volume of 1% HSA to the vial.

Storage of rhIL-2 solution. For short-term storage (<72 hrs), stored vial at 4°C. For long-term storage (>72 hrs), aliquoted vial into smaller volumes and stored in cryovials at -20°C until ready to use. Avoided freeze/thaw cycles.

Expired 6 months after date of preparation. Rh-IL-2 labels included vendor and catalog number, lot number, expiration date, operator initials, concentration and volume of aliquot.

Example 4. TIL Cryopreservation Processes.

This example describes the cryopreservation process method for TILs prepared with the abbreviated, closed procedure described in Example 5 using the CryoMed Controlled Rate Freezer, Model 7454 (Thermo Scientific).

The equipment used was as follows: aluminum cassette holder rack (compatible with CS750 freezer bags), cryostorage cassettes for 750 mL bags, low pressure (22 psi) liquid nitrogen tank, refrigerator, thermocouple sensor (ribbon type for bags), and CryoStore CS750 Freezing bags (OriGen Scientific).

The freezing process provides for a 0.5 °C rate from nucleation to -20 °C and 1 °C per minute cooling rate to -80 °C end temperature. The program parameters are as follows: Step 1 - wait at 4 °C; Step 2: 1.0 °C/min (sample temperature) to -4 °C; Step 3: 20.0 °C/min (chamber temperature) to -45 °C; Step 4: 10.0 °C/min (chamber temperature) to -10.0 °C; Step 5: 0.5 °C/min (chamber temperature) to -20 °C; and Step 6: 1.0 °C/min (sample temperature) to -80 °C.

Example 5. Production of a Cryopreserved TIL Cell Therapy Using a Closed System.

This example describes the procedure for cGMP manufacture of TIL cell therapy process in gas permeable containers, such as G-Rex Flasks (Wilson Wolf Manufacturing Corp., New Brighton, MN, USA), according to current Good Tissue Practices and current Good Manufacturing Practices. This material will be manufactured under US FDA Good Manufacturing Practices Regulations (21 CFR Part 210, 211, 1270, and 1271), and applicable ICH Q7 standards for Phase I through Commercial Material. This method may be combined with the tumor cryopreservation and thawing processes described herein.

The process summary is provided in Table 23 below.

TABLE 23. Process Summary.

| Estimated Day (post-seed) | Activity | Target Criteria | Anticipated Vessels | Estimated Total Volume (mL) |
|---|---|---|---|---|
| 0 | Tumor Dissection | ≤ 50 desirable tumor fragments per G-Rex100MCS | G-Rex100MCS 1 flask | ≤1000 |
| 11 | REP Seed | 5 - 200 x $10^6$ viable cells per G-Rex500MCS | G-Rex500MCS 1 flasks | ≤5000 |
| 16 | REP Split | 1 x $10^9$ viable cells per G-Rex500MCS | G-Rex500MCS ≤5 flasks | ≤25000 |
| 22 | Harvest | Total available cells | 3-4 CS-750 bags | ≤530 |

Throughout this Example, it is assumed that 1.0 mL/L = 1.0 g/kg, unless otherwise specified. Once opened, the following expiries apply at 2°C - 8°C: Human Serum, type AB (HI) Gemini, 1 month; 2-mercaptoethanol, 1 month. Gentamicin Sulfate, 50 mg/mL stock may be kept at room temperature for 1 month. Bags containing 10L of AIM-V media may be warmed at room temperature once only for up to 24 hours prior to use. During the Day 22 harvest two Gatherex™ pumps may be used to harvest the TIL from the G-Rex500MCS flasks.

*Day 0 - CM1 Media Preparation*

Prepare RPMI 1640 Media. In the BSC, using an appropriately sized pipette, remove 100.0 mL from 1000 mL RPMI 1640 Media and place into an appropriately sized container labeled "Waste."

In the BSC, add reagents to RPMI 1640 Media bottle. Add the following reagents to the RPMI 1640 Media bottle as shown in Table 21. Record volumes added. Amount added per bottle: Heat Inactivated Human AB Serum (100.0 mL); GlutaMax (10.0 mL); Gentamicin sulfate, 50 mg/mL (1.0 mL); 2-mercaptoethanol (1.0 mL)

Cap RPMI 1640 Media bottle and swirl bottle to ensure reagents were mixed thoroughly. Filter RPMI 1640 Media through 1L 0.22-micron filter unit. Label filtered media. Aseptically cap the filtered media and label.

111

Thaw one 1.1 mL IL-2 aliquot ($6 \times 10^6$ IU/mL) (BR71424) until all ice had melted. Transfer IL-2 stock solution to media. In the BSC, transfer 1.0 mL of IL-2 stock solution to the CM1 Day 0 Media Bottle prepared above. Add CM1 Day 0 Media 1 bottle and IL-2 ($6 \times 10^6$ IU/mL) 1.0 mL. Cap and swirl the bottle to mix media containing IL-2. Relabeled as "Complete CM1 Day 0 Media".

Remove 20.0 mL of media using an appropriately sized pipette and dispensed into a 50mL conical tube. In BSC, transfer 25.0 mL of "Complete CM1 Day 0 Media" to a 50 mL conical tube. Label the tube as "Tissue Pieces". Aseptically pass G-Rex100MCS (W3013130) into the BSC. In the BSC, closed all clamps on the G-Rex100MCS, leaving vent filter clamp open. Connect the red line of G-Rex100MCS flask to the larger diameter end of the repeater pump fluid transfer set (W3009497) via luer connection. Stage Baxa pump next to BSC. Remove pump tubing section of repeater pump fluid transfer set from BSC and install in repeater pump. Within the BSC, remove the syringe from Pumpmatic Liquid-Dispensing System (PLDS) (W3012720) and discard.

Connect PLDS pipette to the smaller diameter end of repeater pump fluid transfer set via luer connection and placed pipette tip in "Complete CM1 Day 0 Media" for aspiration. Open all clamps between media and G-Rex100MCS. Pump Complete CM1 media into G-Rex100MCS flask. Set the pump speed to "High" and "9" and pumped all Complete CM1 Day 0 Media into G-Rex100MCS flask. Once all media is transferred, clear the line and stop pump.

Disconnect pump from flask. Ensure all clamps were closed on the flask, except vent filter. Remove the repeater pump fluid transfer set from the red media line, and place a red cap (W3012845) on the red media line. Remove G-Rex100MCS flask from BSC, heat seal off the red cap from the red line near the terminal luer. Incubator parameters: $37.0 \pm 2.0$ °C; $CO_2$ Percentage: $5.0 \pm 1.5$ %$CO_2$.

Place the 50mL conical tube" in incubator for $\geq$ 30 minutes of warming.

*Day 0 - Tumor Wash Media Preparation*

Add gentamicin to HBSS. In the BSC, add 5.0 mL gentamicin (W3009832 or W3012735) to $1 \times 500$ mL HBSS Media (W3013128) bottle. Record volumes. Add per bottle: HBSS (500.0 mL); Gentamicin sulfate, 50 mg/ml (5.0 mL). Mix reagents thoroughly. Filter HBSS containing gentamicin through a 1L 0.22-micron (0.22 $\mu$m) filter unit (W1218810). Aseptically cap the filtered media and labeled with the following information.

*Day 0 - Tumor Processing*

Obtain tumor specimen (from cryopreserved tumor) and transfer into suite at 2°C - 8°C immediately for processing and recorded tumor information.

Wash tumor specimens three times at 2°C - 8°C, with gentle agitation for at least 3 minutes for each wash. The wash solution is removed and replaced with fresh solution after each wash.

Using a transfer pipette, place 4 individual drops of Tumor Wash Media from the conical into each of the 6 circles on the upturned lids of the 6-well plates (2 lids). Place an extra drop on two circles for a total of 50 drops.

Tumor Wash 3: Using forceps, remove the tumor from the "Wash 2" dish and transferred to the "Wash 3" dish. Using forceps, wash tumor specimen by gently agitating and allow it to sit for $\geq$ 3 minutes. Record time.

Place a ruler under 150 mm dish lid. Using forceps, aseptically transferred tumor specimen to the 150 mm dissection dish lid. Arrange all pieces of tumor specimen end to end and recorded the approximate overall length and number of fragments. Assess the tumor for necrotic/fatty tissue. Assess whether > 30% of entire tumor area observed to be necrotic and/or fatty tissue; if yes, ensure tumor was of appropriate size if so proceed. Assess whether < 30% of entire tumor area were observed to be necrotic or fatty tissue; if yes, proceed.

Clean-Up Dissection. If tumor was large and >30% of tissue exterior was observed to be necrotic/fatty, performed "clean up dissection" by removing necrotic/fatty tissue while preserving tumor inner structure using a combination of scalpel and/or forceps. To maintain tumor internal structure, use only vertical cutting pressure. Did not cut in a sawing motion with scalpel.

Using a combination of scalpel and/or forceps, cut the tumor specimen into even, appropriately sized fragments (up to 6 intermediate fragments). To maintain tumor internal structure, use only vertical cutting pressure. Again, do not cut in a sawing motion with scalpel. Keep non-dissected intermediate fragments completely submerged in "Tumor Wash Media". Transfer each intermediate fragment to the "holding" dish.

Manipulate one intermediate fragment at a time, dissected the tumor intermediate fragment in the dissection dish into pieces approximately $3 \times 3 \times 3$ mm in size, minimizing the amount of hemorrhagic, necrotic, and/or fatty tissues on each piece. To maintain tumor internal structure, use only vertical cutting pressure. Again, do not cut in a sawing motion with scalpel.

Select up to eight (8) tumor pieces without hemorrhagic, necrotic, and/or fatty tissue. Used the ruler for reference. Continue dissection until 8 favorable pieces have been obtained, or the entire intermediate fragment has been dissected. Transfer each selected piece to one of the drops of "Tumor Wash Media".

After selecting up to eight (8) pieces from the intermediate fragment, place remnants of intermediate fragment into a new single well of "favorable intermediate fragments" 6-well plate.

If desirable tissue remains, selected additional Favorable Tumor Pieces from the "favorable intermediate fragments" 6-well plate to fill the drops for a maximum of 50 pieces. Recorded the total number of dissected pieces created.

Remove the "Tissue Pieces" 50 mL conical tube from the incubator. Ensured conical tube was warmed for ≥30 min. Pass "Tissue Pieces" 50 mL conical into the BSC, ensuring not to compromise the sterility of open processing surfaces.

Using a transfer pipette, scalpel, forceps or combination, transfer the selected 50 best tumor fragments from favorable dish lids to the "Tissue Pieces" 50 mL conical tube. If a tumor piece was dropped during transfer and desirable tissue remains, additional pieces from the favorable tumor intermediate fragment wells were added. Recorded numbers of pieces.

Remove G-Rex100MCS containing media from incubator. Aseptically passed G-Rex100MCS flask into the BSC. When transferring the flask, do not hold from the lid or the bottom of the vessel. Transfer the vessel by handling the sides. In the BSC, lift G-Rex100MCS flask cap, ensuring that sterility of internal tubing was maintained. Swirl conical tube with tumor pieces to suspend and quickly pour the contents into the G-Rex100MCS flask. Ensure that the tumor pieces are evenly distributed across the membrane of the flask. Gently tilted the flask back and forth if necessary to evenly distribute the tumor pieces. Record number of tumor fragments on bottom membrane of vessel and number of observed to be floating in vessel. NOTE: If the number of fragments seeded were NOT equivalent to number of collected, contacted Area Management, and documented in Section 10.0.

Incubate G-Rex100MCS at the following parameters: Incubate G-Rex flask: Temperature LED Display: 37.0 ± 2.0 °C; $CO_2$ Percentage: 5.0 ± 1.5 % $CO_2$. Perform calculations to determine the proper time to remove G-Rex100MCS incubator on Day 11. Calculations: Time of incubation; lower limit = time of incubation + 252 hours; upper limit = time of incubation + 276 hours.

*Day 11 - Media Preparation*

Monitor Incubator. Incubator parameters: Temperature LED Display: 37.0 ± 2.0 °C; $CO_2$ Percentage: 5.0 ± 1.5 % $CO_2$. Warm 3×1000 mL RPMI 1640 Media (W3013112) bottles and 3×1000 mL AIM-V (W3009501) bottles in an incubator for ≥ 30 minutes. Recorded time. Media: RPMI 1640 and AIM-V. Placed an additional 1×1000 mL bottle of AIM-V Media (W3009501) at room temperature for further use.

Remove the RPMI 1640 Media when time was reached. Record end incubation time in previous step. Ensure media is warmed for ≥30 min. In the BSC, removed 100.0 mL from each of the three pre-warmed 1000 mL RPMI 1640 Media bottles and place into an appropriately sized container labeled "Waste". In the BSC, add the following reagents to each of the three RPMI 1640 Media bottles and recorded volumes added to each bottle. GemCell Human serum, Heat Inactivated Type AB (100.0 mL), GlutaMax (10.0 mL), Gentamicin sulfate, 50 μg/mL (1.0 mL), 2-mercaptoethanol (1.0 mL).

Cap bottles and swirl to ensure reagents were mixed thoroughly. Filtered each bottle of media through a separate 1L 0.22-micron filter unit. Aseptically cap the filtered media and label each bottle with CM1 Day 11 Media. Thawed 3 × 1.1 mL aliquots of IL-2 ($6 \times 10^6$ IU/mL) (BR71424) until all ice had melted Recorded IL 2 lot # and expiration date/time.

Remove the three bottles of AIM-V Media from the incubator. Record end incubation time. Ensure media had been warmed for ≥ 30 minutes. Using a micropipette, add 3.0mL of thawed IL-2 into one 1L bottle of pre-warmed AIM-V media. Rinse micropipette tip with media after dispensing IL-2. Use a new sterile micropipette tip for each aliquot. Record the total volume added. Label bottle as "AIM-V Containing IL-2". Aseptically transfer a 10L Labtainer Bag and a repeater pump transfer set into the BSC. Closed all lines on a 10L Labtainer bag. Attach the larger diameter tubing end of a repeater pump transfer set to the middle female port of the 10L Labtainer Bag via luer lock connection.

Stage the Baxa pump next to the BSC. Feed the transfer set tubing through the Baxa pump. Set the Baxa Pump to "High" and "9". Remove syringe from Pumpmatic Liquid-Dispensing System (PLDS) and discarded. Do not compromise the sterility of the PLDS pipette.

Connect the PLDS pipette to smaller diameter end of repeater pump fluid transfer set via luer connection and place pipette tip in AIM-V media containing IL-2 bottle for aspiration. Opened all clamps between media bottle and 10L Labtainer.

Using the PLDS, transfer pre-warmed AIM-V media containing IL-2 prepared, as well as two additional AIM-V bottles into the 10L Labtainer bag. Add the three bottles of filtered CM1 Day 11 Media. After addition of final bottle, clear the line to the bag. Stop the pump between addition of each bottle of media. Remove PLDS from the transfer set and place a red cap on the luer of the line in the BSC. Gently massage the bag to mix. Label the media bag with the following information. Expiration date is 24 hours from the preparation date.

Attach a 60 mL syringe to the available female port of the "Complete CM2 Day 11 Media" bag. Remove 20.0 mL of media and place in a 50 mL conical tube. Place a red cap on the female port of the "Complete CM2 Day 11 Media" Bag. Label and stored Media Retain Sample at 2°C-8°C until submitted for testing. Heat seal off the red cap on the transfer set line, close to red cap. Keep the transfer set on the bag.

In the BSC, add 4.5 mL of AIM-V Media that had been labelled with "For Cell Count Dilutions" and lot number to four 15mL conical tubes. Label the tubes with the lot number and tube number (1-4). Labeled 4 cryovials "Feeder" and vial number (1-4). Transfer any remaining 2-mercaptoethanol, GlutaMax, and human serum from the BSC to 2°C to 8°C.

Outside of the BSC, weld a 1L Transfer Pack to the transfer set attached to the "Complete CM2 Day 11 Media" bag prepared. Labeled transfer pack as "Feeder Cell CM2 Media" and lot number. Make a mark on the tubing of the 1L Transfer Pack tubing a few inches away from the bag. Place the empty Transfer Pack onto the scale so that the tubing was on the scale to the point of the mark. Tare the scale and leave the empty Transfer Pack on the scale.

Set the Baxa pump to "Medium" and "4." Pump 500.0 $\pm$ 5.0 mL of "Complete CM2 Day 11" media into Cell CM2 Media" transfer pack. Measure by weight and record the volume of Complete CM2 media added to the Transfer Pack.

Once filled, heat seal the line. Separate CM2 Day 11 media bag with transfer set from feeder cell media transfer pack, kept weld toward 1L transfer pack. Place "Complete CM2 Day 11 Media" prepared in incubator until use.

*Day 11 - TIL Harvest*

Incubator parameters: Temperature LED Display: 37.0 $\pm$ 2.0 °C; $CO_2$ Percentage: 5.0 $\pm$ 1.5 % $CO_2$. Performed check to ensure incubation parameters are met before removing G-Rex100MCS from incubator. Lower limits are the same as described above.

Record time of removal from incubator. Carefully remove G-Rex100MCS from incubator and ensured all clamps were closed except large filter line. Record processing start time.

Label a 300 mL Transfer pack as "TIL Suspension". Sterile weld the TIL Suspension transfer (single line) of a Gravity Blood Filter. Place the 300mL Transfer Pack on a scale and record dry weight. Labeled 1L Transfer Pack as "Supernatant".

Sterile weld the red media removal line from the G-Rex100MCS to the "Supernatant" transfer pack. Sterile weld the clear cell removal line from the G-Rex100MCS to one of the two spike lines on the top of the blood filter connected to the "TIL Suspension" transfer pack. Place G-Rex100MCS on the left side of the GatheRex and the "Supernatant" and "TIL Suspension" transfer packs to the right side.

Install the red media removal line from the G Rex100MCS to the top clamp (marked with a red line) and tubing guides on the GatheRex. Install the clear harvest line from the G-Rex100MCS to the bottom clamp (marked with a blue line) and tubing guides on the GatheRex. Attach the gas line from the GatheRex to the sterile filter of the G-Rex100MCS flask. Before removing the supernatant from the G-Rex100MCS flask, ensure all clamps on the cell removal lines were closed. Transfer ~900 mL of culture supernatant from the G-Rex100MCS to the 1L Transfer Pack. Visually inspect G-Rex100MCS flask to ensure flask is level and media has been reduced to the end of the aspirating dip tube.

After removal of the supernatant, close all clamps to the red line.

Vigorously tap flask and swirl media to release cells. Perform an inspection of the flask to ensure all cells have detached. Tilt flask away from collection tubing and allow tumor pieces to settle along edge. Slowly tip flask toward collection tubing so pieces remained on the opposite side of the flask. If the cell collection straw is not at the junction of the wall and bottom membrane, rap the flask while tilted at a 45° angle is usually sufficient to properly position the straw.

Release all clamps leading to the TIL Suspension transfer pack. Using the GatheRex, transfer the cell suspension through the blood filter into the 300 mL transfer pack. Maintain the tilted edge until all cells and media are collected. Inspect membrane for adherent cells. Rinse the bottom of the G-Rex100MCS. Cover ~1/4 of gas exchange membrane with rinse media. Ensure all clamps are closed. Heat seal the TIL suspension transfer pack as close to the weld as possible so that the overall tubing length remains approximately the same. Heat sealed the "Supernatant" transfer pack. Maintain enough line to weld. Record weight of TIL Suspension transfer pack and calculate the volume of cell suspension.

Weld a 4-inch (4") plasma transfer set to "supernatant" transfer pack, retaining the luer connection on the 4-inch plasma transfer set, and transfer into the BSC. Weld a 4-inch plasma transfer set to 300 mL "TIL Suspension" transfer pack, retain the luer connection on the 4-inch plasma transfer set, and transfer into the BSC.

Draw up approximately 20.0 mL of supernatant from the 1L "Supernatant" transfer pack and dispense into a sterile 50mL conical tube labeled "Bac-T." Remove a 1.0 mL sample from the 50 mL conical labeled BacT using an appropriately sized syringe and inoculate the anaerobic bottle.

Label 4 cryovials with vial number (1-4). Using separate 3mL syringes, pull 4×1.0mL cell count samples from TIL Suspension Transfer Pack using the luer connection, and place in respective cryovials. Placed a red cap (W3012845) on the line. Place TIL Transfer Pack in incubator until needed. Perform cell counts and calculations. Perform initial cell counts undiluted. If no dilution needed, "Sample [$\mu$L]" = 200, "Dilution [$\mu$L]" = 0.

Record cell counts and TIL numbers. If Total Viable TIL Cells is < 5 $\times$ 10$^6$ cells, proceeded to "Day 11 G-Rex Fill and Seed". If Total Viable TIL Cells is > 5 $\times$ 10$^6$, proceed to "Calculation for flow cytometry".

If the Total Viable TIL Cell count was $\geq 4.0 \times 10^7$, calculated the volume to obtain $1.0 \times 10^7$ cells for the flow cytometry sample. Total viable cells required for flow cytometry: $1.0 \times 10^7$ cells. Volume of cells required for flow cytometry: Viable cell concentration divided by $1.0 \times 10^7$ cells.

If Applicable: Recalculate Total Viable Cells and Volume flow. Calculate the remaining Total Viable Cells and remaining volume after the removal of cytometry sample below.

If applicable: Calculate volume for cryopreservation. Calculate the volume of cells required to obtain $1 \times 10^7$ cells for cryopreservation.

Table 24. TIL Cryopreservation Calculation.

| Total Viable TIL required for cryopreservation | Viable Cell Concentration | Volume of Cells required for cryopreservation $C-A \div B$ | | |
|---|---|---|---|---|
| **A. 1x10$^7$ cells** | **B.** | cells/mL | **C.** | mL |

If applicable: Remove sample for Cryopreservation. Remove the calculated volume from the TIL Suspension transfer pack. Place in appropriately sized conical tube and label as "Cryopreservation Sample $1 \times 10^7$ cells," dated, and lot number. Place a red cap (W3012845) on the TIL Suspension transfer pack.

Centrifuge the "Cryopreservation Sample $1 \times 10^7$ cells" according to the following parameters: Speed: $350 \times$ g, Time: 10:00 minutes, Temperature: Ambient, Brake: Full (9); Acceleration: Full (9).

Add CS-10. In BSC, aseptically aspirate supernatant. Gently tap bottom of tube to resuspend cells in remaining fluid. Added CS-10. Slowly added 0.5 mL of CS10. Record volume added. Cryopreservation Sample Vials Filled at ~0.5 mL.

*Day 11 - Feeder Cells*

Obtain 3 bags of feeder cells with at least two different lot numbers from LN$_2$ freezer. Keep cells on dry ice until ready to thaw. Record feeder cell information. Confirm that at least two different lots of feeder cells were obtained. Place the Feeder Cell bags into individual zip top bags, based on Lot number, and thawed $37.0 \pm 2.0°C$ water bath or cytotherm for ~3-5 minutes or until ice has just disappeared.

Feeder cell harness preparation. Weld 4S-4M60 to a CC2 Cell Connect (W3012820), replacing a single spike of the Cell Connect apparatus with the 4-spike end of the 4S-4M60 manifold. Weld as needed.

Attach media transfer pack: Weld the "Feeder Cell CM2 Media" transfer pack to a CC2 luer. The bag will be attached to the side of the harness with the needless injection port. Transferred the assembly containing the Complete CM2 Day 11 Media into the BSC.

Pool thawed feeder cells. Within the BSC, pull 10 mL of air into a 100 mL syringe. Used this to replace the 60 mL syringe on the CC2. Wipe each port on the feeder cell bags with an alcohol pad prior to removing the cover. Spike the three feeder bags using three of the spikes of the CC2. Maintain constant pressure while turning the spike in one direction. Do not puncture the side of the port. Opened the stopcock so that the line from the feeder cell bags is open and the line to the needless injection port is closed. Draw up the contents of the feeder cell bags into the syringe. All three bags drained at once. Once feeder cell bags had been drained, while maintaining pressure on the syringe, clamp off the line to the feeder cell bags. Do not detach syringe below. the syringe from the harness. Record the total volume of feeder cells in the syringe.

Add feeder cells to transfer pack. Turn the stopcock so the line to the feeder cell bag was closed and the line to the media Transfer Pack was open. Ensure the line to media transfer pack is unclamped. Dispense the feeder cells from the syringe into the "Feeder Cell CM2 Media" transfer pack. Clamp off the line to the transfer pack containing the feeder cells and leave the syringe attached to the harness. Massage bag to mix the pooled feeder cells in the transfer pack. Labeled bag as "Feeder Cell Suspension".

Calculated the total volume of feeder cell suspension. Remove cell count samples. Using a separate 3 mL syringe for each sample, pulled $4 \times 1.0$ mL cell count samples from Feeder Cell Suspension Transfer Pack using the needless injection port. Aliquot each sample into labeled cryovials.

Perform cell counts and calculations utilizing NC-200 and Process Note 5.14. Dilute cell count samples by adding 0.5 mL of cell suspension into 4.5 mL of AIM-V media labelled with the lot number and "For Cell Count Dilutions". This will give a 1:10 dilution.

Record Cell Count and Sample volumes. If Total Viable Cells are $< 5 \times 10^9$, proceed. If Total Viable Cells are $\geq 5 \times 10^9$, proceed as above for higher cells counts. Obtain additional feeder cells as needed and added to transfer pack as discussed above. Calculate the volume of feeder cell suspension that was required to obtain $5 \times 10^9$ viable

feeder cells. Calculate the volume of excess feeder cells to remove. Round down to nearest whole number.

Remove excess feeder cells. In a new 100 mL syringe, pull up 10mL of air and attached the syringe to the harness. Open the line to the "Feeder Cell Suspension" transfer pack. Using the syringe, draw up the volume of feeder cells calculated plus an additional 10.0 mL from the Transfer Pack into a 100 mL syringe. Close the line to the Feeder Cell Suspension transfer pack once the volume of feeder cells is removed. Do not remove final syringe. Once a syringe has been filled, replace it with a new syringe. Multiple syringes could be used to remove total volume. With each new syringe, pull in 10mL of air. Record the total volume (including the additional 10 mL) of feeder cells removed.

Added OKT3. In the BSC, using a 1.0 mL syringe and 16 gauge (16G or 16 Ga) needle, draw up 0.15 mL of OKT3. Aseptically remove the needle from the syringe and attach the syringe to the needless injection port. Injected the OKT3. Open the stopcock to the "Feeder Cell Suspension" transfer pack and add 10 mL of feeder cells removed previously to flush OKT3 through the line. Turn the syringe upside down and push air through to clear the line to the Feeder Cell Suspension transfer pack. Leave the remaining feeder cell suspension in the syringe. Close all clamps and remove the harness from the BSC. Heat seal the Feeder Cell Suspension transfer pack, leaving enough tubing to weld.

*Day 11 - G-Rex Fill and Seed*

Set up G-Rex500MCS. Remove a G-Rex500MCS from packaging and inspected the flask for any cracks or kinks in the tubing. Ensure all luer connections and closures were tight. Closed all clamps on the G-Rex500MCS lines except for the vent filter line. Using a marker draw a line at the 4.5L gradation. Removed the "Complete CM2 Day 11 Media", from the incubator.

Prepare to pump media. Weld the red line of the G-Rex500MCS to the repeater pump transfer set attached to the complete CM2 Day 11 Media. Hang the "Complete CM2 Day 11 Media" bag on an IV pole. Fed the pump tubing through the Baxa pump. Pump media into G-Rex500MCS. Set the Baxa pump to "High" and "9". Pump 4.5L of media into the G-Rex500MCS, filling to the line marked on the flask at the 4.5L gradation. Heat seal the red line of the G-Rex500MCS near the weld. Label the flask with the "Day 11" label. Weld the Feeder Cell: Suspension transfer pack to the flask. Sterile weld the red line of the G-Rex500MCS to the "Feeder Cell Suspension" transfer pack.

Add Feeder Cells to G-Rex500MCS. Open all clamps between Feeder Cell Suspension and G-Rex500MCS and added Feeder Cell Suspension to flask by gravity feed. Heat seal the red line near the weld. Welded the TIL Suspension transfer pack to the flask. Sterile weld the red line of the G-Rex500MCS to the "TIL Suspension" transfer pack.

Add TIL to G-Rex500MCS. Open all clamps between TIL Suspension and G-Rex500MCS and added TIL Suspension to flask by gravity feed. Heat seal the red line near the weld to remove the TIL suspension bag.

Incubate G-Rex500MCS. Check that all clamps on the G-Rex500MCS were closed except the large filter line and place in the incubator. Incubator parameters: Temperature LED Display: 37.0 $\pm$ 2.0 °C, $CO_2$ Percentage: 5.0 $\pm$ 1.5 %$CO_2$.

Calculate incubation window. Perform calculations to determine the proper time to remove G-Rex500MCS from incubator on Day 16. Lower limit: Time of incubation + 108 hours. Upper limit: Time of incubation + 132 hours.

*Day 11 - Excess TIL Cryopreservation*

Freeze Excess TIL Vials. Record and verify the total number of vials placed into the Control Rate Freezer (CRF). Upon completion of freeze, transfer vials from CRF to the appropriate storage container.

*Day 16 - Media Preparation*

Pre-warm AIM-V Media. Remove three CTS AIM V 10L media bags from 2°C to 8°C at least 12 hours prior to use and place at room temperature protected from light. Label each bag. Record warming start time and date. Ensure all bags have been warmed for a duration between 12 and 24 hours.

Attach the larger diameter end of a fluid pump transfer set to one of the female ports of a 10L Labtainer bag using the Luer connectors. Setup 10L Labtainer for Supernatant. Label as "Supernatant". Setup 10L Labtainer for Supernatant. Ensure all clamps were closed prior to removing from the BSC.

Thaw 5 $\times$ 1.1 mL aliquots of IL-2 ($6\times10^6$ IU/mL) (BR71424) per bag of CTS AIM V media until all ice had melted. Aliquot 100.0mL of Glutamax into an appropriately sized receiver. Record the volume added to each receiver and labeled each receiver as "GlutaMax."

Add IL-2 to GlutaMax. Using a micropipette, add 5.0 mL of IL-2 to each GlutaMax receiver. Rinse the tip per process and use a new pipette tip for each mL added. Record volume added to each Glutamax receiver and labeled each receiver as "GlutaMax + IL-2" and receiver number.

Prepare CTS AIM V media bag for formulation. Ensure CTS AIM V 10L media bag (W3012717) was warmed at room temperature and protected from light for 12-24 hours prior to use. Record end incubation time. In the BSC, close clamp on a 4" plasma transfer set, then connected to the bag using the spike ports. Maintain constant pressure while turning the spike in one direction. Ensure to not puncture the side of the port. Connect the larger diameter end of a repeater pump fluid transfer set to the 4" plasma transfer set via luer.

Stage Baxa pump next to BSC. Remove pump tubing section of repeater pump fluid transfer set from BSC and installed in repeater pump.

Prepare to formulate media. In BSC, remove syringe from Pumpmatic Liquid-Dispensing System (PLDS) and discarded. Ensure to not compromise the sterility of the PLDS pipette. Connect PLDS pipette to smaller diameter end of repeater pump fluid transfer set via luer connection and placed pipette tip in "GlutaMax + IL-2" prepared above for aspiration. Open all clamps between receiver and 10L bag.

Pump GlutaMax +IL-2 into bag. Set the pump speed to "Medium" and "3" and pump all "GlutaMax + IL-2" into 10L CTS AIM V media bag. Once no solution remains, clear line and stop pump. Record the volume of GlutaMax containing IL-2 added to each Aim V bag below.

Remove PLDS. Ensure all clamps are closed, and removed the PLDS pipette from the repeater pump fluid transfer set. Remove repeater pump fluid transfer set and red cap the 4" plasma transfer set.

Label each bag of "Complete CM4 Day 16 media" prepared.

Remove Media Retain per Sample Plan. Using a 30 mL syringe, remove 20.0 mL of "Complete CM4 Day 16 media" by attaching syringe to the 4" plasma transfer set and dispensed sample into a 50 mL conical tube. Ensure 4" plasma transfer set was either clamped or red capped after removal of syringe.

Attach new repeater pump fluid transfer set. Attach the larger diameter end of a new fluid pump transfer set onto the 4" plasma transfer set that was connected to the "Complete CM4 Day 16 media" bag. Label with sample plan inventory label and stored media retain sample at 2-8°C until submitted for testing.

Monitored Incubator. If applicable, monitor for additional bags prepared. Incubator parameters: Temperature LED Display: 37.0 $\pm$ 2.0 °C, $CO_2$ Percentage: 5.0 $\pm$ 1.5 %$CO_2$.

Warmed Complete CM4 Day 16 Media. Warm the first bag of Complete CM4 Day 16 Media in incubator for $\geq$ 30 minutes until ready for use. If applicable, warm additional bags.

Prepared Dilutions. In the BSC, added 4.5 mL of AIM-V Media that had been labelled with "For Cell Count Dilutions" to each 4 each, 15 mL conical tube. Label the conical tubes. Labeled 4 cryovials.

*Day 16 - REP Spilt*

Monitor Incubator. Incubator parameters: Temperature LED Display: 37.0 $\pm$ 2.0 °C, $CO_2$ Percentage: 5.0 $\pm$ 1.5 %$CO_2$.

Remove G-Rex500MCS from Incubator. Perform check below to ensure incubation parameters are met before removing G-Rex500MCS from incubator: upper limit, lower limit, time of removal. Remove G-Rex500MCS from the incubator.

Heat seal a 1L transfer pack (W3006645), leaving ~12 inches of line. Label 1L transfer pack as TIL Suspension. Place 1L transfer pack, including the entire line, on a scale and record dry weight.

GatheRex Setup. Sterile weld the red media removal line from the G-Rex500MCS to the repeater pump transfer set on the 10L labtainer bag "Supernatant" prepared above. Sterile weld the clear cell removal line from the G-Rex500MCS to the TIL Suspension transfer pack prepared above. Place G-Rex500MCS flask on the left side of the GatheRex. Place the supernatant labtainer bag and TIL suspension transfer pack to the right side. Install the red media removal line from the G-Rex500MCS to the top clamp (marked with a red line) and tubing guides on the GatheRex. Install the clear harvest line from the G-Rex500MCS to the bottom clamp (marked with a blue line) and tubing guides on the GatheRex. Attach the gas line from the GatheRex to the sterile filter of the G-Rex500 MCS. Before removing the supernatant from the G-Rex500MCS, ensure all clamps on the cell removal lines were closed.

Volume Reduction of G-Rex500MCS. Transfer ~4.5L of culture supernatant from the G-Rex500MCS to the 10L Labtainer per SOP-01777. Visually inspect G-Rex500MCS to ensure flask as level and media had been reduced to the end of the aspirating dip tube.

Prepare flask for TIL Harvest. After removal of the supernatant, close all clamps to the red line.

Initiation of TIL Harvest. Record the start time of the TIL harvest. Vigorously tap flask and swirl media to release cells. Perform an inspection of the flask to ensure all cells have detached. Tilt the flask to ensure hose is at the edge of the flask. If the cell collection straw is not at the junction of the wall and bottom membrane, rapping the flask while tilted at a 450 angle is usually sufficient to properly position the straw.

TIL Harvest. Release all clamps leading to the TIL suspension transfer pack. Using the GatheRex, transfer the cell suspension into the TIL Suspension transfer pack. Be sure to maintain the tilted edge until all cells and media are collected. Inspect membrane for adherent cells.

Rinsed flask membrane. Rinsed the bottom of the G-Rex500MCS. Cover ~1/4 of gas exchange membrane with rinse media. Closed clamps on G-Rex500MCS. Ensured all clamps were closed on the G-Rex500MCS.

Heat seal the Transfer Pack containing the TIL as close to the weld as possible so that the overall tubing length remained approximately the same. Heat seal the 10L Labtainer containing the supernatant and passed into the BSC for sample collection.

Record weight of Transfer Pack with cell suspension and calculate the volume suspension. Prepare transfer pack for sample removal. Weld a 4" plasma transfer set to the TIL Suspension transfer pack from above, leaving the female luer end attached as close to the bag as possible.

Remove testing samples from cell supernatant. In the BSC, remove 10.0 mL of supernatant from 10L labtainer using female luer port and appropriately sized syringe. Place into a 15 mL conical tube and label as "BacT" and Retain the tube for BacT sample. Using a separate syringe, removed 10.0 mL of supernatant and placed into a 15 mL conical tube. Retained the tube for mycoplasma sample for testing. Label tube as "Mycoplasma diluent". Closed supernatant bag. Place a red cap on the luer port to close the bag, and pass out of BSC.

Remove Cell Count Samples. In the BSC, using separate 3 mL syringes for each sample, removed $4 \times 1.0$ mL cell count samples from "TIL Suspension" transfer pack using the luer connection. Place samples in cryovials prepared above.

Remove Mycoplasma Samples. Using a 3 mL syringe, removed 1.0 mL from TIL Suspension transfer pack and place into 15 mL conical labeled "Mycoplasma diluent" prepared above. Label and stored Mycoplasma sample at 2-8°C until submitted for testing.

Prepare Transfer Pack for Seeding. In the BSC, attach the large diameter tubing end of a Repeater Pump Fluid Transfer Set to the Luer adapter on the transfer pack containing the TIL. Clamp the line close to the transfer pack using a hemostat. Place a red cap onto the end of the transfer set.

Place TIL in incubator. Remove cell suspension from the BSC and place in incubator until needed. Record time.

Perform cell counts and calculations utilizing NC-200. Diluted cell count samples initially by adding 0.5 mL of cell suspension into 4.5 mL of AIM-V media prepared above. This gave a 1:10 dilution.

Calculate flasks for subculture. Calculate the total number of flasks to seed. Rounded the number of G-Rex500MCS flasks to seed up to the neared whole number.

Example 6. Small-scale Production of TILs from Cryopreserved Tumor Tissue.

Preparing the tumor tissue for freezing: The tumor was aseptically collected, trimmed to remove extraneous non-tumor or necrotic tissue using sterile tweezers and a scalpel and/or scissors. Tumors with the minimum size of 1.5 cm diameter were further fragmented into 6 mm size fragments. For freezing the tumor, up to 10 pieces of 6 mm diameter fragments were placed in a single 15 mL cryovial (Thermo, USA) containing 10 mL of CryoStorIO (Biolife, USA) storage medium. Additional cryovials were used if the 6 mm diameter fragments exceeded 10 fragments. Cryovial lids were screwed tightly; then the tumor fragments were mixed gently with the storage medium by iteratively inverting the cryovial at least 3 and up to about 5 times. Then, the cryovials were incubated for 15 to 60 minutes at 2°C-8°C in a refrigerator. After incubation, cryovials slotted into a cryoshipper sleeve.

Freezing the tumor tissue: The cryoshipper sleeve containing the cryovials was placed in into a liquid nitrogen dry cryoshipper, thereby flash freezing the contents of the vials using the vapor phase liquid nitrogen temperature inside the $LN_2$ charged and temperature equilibrated dry $LN_2$ cryoshipper. For short term storage or transport, vials remained in the cryoshipper. For long term storage, vials were transferred and stored at liquid nitrogen temperatures in a liquid nitrogen freezer.

Thawing the stored tumor tissue: Frozen tumor samples were thawed by submerging the cryovials in a 37°C water bath for $5 \pm 1$ min. Using forceps, 6 mm diameter fragments were removed, washed three times with sterile Hank's Balanced Salt Solution (HBSS) (Gibco, USA) with gentamicin (Gibco, USA). Gentamicin was added to the HBSS at 50 μg/mL. Each 6 mm diameter fragment were further dissected to approximately 8 individual 1 to 3 mm diameter fragments. Typical tumor fragments were about 1 to 3 mm diameter and about 1 to 3 mm thick.

TIL Expansion, using a 1/100th Scale procedure: This procedure replicates the full-scale process 2A TIL Manufacturing method on a 1/100th scale. The scope of the study is to give instruction for the miniaturized 2A process using G-Rex 10M and G-Rex 5M flasks. PreREP culture was initiated using G-Rex 10M flasks, 1:10 of the full-scale pre-REP process. The REP was initiated using 10% of the pre-REP product in G-Rex 5M which equates to 1:10 of the full-scale REP process. This maintained a 1:100 scale for the remainder of the experiment.

The pre-REP was initiated with tumor processing and four fragments placed in an individual G-Rex 10M flask and cultured with CM1+IL-2 until Day 11. The REP was initiated on Day 11 using pre-REP TIL (10% of pre-REP product), PBMC feeders, OKT3 and IL-2 into a G-Rex 5M. Day 16 will have retained the appropriate amount of TIL and a multiplication factor will be used to extrapolate the full-scale process. Figure 7 summarizes the proecedure employed for this experimental scale TIL production.

Table 25 lists the equipment used for this experimental scale TIL production.

TABLE 25. Equipment.

| Item | Specifications | Company | Model # | Calibration requirements |
|---|---|---|---|---|
| Incubator | Set up at 37°C, 5%CO2 | Thermo Scientific | Forma Steri-Cycle i160 | Temperature is monitored every week. |
| Centrifuge | With swinging bucket rotor | Thermo Scientific or equivalent | Sorvall Legend XTR-7500-4521 | NA |
| Centrifuge | M icroce ntrifuge table top | Eppendorf or equivalent | NA | NA |
| Flow cytometer | Equipped with 3 lasers (red, blue, violet) | BD Biosciences | 8D FACS Canto II | Yearly Preventive Maintenance. CST Baseline and daily performance |
| Vortex Mixer | NA | Labnet International or equivalent | NA | NA |
| Pipet Aid | NA | Drummond or equivalent | NA | NA |
| 2 - 20-$\mu$L pipet | NA | Rainin or equivalent | L-20XLS or equivalent | Every six months |
| 20 - 200-$\mu$L pipet | NA | Rainin or equivalent | L-200XLS or equivalent | Every six months |
| 100 - 1000-pL pipet | NA | Rainin or equivalent | L-1000XLS or equivalent | Every six months |
| Biological Safety Cabinet (BSC) | NA | Thermo Scientific or equivalent | NA | Every six months |
| Water Bath | Set and maintain at 37°C +/- 2 degrees | Any in use | NA | NA |
| Cell counter | Dual fluorescence | Nexelom or equivalent | K2 | As recommended by vendor |
| Refrige rator | Set and maintain at 4°C +/- 2 degrees | Any in use | NA | NA |
| Freeze r | Set and maintain at - 20°C +/- 5 or 4 °C degrees | Any in use | NA | NA |

Table 26 lists the software used for this experimental scale TIL production.

TABLE 26. Software.

| Name | Company | Version |
|---|---|---|
| FACSDiva | BD Biosciences | 7 or equivalent / upgraded |
| FlowJo | Flow Jo | 10 |
| Cellometer K2 | Nexcelom | 2.1.4.2 |

Table 27 lists the materials used in this experimental scale TIL production.

TABLE 27. Materials.

| Product | Specifications | Vendor | Catalog | Storage |
|---|---|---|---|---|
| 50-mL conical tubes | NA | VWR or equivalent | 89004-364 | RT |
| G-Rex 5M | NA | Wilson Wolf | NA | RT |
| G-Rex 10M | NA | Wilson Wolf | NA | RT |
| 110-ml pipet | NA | Denville or equivalent | P7134 | RT |
| 5-mL pipet | NA | Fisher Scientific | 13-678-11D | RT |
| 25-mL pipet | NA | Fisher Scientific | 13-678-11 | RT |
| 50-mL pipet | NA | Fisher Scientific | 13-675-27 | RT |
| 20-pL pipet tips | NA | VWR or equivalent | 10111-260-000 | RT |
| 200-$\mu$L pipet tips | NA | VWR or equivalent | 1018-260-000 | RT |
| 1000-$\mu$L pipet tips | NA | VWR or equivalent | 1019-260-000 | RT |
| 110-mL pipet | NA | VWR or equivalent | 89130-898 | RT |
| Slides for cellometer | NA | Nexcelom Biosciences | SD100 | RT |
| FACS tubes | NA | BD Biosciences | 343675 | RT |
| Microfuge tubes | 1.5 ml | Eppendorf or equivalent | 022363328 | RT |
| Aluminum Foil | NA | VWR or equivalent | N/A | RT |
| BD FACS Flow Sheath Fluid | NA | BD Biosciences | 342003 | RT |
| 500 mL Stericup Filter system | NA | Millipore | SCGPU05RE | RT |
| 1X D-PBS | Without Ca$^{2+}$/Mg$^{2+}$ | Sigma or equivalent | D-8537 | RT |
| BD Cytometer Setup and Tracking Beads | NA | BD | 655051 | 4°C |
| BD FACS Clean solution | NA | BD | 340345 | RT |
| BD Shutdown solution | NA | BD | 334224 | RT |

Table 28 lists additional materials and reagents used.

TABLE 28. Additional Materials and Reagents.

| Product | Specifications | Vendor | Catalog # | Storage |
|---|---|---|---|---|
| BD Stain buffer | Dulbecco's Phosphate-Buffered Saline (DPBS) with 2% (w/v) heat-inactivated FBS and 0.09% (w/v) sodium azide, pH 7.4 (0.2 $\mu$m-pore filtered) | BD Pharmingen | 554656 | 4°C |
| 10% Bleach in Distilled water | Any in use | NA | NA | RT |
| Distilled water | Any in use | NA | NA | RT |
| FACS tube racks | Any in use | NA | NA | RT |
| Gloves, Latex J Nitrile | Any in use | NA | NA | RT |
| Lint free wipes | Any in use | NA | NA | RT |
| 70% Ethanol | Any in use | NA | NA | RT |

(continued)

| Product | Specifications | Vendor | Catalog # | Storage |
|---------|----------------|--------|-----------|---------|
| Paper towels | Any in use | NA | NA | RT |
| CaviCide | NA | Fisher or equivalent | 22-998-805 | RT |
| CryoStor 10 | NA | Starneall Technologies | 07930 | 4°C |

Table 29 lists reagents used for flow cytometry (FACS) analysis of cells to characterize various marker expression.

TABLE 29. FACS Reagents.

| Flow reagents | | | |
|---------------|--------------|--------|----------|
| Marker | Fluorochrome | Vendor | Catalog # |
| Live/dead Aqua | Aqua | Life Technologies | L34957 |
| CD4 | FITC | eBioscience | 11-0037-42 |
| CD8 | Pacific Blue | Biolegend | 301033 |
| TCR $\alpha/\beta$ | PE | Biolegend | 306708 |
| CD57 | PerCP/Cy5.5 | Biolegend | 359622 |
| CD27 | APC H7 | BD Biosciences | 560222 |
| CD28 | PECy7 | BD Biosciences | 560684 |
| CD56 | APC | Beckman | IM2474U |

Table 30 lists reagents used for cell culture media.

TABLE 30. Media Reagents

| Product | Specifications | Vendor | Catalog | Storage |
|---------|----------------|--------|---------|---------|
| RPMI | Contains: L-glutamine and Phenol red. Without: HEPES | Life Technology | 11875-119 | 4°C |
| CTS Optimizer | -L-Glutamine Phenol Red | Life Technologies | A1048501 | 4°C |
| Serum replacement | L-Glutamine | Life Technologies | A2596101 | -20°C |
| Human AB Serum | GemCell™ human serum AB is collected from healthy male donors of the AB serotype at FDA-licensed facilities in the United States. 0.1 $\mu$m sterile-filtered | Gemini BioProducts | 100-512 | -20°C |
| L-Glutamine | NA | Lonza | BW17-605E | -80°C |
| Gentamycin | NA | Gibcol Life Technology | 15710-064 | RT |
| 2-Mercaptoethanol | NA | Gibcol Life Technology | 21985-023 | 4°C |
| GMP Recombinant Human Interleukin-2 (rhIL-2), 6e6 stock solution | NA | CellGenix | 1020-1000 | 20°C |

(continued)

| Product | Specifications | Vendor | Catalog | Storage |
|---|---|---|---|---|
| AIM-V Medium CTS | NA | Gibco/Life Technologies | 087-0112DK | 4°C |
| GlutaMax Supplement | NA | Gibco Life Technology | 35050-061 | RT |
| AO/PI dye | NA | Nexcelom | CS2-0106-5 ml | 4°C |

*Procedure for mini-scale TIL expansion (1/100th scale 2A Process)*

*Day 0*

Media Preparation, if necessary, prepare IL-2. Prepare 350 mL CM1 for every 3 G-Rex 10M flasks. Add 6000 IU/mL IL-2 to CM1. Warm in 37°C water bath while processing tumor.

Tumor Processing: Add 100mL of media and IL-2 to each G-Rex 10M flasks and randomly add 4 fragments to each flask. Be sure to randomize fragments to increase heterogeneity of results. Place in the incubator at 37°C/5% $CO_2$ for 11 days.

*Day 11*

Media Preparation, if necessary, prepare IL-2. Prepare 300 mL CM2 for every 3 G-Rex 5M flasks. Add 3000 IU/mL IL-2 to CM2, then warm in 37°C water bath.

Prepare the TIL from first culture: Carefully remove each G-Rex 10M flask from incubator without disturbing cells. Aspirate media and using a 10 mL pipette, gently mix up and down to liberate cells from membrane. Slowly pull culture up and record volume from each flask, transfer to a 50 mL conical vial.

Obtain a 200 μL aliquot for cell counts, then loosely cap the 3 vials and place in 37°C/5% $CO_2$ incubator until ready to seed flasks. Count TIL and record on cell counting worksheet.

Prepare feeder cells: Obtain an adequate number of feeders (50 × 10^6 cells/flask); thaw feeder cells in 37°C water bath for 2 minutes, then dilute feeder cells 1:10 in CM2+IL-2. Remove a 200 μL aliquot for cell counting. loosely cap vial and place in 37°C/5% $CO_2$ incubator until ready to seed flasks. Dilute 20uL PBMC feeder cells 1:10 and count PBMC feeders. Record on cell counting worksheet. Based on the count, dilute feeders to 10 × 10^6 cells/mL in CM2+IL-2, then place 5 mL of solution into a 50 mL conical tube for each flask. For each flask, add 1.5 mL of 1:1000 diluted stock OKT-3 to the 5mL of feeders and incubate for 15 minutes.

Seed flasks by adding TIL, PBMC feeder cells, OKT-3, and media to each flask. For TILs, add about 10% of the preREP produce, generally up to a maximum of about 2 × 10^6 cells per flask. PBMC feeders, add 5 mL prepare as above, containing 50 × 10^6 cells total. Add 1.5 mL of 1:1000 diluted OKT-3 stock, then bring to a final volume of 50 mL by toping with CM2 medium.

*Day 16*

Media preparation for the split: If necessary, prepare IL-2; then prepare 150 mL CM4 for every 3 flasks. Next, add 3000 IU/mL IL-2 to media. Place in 37°C water bath until needed.

Split: Carefully remove G-Rex 5M flasks from incubator as not to disturb cells, then aspirate approximately 40 mL media, taking care to not aspirate cells. Using a 10 mL pipette, gently mix up and down to liberate cells from membrane; then, slowly pull culture up and record volume from each flask, transfer to a 50 mL conical vial. Take a 200 μL aliquot for cell counts, and then loosely cap the 3 vials, placing them in 37°C/5% $CO_2$ incubator until ready to seed flasks. Count TIL and record on cell counting worksheet. Aspirate remaining liquid from bottom of G-Rex 5M flasks to ensure no extra TIL remain in flasks.

Calculate number of daughter flasks for each flask (TVC/10^6), round up (max 5). Divide TVC by number of daughter flasks and adjust flask to the calculated amount. Some experiments may allow only one flask to be continued. This will also be extrapolated and accounted for at the end of the harvest. Fill flasks to 50 mL with CM4+IL2 and then place flasks in 37°C/5% $CO_2$.

*Day 22*

Harvest: Carefully remove G-Rex 5M flask from incubator as not to disturb cells, then remove supernatant, if needed for metabolite analysis. Aspirate media and using a 10 mL pipette, gently mix up and down to liberate cells from membrane. Slowly pull culture up and record volume from each flask, then transfer to a 50 mL conical vial. Remove a 200 $\mu$L aliquot for cell counts; loosely cap the 3 vials and place in 37°C/5% $CO_2$ incubator until ready to seed flasks.

Count TIL record on cell counting worksheet; extrapolate and record theoretical full-scale yield (TVC*100). If only one flask is continued in split, also multiply by # of flasks calculated above. Then, allocate the cells for the assays listed in Table 31.

TABLE 31. Functional Assays.

| Functional Assay | Number of Cells/Culture Supernatant |
|---|---|
| Flow Phenotyping (DF1/DF2) - WRK LAB-041 | ≥10e6 |
| IFN-γ Re-stimulation -WRK LAB-016 | ≥5e6 |
| Cells to freeze | All Remaining cells |

Wash remaining cells two times with a 1:10 dilution of 1% HSA/PL-A. Freeze remaining cells in a thermally-insulated container (such as a Mr. Frosty container) or CRF in a 1:1 formulation with CS10 (30 $\times$ $10^6$ cells/mL/Vial) for other studies.

Table 32 summarizes the critical process parameters for the TIL production process.

TABLE 32. Process Parameters.

| IL-2 Concentration | CM1-6000IU/mL CM2/CM4-3000IU/mL |
|---|---|
| OKT-3 Concentration | Day 11 - 30ng/mL |
| Feeder seeding density | Day 11 - 50e6/flask |
| TIL seeding density | Day 11 - ≤2e6/flask (10% of pre-REP product) |
| Preloading of OKT-3 | 15 Minutes incubation of OKT-3 and feeder cells |
| TIL seeding density | Day 16 - Divide evenly across calculated daughter flasks |
| Calculation of Daughter flasks | Day 16 TVC / 1e9, maximum 5. |
| Media Preparation | Property prepared according to LAB-005 |
| Product Formulation | 2X wash with 1% HSA/PL formulated with 1:1 TIL/CS10 ratio |

## Example 7. Early REP with Frozen Tumor Tissue TILs.

This data was generated using a modified version of the TIL manufacturing protocol detailed in Example 5. Aspects of the experimental method that differ from Example 5 are highlighted.

Tumor tissue preparation: Care was taken to keep the tumor hydrated by adding Hank's Balanced Salt Solution (HBSS) to the tissue as needed, keeping it hydrated through drop-wise addition. The tumor was trimmed to remove extraneous non-tumor tissue using sterile tweezers, a scalpel, and scissors. Tumor fragments with a minimum size of 1.5 cm diameter were further fragmented into 6 mm diameter pieces. Ideal thickness for each fragment was roughly 6 mm

Tumor tissue freezing: Up to ten tumor fragments (nominal size 6 mm $\times$ 6 mm $\times$ 6 mm) were placed in each cryovial. 15 mL cryovials (Thermo, USA) were used. 10 mL of CryoStorlO (Biolife, USA) was added to each tube. Cryovial lids were screwed tightly and the contents of the cryovial were mixed gently by inverting the tube 5 times. The cryovials were then incubated at 2°C-8°C in a refrigerator, for 15 minutes, 30 minutes, or 60 minutes. After the refrigerated incubation in storage medium, cryovials were packed in a sleeve, then flash frozen using the vapor phase of liquid nitrogen ($LN_2$) in a properly charged and equilibrated dry liquid nitrogen shipper (Cryoport). For storage, fully frozen cryovials were transferred to a liquid nitrogen cell storage freezer, to maintain the frozen tumor tissue at liquid nitrogen temperatures.

Tumor thawing: On Day 0 of the TIL manufacturing process, cryovials containing the tumor tissue fragments were removed from the sleeve, placed in a sealable plastic bag, then thawed by submerging the sealed bag in a 37°C water bath for 5 + 1 minute.

By using forceps, tumor fragments were carefully removed into sterile HBSS (Gibco, USA) + Gentamicin (Gibco, USA), serially washed 3 times in HBSS containing gentamicin (50 μg/mL) for ≥ 3 minutes each wash. Individual fragments approximately 5-6 mm in all dimensions were cut from the original tumor samples and staged in individual drops of HBSS on a new sterile plastic surface.

Up to 60 of these fragments were placed into a G-Rex 100MCS flasks containing 1 L of Cell Culture Medium 1 (CM1), supplemented with 6000 IU/mL recombinant human IL-2, and the flask was placed in a humidified incubator (target 37 °C, 5% $CO_2$ in air) to initiate the pre-Rapid Expansion Phase (pre-REP) unit operation.

Summary of TIL manufacture: PreREP and REP. A small-scale Gen 2 process (1/100th scale) was used to test the "fresh" control and the several frozen tumor conditions. Pre-REP culture was initiated using G-Rex 10M flasks, at 1:10 scale of the full-scale pre-REP process. REP was initiated using 10% of the pre-REP product in a G-Rex 5M, which equates to 1:100 of the full-scale REP process.

For "fresh" control process, fresh tumor was washed in sterile HBSS+ Gentamicin, sectioned to nominal 6 mm × 6 mm × 6 mm fragments. About 4 fragments were placed into a G-Rex 10M flask with CM1 media containing 6,000 IU/mL of rhIL-2. G-Rex cultures were incubated for 11 days in a 37°C incubator. On day 11 of the culture, pre-REP cells were harvested, and the REP was initiated by coculturing 10% of the Pre-REP TIL with $50 \times 10^6$ PBMC feeder cells in CM2 media containing 3,000 IU/mL rhIL-2 and 30 ng/mL of OKT-3 in a G-Rex 5M flask. On day 16 of the co-culture, the culture was split into G-Rex 5M flasks containing no more than $10 \times 10^6$ cells. The calculation of the number of split "daughter" flasks was performed as follows: $TVC/10^6$ = number of Daughter flasks, rounded up.

The split cultures were incubated for 6 additional days in CM4 media containing 3,000 IU/mL rhIL-2. On day 22, the cells were harvested and frozen using CryoStorlO (Biolife, USA).

For frozen tumor expansion process, the nominal 6 mm × 6 mm × 6 mm fragments were processed into smaller 2 mm - 3 mm fragments as explained above. Pre-REP setup, REP initiation, Split process, and reagents used for frozen tumor expansion were performed exactly like the "fresh" control process, with the following exceptions:

The duration of the Pre-REP culture was 7 days (vs 11 days for "fresh" control)

The duration of the REP culture was 14 days with a Split on Day 7 (vs 11 days with a Split on Day 5 for "fresh" control). Thus, the total duration of the frozen tumor was 21 days, vs 22 days for the "fresh" control.

Characterization or REP TILs: TILs produced from each of the tumor processing conditions (fresh, frozen with 0, 30 minutes, or 60 minute refrigerated incubations in storage medium). To characterize TIL purity, identity, memory subset, activation, and exhaustion markers, flow cytometry was conducted with the following antibodies: CD3-BUV395, CD62LBV421, CD57-PB, CD11c-BV711, CD28-BB515, CD19-BUV563, CCR7-PE, CD123-BV605, CD27PE-CF594, CD14-BV-650, TCRγ/δ-APC CD45-PerCP-Cy5.5, CD45RA-A700, CD56-BUV737, CD8-BV786, CD4-PE-Cy7AND CD16-APC-Vio770) and TIL-2 panel (CD3-BUV395, PD-1-BV421, 2B4/CD244-PB, CD8-BB515, CD25-BUV563, BTLA-PE, KLRG1-PE-Dazzle 594, TIM-3-BV650, CD194/CCR4-APC, CD4-VioGreen, TIGIT-PerCP-eFluor 710, CD183-BV711, CD69-APC-R700, CD95-BUV737, CD127-PE-Cy7, CD103-BV786, LAG-3-APC-eFluor 780. TIL function was further determined based on secretion of IFN-γ, Granzyme-B and CD107A. Briefly, about $10^5$ cells were stimulated with anti-CD3 coated plate for 24 h. Culture supernatant was collected and tested using ELISA for IFN-γ and Granzyme-B. For CD107A levels, TIL were stimulated with or without stimulation of PMA/IO for 2 h, stained for CD107A-APC700, and further analyzed by flow cytometry.

Flow-FISH was performed using Dako/Agilent Pathology Solutions Telomere PNA Kit/FITC for Flow Cytometry kit to determine telomere length, following the manufacturer's instructions. 1301 T-cell leukemia cell line (Sigma-Aldrich) was used as the internal reference standard.

*Results*

Figure 8 shows the pre-REP total viable cells (TVC) based on the tumor tissue freezing procedure. Incubation in the storage medium for about 30 minutes under refrigerated conditions (2°C - 8°C) yielded the most viable cells, compared both to immediate flash freezing and longer incubations before freezing. Figure 9 shows the REP TVC results at day 21. The freezing protocol affects the early viability in pre-REP culture. Further function was assessed by measuring IFN-y secretion, and is summarized in Figure 10. TILs expanded from frozen tumor tissue first incubated for 30 minutes in storage medium expressed the highest level of IFN-γ. All markers evaluated in the cell phenotype panel (*see* Table 29) demonstrate that TILs produced from frozen tumor tissue have similar phenotypes as TILs produced from fresh tumor tissue.

Figures 11 and 12 overview the major steps and highlights the differences between the cell populations compared in this exemplary experiment.

Figures 13-23 illustrate the results of the TIL phenotypic characterization of fresh and frozen tumor samples in 3 to 5 patients. Patient M1125 was tested at flash freezing and 30 minute and 60 minute incubation at 2°C to 8°C prior to freezing in the vapor phase of liquid nitrogen, and all other patients were tested at the 60 minute incubation at 2°C to 8°C prior to freezing in the vapor phase of liquid nitrogen. Overall, frozen tumor samples performed as well as or better than fresh tumor samples.

Example 8. Tumor Cryopreservation Process.

Tumors may be cryopreserved in an exemplary embodiment of the present invention according to the following process. Tumor tissue must be handled aseptically and remain free of contamination throughout processing.

After the tissue is removed from the patient it should be kept hydrated by adding Hank's Balanced Salt Solution (HBSS) to the tissue as needed through drop-wise addition with a transfer pipette. The tumor is aseptically placed in a sterile specimen container with a lid and transferred to a sterile surface in the surgical suite or a laminar flow hood if sent to pathology for prosection. Using sterile tweezers and a sterile scalpel or scissors, the tumor should be trimmed to remove extraneous non-tumor or necrotic tissue by the resecting physician or an experienced pathologist designated at each site. Hemorrhagic, liquid and necrotic tissue should be excluded whenever possible. The physician or pathologist should evaluate the prosected specimen to ensure tissue is viable, non- hemorrhagic, free of necrosis, and that the minimum size specification is met. Tissue should have pigment heterogeneity if possible.

If the tumor has a diameter of at least 1.5 cm, it may be cut in half to provide both a frozen specimen and a fresh specimen for comparison. One half of the trimmed tumor is to be placed into a sterile bottle of Hypothermosol that has been maintained at 4-8°C with gentamicin and amphotericin added. The lid should be screwed tightly. This provides the fresh specimen.

The other half of the tumor should be fragmented into $10 \times 6$ mm fragments and placed into a prepared sterile 15 mL cryovial containing 10 mL of sterile CryoStor® 10. If more than ten 6 mm fragments are obtained, use additional 15 mL cryovial containing 10 mL of sterile CryoStor 10. The lid should be screwed tightly and the frozen specimen container should be placed at 4°C for 1 hour and then flash frozen over liquid nitrogen. This provides the frozen specimen.

The frozen tumor specimen should be packaged into a cryoshipper per cryoshipper instructions.

Example 9. Sixteen Day Frozen Tumor Expansion Processes from Ovarian Cancer Tumors.

A small scale TIL expansion process (1/100th scale) was used to test "fresh" control tumor samples and frozen tumor samples. Frozen tumor samples were frozen according to the cryopreservation process discussed above in Examples 7 and 8. Pre-REP was initiated using G-Rex 10M flasks at 1:10 of the full-scale pre-REP process. REP was initiated using 10% of the pre-REP product in a G-Rex 5M flask, which equates to about 1:100 of the full-scale REP process. Ovarian tumor samples were used in this example.

Fresh Tumor Preparation. Six 3 mm diameter fresh tumor fragments were washed in sterile Hanks Balanced Salt Solution (HBSS) supplemented with gentamicin. The fragments were placed into a G-Rex 10M flask (Wilson Wolf Mfg., New Brighton, MN) with CM1 media containing about 6,000 IU/mL of rhIL-2. These cultures were incubated for 11 days in a 37°C incubator. On day 11, the pre-REP cells were harvested, and the REP was initiated by co-culturing 10% of the pre-REP TILs with $50 \times 10^6$ PBMC feeder cells in CM2 media containing 3,000 IU/mL rhIL-2 and 30 ng/mL of OKT-3 in a G-Rex 5M flask (Wilson Wolf Mfg., New Brighton, MN). On day 16, the culture was split into G-Rex 5M flasks containing no more than $10 \times 10^6$ cells. The split cultures were incubated for 6 additional days in CM4 media containing 3,000 IU/mL rhIL-2. On day 22, the cells were harvested and frozen using CryoStorlO (Biolife, USA).

Frozen Tumor Preparation - Early REP Method 1 ("ER-1"). Six 2-3 mm diameter frozen tumor fragments were thawed and washed in sterile Hanks Balanced Salt Solution (HBSS) supplemented with gentamicin. The fragments were placed into a G-Rex 10M flask (Wilson Wolf Mfg., New Brighton, MN) with CM1 media containing about 6,000 IU/mL of rhIL-2. These cultures were incubated for 5 days in a 37°C incubator. On day 5, the pre-REP cells were harvested, and the REP was initiated by co-culturing 10% of the pre-REP TILs with either $25 \times 10^6$ or $50 \times 10^6$ PBMC feeder cells in CM2 media containing 3,000 IU/mL rhIL-2 and 30 ng/mL of OKT-3 in a G-Rex 5M flask (Wilson Wolf Mfg., New Brighton, MN). On day 10, the culture was split into G-Rex 5M flasks containing no more than $10 \times 10^6$ cells. The split cultures were incubated for 6 additional days in CM4 media containing 3,000 IU/mL rhIL-2. On day 16, the cells were harvested and frozen using CryoStorlO (Biolife, USA).

Frozen Tumor Preparation - Early REP Method 2 ("ER-2"). Six 2-3 mm diameter frozen tumor fragments were thawed and washed in sterile Hanks Balanced Salt Solution (HBSS) supplemented with gentamicin. The fragments were placed into a G-Rex 10M flask (Wilson Wolf Mfg., New Brighton, MN) with CM1 media containing about 6,000 IU/mL of rhIL-2. These cultures were incubated for 3 days in a 37°C incubator. On day 3, the pre-REP cells were harvested, and the REP was initiated by co-culturing 10% of the pre-REP TILs with either $25 \times 10^6$ or $50 \times 10^6$ PBMC feeder cells in CM2 media containing 3,000 IU/mL rhIL-2 and 30 ng/mL of OKT-3 in a G-Rex 5M flask (Wilson Wolf Mfg., New Brighton, MN). On day 9, the culture was split into G-Rex 5M flasks containing no more than $10 \times 10^6$ cells. The split cultures were incubated for 7 additional days in CM4 media containing 3,000 IU/mL rhIL-2. On day 16, the cells were harvested and frozen using CryoStorlO (Biolife, USA).

Figure 24 illustrates a flow chart comparing these three expansion methods. Results are illustrated below in Table 33. Expansion of TILs using both ER-1 and ER-2 methods produced a much larger REP fold expansion as compared with using the fresh tumor sample method GEN2.

Table 33: Expansion Results from Example 9 Extrapolated to Full Scale.

|  | Gen2 | ER-1 Frozen | ER-1 Frozen with half feeders | ER-2 Frozen | ER-2 Frozen with half feeders |
|---|---|---|---|---|---|
| **Day 0 No. of fragments/flasks** | 60 | 60 | 60 | 60 | 60 |
| **Day 11 - Pre-REP TVC (% viability)** | $2.93 \times 10^6$ (88.7) | $1.23 \times 10^6$ (64) | $1.23 \times 10^6$ (64) | $6.1 \times 10^5$ (55) | $6.1 \times 10^5$ (55) |
| **Day 11 REP Seeded (TIL)** | $2.93 \times 10^6$ | $1.23 \times 10^6$ | $1.23 \times 10^6$ | $6.1 \times 10^5$ | $6.1 \times 10^5$ |
| **Day 11 REP Seeded (Feeders)** | $5 \times 10^9$ | $5 \times 10^9$ | $2.5 \times 10^9$ | $5 \times 10^9$ | $2.5 \times 10^9$ |
| **Split Day Scale up TVC (% Viability)** | Day 16 $1.49 \times 10^9$ (83.2) | Day 10 $1.66 \times 10^9$ (91.2) | Day 10 $1.2 \times 10^9$ (91.4) | Day 9 $2.17 \times 10^9$ (92) | Day 9 $1.23 \times 10^9$ (86.8) |
| **Harvest Day TVC (% viability)** | Day 22 $10.9 \times 10^9$ (92.9) | Day 16 $29.1 \times 10^9$ (88.3) | Day 16 $25.9 \times 10^9$ (89) | Day 16 $49.8 \times 10^9$ (92.3) | Day 16 $34.2 \times 10^9$ (89.6) |
| **REP fold expansion** | 3728 | 23714 | 21060 | 81684 | 55998 |

Example 10: Sixteen Day Frozen Tumor Expansion Processes with Other Tumor Types.

The processes disclosed in Example 9 are performed with tumor samples collected from at least two patients in the following cancer types: melanoma, lung, cervical, and head and neck squamous cell carcinoma (HNSCC). The expansion processes are performed for each cancer type in substantially the same manner as described above for the ER-1 and ER-2 methods. Data are collected as described above.

Example 11: Sixteen Day Frozer Tumor Expansion Processes with Defined Medium.

The processes disclosed in Example 9 are performed with tumor samples collected from at least two patients in the following cancer types: melanoma, lung, cervical, head and neck squamous cell carcinoma (HNSCC), and ovarian, but substituting the CM1 and CM2 media with a defined medium according to the present invention (e.g., CTS™ OpTmizer™ T-Cell Expansion SFM with 3% CTS™ Immune Cell Serum Replacement (ThermoFisher Scientific) and 2-mercaptoethanol at 55mM).

Example 12: Sixteen Day Frozen Tumor Expansion Processes with OMNI C3™ culture bags and EXP-PAK® Bio-Containers.

The processes disclosed in Example 9 are performed with tumor samples collected from at least two patients in the following cancer types: melanoma, lung, cervical, head and neck squamous cell carcinoma (HNSCC), and ovarian, but substituting the GRex gas permeable containers with either the OMNI C3™ culture bags (Lampire Biological Laboratories) or the EXP-PAK® Cell Expansion Bio-Containers (Charter Medical). The OMNI C3™ culture bag is substituted at the pre-REP/first expansion stage, and the EXP-PAK® bio-container is substituted at the REP/second expansion stage.

Example 13: Sixteen Day Frozen Tumor Expansion Processes with Defined Medium, OMNI C3™ culture bags and EXP-PAK® Bio-Containers.

The processes disclosed in Example 9 are performed with tumor samples collected from at least two patients in the following cancer types: melanoma, lung, cervical, head and neck squamous cell carcinoma (HNSCC), and ovarian, but substituting the CM1 and CM2 media with a defined medium according to the present invention (e.g., CTS™ OpTmizer™ T-Cell Expansion SFM, ThermoFisher) and substituting the GRex gas permeable containers with either the OMNI C3™ culture bags (Lampire Biological Laboratories) or the EXP-PAK® Cell Expansion Bio-Containers (Charter Medical). The OMNI C3™ culture bag is substituted at the pre-REP/first expansion stage, and the EXP-PAK® bio-container is substituted at the REP/second expansion stage.

**Claims**

1.  A method of cryopreserving tumor tissue for the manufacture of tumor infiltrating lymphocytes (TILs), the method comprising:

    (i) fragmenting the tumor tissue;
    (ii) incubating the fragments in a cryopreservation medium for 30 minutes to 80 minutes at 2°C to 8°C; and,
    (iii) freezing the fragments wherein the freezing is flash freezing using the vapor phase of liquid nitrogen.

2.  The method of claim 1, wherein the tumor tissue is:

    (i) fragmented into approximately spherical fragments having a diameter of 1.5 mm to 6 mm, optionally wherein the approximately spherical fragments have a diameter of 6mm or 3mm;
    (ii) fragmented into generally rectangular fragments having a shortest edge length of at least 1.5 mm and a longest edge length of 6 mm;
    (iii) fragmented into generally cubical fragments having edge lengths of between 1.5 mm and 6 mm, optionally wherein the cubical fragments have edge lengths of 6 mm or 3mm;
    (iv) from a dissected tumor, optionally wherein the dissected tumor is less than eight hours old; or
    (v) is washed in a physiologically buffered isotonic saline solution prior to incubation, optionally wherein the washing comprises three serial washes of at least three minutes each, with the physiologically buffered isotonic saline solution replaced after each serial wash.

3.  The method of claim 1 or 2, wherein the cryopreservation medium comprises:

    (i) 2% v/v to 15% v/v dimethyl sulfoxide (DMSO), optionally wherein the cryopreservation medium comprises 10% v/v DMSO; or
    (ii) at least one antimicrobial agent, optionally wherein the at least one antimicrobial agent is gentamicin at a concentration of at least 50 $\mu$g/mL.

4.  The method of any one of claims 1 to 3, wherein the freezing takes place at a temperature in the range of -125°C to -196°C, optionally wherein the freezing takes place at a temperature of -140°C to -175°C, further optionally wherein the freezing takes place at - 145°C.

5.  The method of any one of claims 1 to 4, further comprising the step of (iv) storing the frozen fragments below at least -130°C, optionally wherein the frozen fragments are stored in the vapor phase of liquid nitrogen or are stored submerged in liquid nitrogen.

6.  The method of any one of claims 1 to 5, wherein the tumor fragments are incubated in the cryopreservation medium for 30 minutes to 70 minutes and optionally for 30 minutes to 60 minutes.

7.  A cryopreserved tumor fragment for the manufacture of tumor infiltrating lymphocytes (TILs), prepared by a process comprising the steps of:

    (i) fragmenting a dissected tumor or tumor biopsy sample;
    (ii) incubating the fragments in a cryopreservation medium; and,
    (iii) freezing the fragments wherein the freezing is flash freezing using the vapor phase of liquid nitrogen,

    wherein step (ii) further comprises incubating the fragments for 30 minutes to 60 minutes at 2°C to 8°C in a cryopreservation medium comprising 10% v/v DMSO.

8.  A method for preparing tumor infiltrating lymphocytes (TILs) for adoptive T-cell therapy, the method comprising:

(a) storing the tumor tissue in a frozen state, the method of storing the tumor tissue comprising:

(i) trimming the tumor tissue to remove excess non-tumor tissue;
(ii) placing the tumor tissue in a closable vessel containing a storage medium;
(iii) incubating the vessel, which contains the tumor tissue and the storage medium, at a temperature of 2 to 8°C for a period of 30 minutes to 60 minutes;
(iv) freezing the vessel, wherein the freezing is flash freezing using the vapor phase of liquid nitrogen; and
(v) storing the vessel at or below -130°C;

(b) thawing the vessel;
(c) treating the tumor tissue in a gas permeable container with a first cell culture medium comprising interleukin 2 (IL-2) and optionally OKT-3 to provide TILs;
(d) expanding the TILs in a gas permeable container using a second cell culture medium comprising cell culture medium, irradiated feeder cells, OKT-3, and IL-2, to provide an expanded number of TILs.

9. The method of claim 8, wherein the tumor tissue in step (i) is:

(a) trimmed to between 1.5 mm and 6 mm in diameter, optionally wherein the tumor tissue in step (i) is trimmed to 6 mm by 6 mm by 6 mm; and/or
(b) first washed in Hank's Balanced Salt Solution (HBSS) before performing step (i), optionally wherein the washing comprises three serial washes of at least three minutes each, with the HBSS replaced after each serial wash.

10. The method of claim 8 or 9, wherein the storage medium further comprises:

(i) an antibacterial agent and an antifungal agent, optionally wherein the storage medium comprises gentamicin at a concentration of at least 50 $\mu$g/mL; and/or
(ii) 2 % v/v to 12 % v/v dimethyl sulfoxide (DMSO), optionally wherein the storage medium comprises 10% DMSO.

11. The method of any one of claims 8 to 10, wherein step (b) comprises immersing the vessel in water bath at a temperature of 37°C for 5 minutes.

12. The method of any one of claims 1 to 6 or claims 8 to 11, wherein the tumor tissue is selected from the group consisting of melanoma tumor tissue, head and neck tumor tissue, breast tumor tissue, renal tumor tissue, pancreatic tumor tissue, glioblastoma tumor tissue, lung tumor tissue, colorectal tumor tissue, sarcoma tumor tissue, triple negative breast tumor tissue, cervical tumor tissue, ovarian tumor tissue, or HPV-positive tumor tissue.

13. The method of any one of claims 8 to 12, the method further comprising a split between step (c) and step (d), optionally wherein the split occurs 16 days after step (c) is initiated.

14. The method of any one of claims 8 to 13, wherein the first culture step is 11 days, or wherein steps (c) through (d) are performed over a period of 22 days, or over a period of less than 20 days.

15. The method of any one of claims 8 to 14, wherein:

(a) step (iv) comprises freezing the vessel at -125 °C to -150 °C;
(b) step (v) comprises storing the vessel below at least -130 °C; or
(c) step (v) comprises storing the vessel submerged in liquid nitrogen.

16. A therapeutic population of TILs for use in a method for treating a subject with cancer, the method comprising providing expanded tumor infiltrating lymphocytes (TILs) for administration produced by a method comprising:

(a) storing the tumor tissue in a frozen state, the method of storing the tumor tissue comprising:

(i) trimming the tumor tissue to remove excess non-tumor tissue;
(ii) placing the tumor tissue in a closable vessel containing a storage medium;
(iii) incubating the vessel, which contains the tumor tissue and the storage medium, at 2 - 8°C for 30 minutes;
(iv) flash freezing the vessel using the vapor phase of liquid nitrogen; and

(v) storing the vessel at the vapor phase of liquid nitrogen temperature,

(b) thawing the tumor tissue;

(c) adding the tumor fragments into a closed system;

(d) performing a first expansion by culturing the first population of TILs in a cell culture medium comprising IL-2 to produce a second population of TILs, wherein the first expansion is performed in a closed container providing a first gas-permeable surface area, wherein the first expansion is performed for 3-14 days to obtain the second population of TILs, wherein the second population of TILs is at least 50-fold greater in number than the first population of TILs, and wherein the transition from step (c) to step (d) occurs without opening the system;

(e) performing a second expansion by supplementing the cell culture medium of the second population of TILs with additional IL-2, OKT-3, and antigen presenting cells (APCs), to produce a third population of TILs, wherein the second expansion is performed for 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (d) to step (e) occurs without opening the system;

(f) harvesting the therapeutic population of TILs obtained from step (e), wherein the transition from step (e) to step (f) occurs without opening the system;

(g) transferring the harvested TIL population from step (f) to an infusion bag, wherein the transfer from step (f) to (g) occurs without opening the system;

(h) cryopreserving the infusion bag comprising the harvested TIL population from step (g) using a cryopreservation process; and

(i) providing a therapeutically effective dosage of the third population of TILs for administration from the infusion bag in step (h) to the subject.

17. The therapeutic population of TILs of claim 16, wherein the method for treating the subject with cancer further comprises the steps of (j) concomitantly providing a therapeutically effective dosage of aldesleukin or a biosimilar thereof for administration to the subject; and (k) concomitantly providing a therapeutically effective dosage of a PD-1/PD-L1 inhibitor for administration to the subject, optionally wherein the PD-1/PD-L1 inhibitor is selected from the group consisting of pembrolizumab, nivolumab, avelumab, durvalumab, atezolizumab, and biosimilars thereof.

18. The therapeutic population of TILs of claim 16 or claim 17, wherein the cancer is selected from the group consisting of melanoma, cervical cancer, head and neck squamous cell cancer, non-small cell lung cancer, bladder cancer, ovarian cancer, pancreatic cancer, and sarcoma.

19. A method for expanding tumor infiltrating lymphocytes (TILs) from frozen tumor tissue comprising:

(a) providing tumor tissue obtained from a patient;

(b) incubating the tumor tissue for at least 30 minutes in a storage medium at a temperature in the range of 2°C to 8°C;

(c) flash freezing the tumor tissue;

(d) storing the tumor tissue in a frozen state;

(e) thawing the tumor tissue;

(f) treating the tumor tissue in a gas permeable container with a first cell culture medium and interleukin 2 (IL-2) to provide TILs;

(g) removing at least a plurality of the TILs; and

(h) expanding the TILs with a second cell culture medium comprising cell culture media, irradiated feeder cells, OKT-3 antibody, and IL-2 in a gas permeable container to provide an expanded number of TILs.

20. A method for expanding TILs from frozen tumor tissue comprising:

(a) providing tumor tissue obtained from a patient;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range of 2°C to 8°C for 20 minutes to 70 minutes;

(e) flash freezing the vessel;

(f) storing the vessel such that the vessel remains frozen;

(g) thawing the vessel;

(h) treating the tumor tissue in a gas permeable container with a first cell culture medium and interleukin 2 (IL-2) to provide TILs;

(i) removing at least a plurality of the TILs; and

(j) expanding the TILs with a second cell culture medium comprising cell culture media, irradiated feeder cells, OKT-3, and IL-2 in a gas permeable container to provide an expanded number of TILs.

21. A therapeutic population of TILs for use in a method of treating cancer for a human subject in need thereof, the method comprising providing expanded tumor infiltrating lymphocytes (TILs) for administration produced by a method comprising:

(a) providing tumor tissue obtained from the subject;

(b) trimming the tumor tissue to remove excess non-tumor tissue;

(c) placing the tumor tissue in a closable vessel containing a storage medium;

(d) incubating the vessel, which contains the tumor tissue and the storage medium, at a temperature in the range of 2°C to 8°C for 20 minutes to 70 minutes;

(e) flash freezing the vessel using the vapor phase of liquid nitrogen;

(f) storing the vessel at the vapor phase of liquid nitrogen temperature;

(g) thawing the tumor tissue;

(h) adding the tumor tissue into a closed system;

(i) performing a first expansion by culturing a first population of TILs from the tumor tissue in a cell culture medium comprising IL-2 to produce a second population of TILs, wherein the first expansion is performed in a closed container providing a first gas-permeable surface area, wherein the first expansion is performed for 3-14 days to obtain the second population of TILs, wherein the second population of TILs is at least 50-fold greater in number than the first population of TILs, and wherein the transition from step (h) to step (i) occurs without opening the system;

(j) performing a second expansion by supplementing the cell culture medium of the second population of TILs with additional IL-2, OKT-3, and antigen presenting cells (APCs), to produce a third population of TILs, wherein the second expansion is performed for 7-14 days to obtain the third population of TILs, wherein the third population of TILs is a therapeutic population of TILs which comprises an increased subpopulation of effector T cells and/or central memory T cells relative to the second population of TILs, wherein the second expansion is performed in a closed container providing a second gas-permeable surface area, and wherein the transition from step (i) to step (j) occurs without opening the system;

(k) harvesting the therapeutic population of TILs obtained from step (j), wherein the transition from step (j) to step (k) occurs without opening the system;

(l) transferring the therapeutic population of TILs harvested in step (k) to an infusion bag, wherein the transfer from step (k) to (l) occurs without opening the system;

(m) cryopreserving the infusion bag comprising the therapeutic population of TILs from step (l) using a cryopreservation process; and

(n) providing a therapeutically effective dosage of the therapeutic population of TILs for administration from the infusion bag in step (m) to the subject.

22. The method according to claim 19, wherein the tumour tissue in step (b) is incubated for 30 minutes and the first cell culture medium in step (f) comprises OKT-3 antibody.

23. The method of claim 22, wherein the TILs in step (f):

(i) are cultured over a period of from 3 to 5 days, and wherein the TILs in step (h) are cultured over a period of from 11 to 13 days;

(ii) are cultured over a period of 3 days, and wherein the TILs in step (h) are cultured over a period of 13 days; or

(iii) are cultured over a period of 5 days, and wherein the TILs in step (h) are cultured over a period of 11 days.

24. The method of claim 22 or claim 23, further comprising (i) harvesting the TILs, optionally wherein the harvested TILs represent at least 10000-fold increase in number.

**Patentansprüche**

1. Verfahren zum Kryokonservieren von Tumorgewebe zur Fertigung von tumorinfiltrierenden Lymphozyten (TILs), wobei das Verfahren umfasst:

   (i) Fragmentieren des Tumorgewebes;
   (ii) Inkubieren der Fragmente in einem Kryokonservierungsmedium für 30 Minuten bis 80 Minuten bei 2 °C bis 8 °C; und
   (iii) Gefrieren der Fragmente, wobei es sich bei dem Gefrieren um Schockgefrieren unter Verwendung der Dampfphase von Flüssigstickstoff handelt.

2. Verfahren nach Anspruch 1, wobei das Tumorgewebe:

   (i) in ungefähr kugelförmige Fragmente mit einem Durchmesser von 1,5 mm bis 6 mm fragmentiert wird, optional, wobei die ungefähr kugelförmigen Fragmente einen Durchmesser von 6 mm oder 3 mm aufweisen;
   (ii) in im Allgemeinen rechteckige Fragmente mit einer kürzesten Kantenlänge von mindestens 1,5 mm und einer längsten Kantenlänge von 6 mm fragmentiert wird;
   (iii) in im Allgemeinen kubische Fragmente mit Kantenlängen von zwischen 1,5 mm und 6 mm fragmentiert wird, optional, wobei die kubischen Fragmente Kantenlängen von 6 mm oder 3 mm aufweisen;
   (iv) aus einem sezierten Tumor stammt, optional, wobei der sezierte Tumor weniger als acht Stunden alt ist; oder
   (v) wird in einer physiologisch gepufferten isotonischen Kochsalzlösung vor der Inkubation gewaschen wird, optional, wobei das Waschen drei serielle Wäschen von jeweils mindestens drei Minuten umfasst, wobei die physiologisch gepufferte isotonische Kochsalzlösung nach jeder seriellen Wäsche ersetzt wird.

3. Verfahren nach Anspruch 1 oder 2, wobei das Kryokonservierungsmedium umfasst:

   (i) 2 % v/v bis 15 % v/v Dimethylsulfoxid (DMSO), optional, wobei das Kryokonservierungsmedium 10 % v/v DMSO umfasst; oder
   (ii) mindestens ein antimikrobielles Mittel, optional, wobei das mindestens eine antimikrobielle Mittel Gentamicin mit einer Konzentration von mindestens 50 $\mu$g/ml ist.

4. Verfahren nach einem der Ansprüche 1 bis 3, wobei das Gefrieren bei einer Temperatur im Bereich von -125 °C bis -196 °C stattfindet, optional, wobei das Gefrieren bei einer Temperatur von -140 °C bis -175 °C stattfindet, ferner optional, wobei das Gefrieren bei -145 °C stattfindet.

5. Verfahren nach einem der Ansprüche 1 bis 4, ferner umfassend den Schritt (iv) des Aufbewahrens der gefrorenen Fragmente unterhalb von mindestens -130 °C, optional, wobei die gefrorenen Fragmente in der Dampfphase von Flüssigstickstoff aufbewahrt werden oder in Flüssigstickstoff eingetaucht aufbewahrt werden.

6. Verfahren nach einem der Ansprüche 1 bis 5, wobei die Tumorfragmente in dem Kryokonservierungsmedium für 30 Minuten bis 70 Minuten und optional für 30 Minuten bis 60 Minuten inkubiert werden.

7. Kryokonserviertes Tumorfragment zur Fertigung tumorinfiltrierender Lymphozyten (TILs), hergestellt durch einen Prozess, umfassend die Schritte:

   (i) Fragmentieren eines sezierten Tumors oder einer Tumorbiopsieprobe;
   (ii) Inkubieren der Fragmente in einem Kryokonservierungsmedium; und
   (iii) Gefrieren der Fragmente, wobei es sich bei dem Gefrieren um Schockgefrieren unter Verwendung der Dampfphase von Flüssigstickstoff handelt,

   wobei Schritt (ii) ferner das Inkubieren der Fragmente für 30 Minuten bis 60 Minuten bei 2 °C bis 8 °C in einem 10% v/v DMSO umfassenden Kryokonservierungsmedium umfasst.

8. Verfahren zur Herstellung tumorinfiltrierender Lymphozyten (TILs) für adoptive T-Zell-Therapie, wobei das Verfahren umfasst:

   (a) Aufbewahren des Tumorgewebes in einem gefrorenen Zustand, wobei das Verfahren zum Aufbewahren des Tumorgewebes umfasst:

(i) Trimmen des Tumorgewebes, um überschüssiges Nicht-Tumorgewebe zu entfernen;

(ii) Platzieren des Tumorgewebes in einem verschließbaren Gefäß, das ein Aufbewahrungsmedium enthält;

(iii) Inkubieren des Gefäßes, das das Tumorgewebe und das Aufbewahrungsmedium enthält, bei einer Temperatur von 2 bis 8 °C für einen Zeitraum von 30 Minuten bis 60 Minuten;

(iv) Gefrieren des Gefäßes, wobei es sich bei dem Gefrieren um Schockgefrieren unter Verwendung der Dampfphase von Flüssigstickstoff handelt; und

(v) Aufbewahren des Gefäßes bei oder unterhalb von -130 °C;

(b) Auftauen des Gefäßes;

(c) Behandeln des Tumorgewebes in einem gaspermeablen Behälter mit einem ersten Zellkulturmedium umfassend Interleukin 2 (IL-2) und optional OKT-3, um TILs bereitzustellen;

(d) Expandieren der TILs in einem gaspermeablen Behälter unter Verwendung eines zweiten Zellkulturmediums umfassend Zellkulturmedium, bestrahlte Feeder-Zellen, OKT-3 und IL-2, um eine expandierte Anzahl von TILs bereitzustellen.

9. Verfahren nach Anspruch 8, wobei das Tumorgewebe in Schritt (i):

(a) auf einen Durchmesser zwischen 1,5 mm und 6 mm getrimmt wird, optional, wobei das Tumorgewebe in Schritt (i) auf 6 mm mal 6 mm mal 6 mm getrimmt wird; und/oder

(b) zuerst in Hank's Balanced Salt Solution (HBSS) gewaschen wird, bevor Schritt (i) durchgeführt wird, optional, wobei das Waschen drei serielle Wäschen von jeweils mindestens drei Minuten umfasst, wobei die HBSS nach jeder seriellen Wäsche ersetzt wird.

10. Verfahren nach Anspruch 8 oder 9, wobei das Aufbewahrungsmedium ferner umfasst:

(i) ein antibakterielles Mittel und ein Antimykotikum, optional, wobei das Aufbewahrungsmedium Gentamicin mit einer Konzentration von mindestens 50 μg/ml umfasst; und/oder

(ii) 2 % v/v bis 12 % v/v Dimethylsulfoxid (DMSO), optional, wobei das Aufbewahrungsmedium 10 % DMSO umfasst.

11. Verfahren nach einem der Ansprüche 8 bis 10, wobei Schritt (b) das Eintauchen des Gefäßes in Wasserbad bei einer Temperatur von 37 °C für 5 Minuten umfasst.

12. Verfahren nach einem der Ansprüche 1 bis 6 oder Ansprüche 8 bis 11, wobei das Tumorgewebe ausgewählt ist aus der Gruppe bestehend aus Melanomtumorgewebe, Kopf-Hals-Tumorgewebe, Brusttumorgewebe, Nierentumorgewebe, Bauchspeicheldrüsentumorgewebe, Glioblastomtumorgewebe, Lungentumorgewebe, kolorektalem Tumorgewebe, Sarkomtumorgewebe, dreifach negativem Brusttumorgewebe, Gebärmutterhalstumorgewebe, Eierstocktumorgewebe oder HPV-positivem Tumorgewebe.

13. Verfahren nach einem der Ansprüche 8 bis 12, wobei das Verfahren ferner eine Aufteilung zwischen Schritt (c) und Schritt (d) umfasst, optional, wobei die Aufteilung 16 Tage nach Einleitung von Schritt (c) erfolgt.

14. Verfahren nach einem der Ansprüche 8 bis 13, wobei der erste Kulturschritt 11 Tage beträgt oder wobei Schritte (c) bis (d) über einen Zeitraum von 22 Tagen oder über einen Zeitraum von weniger als 20 Tagen durchgeführt werden.

15. Verfahren nach einem der Ansprüche 8 bis 14, wobei:

(a) Schritt (iv) das Gefrieren des Gefäßes bei -125 °C bis -150 °C umfasst;

(b) Schritt (v) das Aufbewahren des Gefäßes unterhalb von mindestens -130 °C umfasst; oder

(c) Schritt (v) das Aufbewahren des in Flüssigstickstoff eingetauchten Gefäßes umfasst.

16. Therapeutische Population von TILs zur Verwendung in einem Verfahren zum Behandeln eines Subjekts mit Krebs, wobei das Verfahren das Bereitstellen expandierter tumorinfiltrierender Lymphozyten (TILs) zur Verabreichung umfasst, die durch ein Verfahren hergestellt werden, das Folgendes umfasst:

(a) Aufbewahren des Tumorgewebes in einem gefrorenen Zustand, wobei das Verfahren zum Aufbewahren des Tumorgewebes umfasst:

(i) Trimmen des Tumorgewebes, um überschüssiges Nicht-Tumorgewebe zu entfernen;
(ii) Platzieren des Tumorgewebes in einem verschließbaren Gefäß, das ein Aufbewahrungsmedium enthält;
(iii) Inkubieren des Gefäßes, das das Tumorgewebe und das Aufbewahrungsmedium enthält, bei 2 - 8 °C für 30 Minuten;
(iv) Schockgefrieren des Gefäßes unter Verwendung der Dampfphase von Flüssigstickstoff; und
(v) Aufbewahren des Gefäßes bei der Temperatur der Dampfphase von Flüssigstickstoff;

(b) Auftauen des Tumorgewebes;
(c) Hineingeben der Tumorfragmente in ein geschlossenes System;
(d) Durchführen einer ersten Expansion durch Kultivieren der ersten Population von TILs in einem Zellkulturmedium, das IL-2 umfasst, um eine zweite Population von TILs zu erzeugen, wobei die erste Expansion in einem geschlossenen Behälter durchgeführt wird, der eine erste gaspermeable Oberflächen bereitstellt, wobei die erste Expansion für 3-14 Tage durchgeführt wird, um die zweite Population von TILs zu erhalten, wobei die zweite Population von TILs zahlenmäßig mindestens 50-mal größer als die erste Population von TILs ist, und wobei der Übergang von Schritt (c) zu Schritt (d) ohne Öffnen des Systems erfolgt;
(e) Durchführen einer zweiten Expansion durch Ergänzen des Zellkulturmediums der zweiten Population von TILs mit zusätzlichem IL-2, OKT-3 und antigenpräsentierenden Zellen (APCs), um eine dritte Population von TILs zu erzeugen, wobei die zweite Expansion für 7-14 Tage durchgeführt wird, um die dritte Population von TILs zu erhalten, wobei die dritte Population von TILs eine therapeutische Population von TILs ist, die eine erhöhte Subpopulation von Effektor-T-Zellen und/oder zentralen Gedächtnis-T-Zellen relativ zu der zweiten Population von TILs umfasst, wobei die zweite Expansion in einem geschlossenen Behälter durchgeführt wird, der eine zweite gaspermeable Oberfläche bereitstellt, und wobei der Übergang von Schritt (d) zu Schritt (e) ohne Öffnen des Systems erfolgt;
(f) Ernten der aus Schritt (e) erhaltenen therapeutischen Population von TILs, wobei der Übergang von Schritt (e) zu Schritt (f) ohne Öffnen des Systems erfolgt;
(g) Übertragen der geernteten TIL-Population aus Schritt (f) auf einen Infusionsbeutel, wobei die Übertragung von Schritt (f) zu (g) ohne Öffnen des Systems erfolgt;
(h) Kryokonservieren des Infusionsbeutels, der die geerntete TIL-Population aus Schritt (g) umfasst, unter Verwendung eines Kryokonservierungsprozesses; und
(i) Bereitstellen einer therapeutisch wirksamen Dosierung der dritten Population von TILs zur Verabreichung aus dem Infusionsbeutel in Schritt (h) an das Subjekt.

**17.** Therapeutische Population von TILs nach Anspruch 16, wobei das Verfahren zum Behandeln des Subjekts mit Krebs ferner die Schritte des (j) gleichzeitigen Bereitstellens einer therapeutisch wirksamen Dosierung von Aldesleukin oder eines Biosimilars davon zur Verabreichung an das Subjekt; und (k) gleichzeitigen Bereitstellens einer therapeutisch wirksamen Dosierung eines PD-1/PD-L1-Inhibitors zur Verabreichung an das Subjekt, optional, wobei der PD-1/PD-L1-Inhibitor ausgewählt ist aus der Gruppe bestehend aus Pembrolizumab, Nivolumab, Avelumab, Durvalumab, Atezolizumab und Biosimilars davon, umfasst.

**18.** Therapeutische Population von TILs nach Anspruch 16 oder Anspruch 17, wobei der Krebs ausgewählt ist aus der Gruppe bestehend aus Melanom, Gebärmutterhalskrebs, Kopf-Hals-Plattenepithelkrebs, nicht-kleinzelligem Lungenkrebs, Blasenkrebs, Eierstockkrebs, Bauchspeicheldrüsenkrebs und Sarkom.

**19.** Verfahren zum Expandieren tumorinfiltrierender Lymphozyten (TILs) aus gefrorenem Tumorgewebe, umfassend:

(a) Bereitstellen von Tumorgewebe, das aus einem Patienten erhalten wurde;
(b) Inkubieren des Tumorgewebes für mindestens 30 Minuten in einem Aufbewahrungsmedium bei einer Temperatur im Bereich von 2 °C bis 8 °C;
(c) Schockgefrieren des Tumorgewebes;
(d) Aufbewahren des Tumorgewebes in einem gefrorenen Zustand;
(e) Auftauen des Tumorgewebes;
(f) Behandeln des Tumorgewebes in einem gaspermeablen Behälter mit einem ersten Zellkulturmedium und Interleukin 2 (IL-2), um TILs bereitzustellen;
(g) Entfernen mindestens einer Vielzahl der TILs; und
(h) Expandieren der TILs mit einem zweiten Zellkulturmedium umfassend Zellkulturmedien, bestrahlte Feeder-Zellen, OKT-3-Antikörper und IL-2 in einem gaspermeablen Behälter, um eine expandierte Anzahl von TILs bereitzustellen.

20. Verfahren zum Expandieren von TILs aus gefrorenem Tumorgewebe, umfassend:

   (a) Bereitstellen von Tumorgewebe, das aus einem Patienten erhalten wurde;
   (b) Trimmen des Tumorgewebes, um überschüssiges Nicht-Tumorgewebe zu entfernen;
   (c) Platzieren des Tumorgewebes in einem verschließbaren Gefäß, das ein Aufbewahrungsmedium enthält;
   (d) Inkubieren des Gefäßes, das das Tumorgewebe und das Aufbewahrungsmedium enthält, bei einer Temperatur im Bereich von 2 °C bis 8 °C für 20 Minuten bis 70 Minuten;
   (e) Schockgefrieren des Gefäßes;
   (f) Aufbewahren des Gefäßes derart, dass das Gefäß gefroren bleibt;
   (g) Auftauen des Gefäßes;
   (h) Behandeln des Tumorgewebes in einem gaspermeablen Behälter mit einem ersten Zellkulturmedium und Interleukin 2 (IL-2), um TILs bereitzustellen;
   (i) Entfernen mindestens einer Vielzahl der TILs; und
   (j) Expandieren der TILs mit einem zweiten Zellkulturmedium umfassend Zellkulturmedien, bestrahlte Feeder-Zellen, OKT-3 und IL-2 in einem gaspermeablen Behälter, um eine expandierte Anzahl von TILs bereitzustellen.

21. Therapeutische Population von TILs zur Verwendung in einem Verfahren zum Behandeln von Krebs bei einem menschlichen Subjekt mit Bedarf daran, wobei das Verfahren das Bereitstellen expandierter tumorinfiltrierender Lymphozyten (TILs) zur Verabreichung umfasst, die durch ein Verfahren hergestellt werden, das Folgendes umfasst:

   (a) Bereitstellen von Tumorgewebe, das aus dem Subjekt erhalten wurde;
   (b) Trimmen des Tumorgewebes, um überschüssiges Nicht-Tumorgewebe zu entfernen;
   (c) Platzieren des Tumorgewebes in einem verschließbaren Gefäß, das ein Aufbewahrungsmedium enthält;
   (d) Inkubieren des Gefäßes, das das Tumorgewebe und das Aufbewahrungsmedium enthält, bei einer Temperatur im Bereich von 2 °C bis 8 °C für 20 Minuten bis 70 Minuten;
   (e) Schockgefrieren des Gefäßes unter Verwendung der Dampfphase von Flüssigstickstoff;
   (f) Aufbewahren des Gefäßes bei der Temperatur der Dampfphase von Flüssigstickstoff;
   (g) Auftauen des Tumorgewebes;
   (h) Hineingeben des Tumorgewebes in ein geschlossenes System;
   (i) Durchführen einer ersten Expansion durch Kultivieren der ersten Population von TILs aus dem Tumorgewebe in einem Zellkulturmedium, das IL-2 umfasst, um eine zweite Population von TILs zu erzeugen, wobei die erste Expansion in einem geschlossenen Behälter durchgeführt wird, der eine erste gaspermeable Oberfläche bereitstellt, wobei die erste Expansion für 3-14 Tage durchgeführt wird, um die zweite Population von TILs zu erhalten, wobei die zweite Population von TILs zahlenmäßig mindestens 50-mal größer als die erste Population von TILs ist, und wobei der Übergang von Schritt (h) zu Schritt (i) ohne Öffnen des Systems erfolgt;
   (j) Durchführen einer zweiten Expansion durch Ergänzen des Zellkulturmediums der zweiten Population von TILs mit zusätzlichem IL-2, OKT-3 und antigenpräsentierenden Zellen (APCs), um eine dritte Population von TILs zu erzeugen, wobei die zweite Expansion für 7-14 Tage durchgeführt wird, um die dritte Population von TILs zu erhalten, wobei die dritte Population von TILs eine therapeutische Population von TILs ist, die eine erhöhte Subpopulation von Effektor-T-Zellen und/oder zentralen Gedächtnis-T-Zellen relativ zu der zweiten Population von TILs umfasst, wobei die zweite Expansion in einem geschlossenen Behälter durchgeführt wird, der eine zweite gaspermeable Oberfläche bereitstellt, und wobei der Übergang von Schritt (i) zu Schritt (j) ohne Öffnen des Systems erfolgt;
   (k) Ernten der aus Schritt (j) erhaltenen therapeutischen Population von TILs, wobei der Übergang von Schritt (j) zu Schritt (k) ohne Öffnen des Systems erfolgt;
   (l) Übertragen der in Schritt (k) geernteten therapeutischen Population von TILs auf einen Infusionsbeutel, wobei die Übertragung von Schritt (k) zu (l) ohne Öffnen des Systems erfolgt;
   (m) Kryokonservieren des Infusionsbeutels, der die therapeutische TIL-Population aus Schritt (l) umfasst, unter Verwendung eines Kryokonservierungsprozesses; und
   (n) Bereitstellen einer therapeutisch wirksamen Dosierung der therapeutischen Population von TILs zur Verabreichung aus dem Infusionsbeutel in Schritt (m) an das Subjekt.

22. Verfahren nach Anspruch 19, wobei das Tumorgewebe in Schritt (b) für 30 Minuten inkubiert wird und das erste Zellkulturmedium in Schritt (f) OKT-3-Antikörper umfasst.

23. Verfahren nach Anspruch 22, wobei die TILs in Schritt (f):

   (i) über einen Zeitraum von 3 bis 5 Tagen kultiviert werden und wobei die TILs in Schritt (h) über einen Zeitraum

EP 3 852 524 B1

von 11 bis 13 Tagen kultiviert werden;

(ii) über einen Zeitraum von 3 Tagen kultiviert werden und wobei die TILs in Schritt (h) über einen Zeitraum von 13 Tagen kultiviert werden; oder

(iii) über einen Zeitraum von 5 Tagen kultiviert werden und wobei die TILs in Schritt (h) über einen Zeitraum von 11 Tagen kultiviert werden.

24. Verfahren nach Anspruch 22 oder Anspruch 23, ferner umfassend (i) Ernten der TILs, optional, wobei die geernteten TILs eine mindestens 10000-fache Erhöhung der Anzahl darstellen.

**Revendications**

1. Méthode de cryoconservation d'un tissu tumoral pour la fabrication de lymphocytes infiltrant les tumeurs (TIL), la méthode comprenant :

(i) la fragmentation du tissu tumoral ;

(ii) l'incubation des fragments dans un milieu de cryoconservation pendant 30 minutes à 80 minutes à une température de 2°C à 8°C ; et,

(iii) la congélation des fragments, ladite congélation étant une congélation instantanée à l'aide de la phase vapeur d'azote liquide.

2. Méthode selon la revendication 1, ledit tissu tumoral :

(i) étant fragmenté en fragments approximativement sphériques comportant un diamètre de 1,5 mm à 6 mm, éventuellement lesdits fragments approximativement sphériques comportant un diamètre de 6 mm ou 3 mm ;

(ii) étant fragmenté en fragments généralement rectangulaires comportant une longueur de bord la plus courte d'au moins 1,5 mm et une longueur de bord la plus longue de 6 mm ;

(iii) étant fragmenté en fragments généralement cubiques comportant des longueurs de bord comprises entre 1,5 mm et 6 mm, éventuellement lesdits fragments cubiques comportant des longueurs de bord de 6 mm ou 3 mm ;

(iv) provenant d'une tumeur disséquée, éventuellement ladite tumeur disséquée datant de moins de huit heures ; ou

(v) étant lavé dans une solution saline isotonique physiologiquement tamponnée avant incubation, éventuellement ledit lavage comprenant trois lavages en série d'au moins trois minutes chacun, la solution saline isotonique physiologiquement tamponnée étant remplacée après chaque lavage en série.

3. Méthode selon la revendication 1 ou 2, ledit milieu de cryoconservation comprenant :

(i) 2 % en v/v à 15 % en v/v de diméthylsulfoxyde (DMSO), éventuellement ledit milieu de cryoconservation comprenant 10 % en v/v de DMSO ; ou

(ii) au moins un agent antimicrobien, éventuellement ledit au moins un agent antimicrobien étant la gentamicine à une concentration d'au moins 50 μg/ml.

4. Méthode selon l'une quelconque des revendications 1 à 3, ladite congélation ayant lieu à une température comprise dans la plage de -125°C à -196°C, éventuellement ladite congélation ayant lieu à une température de -140°C à -175°C, en outre éventuellement ladite congélation ayant lieu à-145°C.

5. Méthode selon l'une quelconque des revendications 1 à 4, comprenant en outre l'étape de (iv) stockage des fragments congelés en dessous d'au moins -130°C, éventuellement lesdits fragments congelés étant stockés dans la phase vapeur d'azote liquide ou étant stockés immergés dans l'azote liquide.

6. Méthode selon l'une quelconque des revendications 1 à 5, lesdits fragments tumoraux étant incubés dans le milieu de cryoconservation pendant 30 minutes à 70 minutes et éventuellement pendant 30 minutes à 60 minutes.

7. Fragment tumoral cryoconservé pour la fabrication de lymphocytes infiltrant les tumeurs (TIL), préparé par une méthode comprenant les étapes de :

(i) fragmentation d'une tumeur disséquée ou d'un échantillon de biopsie tumorale ;

135

(ii) incubation des fragments dans un milieu de cryoconservation ; et,
(iii) congélation des fragments, ladite congélation étant une congélation instantanée à l'aide de la phase vapeur d'azote liquide,

ladite étape (ii) comprenant en outre l'incubation des fragments pendant 30 minutes à 60 minutes à une température de 2°C à 8°C dans un milieu de cryoconservation comprenant 10 % en v/v de DMSO.

8. Méthode permettant la préparation de lymphocytes infiltrant les tumeurs (TIL) pour une thérapie adoptive par lymphocytes T, la méthode comprenant :

(a) le stockage du tissu tumoral dans un état congelé, la méthode de stockage du tissu tumoral comprenant :

(i) la coupe du tissu tumoral pour retirer le tissu non tumoral en excès ;
(ii) le placement du tissu tumoral dans un récipient refermable contenant un milieu de stockage ;
(iii) l'incubation du récipient, qui contient le tissu tumoral et le milieu de stockage, à une température de 2 à 8°C pendant une durée de 30 minutes à 60 minutes ;
(iv) la congélation du récipient, la congélation étant une congélation instantanée à l'aide de la phase vapeur d'azote liquide ; et
(v) le stockage du récipient à ou en dessous de -130°C ;

(b) la décongélation du récipient ;
(c) le traitement du tissu tumoral dans un récipient perméable aux gaz avec un premier milieu de culture cellulaire comprenant de l'interleukine 2 (IL-2) et éventuellement de l'OKT-3 pour fournir des TIL ;
(d) l'expansion des TIL dans un récipient perméable aux gaz à l'aide d'un second milieu de culture cellulaire comprenant un milieu de culture cellulaire, des cellules nourricières irradiées, de l'OKT-3 et de l'IL-2, pour fournir un nombre accru de TIL.

9. Méthode selon la revendication 8, ledit tissu tumoral à l'étape (i) étant :

(a) coupé à un diamètre compris entre 1,5 mm et 6 mm, éventuellement ledit tissu tumoral à l'étape (i) étant coupé à 6 mm sur 6 mm sur 6 mm ; et/ou
(b) d'abord lavé dans une solution saline équilibrée de Hank (HBSS) avant d'effectuer l'étape (i), éventuellement ledit lavage comprenant trois lavages en série d'au moins trois minutes chacun, le HBSS étant remplacé après chaque lavage en série.

10. Méthode selon la revendication 8 ou 9, ledit milieu de stockage comprenant en outre :

(i) un agent antibactérien et un agent antifongique, éventuellement ledit milieu de stockage comprenant de la gentamicine à une concentration d'au moins 50 µg/ml ; et/ou
(ii) 2 % en v/v à 12 % en v/v de diméthylsulfoxyde (DMSO), éventuellement ledit milieu de stockage comprenant 10 % de DMSO.

11. Méthode selon l'une quelconque des revendications 8 à 10, ladite étape (b) comprenant l'immersion du récipient dans un bain d'eau à une température de 37°C pendant 5 minutes.

12. Méthode selon l'une quelconque des revendications 1 à 6 ou des revendications 8 à 11, ledit tissu tumoral étant choisi dans le groupe constitué par un tissu tumoral de mélanome, un tissu tumoral de la tête et du cou, un tissu tumoral mammaire, un tissu tumoral rénal, un tissu tumoral pancréatique, un tissu tumoral de glioblastome, un tissu tumoral pulmonaire, un tissu tumoral colorectal, un tissu tumoral de sarcome, un tissu tumoral mammaire triple négatif, un tissu tumoral du col de l'utérus, un tissu tumoral ovarien ou un tissu tumoral positif au VPH.

13. Méthode selon l'une quelconque des revendications 8 à 12, la méthode comprenant en outre une séparation entre l'étape (c) et l'étape (d), éventuellement ladite séparation se produisant 16 jours après le début de l'étape (c).

14. Méthode selon l'une quelconque des revendications 8 à 13, ladite première étape de culture durant 11 jours, ou lesdites étapes (c) à (d) étant réalisées sur une période de 22 jours, ou sur une période inférieure à 20 jours.

15. Méthode selon l'une quelconque des revendications 8 à 14 :

(a) ladite étape (iv) comprenant la congélation du récipient à une température de -125 °C à -150°C ;
(b) ladite étape (v) comprenant le stockage du récipient en dessous d'au moins -130°C ; ou
(c) ladite étape (v) comprenant le stockage du récipient immergé dans de l'azote liquide.

16. Population thérapeutique de TIL destinée à être utilisée dans une méthode permettant le traitement d'un sujet atteint d'un cancer, la méthode comprenant la fourniture de lymphocytes infiltrant les tumeurs (TIL) expansés pour l'administration produits par une méthode comprenant :

(a) le stockage du tissu tumoral dans un état congelé, la méthode de stockage du tissu tumoral comprenant :

(i) la coupe du tissu tumoral pour retirer le tissu non tumoral en excès ;
(ii) le placement du tissu tumoral dans un récipient refermable contenant un milieu de stockage ;
(iii) l'incubation du récipient, qui contient le tissu tumoral et le milieu de stockage, à une température de 2 à 8°C pendant 30 minutes ;
(iv) la congélation instantanée du récipient à l'aide de la phase vapeur d'azote liquide ; et
(v) le stockage du récipient à la température de la phase vapeur d'azote liquide,

(b) la décongélation du tissu tumoral ;
(c) l'ajout des fragments de tumeur dans un système fermé ;
(d) la réalisation d'une première expansion en cultivant la première population de TIL dans un milieu de culture cellulaire comprenant de l'IL-2 pour produire une deuxième population de TIL, ladite première expansion étant effectuée dans un récipient fermé fournissant une première surface perméable aux gaz, ladite première expansion étant effectuée pendant 3 à 14 jours pour obtenir la deuxième population de TIL, ladite deuxième population de TIL étant au moins 50 fois plus grande en nombre que la première population de TIL, et ladite transition de l'étape (c) à l'étape (d) se produisant sans ouvrir le système ;
(e) la réalisation d'une seconde expansion en complétant le milieu de culture cellulaire de la deuxième population de TIL avec un complément d'IL-2, de l'OKT-3 et des cellules présentant des antigènes (APC), pour produire une troisième population de TIL, ladite seconde expansion étant effectuée pendant 7 à 14 jours pour obtenir la troisième population de TIL, ladite troisième population de TIL étant une population thérapeutique de TIL qui comprend une sous-population accrue de lymphocytes T effecteurs et/ou de lymphocytes T de mémoire centrale par rapport à la deuxième population de TIL, ladite seconde expansion étant effectuée dans un récipient fermé fournissant une seconde surface perméable aux gaz, et ladite transition de l'étape (d) à l'étape (e) se produisant sans ouvrir le système ;
(f) la récolte de la population thérapeutique de TIL obtenue à l'étape (e), ladite transition de l'étape (e) à l'étape (f) se produisant sans ouvrir le système ;
(g) le transfert de la population de TIL récoltée de l'étape (f) vers une poche de perfusion, ledit transfert de l'étape (f) à l'étape (g) se produisant sans ouvrir le système ;
(h) la cryoconservation de la poche de perfusion comprenant la population de TIL récoltée de l'étape (g) à l'aide d'un procédé de cryoconservation ; et
(i) la fourniture d'une dose thérapeutiquement efficace de la troisième population de TIL pour l'administration à partir de la poche de perfusion à l'étape (h) au sujet.

17. Population thérapeutique de TIL selon la revendication 16, ladite méthode permettant le traitement du sujet atteint d'un cancer comprenant en outre les étapes de (j) fourniture concomitante d'une dose thérapeutiquement efficace d'aldesleukine ou d'un biosimilaire de celle-ci pour l'administration au sujet ; et (k) fourniture concomitante d'une dose thérapeutiquement efficace d'un inhibiteur de PD-1/PD-L1 pour l'administration au sujet, éventuellement ledit inhibiteur de PD-1/PD-L1 étant choisi dans le groupe constitué par le pembrolizumab, le nivolumab, l'avélumab, le durvalumab, l'atézolizumab et leurs biosimilaires.

18. Population thérapeutique de TIL selon la revendication 16 ou la revendication 17, ledit cancer étant choisi dans le groupe constitué par un mélanome, le cancer du col de l'utérus, le cancer à cellules squameuses de la tête et du cou, le cancer du poumon non à petites cellules, le cancer de la vessie, le cancer de l'ovaire, le cancer du pancréas, et un sarcome.

19. Méthode permettant l'expansion de lymphocytes infiltrant les tumeurs (TIL) à partir d'un tissu tumoral congelé comprenant :

(a) la fourniture d'un tissu tumoral prélevé chez un patient ;

(b) l'incubation du tissu tumoral pendant au moins 30 minutes dans un milieu de stockage à une température comprise dans la plage de 2°C à 8°C ;

(c) la congélation instantanée du tissu tumoral ;

(d) le stockage du tissu tumoral dans un état congelé ;

(e) la décongélation du tissu tumoral ;

(f) le traitement du tissu tumoral dans un récipient perméable aux gaz avec un premier milieu de culture cellulaire et de l'interleukine 2 (IL-2) pour fournir des TIL ;

(g) le retrait d'au moins une pluralité des TIL ; et

(h) l'expansion des TIL avec un second milieu de culture cellulaire comprenant des milieux de culture cellulaire, des cellules nourricières irradiées, un anticorps OKT-3 et de l'IL-2 dans un récipient perméable aux gaz pour fournir un nombre accru de TIL.

**20.** Méthode permettant l'expansion de TIL à partir d'un tissu tumoral congelé comprenant :

(a) la fourniture d'un tissu tumoral prélevé chez un patient ;

(b) la coupe du tissu tumoral pour retirer le tissu non tumoral en excès ;

(c) le placement du tissu tumoral dans un récipient refermable contenant un milieu de stockage ;

(d) l'incubation du récipient, qui contient le tissu tumoral et le milieu de stockage, à une température comprise dans la plage de 2°C à 8°C pendant 20 minutes à 70 minutes ;

(e) la congélation instantanée du récipient ;

(f) le stockage du récipient de sorte que le récipient reste congelé ;

(g) la décongélation du récipient ;

(h) le traitement du tissu tumoral dans un récipient perméable aux gaz avec un premier milieu de culture cellulaire et de l'interleukine 2 (IL-2) pour fournir des TIL ;

(i) le retrait d'au moins une pluralité des TIL ; et

(j) l'expansion des TIL avec un second milieu de culture cellulaire comprenant des milieux de culture cellulaire, des cellules nourricières irradiées, de l'OKT-3 et de l'IL-2 dans un récipient perméable aux gaz pour fournir un nombre accru de TIL.

**21.** Population thérapeutique de TIL destinée à être utilisée dans une méthode de traitement d'un cancer pour un sujet humain en ayant besoin, la méthode comprenant la fourniture de lymphocytes infiltrant les tumeurs (TIL) expansés pour l'administration produits par une méthode comprenant :

(a) la fourniture d'un tissu tumoral prélevé chez un sujet ;

(b) la coupe du tissu tumoral pour retirer le tissu non tumoral en excès ;

(c) le placement du tissu tumoral dans un récipient refermable contenant un milieu de stockage ;

(d) l'incubation du récipient, qui contient le tissu tumoral et le milieu de stockage, à une température comprise dans la plage de 2°C à 8°C pendant 20 minutes à 70 minutes ;

(e) la congélation instantanée du récipient à l'aide de la phase vapeur d'azote liquide ;

(f) le stockage du récipient à la température de la phase vapeur d'azote liquide ;

(g) la décongélation du tissu tumoral ;

(h) l'ajout du tissu tumoral dans un système fermé ;

(i) la réalisation d'une première expansion en cultivant une première population de TIL provenant du tissu tumoral dans un milieu de culture cellulaire comprenant de l'IL-2 pour produire une deuxième population de TIL, ladite première expansion étant effectuée dans un récipient fermé fournissant une première surface perméable aux gaz, ladite première expansion étant effectuée pendant 3 à 14 jours pour obtenir la deuxième population de TIL, ladite deuxième population de TIL étant au moins 50 fois plus grande en nombre que la première population de TIL, et ladite transition de l'étape (h) à l'étape (i) se produisant sans ouvrir le système ;

(j) la réalisation d'une seconde expansion en complétant le milieu de culture cellulaire de la deuxième population de TIL avec un complément d'IL-2, de l'OKT-3 et des cellules présentant des antigènes (APC), pour produire une troisième population de TIL, ladite seconde expansion étant effectuée pendant 7 à 14 jours pour obtenir la troisième population de TIL, ladite troisième population de TIL étant une population thérapeutique de TIL qui comprend une sous-population accrue de lymphocytes T effecteurs et/ou de lymphocytes T de mémoire centrale par rapport à la deuxième population de TIL, ladite seconde expansion étant effectuée dans un récipient fermé fournissant une seconde surface perméable aux gaz, et ladite transition de l'étape (i) à l'étape (j) se produisant sans ouvrir le système ;

(k) la récolte de la population thérapeutique de TIL obtenue à l'étape (j), ladite transition de l'étape (j) à l'étape (k) se produisant sans ouvrir le système ;

(l) le transfert de la population thérapeutique de TIL récoltée à l'étape (k) dans une poche de perfusion, ledit transfert de l'étape (k) à (l) se produisant sans ouvrir le système ;

(m) la cryoconservation de la poche de perfusion comprenant la population thérapeutique de TIL de l'étape (l) à l'aide d'un procédé de cryoconservation ; et

(n) la fourniture d'une dose thérapeutiquement efficace de la population thérapeutique de TIL pour l'administration à partir de la poche de perfusion à l'étape (m) au sujet.

22. Méthode selon la revendication 19, ledit tissu tumoral à l'étape (b) étant incubé pendant 30 minutes et ledit premier milieu de culture cellulaire à l'étape (f) comprenant l'anticorps OKT-3.

23. Méthode selon la revendication 22, lesdits TIL à l'étape (f) :

(i) étant cultivés sur une période allant de 3 à 5 jours, et lesdits TIL à l'étape (h) étant cultivés sur une période allant de 11 à 13 jours ;

(ii) étant cultivés sur une période de 3 jours, et lesdits TIL à l'étape (h) étant cultivés sur une période de 13 jours ; ou

(iii) étant cultivés sur une période de 5 jours, et lesdits TIL à l'étape (h) étant cultivés sur une période de 11 jours.

24. Méthode selon la revendication 22 ou la revendication 23, comprenant en outre (i) la récolte des TIL, éventuellement lesdits TIL récoltés représentant une augmentation d'au moins 10 000 fois en nombre.

# Figure 1

**Process 2A: about 22 days from initiation of First
Expansion – Step E**

### 1. STEP A

Obtain Patient Tumor Sample

### 2. STEP B

Fragmentation
Cryopreservation of Tumor Tissue Fragments
First Expansion
3 days to 14 days

### 3. STEP C

First Expansion to Second Expansion Transition

No Storage and Closed System

### 4. STEP D

Second Expansion

IL-2, OKT-3, and antigen-presenting feeder cells

Closed System

### 5. STEP E

Harvest TILS from Step D

Closed System

### 6. STEP F

Final Formulation and/or Transfer to Infusion Bag

(optionally cryopreserve)

# Figure 2A

# Figure 2B

# Figure 2C

Figure 2B

Spent Medium removal ≤ 5x G-Rex 500MCS → supernate → Pool supernate 10L Bioprocess bag(s) → Mycoplasma PCR Sample Diluent

3L Cell culture Bag (Origen EV3000 or equivalent)

Cell Count & Viability NC-200 4x 1mL sample

Wash Buffer PL-A + 1% HSA → Volume Reduction LOVO 2 Cycles, 10:1 concentration 4000 RPM, 75mL/min → Formulate 1:1 with cold CS10*

IL-2 300 IU/mL → Add IL-2

*Formulate bulk Product Bag stored at 2-8°C in between processing steps

Satellite Vial Prep

Cryopreserved Satellite Vial Sample 10 x 0.5mL/vial ← Remove QC Release Test Samples & Air (Mycoplasma, Endotoxin, Sterility, Gram Stain, Cell Count, viability, flow, INF-g, Retain) (20mL) ← Aliquot into CS750 cryobags 90-120mL/bag

Manual Fill 0.5 mL / vial → Controlled Rate Freeze Preset-6 Profile

Visually Inspect

Store in vapor phase LN

Ship*

*Figure 3*

**Figure 4**

# Figure 5

| Process 1C<br>43 – 55 Days for Steps A - E | Process 2A<br>About 22 Days from initiation of preREP to Step E |
|---|---|
| **1. Step A**<br>Obtain Patient Tumor Sample | **1. Step A**<br>Obtain Patient Tumor Sample |
| **2. Step B**<br>Fragmentation and First Expansion<br>11 days to 21 days | **2. Step B**<br>Fragmentation, optional cryopreservation of tumor fragments, and then First Expansion<br>11 days to 21 days<br>(beginning from start of<br>first expansion culture) |
| **3. Step C**<br>First Expansion to Second Expansion Transition<br>Optional Storage until Selection | **3. Step C**<br>First Expansion to Second Expansion Transition<br>No Storage and Closed System |
| **4. Step D**<br>Second Expansion<br>IL-2, OKT-3, antigen-presenting feeder cells<br>Optionally repeat one or more times | **4. Step D**<br>Second Expansion<br>IL-2, OKT-3, antigen-presenting feeder cells<br>Closed System |
| **5. Step E**<br>Harvest TILs from Step D | **5. Step E**<br>Harvest TILs from Step D<br>Closed System |
| **6. Step F**<br>Final Formulation and/or Transfer to Infusion Bag | **6. Step F**<br>Final Formulation and/or Transfer to Infusion Bag<br>Optional cryopreservation |

# Figure 6

| Process Step | Process 1C Embodiment | Process 2A Embodiment | Advantages |
|---|---|---|---|
| Pre-REP | • 4 fragments per 10 GREX-10 flasks<br><br>• 11-21 day duration | • 40 fragments per 1 GREX-100M flask<br><br>• 11 day duration | • Increased tumor fragments per flask<br>• Shortened culture time<br>• Reduced number of steps<br>• Amenable to closed system |
| Pre-REP to REP Transition | • Pre-REP TIL are frozen until phenotyped for selection then thawed to proceed to the REP (~day 30)<br><br>• REP requires >40×10$^9$ TIL | • Pre-REP TIL directly move to REP on day 11<br><br>• REP requires 25-200×10$^6$ TIL | • Shortened pre-REP-to-REP process<br><br>• Reduced number of steps<br><br>• Eliminated phenotyping selection<br>• Amenable to closed system |
| REP | • 6 GREX-100M flasks on REP day 0<br>• 5×10$^6$ TIL and 5×10$^8$ PBMC feeders per flask on REP day 0<br>• Split to 18-36 flasks on REP day 7<br>• 14 day duration | • 1 GREX-500M flask on day 11<br>• 25-200×10$^6$ TIL and 5x10$^9$ PBMC feeders on day 11<br>• Split to ≤ 6 GREX-500M flasks on day 16<br>• 11 day duration | • Reduced number of steps<br><br>• Shorter REP duration<br><br>• Closed system transfer of TIL between flasks<br><br>• Closed system media exchanges |
| Harvest | • TIL harvested via centrifugation | • TIL harvested via LOVO automated cell washing system | • Reduced number of steps<br>• Automated cell washing<br>• Closed system<br>• Reduced loss of product during wash |
| Final Formulation | • Fresh product in Hypothermosol<br><br>• Single infusion bag<br>• Limited shipping stability | • Cryopreserved product in PlasmaLyte-A + 1% HSA and CS10 stored in LN$_2$<br>• Multiple aliquots<br>• Longer shipping stability | • Shipping flexibility<br><br>• Flexible patient scheduling<br>• More timely release testing |
| Overall Estimated Process Time | • 43-55 days | • 22 days | • Faster turnaround to patient |

Figure 7

## Figure 8

- Pre-REP TVC from Gen2 were 100e6 on Day 11

- Pre-REP TVC from Gen 2.2 were 0.8e6 on Day 7

- Pre-REP yield from Gen 2.2 were ~ 1% of Gen 2 control

| PreREP | Gen 2 (Day 11) | Gen 2.2 (Day 7) |
|---|---|---|
| 24h post HTS | 1.00E+08 | 7.83E+06 |
| 0 min | 7.00E+05 | 6.69E+05 |
| 30 min | 5.00E+05 | 1.47E+06 |
| 60 min | 0.00E+00 | 1.91E+05 |

| PreREP | Gen 2 (Day 11) | Gen 2.2 (Day 7) |
|---|---|---|
| 24h post HTS | 1.00E+08 | -- |
| 0 min | -- | 6.69E+05 |
| 30 min | -- | 1.47E+06 |
| 60 min | -- | 1.91E+05 |

EP 3 852 524 B1

# Figure 9

- Total REP TVC from Gen 2 were 2.5e9 on Day 22

- Total REP TVC from Gen 2.2 were 2.2, 2, 2, e9 on Day 21

- Total REP yield from Gen 2.2 were ~ 86% of Gen 2

| REP | Gen 2 (Day 22) | Gen 2.2 (Day 21) |
|---|---|---|
| 24h post HTS | 2.47E+09 | 4.89E+09 |
| 0 min | 4.20E+08 | 2.26E+09 |
| 30 min | 5.89E+08 | 1.97E+09 |
| 60 min | 2.28E+08 | 2.12E+09 |

| REP | Gen 2 (Day 22) | Gen 2.2 (Day 21) |
|---|---|---|
| 24h post HTS | 2.47E+09 | - |
| 0 min | - | 2.26E+09 |
| 30 min | - | 1.97E+09 |
| 60 min | - | 2.12E+09 |

# Figure 10

- REP TIL from Gen2 produced 6180 pg/mL IFNγ on Day 22

- REP TIL from Gen2.2 produced 10520, 12612, 11618 pg/mL IFNγ on Day 22

**Figure 11**
Tumor Processing
Example 8

Gen 2 Process

Process tumor after 24 h HTS

↓

Mini-scale Gen2 process: Pre-REP using 4 Fragments/G-Rex 10M

↓

Count on Day 11, Initiate REP (10%) and Harvest on Day 22 for functional studies

Gen 2.2 Process (Frozen tumor)

None
Direct Flash Freeze

Process tumor fresh

↓

Fragment 6 mm Tumor pieces, place in CryoVial (5mL), Add 5 mL of CS10

↓

Stored in LN₂ for 1-7 days

↓

Thaw tumor pieces and process mini-scale Gen 2 process

Incubation in Storage Medium

30 Min

Process tumor fresh

↓

Fragment 6 mm Tumor pieces, place in CryoVial (5mL), Add 5 mL of CS10

↓ Incubate for 30 min at 2-8°C

Stored in LN₂ for 1-7 days

↓

Thaw tumor pieces and process mini-scale Gen 2 process

60 min

Process tumor fresh

↓

Fragment 6 mm Tumor pieces, place in CryoVial (5mL), Add 5 mL of CS10

↓ Incubate for 30 min at 2-8°C

Stored in LN₂ for 1-7 days

↓

Thaw tumor pieces and process mini-scale Gen 2 process

# Figure 12
## 1/100 Scale TIL Production

Day 0 (START)
- Process tumor according to LAB-008
- Place 4 fragments/GREX-10M flask in appropriate # of flasks per experiment design
- Fill flasks with 100mL of CM1 (6000IU/mL IL2)

Day 11 (split)
- Volume reduce and count PreREP
- Initiate REP with 10% of the PreREP product into appropriate # of GREX-5M flasks
- To accuratly reflect the 2A process, be sure to load feeder cells with OKT-3 prior to adding TIL
- Fill flasks to 50mL with CM2 (3000IU/mL IL2)

Day 16 (split)
- Volume reduce flasks and count
- calculate appropriate # of daughter flasks (TVC/10e6), record for future extrapolation
- divide TVC/# of flasks, and carry forward one flask with appropriate number of cells

Day 22 (Harvest)
- Volume reduce flasks and count
- Perform any necessary testing

FIG. 13A

| Pre-REP | Fresh (Day 11) | Frozen (Day 7) |
|---|---|---|
| Gen 2 Control | 1.0E+08 | - |
| Flash 0 min | - | 6.7E+05 |
| 30 min inc.. | - | 1.6E+06 |
| 60 min inc. | - | 1.9E+05 |

FIG. 13B

| Pre-REP | Fresh (Day 11) | Frozen (Day 7) |
|---|---|---|
| Gen 2.1 Control | 12E+06 | - |
| Frozen/eREP | - | 3E+06 |

FIG. 13C

| Pre-REP | Fresh (Day 11) | Frozen (Day 7) |
|---|---|---|
| Gen 2.1 Control | 1E+06 | - |
| Frozen/eREP | - | 2E+06 |

FIG. 13D

| Pre-REP | Fresh (Day 11) | Frozen (Day 7) |
|---|---|---|
| Gen 2.1 Control | 9.5E+06 | - |
| Frozen/eREP | - | 9.2E+06 |

FIG. 13E

| Pre-REP | Fresh (Day 11) | Frozen (Day 7) |
|---|---|---|
| Gen 2.1 Control | 8.5E+06 | - |
| Frozen/eREP | - | 2.4E+06 |

## FIG. 14A

| Ideal REP Yield | Gen 2 (Day 22) | Frozen/Early REP (Day 21) |
|---|---|---|
| Gen 2 Control | 24.7E+09 | - |
| Flash 0 min | - | 22.6E+09 |
| 30 min inc.. | - | 19.7E+09 |
| 60 min inc. | - | 21.2E+09 |

## FIG. 14B

| Ideal REP Yield | Gen 2 (Day 21) | Frozen/Early REP (Day 21) |
|---|---|---|
| Gen 2.1 Control | 25.8E+09 | - |
| Frozen/eREP | - | 25.3E+09 |

## FIG. 14C

| Ideal REP Yield | Gen 2 (Day 21) | Frozen/Early REP (Day 21) |
|---|---|---|
| Gen 2.1 Control | 0.6E+09 | - |
| Frozen/eREP | - | 0.8E+09 |

## FIG. 14D

| Ideal REP Yield | Gen 2.1 (Day 21) | Frozen/Early REP (Day 21) |
|---|---|---|
| Gen 2.1 Control | 30E+09 | - |
| Frozen/eREP | - | 87E+09 |

FIG. 14E

| Total REP Yield | Gen 2 (Day 22) | Frozen/Early REP (Day 22) |
|---|---|---|
| Gen 2.1 Control | 66E+09 | - |
| Frozen/eREP | - | 97E+09 |

FIG. 15

FIG. 16A

FIG. 16B

FIG. 16C

FIG. 16D

FIG. 16E

FIG. 16F

FIG. 16G

FIG. 16H

FIG. 17A

M1125

■ Gen 2 Control
▨ Flash
▨ 30 Min inc.
▨ 60 Min inc.

% Gated on CD4 or CD8

CD4+ — CD27, CD57, CD28
CD8+ — CD27, CD28, CD57

FIG. 17B

S13018

■ Gen2 1
▨ Frozen/eREP

% Gated on CD4 or CD8

CD4+ — CD27, CD28, CD57
CD8+ — CD27, CD28, CD57

FIG. 17C

T6042

■ Gen2 1
▨ Frozen/eREP

% Gated on CD4 or CD8

CD4+ — CD27, CD28, CD57
CD8+ — CD27, CD28, CD57

FIG. 18A

FIG. 18B

FIG. 18C

FIG. 19A

FIG. 19B

FIG. 19C

FIG. 20A

FIG. 20B

FIG. 20C

EP 3 852 524 B1

FIG. 21C

FIG. 21B

FIG. 21A

FIG. 22A

FIG. 22B

FIG. 22C

FIG. 23B

FIG. 23A

FIG. 23C

FIG. 24

**5 x 6mm Fragments**

**GEN2 FRESH TUMOR**

Day 0 Pre-REP: Dissect one 6mm to 6 x 3mm and culture using 1/100th scale procedure

Day 11: REP 5-200 x10$^4$ TIL with 50x10$^6$ feeders (1/100th scale)

Day 16: TIL-Scale Up (1/100th scale)

Day 22: Harvest

Freeze Fragments

**ER-1 FROZEN TUMOR**

Day 0: PreREP Thaw and dissect one 6mm to 6 x 3mm fragments and culture using 1/100th scale procedure

Day 5: 10% REP 5-200 x 10$^4$ TIL 50 x 10$^6$ or 25 x 10$^6$ Feeders (1/100th scale)

Day 10: TIL-Scale Up (1/100th scale)

Day 16: Harvest

**ER-2 FROZEN TUMOR**

Day 0: PreREP Thaw and dissect one 6mm to 6 x 3mm fragments and culture using 1/100th scale procedure

Day 3: 10% REP 5-200 x 10$^4$ TIL 50 x 10$^6$ or 25 x 10$^6$ Feeders (1/100th scale)

Day 9: TIL-Scale Up (1/100th scale)

Day 16: Harvest

Structure I-A

Structure I-B

*FIG. 25*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018081473 A1
- US 20140328791 A1
- WO 2012065086 A1
- US 4766106 A
- US 5206344 A
- US 5089261 A
- US 4902502 A
- US 6706289 B
- EP 404097 A
- WO 9311161 A
- US 5714350 A
- US 6350861 B
- US 20040110704 A
- EP 1176195 A
- WO 03035835 A
- WO 9954342 A
- EP 0154316 A
- EP 0401384 A
- US 5891617 A, Watson
- US 9938495 B, Comhaire
- US 20120244133 A1
- WO 2015189356 A
- WO 2015189357 A
- US 20160010058 A1
- US 8821867 B
- US 8337850 B
- US 9468678 B
- WO 2012032433 A1
- US 7288638 B
- US 8962804 B
- US 6974863 B
- US 20050095244 A
- US 6887673 B
- US 7214493 B
- US 6303121 B
- US 6569997 B
- US 6905685 B
- US 6362325 B
- US 6210669 B
- US 5928893 A
- WO 2012177788 A
- WO 2015119923 A
- WO 2010042433 A
- WO 2008025516 A1
- WO 2009007120 A1
- WO 2010003766 A1
- WO 2010010051 A1
- WO 2010078966 A1
- US 20110027218 A1
- US 20150126709 A1
- US 20110111494 A1
- US 20150110734 A1
- US 20150126710 A1
- US 9359420 B
- US 9340599 B
- US 8921519 B
- US 8450460 B
- US 7960515 B
- US 8236930 B
- US 9028824 B
- US 9006399 B
- US 9163085 B
- WO 2012027328 A
- WO 9512673 A
- WO 9521925 A
- WO 2006121810 A
- WO 2013028231 A
- WO 2013038191 A
- WO 2014148895 A
- EP 0672141 A
- US 2010136030 A
- US 2014377284 A
- US 2015190506 A
- US 2015132288 A
- US 7504101 B
- US 7550140 B
- US 7622444 B
- US 7696175 B
- US 7961515 B
- US 8133983 B
- US 6312700 B
- US 15863634 B
- WO 1998030679 A
- US 20050106717 A1
- US 20140377739 A1
- WO 2014210036 A1
- US 20130115617 A1
- WO 2013188427 A1
- US 20110136228 A1
- US 8809050 B2
- WO 2011072088 A2
- US 20160208216 A1
- WO 2012129201 A1
- US 20130102075 A1
- US 8956860 B2
- WO 2013173835 A1
- US 20150175966 A1
- US 8008449 B

- US 7943743 B
- US 20110052530 A
- WO 2015026684 A1
- WO 2006121168 A
- WO 2008156712 A1
- US 8354509 B
- US 20100266617 A1
- US 20130108651 A1
- US 20130109843 A2
- US 8287856 B
- US 8580247 B
- US 8168757 B
- US 20090028857 A1
- US 20100285013 A1
- US 20130022600 A1
- US 20110008369 A1
- US 8735553 B1
- US 8686119 B
- US 8907053 B

- US 9096642 B
- US 9044442 B
- US 20150087581 A
- US 20150073024
- US 20150073042 A
- US 20150125491 A
- WO 2015033301 A
- WO 2015036927 A
- WO 201504490 A
- US 20140294898 A
- US 8779108 B
- US 20130034559
- US 8217149 B
- US 20100203056 A1
- US 20130045200 A1
- US 20130045201 A1
- US 20130045202 A1
- US 20140065135 A1

**Non-patent literature cited in the description**

- **GATTINONI et al.** *Nat. Rev. Immunol.,* 2006, vol. 6, 383-393
- **DUDLEY et al.** *Science,* 2002, vol. 298, 850-54
- **DUDLEY et al.** *J. Clin. Oncol.,* 2005, vol. 23, 2346-57
- **DUDLEY et al.** *J. Clin. Oncol.,* 2008, vol. 26, 5233-39
- **RIDDELL et al.** *Science,* 1992, vol. 257, 238-41
- **DUDLEY et al.** *J. Immunother,* 2003, vol. 26, 332-42
- **HILDERS et al.** *International Journal of Cancer,* 1994, vol. 57 (6), 805-813
- *CHEMICAL ABSTRACTS,* 7681-93-8
- *CHEMICAL ABSTRACTS,* 1397-89-3
- **NELSON.** *J. Immunol.,* 2004, vol. 172, 3983-88
- **MALEK.** *Annu. Rev. Immunol.,* 2008, vol. 26, 453-79
- **STEINKE ; BORISH.** *Respir. Res,* 2001, vol. 2, 66-70
- **FRY ; MACKALL.** *Blood,* 2002, vol. 99, 3892-904
- **FEHNIGE ; CALIGIURI.** *Blood,* 2001, vol. 97, 14-32
- **SPOLSKI ; LEONARD.** *Nat. Rev. Drug. Disc.,* 2014, vol. 13, 379-95
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **JONES et al.** *Nature,* 1986, vol. 321, 522-525
- **RIECHMANN et al.** *Nature,* 1988, vol. 332, 323-329

- **PRESTA.** *Curr. Op. Struct. Biol.,* 1992, vol. 2, 593-596
- **BOLLIGER et al.** *Proc. Natl. Acad. Sci. USA,* 1993, vol. 90, 6444-6448
- **YAMANE-OHNUKI et al.** *Biotechnol. Bioeng.,* 2004, vol. 87, 614-622
- **SHIELDS et al.** *J. Biol. Chem.,* 2002, vol. 277, 26733-26740
- **UMANA et al.** *Nat. Biotech.,* 1999, vol. 17, 176-180
- **TARENTINO et al.** *Biochem.,* 1975, vol. 14, 5516-5523
- **SWARTZ et al.** *Cancer Res,* 2012, vol. 72, 2473
- The osmotic rupture hypothesis of intracellular freezing injury. *Biophysical Journa,* 1994, vol. 66, 532-41
- Membrane damage occurs during the formation of intracellular ice. *Cryo Letter,* 2001, vol. 22, 241-54
- **STROBER.** *Curr. Protoc. Immunol.,* 2001, https://dx.doi.org/10.1002/0471142735.ima03bs21
- **BERRIDGE et al.** Tetrazolium dyes as tools in cell biology: new insights into their cellular reduction. *Biotechnology Annual Review,* 2005, vol. 11, 127-152
- **MOSMANN.** Rapid colorimetric assay for cellular growth and survival: application to proliferation and cytotoxicity assays. *J. Immunol. Methods,* 1983, vol. 65 (1-2), 55-63
- **ACKER ; MCGANN.** Protective effect of intracellular ice during freezing?. *Cryobiology,* 2003, vol. 46 (2), 197-202

- **JIN et al.** *J. Immunother.,* 2012, vol. 35 (3), 283-292
- **DUDLEY et al.** *J. Clin. Oncol,* 2008, vol. 26, 5233-39
- **DONIA et al.** *Scandinavian Journal of Immunology,* 2012, vol. 75, 157-167
- **DUDLEY et al.** *Clin Cancer Res,* 2010, vol. 16, 6122-6131
- **HUANG et al.** *J Immunother,* 2005, vol. 28 (3), 258-267
- **BESSER et al.** *Clin Cancer Res,* 2013, vol. 19 (17), 1-9
- **BESSER et al.** *J Immunother,* 2009, vol. 32, 415-423
- **ROBBINS et al.** *J Immunol,* 2004, vol. 173, 7125-7130
- **SHEN et al.** *J Immunother,* 2007, vol. 30, 123-129
- **ZHOU et al.** *J Immunother,* 2005, vol. 28, 53-62
- **TRAN et al.** *J Immunother,* 2008, vol. 31, 742-751
- **TRAN et al.** *J. Immunother,* 2008, vol. 31, 742-51
- **TRAN KQ ; ZHOU J ; DURFLINGER KH et al.** *J Immunother,* 2008, vol. 31, 742-751
- **DUDLEY ME ; WUNDERLICH JR ; SHELTON TE et al.** *J Immunother.,* 2003, vol. 26, 332-342
- Isolation of mononuclear cells: Methodology and Application. *GE Life Sciences technical publication 18-1152-69-AE, https://us.vwr.com/assetsvc/asset/en_US/id/16286835/contents*
- **TSOUKAS et al.** *J. Immunol.,* 1985, vol. 135, 1719
- **LEE et al.** *PLOS One,* 2013, vol. 8, e69677
- **GIEFFERS et al.** *Mol. Cancer Therapeutics,* 2013, vol. 12, 2735-47
- **FISHER et al.** *Cancer Immunolog. & Immunother.,* 2012, vol. 61, 1721-33
- **SEGAL et al.** *Clin. Cancer Res.,* 2016, http:/dx.doi.org/ 10.1158/1078-0432.CCR-16-1272
- **MARCO.** *Microbial Cell Factories,* 2011, vol. 10, 44
- **AHMAD et al.** *Clin. & Dev. Immunol.,* 2012, 980250
- **MONNIER et al.** *Antibodies,* 2013, vol. 2, 193-208
- **SADUN et al.** *J. Immunother.,* 2009, vol. 182, 1481-89
- **CURTI et al.** *Cancer Res.,* 2013, vol. 73, 7189-98
- **WEINBERG et al.** *J. Immunother.,* 2006, vol. 29, 575-585
- **SMITH et al.** Ex vivo expansion of human T cells for adoptive immunotherapy using the novel Xeno-free CTS Immune Cell Serum Replacement. *Clin Transl Immunology,* 2015, vol. 4 (1
- *CHEMICAL ABSTRACTS,* 60-24-2
- **JIN et al.** *J. Immunotherapy,* 2012, vol. 35, 283-292
- **SADEGHI et al.** *Acta Oncologica,* 2013, vol. 52, 978-986
- **JIN et al.** *J. Immunotherapy,* 2012, vol. 35 (3), 283-292
- **GOFF et al.** *J. Clinical Oncology,* 2016, vol. 34 (20), 2389-239
- **DUDLEY et al.** *J Immunother.,* 2003, vol. 26, 332-342
- **GOFF et al.** *J Immunother.,* 2010, vol. 33, 840-847
- **MULLANY et al.** *Endocrinology,* 2012, vol. 153, 1585-92
- **FONG et al.** *J. Ovarian Res,* 2009, vol. 2, 12
- **HERREROS-VILLANUEVA et al.** *World J. Gastroenterol.,* 2012, vol. 18, 1286-1294
- **FANTOZZI.** *Breast Cancer Res.,* 2006, vol. 8, 212
- **DAMSKY et al.** *Pigment Cell & Melanoma Res,* 2010, vol. 23, 853-859
- **MEUWISSEN et al.** *Genes & Development,* 2005, vol. 19, 643-664
- **KIM.** *Clin. Exp. Otorhinolaryngol,* 2009, vol. 2, 55-60
- **SANO.** *Head Neck Oncol.,* 2009, vol. 1, 32
- **GASSNER et al.** *Cancer Immunol. Immunother.,* 2011, vol. 60, 75-85
- **MURANSKI et al.** *Nat. Clin. Pract. Oncol.,* 2006, vol. 3, 668-681
- **DUDLEY et al.** *J. Clin. Oncol.,* 2008, vol. 26, 5233-5239
- **DUDLEY et al.** *J. Clin. Oncol.,* 2005, vol. 23, 2346-2357
- **O'DAY et al.** *J. Clin. Oncol.,* 1999, vol. 17, 2752-61
- **ETON et al.** *Cancer,* 2000, vol. 88, 1703-9
- **KEIR et al.** *Annu. Rev. Immunol.,* 2008, vol. 26, 677-704
- **TOPALIAN et al.** *N. Eng. J. Med.,* 2012, vol. 366, 2443-54
- *CHEMICAL ABSTRACTS,* 946414-94-4
- **WANG et al.** *Cancer Immunol Res.,* 2014, vol. 2, 846-56
- **PAGE et al.** *Ann. Rev. Med.,* 2014, vol. 65, 185-202
- **WEBER et al.** *J. Clin. Oncology,* 2013, vol. 31, 4311-4318
- **FUERST.** *Oncology Times,* 2014, vol. 36, 35-36
- **ROBERT et al.** *Lancet,* 2014, vol. 384, 1109-17
- **THOMAS et al.** *Exp. Opin. Biol. Ther.,* 2014, vol. 14, 1061-1064
- **BRAHMER et al.** *J. Clin. Oncol,* 2014, vol. 32 (8021), 5s

- **MCDERMOTT et al.** *Cancer Treatment Rev,* 2014, vol. 40, 1056-64

- **SANTEGOETS, S. J.** *J. Transl Med.,* 2013, vol. 11, 37